(19) Europäisches Patentamt
European Patent Office
Office européen des brevets

(11) EP 4 692 351 A1

(12) EUROPEAN PATENT APPLICATION
published in accordance with Art. 153(4) EPC

(43) Date of publication:
**11.02.2026 Bulletin 2026/07**

(21) Application number: **24780728.2**

(22) Date of filing: **28.03.2024**

(51) International Patent Classification (IPC):
***C12N 15/13*** (2006.01) ***A61K 31/4745*** (2006.01)
***A61K 39/395*** (2006.01) ***A61K 45/00*** (2006.01)
***A61K 47/68*** (2017.01) ***A61P 35/00*** (2006.01)
***A61P 43/00*** (2006.01) ***C07K 16/28*** (2006.01)
***C12N 1/15*** (2006.01) ***C12N 1/19*** (2006.01)
***C12N 5/10*** (2006.01) ***C12N 15/63*** (2006.01)
***C12P 21/08*** (2006.01)

(52) Cooperative Patent Classification (CPC):
**A61K 31/4745; A61K 39/395; A61K 45/00; A61K 47/68; A61P 35/00; A61P 43/00; C07K 16/00; C07K 16/28; C12N 5/10; C12N 15/63; C12N 15/80; C12N 15/81**

(86) International application number:
**PCT/JP2024/012855**

(87) International publication number:
**WO 2024/204629 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **29.03.2023 JP 2023053446**
**13.06.2023 JP 2023096690**
**08.09.2023 JP 2023145696**

(71) Applicant: **Daiichi Sankyo Company, Limited**
**Chuo-ku**
**Tokyo 103-8426 (JP)**

(72) Inventors:
- **SAWANO, Kota**
  **Tokyo 103-8426 (JP)**
- **DOKE, Yukiko**
  **Tokyo 103-8426 (JP)**
- **ISUMI, Yoshitaka**
  **Tokyo 103-8426 (JP)**
- **MATSUMOTO, Ryota**
  **Tokyo 103-8426 (JP)**

(74) Representative: **Hoffmann Eitle**
**Patent- und Rechtsanwälte PartmbB**
**Arabellastraße 30**
**81925 München (DE)**

Remarks:
The complete document including Reference Table(s) and the Sequence Listing(s) can be downloaded from the EPO website

# (54) ANTI-CD25 ANTIBODY AND ANTI-CD25 ANTIBODY-DRUG CONJUGATE

(57) An object of the present invention is to provide an antibody that binds to CD25 and has internalization activity, an antibody-drug conjugate that comprises the antibody and has antitumor activity, a medicament having a therapeutic effect on tumor using the antibody-drug conjugate, and a method for treating tumor using the antibody, the antibody-drug conjugate, or the medicament, etc. The present invention provides a CD25 antibody or an antigen binding fragment of the antibody, having the following characteristics: (1) having no ability to block IL-2, and (2) having activity of being internalized in CD25-expressing cells by binding to CD25. This antibody or the antigen binding fragment of the antibody is capable of exhibiting the ability to remove regulatory T cells and/or the ability to promote the proliferation of granzyme-positive CD8-positive cells by conjugation to a cytotoxic active compound.

EP 4 692 351 A1

Fig. 4

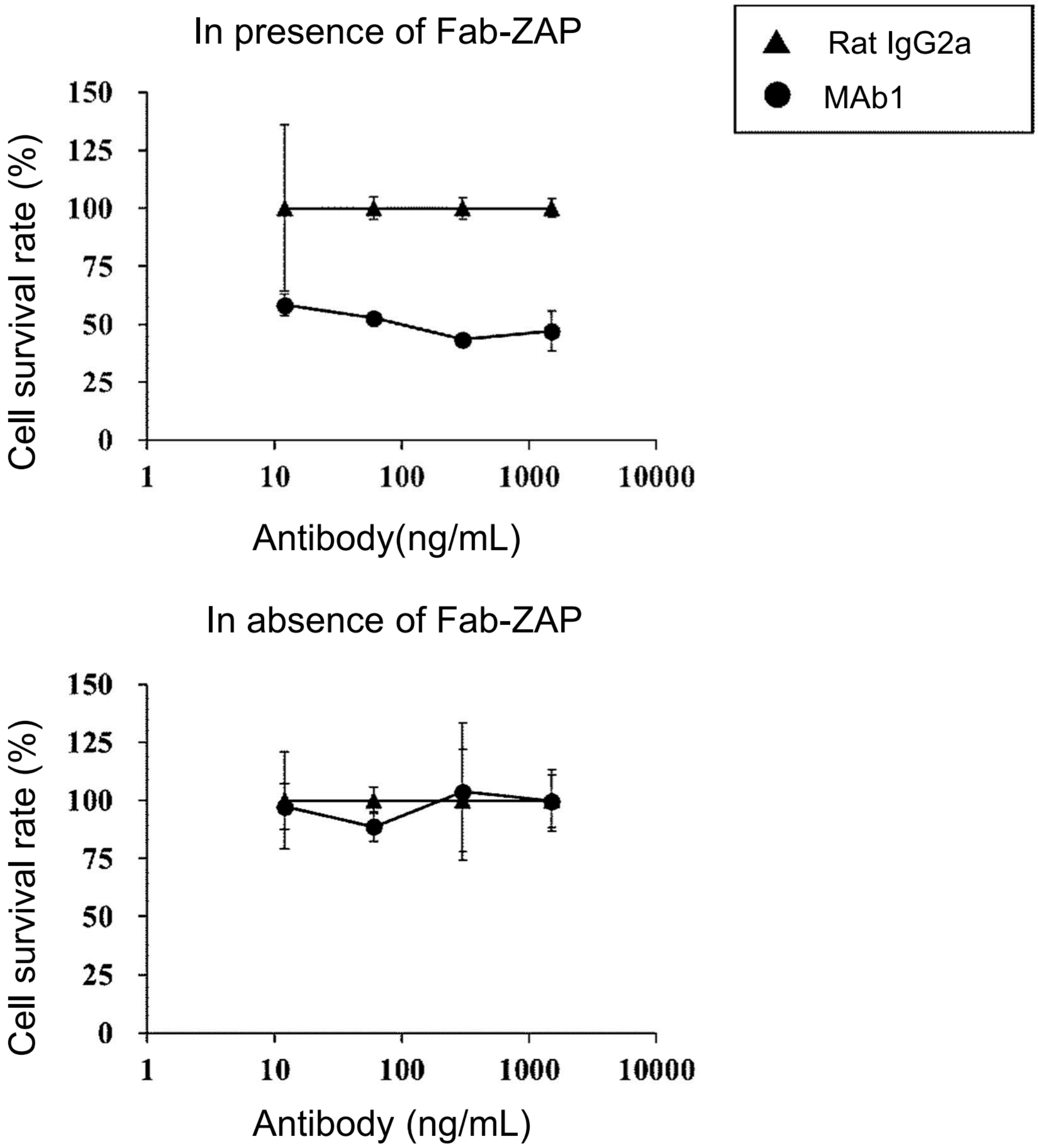
In presence of Fab-ZAP
Rat IgG2a
MAb1
Cell survival rate (%)
150
125
100
75
50
25
0
1
10
100
1000
10000
Antibody(ng/mL)
In absence of Fab-ZAP
Cell survival rate (%)
150
125
100
75
50
25
0
1
10
100
1000
10000
Antibody (ng/mL)

## Description

Technical Field

The present invention relates to an anti-CD25 antibody having an effect of binding to CD25, a method for producing the anti-CD25 antibody, an antibody-drug conjugate comprising the antibody, an antitumor agent comprising the antibody-drug conjugate, etc.

Background Art

Regulatory T cells (Treg) are a cell population having a role in suppressing immune response or establishing and maintaining self-tolerance as a subset of CD4-positive T cells (Non Patent Literature 1). Treg accounts for 10% of peripheral CD4-positive T cells, and Forkhead box P3 (FoxP3) has been identified as a master transcriptional factor (Non Patent Literature 2). Excessive proliferation of CD4-positive cells or elevated levels of cytokines have been confirmed in scurfy mice having a mutation in FoxP3 (Non Patent Literatures 3 and 4). A mutation in FoxP3 gene in humans is known to cause autoimmune abnormality called IPEX (immune dysregulation, polyendocrinopathy, enteropathy, X-linked) syndrome ascribable to the dysfunction of Treg (Non Patent Literature 5). Thus, Treg plays an important role in immune tolerance.

Cancers are diseases that rank high as factors of death (Non Patent Literature 6). Abnormal proliferation of cancer cells damages normal tissues, resulting in a lethal disease. Examples of the features of cancer cells include unregulated cell proliferation, resistance to apoptosis, and migration to other tissues (Non Patent Literature 7). Other examples of the features of cancer cells include the avoidance of immune response to cancer cells.

Gene mutations including not only driver mutations but passenger mutations are accumulated in cancer cells due to gene instability. A protein obtained by transcription from such a gene mutation and translation is presented as a neoantigen. This neoantigen differs from an autoantigen and is therefore normally recognized as being not normal autologous cells by immunocytes and thus eliminated (Non Patent Literature 8). However, in the cancer microenvironment, an environment that suppresses immunity in various forms including high expression of immunosuppressive cytokines such as TGF-β or IL-10 or immunosuppressive molecules such as PD-1 or PD-L1 and infiltration of immunosuppressive cells is constructed, and it is considered that the elimination of cancer cells by immunocytes is unlikely to occur (Non Patent Literature 9). In recent years, immune checkpoint inhibitors such as anti-PD-1 antibodies have exhibited promising results in clinical trials and been approved for many types of advanced cancers including melanoma and non-small cell lung cancer (NSCLC) (Non Patent Literatures 10 and 11). However, a limited number of patients responds to such an immune checkpoint inhibitor. As one of the reasons therefor, the immunosuppressive cells (particularly, Treg) mentioned above are considered as major cells that suppress tumor immunity. Treg present in tumor suppresses the function of effector T cells (Teff) or cytotoxic T cells(CTL) so that cancer cells can no longer be eliminated as foreign matter, contributing to the aggravation of pathological conditions. It has also been reported that the balance of Treg and CTL in tumor correlates with prognosis (Non Patent Literature 12).

CD25 (also called IL-2 receptor α chain or Tac antigen) is a type I transmembrane protein extracellularly having two Sushi domains (Non Patent Literatures 13 and 14) and is known as IL-2 receptor α chain. CD25 plays a role in transducing IL-2 signals into cells upon binding to IL-2, which is known as a factor having various roles including the differentiation and proliferation of T cells (Non Patent Literature 15). It has been reported that CD25 is expressed at a low level in limited types of immunocytes such as activated T cells or B cells (Non Patent Literatures 16 and 17) and is highly expressed in Treg (Non Patent Literature 18). The immunosuppressive activity of Treg has been found to correlate with strong or weak CD25 expression (Non Patent Literature 18). For example, IL-2 signals essential for the survival of Treg (Non Patent Literature 19) and binding to IL-2 and consumption of IL-2 resulting in decreased chance of IL-2 to bind to other immunocytes have been reported as roles of CD25 in Treg (Non Patent Literature 19). Also, CD25 expression in blood cancer cells such as non-Hodgkin's lymphoma has been reported (Non Patent Literature 20).

Some anti-CD25 antibodies have been prepared so far. These antibodies include two types: an antibody that can bind to CD25 while IL-2 signals of CD25 are retained; and an antibody that inhibits IL-2 signals and binds to CD25. The antibody that inhibits IL-2 signals includes, for example, Anti-Tac (anti-human CD25 antibody), and the antibody that does not inhibit IL-2 signals includes, for example, 7G7B6 (anti-human CD25 antibody). It has been reported that Anti-Tac inhibits IL-2-dependent T cell proliferation, whereas 7G7B6 does not inhibit it (Non Patent Literature 21). Basiliximab, an antibody that inhibits IL-2 signals, reportedly suppresses immune response mediated by T cells and is used in the prevention of rejection in organ transplantation. Thus, the anti-CD25 antibody that inhibits IL-2 signals suppresses immune activation. On the other hand, the anti-CD25 antibody that does not inhibit IL-2 signals is considered to not suppress immune response mediated by T cells (Non Patent Literature 22).

Studies using anti-mouse CD25 antibodies have been reported so far. In recent years, results have been reported which suggest that the therapeutic effects and effects of these antibodies in mouse tumor models are based on

the removal of Treg from the tumor and the activation of Teff in the tumor (Non Patent Literature 22). It has been reported that human tumor contains Treg having strong immunosuppressive activity among Treg cells (Non Patent Literature 23). Since this cell population strongly expresses CD25, CD25 is a promising target for aiming Treg in tumor.

An antibody-drug conjugate (ADC) is a compound in which an antibody that specifically binds to a target antigen is conjugated to a drug having cytotoxic activity (Non Patent Literature 24). Owing to this feature, ADC can be expected to kill cells expressing a target antigen while reducing its influence on cells expressing no target antigen. The development of ADC as a therapeutic drug in the cancer field has been underway so far. For example, Enhertu(TM) (trastuzumab deruxtecan), a conjugate of an anti-HER2 monoclonal antibody and deruxtecan, is used in the treatment of HER2-positive advanced or recurrent breast cancer.

Antibody-dependent cellular cytotoxic activity (ADCC) and antibody-dependent cell-mediated phagocytic activity (ADCP) are known as mechanisms in which an antibody is used to specifically eliminate cells expressing a target antigen. Antibody techniques of enhancing the ADCC effect have been developed by focusing on this effect (Non Patent Literature 25). An anti-human CCR4 antibody Poteligeo(TM) (mogamulizumab) having the ADCC effect thus enhanced exhibits a limited effect on T cell lymphoma (Non Patent Literature 26). On the other hand, the expansion of an antibody having the enhanced ADCC effect into solid cancers is controversial in terms of efficacy (Non Patent Literature 27). A possible reason why the expansion into solid cancers does not progress is low abundance of NK cells which are main immunocytes that exert an ADCC effect (Non Patent Literature 28).

Thus, application to therapeutic drugs for cancers aiming at removing Treg by targeting CD25 has been contemplated. However, there is no previous report stating that Treg in the tumor of a solid cancer can be selectively removed. ADCC enhanced Ab RG-6292 targeting CD25, or ADCT-301 which is ADC of an anti-CD25 antibody conjugated to pyrrolobenzodiazepine (PBD) is currently under clinical trial for solid cancers.

Citation List

Non Patent Literature

Non Patent Literature 1: Sakaguchi S, et al., Annu Rev Immunol. 2004; 22: 531-562

Non Patent Literature 2: Sakaguchi S, et al., Annu Rev Immunol. 2020; 38:541-566.

Non Patent Literature 3: Brunkow ME, et al., 2001 Nature Publishing Group http://genetics.nature.com. 2001; 27 (january): 68-73.

Non Patent Literature 4: Sakaguchi S, et al., Cell. 2008; 133(5): 775-787.

Non Patent Literature 5: Bacchetta R, et al.,Ann N Y Acad Sci. 2016; 1417(1): 5-22.

Non Patent Literature 6: Sung H, et al., Global Cancer Statistics 2020: GLOBOCAN Estimates of Incidence and Mortality Worldwide for 36 Cancers in 185 Countries. CA Cancer J Clin. 2021; 71(3): 209-249.

Non Patent Literature 7: Hanahan D, et al., Cell. 2011; 144(5): 646-674.

Non Patent Literature 8: Chen DS, et al., Immunity. 2013; 39(1): 1-10.

Non Patent Literature 9: Huang AC, et al., Nat Immunol. Published online 2022; 23(5), 660-670.

Non Patent Literature 10: Larkin J, et al., N Engl J Med. 2015; 373(1): 23-34.

Non Patent Literature 11: Mok TSK, et al., Lancet. 2019; 393(10183): 1819-1830.

Non Patent Literature 12: O'Callaghan DS, et al., Eur Respir J. 2015; 46(6): 1762-1772.

Non Patent Literature 13: Rickert M, et al., Rickert M, Wang X, Boulanger MJ, Goriatcheva N, Garcia KC. The Structure of Interleukin-2 Complexed with Its Alpha Receptor. 2014; 1477(June): 1477-1481.

Non Patent Literature 14: Wang X, et al.,Science. 2005; 310(5751): 1159-1163.

Non Patent Literature 15: Spolski R, et al., Nat Rev Immunol. 2018; 18(10): 648-659.

Non Patent Literature 16: Tsudo M, et al., J Immunol. 1982; 129(2): 592-595.

Non Patent Literature 17: Brisslert M, et al., Immunology. 2006; 117(4): 548-557.

Non Patent Literature 18: Miyara M, et al., Immunity. 2009; 30(6): 899-911.

Non Patent Literature 19: Chinen T, et al., Nat Immunol. 2016; 17(11): 1322-1333.

Non Patent Literature 20: Flynn MJ, et al., Br J Haematol. 2017; 179(1): 20-35.

Non Patent Literature 21: Rubin LA. et al., A monoclonal antibody 7G7/B6, binds to an epitope on the human interleukin-2 (IL-2) receptor that is distinct from that recognized by IL-2 or anti-Tac Hybridoma 1985; 4(2): 91-102.

Non Patent Literature 22: Solomon I, et al., Nat Cancer. 2020; 1(12): 1153-1166.

Non Patent Literature 23: Saito T, et al., Nat Med. 2016; 22(6): 679-684.

Non Patent Literature 24: Chari RVJ, et al., Angew Chemie - Int Ed. 2014; 53(15): 3796-3827.

Non Patent Literature 25: Niwa R, et al., Cancer Res. 2004; 64(6): 2127-2133.

Non Patent Literature 26: Ishida T, et al., J Clin Oncol. 2012; 30(8): 837-842.

Non Patent Literature 27: Lo Nigro C, et al., Ann Transl Med. 2019; 7(5): 105-105.
Non Patent Literature 28: Marechal R, et al., BMC Cancer. 2010; 10.

Summary of Invention

Technical Problem

An object of the present invention is to provide an antibody that binds to CD25, an antibody-drug conjugate that comprises the antibody and has antitumor activity, a medicament having a therapeutic effect on tumor using the antibody-drug conjugate, and a method for treating tumor using the antibody, the antibody-drug conjugate, or the medicament, etc.

Solution to Problem

The present inventors have conducted diligent studies to attain the object and consequently found that the particular antibody disclosed in the present invention binds to CD25 and is useful as an antibody for an antibody-drug conjugate. The present inventors have further found that the anti-CD25 antibody-drug conjugate disclosed in the present invention is useful as a medicament.
The invention of the present application encompasses the following aspects.

[1] An anti-CD25 antibody or an antigen binding fragment of the antibody, having the following characteristics:

(1) having no ability to block IL-2; and
(2) exhibiting the ability to remove human regulatory T cells and/or the ability to promote the proliferation of human granzyme-positive CD8-positive cells by conjugation to a cytotoxic active compound.

[2] The antibody or the antigen binding fragment of the antibody according to [1], wherein the antibody or the antigen binding fragment has activity of being internalized in CD25-expressing cells by binding to CD25.
[3] The antibody or the antigen binding fragment of the antibody according to [1] or [2], wherein the CD25 is human CD25, cynomolgus monkey CD25, or mouse CD25.
[4] The antibody or the antigen binding fragment of the antibody according to [3], wherein the CD25 is human CD25 or cynomolgus monkey CD25.
[4-2] The antibody or the antigen binding fragment of the antibody according to [3], wherein the CD25 is human CD25.
[5] The antibody or the antigen binding fragment of the antibody according to [3], wherein the CD25 is mouse CD25.
[5-2] The antibody or the antigen binding fragment of the antibody according to any one of [1] to [5], wherein the antibody or the antigen binding fragment has competitive inhibitory activity, for binding to CD25, against at least any one antibody selected from the group consisting of the following antibodies (1) to (6):

(1) an antibody comprising a light chain consisting of an amino acid sequence from positions 21 to 232 of SEQ ID NO: 26 and a heavy chain consisting of an amino acid sequence from positions 20 to 476 of SEQ ID NO: 27,
(2) an antibody comprising a light chain consisting of an amino acid sequence from positions 21 to 233 of SEQ ID NO: 13 and a heavy chain consisting of an amino acid sequence from positions 20 to 476 of SEQ ID NO: 15,
(3) an antibody comprising a light chain consisting of an amino acid sequence from positions 21 to 233 of SEQ ID NO: 14 and a heavy chain consisting of an amino acid sequence from positions 20 to 476 of SEQ ID NO: 16,
(4) an antibody comprising a light chain consisting of an amino acid sequence from positions 21 to 232 of SEQ ID NO: 30 and a heavy chain consisting of an amino acid sequence from positions 20 to 463 of SEQ ID NO: 31,
(5) an antibody comprising a light chain consisting of an amino acid sequence from positions 21 to 239 of SEQ ID NO: 32 and a heavy chain consisting of an amino acid sequence from positions 20 to 463 of SEQ ID NO: 33, and
(6) an antibody comprising a light chain consisting of an amino acid sequence from positions 21 to 239 of SEQ ID NO: 54 and a heavy chain consisting of an amino acid sequence from positions 20 to 463 of SEQ ID NO: 55.

[5-3] The antibody or the antigen binding fragment of the antibody according to any one of [1] to [5], wherein the antibody or the antigen binding fragment has competitive inhibitory activity, for binding to CD25, against at least any one antibody selected from the group consisting of the following antibodies (1) to (3):

(1) an antibody comprising a light chain consisting of an amino acid sequence from positions 21 to 232 of SEQ ID NO: 26 and a heavy chain consisting of an amino acid sequence from positions 20 to 476 of SEQ ID NO: 27,
(2) an antibody comprising a light chain consisting of an amino acid sequence from positions 21 to 233 of SEQ ID NO: 13 and a heavy chain consisting of an amino acid sequence from positions 20 to 476 of SEQ ID NO: 15, and

(3) an antibody comprising a light chain consisting of an amino acid sequence from positions 21 to 233 of SEQ ID NO: 14 and a heavy chain consisting of an amino acid sequence from positions 20 to 476 of SEQ ID NO: 16.

[5-4] The antibody or the antigen binding fragment of the antibody according to any one of [1] to [5], wherein the antibody or the antigen binding fragment has competitive inhibitory activity, for binding to CD25, against at least any one antibody selected from the group consisting of the following antibodies (1) and (2):

(1) an antibody comprising a light chain consisting of an amino acid sequence from positions 21 to 233 of SEQ ID NO: 13 and a heavy chain consisting of an amino acid sequence from positions 20 to 476 of SEQ ID NO: 15, and
(2) an antibody comprising a light chain consisting of an amino acid sequence from positions 21 to 233 of SEQ ID NO: 14 and a heavy chain consisting of an amino acid sequence from positions 20 to 476 of SEQ ID NO: 16.

[6] The antibody or the antigen binding fragment of the antibody according to any one of [1] to [5-4], wherein the antibody comprises

CDRL1, CDRL2, and CDRL3 in any one combination selected from the group consisting of the following combinations (1) and (2):

(1) CDRL1 consisting of the amino acid sequence represented by SEQ ID NO: 1, CDRL2 consisting of the amino acid sequence represented by SEQ ID NO: 2 (KAS), and CDRL3 consisting of the amino acid sequence represented by SEQ ID NO: 3, and
(2) CDRL1 consisting of the amino acid sequence represented by SEQ ID NO: 4, CDRL2 consisting of the amino acid sequence represented by SEQ ID NO: 5 (FVS), and CDRL3 consisting of the amino acid sequence represented by SEQ ID NO: 6, and

CDRH1, CDRH2, and CDRH3 in any one combination selected from the group consisting of the following combinations (3) and (4):

(3) CDRH1 consisting of the amino acid sequence represented by SEQ ID NO: 7, CDRH2 consisting of the amino acid sequence represented by SEQ ID NO: 8, and CDRH3 consisting of the amino acid sequence represented by SEQ ID NO: 9, and
(4) CDRH1 consisting of the amino acid sequence represented by SEQ ID NO: 10, CDRH2 consisting of the amino acid sequence represented by SEQ ID NO: 11, and CDRH3 consisting of the amino acid sequence represented by SEQ ID NO: 12.

[7] The antibody or the antigen binding fragment of the antibody according to any one of [1] to [6], wherein the antibody comprises
CDRL1, CDRL2, and CDRL3, and CDRH1, CDRH2, and CDRH3 in any one combination selected from the group consisting of the following combinations (1) and (2):

(1) CDRL1 consisting of the amino acid sequence represented by SEQ ID NO: 1, CDRL2 consisting of the amino acid sequence represented by SEQ ID NO: 2 (KAS), and CDRL3 consisting of the amino acid sequence represented by SEQ ID NO: 3, and CDRH1 consisting of the amino acid sequence represented by SEQ ID NO: 7, CDRH2 consisting of the amino acid sequence represented by SEQ ID NO: 8, and CDRH3 consisting of the amino acid sequence represented by SEQ ID NO: 9, and
(2) CDRL1 consisting of the amino acid sequence represented by SEQ ID NO: 4, CDRL2 consisting of the amino acid sequence represented by SEQ ID NO: 5 (FVS), and CDRL3 consisting of the amino acid sequence represented by SEQ ID NO: 6, and CDRH1 consisting of the amino acid sequence represented by SEQ ID NO: 10, CDRH2 consisting of the amino acid sequence represented by SEQ ID NO: 11, and CDRH3 consisting of the amino acid sequence represented by SEQ ID NO: 12.

[8] The antibody or the antigen binding fragment of the antibody according to any one of [1] to [7], wherein the antibody or the antigen binding fragment is humanized.
[9] The antibody or the antigen binding fragment of the antibody according to any one of [1] to [8], wherein the antibody comprises
any one light chain variable region selected from the group consisting of the following light chain variable regions (1) to (4):

(1) a light chain variable region consisting of an amino acid sequence from positions 21 to 127 of SEQ ID NO: 13,
(2) a light chain variable region consisting of an amino acid sequence from positions 21 to 127 of SEQ ID NO: 14,
(3) a light chain variable region consisting of an amino acid sequence having at least 95% or higher sequence identity to the sequences of framework regions except for CDR sequences in each of the amino acid sequences of (1) and (2), and
(4) a light chain variable region consisting of an amino acid sequence derived by the deletion, substitution, or addition of one or several amino acids from the sequences of framework regions except for CDR sequences in each of the amino acid sequences of (1) and (2), and any one heavy chain variable region selected from the group consisting of the following heavy chain variable regions (5) to (8):
(5) a heavy chain variable region consisting of an amino acid sequence from positions 20 to 146 of SEQ ID NO: 15,
(6) a heavy chain variable region consisting of an amino acid sequence from positions 20 to 146 of SEQ ID NO: 16,
(7) a heavy chain variable region consisting of an amino acid sequence having at least 95% or higher sequence homology to the sequences of framework regions except for CDR sequences in each of the amino acid sequences of (5) and (6), and
(8) a heavy chain variable region consisting of an amino acid sequence derived by the deletion, substitution, or addition of one or several amino acids from the sequences of framework regions except for CDR sequences in each of the amino acid sequences of (5) and (6).

[10] The antibody or the antigen binding fragment of the antibody according to any one of [1] to [9], wherein the antibody comprises
a light chain variable region and a heavy chain variable region in any of the following combinations (1) and (2):

(1) a light chain variable region consisting of an amino acid sequence from positions 21 to 127 of SEQ ID NO: 13 and a heavy chain variable region consisting of an amino acid sequence from positions 20 to 146 of SEQ ID NO: 15, and
(2) a light chain variable region consisting of an amino acid sequence from positions 21 to 127 of SEQ ID NO: 14 and a heavy chain variable region consisting of an amino acid sequence from positions 20 to 146 of SEQ ID NO: 16.

[11] The antibody or the antigen binding fragment of the antibody according to any one of [1] to [10], wherein the antibody comprises any of the following combinations (1) and (2):

(1) a light chain consisting of an amino acid sequence from positions 21 to 233 of SEQ ID NO: 13 and a heavy chain consisting of an amino acid sequence from positions 20 to 476 of SEQ ID NO: 15, and
(2) a light chain consisting of an amino acid sequence from positions 21 to 233 of SEQ ID NO: 14 and a heavy chain consisting of an amino acid sequence from positions 20 to 476 of SEQ ID NO: 16.

[12] The antibody or the antigen binding fragment of the antibody according to [11], wherein the antibody comprises a light chain consisting of an amino acid sequence from positions 21 to 233 of SEQ ID NO: 13 and a heavy chain consisting of an amino acid sequence from positions 20 to 476 of SEQ ID NO: 15.
[13] The antibody or the antigen binding fragment of the antibody according to [11], wherein the antibody comprises a light chain consisting of an amino acid sequence from positions 21 to 233 of SEQ ID NO: 14 and a heavy chain consisting of an amino acid sequence from positions 20 to 476 of SEQ ID NO: 16.
[14] The antigen binding fragment of the antibody according to any one of [1] to [13], wherein the antigen binding fragment is selected from the group consisting of Fab, F(ab')2, Fab', and Fv.
[15] The antigen binding fragment of the antibody according to any one of [1] to [13], wherein the antigen binding fragment is scFv.
[15-2] A humanized antibody comprising a light chain variable region consisting of an amino acid sequence from positions 21 to 127 of SEQ ID NO: 13 and a heavy chain variable region consisting of an amino acid sequence from positions 20 to 146 of SEQ ID NO: 15.
[15-3] A humanized antibody comprising a light chain consisting of an amino acid sequence from positions 21 to 233 of SEQ ID NO: 13 and a heavy chain consisting of an amino acid sequence from positions 20 to 476 of SEQ ID NO: 15.
[15-4] A humanized antibody comprising a light chain variable region consisting of an amino acid sequence from positions 21 to 127 of SEQ ID NO: 14 and a heavy chain variable region consisting of an amino acid sequence from positions 20 to 146 of SEQ ID NO: 16.
[15-5] A humanized antibody comprising a light chain consisting of an amino acid sequence from positions 21 to 233 of SEQ ID NO: 14 and a heavy chain consisting of an amino acid sequence from positions 20 to 476 of SEQ ID NO: 16.
[15-6] An anti-CD25 antibody or an antigen binding fragment of the antibody, having at least one or more of the

following characteristics (a) to (d):

(a) specifically binding to CD25,
(b) having no ability to block IL-2,
(c) exhibiting the ability to remove human regulatory T cells and/or the ability to promote the proliferation of human granzyme-positive CD8-positive cells by conjugation to a cytotoxic active compound, and
(d) having activity of being internalized in CD25-expressing cells by binding to CD25, wherein the CD25 is human CD25, cynomolgus monkey CD25, or mouse CD25, and

the antibody or the antigen binding fragment of the antibody comprises

CDRL1, CDRL2, and CDRL3 in any one combination selected from the group consisting of the following combinations (1) and (2):

(1) CDRL1 consisting of the amino acid sequence represented by SEQ ID NO: 1, CDRL2 consisting of the amino acid sequence represented by SEQ ID NO: 2 (KAS), and CDRL3 consisting of the amino acid sequence represented by SEQ ID NO: 3, and
(2) CDRL1 consisting of the amino acid sequence represented by SEQ ID NO: 4, CDRL2 consisting of the amino acid sequence represented by SEQ ID NO: 5 (FVS), and CDRL3 consisting of the amino acid sequence represented by SEQ ID NO: 6, and

CDRH1, CDRH2, and CDRH3 in any one combination selected from the group consisting of the following combinations (3) and (4):

(3) CDRH1 consisting of the amino acid sequence represented by SEQ ID NO: 7, CDRH2 consisting of the amino acid sequence represented by SEQ ID NO: 8, and CDRH3 consisting of the amino acid sequence represented by SEQ ID NO: 9, and
(4) CDRH1 consisting of the amino acid sequence represented by SEQ ID NO: 10, CDRH2 consisting of the amino acid sequence represented by SEQ ID NO: 11, and CDRH3 consisting of the amino acid sequence represented by SEQ ID NO: 12.

[15-7] An anti-CD25 antibody or an antigen binding fragment of the antibody, having at least one or more of the following characteristics (a) to (d):

(a) specifically binding to CD25,
(b) having no ability to block IL-2,
(c) exhibiting the ability to remove human regulatory T cells and/or the ability to promote the proliferation of human granzyme-positive CD8-positive cells by conjugation to a cytotoxic active compound, and
(d) having activity of being internalized in CD25-expressing cells by binding to CD25, wherein

the CD25 is human CD25 or cynomolgus monkey CD25, and the antibody or the antigen binding fragment of the antibody comprises

any one light chain variable region selected from the group consisting of the following light chain variable regions (1) to (4):

(1) a light chain variable region consisting of an amino acid sequence from positions 21 to 127 of SEQ ID NO: 13,
(2) a light chain variable region consisting of an amino acid sequence from positions 21 to 127 of SEQ ID NO: 14,
(3) a light chain variable region consisting of an amino acid sequence having at least 95% or higher sequence identity to the sequences of framework regions except for CDR sequences in each of the amino acid sequences of (1) and (2), and
(4) a light chain variable region consisting of an amino acid sequence derived by the deletion, substitution, or addition of one or several amino acids from the sequences of framework regions except for CDR sequences in each of the amino acid sequences of (1) and (2), and

any one heavy chain variable region selected from the group consisting of the following heavy chain variable

regions (5) to (8):

(5) a heavy chain variable region consisting of an amino acid sequence from positions 20 to 146 of SEQ ID NO: 15,
(6) a heavy chain variable region consisting of an amino acid sequence from positions 20 to 146 of SEQ ID NO: 16,
(7) a heavy chain variable region consisting of an amino acid sequence having at least 95% or higher sequence homology to the sequences of framework regions except for CDR sequences in each of the amino acid sequences of (5) and (6), and
(8) a heavy chain variable region consisting of an amino acid sequence derived by the deletion, substitution, or addition of one or several amino acids from the sequences of framework regions except for CDR sequences in each of the amino acid sequences of (5) and (6).

[15-8] An anti-CD25 antibody or an antigen binding fragment of the antibody, having at least one or more of the following characteristics (a) to (d):

(a) specifically binding to CD25,
(b) having no ability to block IL-2,
(c) exhibiting the ability to remove human regulatory T cells and/or the ability to promote the proliferation of human granzyme-positive CD8-positive cells by conjugation to a cytotoxic active compound, and
(d) having activity of being internalized in CD25-expressing cells by binding to CD25, wherein

the CD25 is human CD25 or cynomolgus monkey CD25, and the antibody or the antigen binding fragment of the antibody comprises
any of the following combinations (1) and (2):

(1) a light chain consisting of an amino acid sequence from positions 21 to 233 of SEQ ID NO: 13 and a heavy chain consisting of an amino acid sequence from positions 20 to 476 of SEQ ID NO: 15, and
(2) a light chain consisting of an amino acid sequence from positions 21 to 233 of SEQ ID NO: 14 and a heavy chain consisting of an amino acid sequence from positions 20 to 476 of SEQ ID NO: 16.

[16] A polynucleotide encoding the antibody or the antigen binding fragment of the antibody according to any one of [1] to [15-8].
[17] The polynucleotide according to [16], wherein the polynucleotide comprises
polynucleotides in any one combination selected from the group consisting of the following combinations (1) and (2):

(1) a polynucleotide encoding a light chain variable region comprising CDRL1 consisting of the amino acid sequence represented by SEQ ID NO: 1, CDRL2 consisting of the amino acid sequence represented by SEQ ID NO: 2 (KAS), and CDRL3 consisting of the amino acid sequence represented by SEQ ID NO: 3, and a polynucleotide encoding a heavy chain variable region comprising CDRH1 consisting of the amino acid sequence represented by SEQ ID NO: 7, CDRH2 consisting of the amino acid sequence represented by SEQ ID NO: 8, and CDRH3 consisting of the amino acid sequence represented by SEQ ID NO: 9, and
(2) a polynucleotide encoding a light chain variable region comprising CDRL1 consisting of the amino acid sequence represented by SEQ ID NO: 4, CDRL2 consisting of the amino acid sequence represented by SEQ ID NO: 5 (FVS), and CDRL3 consisting of the amino acid sequence represented by SEQ ID NO: 6, and a polynucleotide encoding a heavy chain variable region comprising CDRH1 consisting of the amino acid sequence represented by SEQ ID NO: 10, CDRH2 consisting of the amino acid sequence represented by SEQ ID NO: 11, and CDRH3 consisting of the amino acid sequence represented by SEQ ID NO: 12.

[18] The polynucleotide according to [16] or [17], wherein the polynucleotide comprises a polynucleotide encoding a light chain consisting of an amino acid sequence from positions 21 to 233 of SEQ ID NO: 13 and a polynucleotide encoding a heavy chain consisting of an amino acid sequence from positions 20 to 476 of SEQ ID NO: 15.
[19] The polynucleotide according to [16] or [17], wherein the polynucleotide comprises a polynucleotide encoding a light chain consisting of an amino acid sequence from positions 21 to 233 of SEQ ID NO: 14 and a polynucleotide encoding a heavy chain consisting of an amino acid sequence from positions 20 to 476 of SEQ ID NO: 16.
[20] An expression vector comprising the polynucleotide according to any one of [16] to [19].
[21] A host cell transformed with the expression vector according to [20].
[22] The host cell according to [21], wherein the host cell is a eukaryotic cell.

[23] A method for producing an antibody or an antigen binding fragment of the antibody, comprising the steps of: culturing the host cell according to [21] or [22]; and collecting the antibody or the antigen binding fragment of the antibody of interest from the cultures obtained in the preceding step.

[24] The antibody or the antigen binding fragment of the antibody according to any one of [1] to [15-8], wherein a heavy chain or a light chain has one or two or more modifications selected from the group consisting of N-linked glycosylation, O-linked glycosylation, N-terminal processing, C-terminal processing, deamidation, isomerization of aspartic acid, oxidation of methionine, addition of a methionine residue to the N terminus, amidation of a proline residue, conversion of N-terminal glutamine or N-terminal glutamic acid to pyroglutamic acid, and deletion of one or two amino acids from the carboxyl terminus.

[25] The antibody according to [24], wherein one or two amino acids are deleted from the carboxyl terminus of a heavy chain.

[26] The antibody according to [25], wherein one amino acid is deleted from each of the carboxyl termini of both of two heavy chains.

[26-2] The humanized antibody according to any one of [15-2] to [15-8], wherein one amino acid is deleted from each of the carboxyl termini of both of two heavy chains of the antibody.

[26-3] The humanized antibody according to [26-2], wherein the deleted amino acid is lysine.

[27] The antibody according to [24], wherein a proline residue at the carboxyl terminus of a heavy chain is further amidate.

[28] The antibody or the antigen binding fragment of the antibody according to any one of [1] to [15-8] and [24] to [27], wherein the antibody or the antigen binding fragment has the modification of a glycan that enhances antibody-dependent cellular cytotoxic activity.

[29] An antibody-drug conjugate in which the antibody or the antigen binding fragment of the antibody according to any one of [1] to [15-8] and [24] to [28] is conjugated to a drug.

[30] The antibody-drug conjugate according to [29], wherein the drug is any one or more drugs selected from the group consisting of a substance having cytotoxic activity, a cytotoxic active compound, a substance having antitumor activity, a chemotherapeutic, a molecular targeting drug, an immunoactivator, an immunosuppressant, a toxin, a photosensitive substance, a drug for diagnosis, a protein, a peptide, an amino acid, a nucleic acid, an antigen, a vitamin, a hormone, a substance effective for blood disease, a substance effective for autoimmune disease, an anti-inflammatory substance, an antibacterial substance, an antifungal substance, an antiparasitic substance, an antiviral substance, and an anti-anesthetic substance.

[31] The antibody-drug conjugate according to [30], wherein the drug is a cytotoxic active compound.

[32] The antibody-drug conjugate according to [31], wherein the cytotoxic active compound is a cytotoxic active compound represented by the following formula:

[Formula 1]

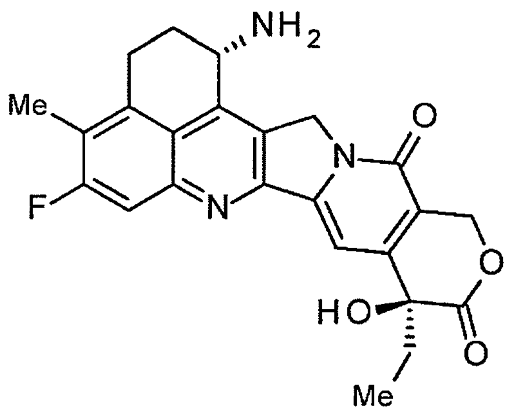

[32-2] The antibody-drug conjugate according to any one of [29] to [32], wherein the antibody is conjugated to the drug via a linker having a structure represented by the following formula: -(Succinimid-3-yl-N)-$(CH_2)n^1$-C(=O)-L-GGFG-NH-X-C(=O) -, wherein $n^1$ represents an integer of 2 to 8, L represents -NH-$(CH_2$-$CH_2$-$O)n^2$-$CH_2$-$CH_2$- or a single bond, $n^2$ represents an integer of 1 to 6, X represents a linear or branched alkylene group having 1 to 20 carbon atoms (one or two or more methylene groups in the chain are optionally replaced with an oxygen atom or a sulfur atom), the alkylene group optionally having a cyclic saturated hydrocarbon group having 1 to 6 carbon atoms as a portion of the structure thereof, the antibody is connected to the end of -(Succinimid-3-yl-N), the drug is connected to the carbonyl

group at another end, GGFG represents an amino acid sequence consisting of glycine-glycine-phenylalanine-glycine linked through peptide bonds, and -(Succinimid-3-yl-N)- has a structure represented by the following formula:

[Formula 2]

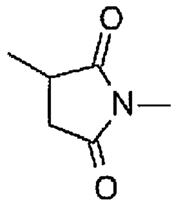

which is connected to the antibody at the 3-position thereof and is connected to a methylene group in the linker structure containing this structure on the nitrogen atom at the 1-position.

[33] The antibody-drug conjugate according to any one of [29] to [32-2], wherein the antibody is conjugated to the drug via a linker having any structure selected from the group consisting of the following formulas (a) to (f):

(a) -(Succinimid-3-yl-N)-$CH_2CH_2$-C(=O)-GGFG-NH-$CH_2CH_2CH_2$-C(=O)-,
(b) - (Succinimid-3-yl-N)-$CH_2CH_2CH_2CH_2CH_2$-C(=O)-GGFG-NH-$CH_2CH_2CH_2$-C(=O)-,
(c) - (Succinimid-3-yl-N)-$CH_2CH_2CH_2CH_2CH_2$-C(=O)-GGFG-NH-$CH_2$-O-$CH_2$-C(=O)-,
(d) - (Succinimid-3-yl-N)-$CH_2CH_2CH_2CH_2CH_2$-C(=O)-GGFG-NH-$CH_2CH_2$-O-$CH_2$-C(=O)-,
(e) - (Succinimid-3-yl-N)-$CH_2CH_2$-C(=O)-NH-$CH_2CH_2$O-$CH_2CH_2$O-$CH_2CH_2$-C(=O)-GGFG-NH-$CH_2CH_2CH_2$-C(=O)-, and
(f)- (Succinimid-3-yl-N)-$CH_2CH_2$-C(=O)-NH-$CH_2CH_2$O-$CH_2CH_2$O-$CH_2CH_2$O-$CH_2CH_2$O-$CH_2CH_2$-C(=O)-GGFG-NH-$CH_2CH_2CH_2$-C(=O)-, wherein the antibody is connected to the end of - (Succinimid-3-yl-N), the drug is connected to the carbonyl group at another end wherein the nitrogen atom of the amino group at the 1-position is used as a connecting position, GGFG represents an amino acid sequence consisting of glycine-glycine-phenylalanine-glycine linked through peptide bonds, and -(Succinimid-3-yl-N)- has a structure represented by the following formula:

[Formula 3]

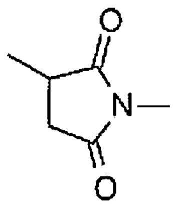

which is connected to the antibody at the 3-position thereof and is connected to a methylene group in the linker structure containing this structure on the nitrogen atom at the 1-position.

[34] The antibody-drug conjugate according to any one of [29] to [33] wherein the linker is represented by any formula selected from the group consisting of the following formulas (c), (d), and (e):

(c) - (Succinimid-3-yl-N)-$CH_2CH_2CH_2CH_2CH_2$-C(=O)-GGFG-NH-$CH_2$-O-$CH_2$-C(=O)-,
(d) - (Succinimid-3-yl-N)-$CH_2CH_2CH_2CH_2CH_2$-C(=O)-GGFG-NH-$CH_2CH_2$-O-$CH_2$-C(=O)-,
(e) - (Succinimid-3-yl-N)-$CH_2CH_2$-C(=O)-NH-$CH_2CH_2$O-$CH_2CH_2$O-$CH_2CH_2$-C(=O)-GGFG-NH-$CH_2CH_2CH_2$-C(=O)-

[35] The antibody-drug conjugate according to any one of [29] to [34] wherein the linker is represented by the following formula (c) or (e):

(c) - (Succinimid-3-yl-N)-$CH_2CH_2CH_2CH_2CH_2$-C(=O)-GGFG-NH-$CH_2$-O-$CH_2$-C(=O)-,
(e) - (Succinimid-3-yl-N)-$CH_2CH_2$-C(=O)-NH-$CH_2CH_2$O-$CH_2CH_2$O-$CH_2CH_2$-C(=O)-GGFG-NH-$CH_2CH_2CH_2$-C(=O)-

[36] The antibody-drug conjugate according to any one of [29] to [35], wherein the antibody-drug conjugate has a structure represented by the following formula:

[Formula 4]

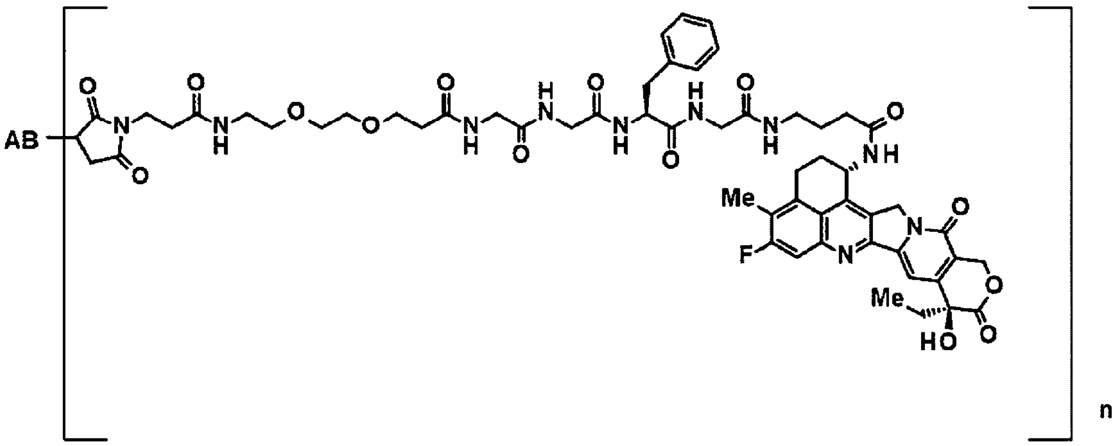

wherein AB represents the antibody or the antigen binding fragment of the antibody, n represents an average number of units of the drug-linker structure conjugated to the antibody or the antigen binding fragment of the antibody per antibody or per antigen binding fragment of the antibody, and the antibody or the antigen binding fragment of the antibody is connected to the linker via a sulfhydryl group derived from the antibody or the antigen binding fragment of the antibody.

[37] The antibody-drug conjugate according to any one of [29] to [35], wherein the antibody-drug conjugate has a structure represented by the following formula:

[Formula 5]

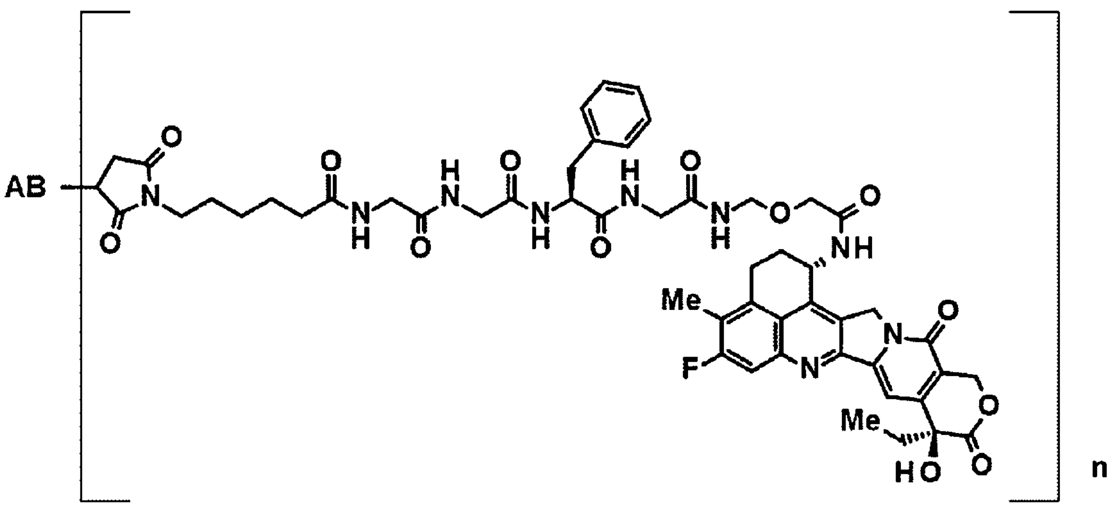

wherein AB represents the antibody or the antigen binding fragment of the antibody, n represents an average number of units of the drug-linker structure conjugated to the antibody or the antigen binding fragment of the antibody per antibody or per antigen binding fragment of the antibody, and the antibody or the antigen binding fragment of the antibody is connected to the linker via a sulfhydryl group derived from the antibody or the antigen binding fragment of the antibody.

[38] The antibody-drug conjugate according to any one of [29] to [37], wherein the antibody is an antibody comprising a light chain variable region and a heavy chain variable region in any one combination selected from the group consisting of the following combinations (1) and (2):

(1) a light chain variable region consisting of an amino acid sequence from positions 21 to 127 of SEQ ID NO: 13

and a heavy chain variable region consisting of an amino acid sequence from positions 20 to 146 of SEQ ID NO: 15, and

(2) a light chain variable region consisting of an amino acid sequence from positions 21 to 127 of SEQ ID NO: 14 and a heavy chain variable region consisting of an amino acid sequence from positions 20 to 146 of SEQ ID NO: 16, or an antigen binding fragment of the antibody.

[39] The antibody-drug conjugate according to [38], wherein the antibody is an antibody comprising a light chain variable region consisting of an amino acid sequence from positions 21 to 127 of SEQ ID NO: 13 and a heavy chain variable region consisting of an amino acid sequence from positions 20 to 146 of SEQ ID NO: 15, or an antigen binding fragment of the antibody.

[40] The antibody-drug conjugate according to [38], wherein the antibody is an antibody comprising a light chain variable region consisting of an amino acid sequence from positions 21 to 127 of SEQ ID NO: 14 and a heavy chain variable region consisting of an amino acid sequence from positions 20 to 146 of SEQ ID NO: 16, or an antigen binding fragment of the antibody.

[41] The antibody-drug conjugate according to any one of [29] to [37], wherein the antibody is an antibody comprising a light chain and a heavy chain in any one combination selected from the group consisting of the following combinations (1) and (2):

(1) a light chain consisting of an amino acid sequence from positions 21 to 233 of SEQ ID NO: 13 and a heavy chain consisting of an amino acid sequence from positions 20 to 476 of SEQ ID NO: 15, and

(2) a light chain consisting of an amino acid sequence from positions 21 to 233 of SEQ ID NO: 14 and a heavy chain consisting of an amino acid sequence from positions 20 to 476 of SEQ ID NO: 16,

or an antigen binding fragment of the antibody.

[42] The antibody-drug conjugate according to [41], wherein the antibody is an antibody comprising a light chain consisting of an amino acid sequence from positions 21 to 233 of SEQ ID NO: 13 and a heavy chain consisting of an amino acid sequence from positions 20 to 476 of SEQ ID NO: 15, or an antigen binding fragment of the antibody.

[43] The antibody-drug conjugate according to [41], wherein the antibody is an antibody comprising a light chain consisting of an amino acid sequence from positions 21 to 233 of SEQ ID NO: 14 and a heavy chain consisting of an amino acid sequence from positions 20 to 476 of SEQ ID NO: 16, or an antigen binding fragment of the antibody.

[44] The antibody-drug conjugate according to any one of [29] to [43], wherein a heavy chain or a light chain has one or two or more modifications selected from the group consisting of N-linked glycosylation, O-linked glycosylation, N-terminal processing, C-terminal processing, deamidation, isomerization of aspartic acid, oxidation of methionine, addition of a methionine residue to the N terminus, amidation of a proline residue, conversion of N-terminal glutamine or N-terminal glutamic acid to pyroglutamic acid, and deletion of one or two amino acids from the carboxyl terminus.

[45] The antibody-drug conjugate according to [44], wherein one or two amino acids are deleted from the carboxyl terminus of a heavy chain.

[46] The antibody-drug conjugate according to [45], wherein one amino acid is deleted from each of the carboxyl termini of both of two heavy chains.

[47] The antibody-drug conjugate according to [44], wherein a proline residue at the carboxyl terminus of a heavy chain is further amidate.

[48] The antibody-drug conjugate according to any one of [29] to [47], wherein the antibody-drug conjugate has the modification of a glycan for enhancing antibody-dependent cellular cytotoxic activity.

[49] The antibody-drug conjugate according to any one of [29] to [48], wherein the average number of units of the drug-linker structure conjugated per antibody is in the range of 1 to 10.

[50] The antibody-drug conjugate according to [49], wherein the average number of units of the drug-linker structure conjugated per antibody is in the range of 2 to 8.

[51] The antibody-drug conjugate according to [50], wherein the average number of units of the drug-linker structure conjugated per antibody is in the range of 5 to 8.

[52] The antibody-drug conjugate according to [51], wherein the average number of units of the drug-linker structure conjugated per antibody is 7 to 8.

[52-2] An antibody-drug conjugate having a structure represented by the following formula:

[Formula 6]

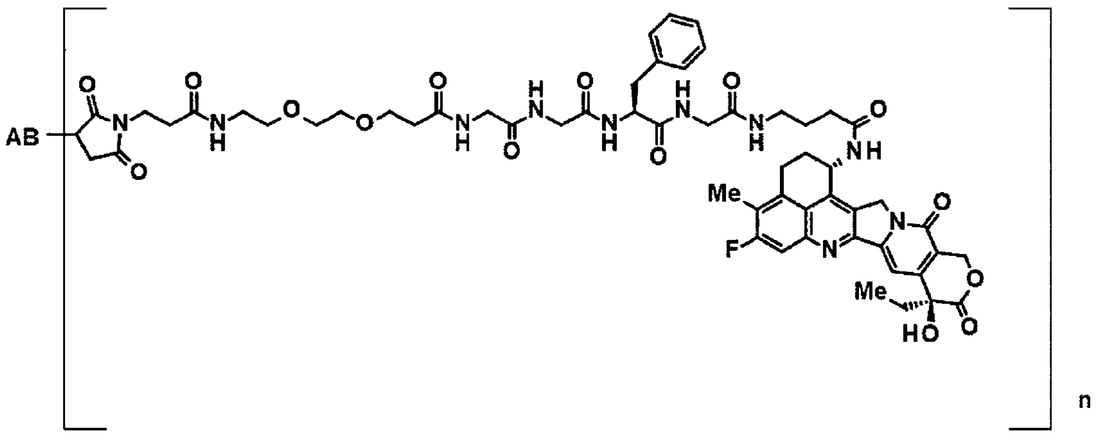

wherein AB represents a humanized antibody comprising a light chain variable region consisting of an amino acid sequence from positions 21 to 127 of SEQ ID NO: 13 and a heavy chain variable region consisting of an amino acid sequence from positions 20 to 146 of SEQ ID NO: 15, n represents an average number of units of the drug-linker structure conjugated to the antibody per antibody, and the average number is in the range of 7 to 8, and the antibody is connected to the linker via a sulfhydryl group derived from the antibody.

[52-3] An antibody-drug conjugate having a structure represented by the following formula:

[Formula 7]

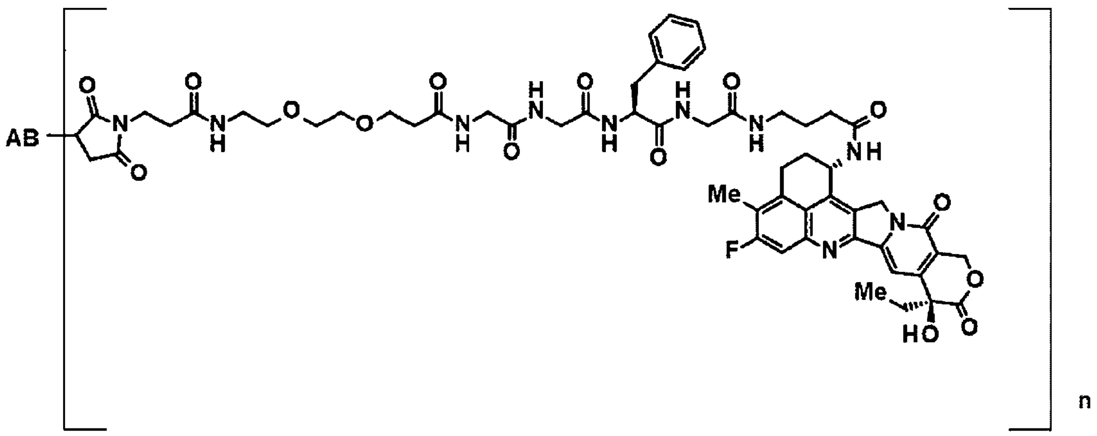

wherein AB represents a humanized antibody comprising a light chain consisting of an amino acid sequence from positions 21 to 233 of SEQ ID NO: 13 and a heavy chain consisting of an amino acid sequence from positions 20 to 476 of SEQ ID NO: 15, n represents an average number of units of the drug-linker structure conjugated to the antibody per antibody, and the average number is in the range of 7 to 8, and the antibody is connected to the linker via a sulfhydryl group derived from the antibody.

[52-4] An antibody-drug conjugate having a structure represented by the following formula:

[Formula 8]

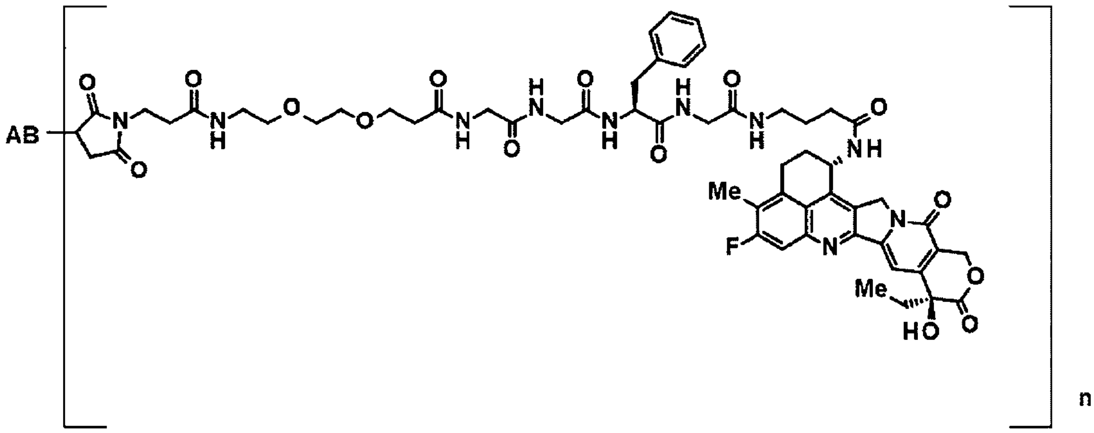

wherein AB represents a humanized antibody comprising a light chain variable region consisting of an amino acid sequence from positions 21 to 127 of SEQ ID NO: 14 and a heavy chain variable region consisting of an amino acid sequence from positions 20 to 146 of SEQ ID NO: 16, n represents an average number of units of the drug-linker structure conjugated to the antibody per antibody, and the average number is in the range of 7 to 8, and the antibody is connected to the linker via a sulfhydryl group derived from the antibody.

[52-5] An antibody-drug conjugate having a structure represented by the following formula:

[Formula 9]

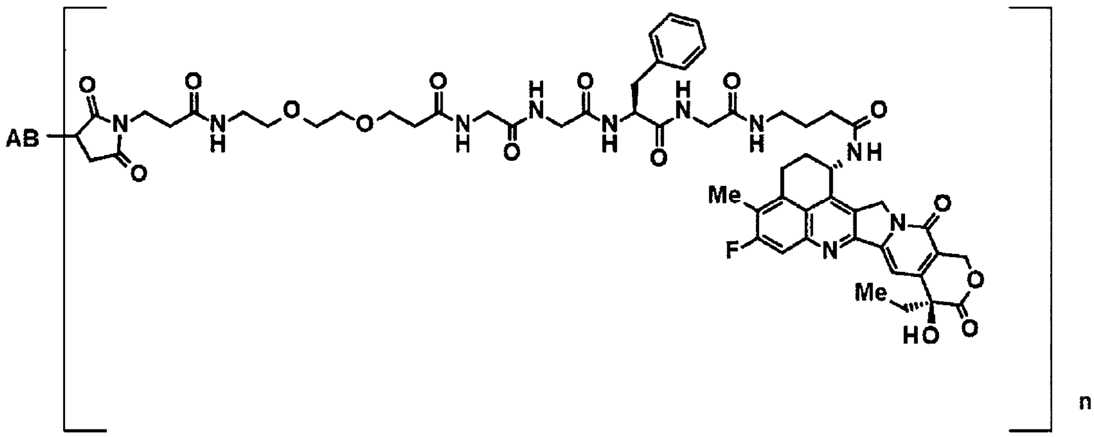

wherein AB represents a humanized antibody comprising a light chain consisting of an amino acid sequence from positions 21 to 233 of SEQ ID NO: 14 and a heavy chain consisting of an amino acid sequence from positions 20 to 476 of SEQ ID NO: 16, n represents an average number of units of the drug-linker structure conjugated to the antibody per antibody, and the average number is in the range of 7 to 8, and the antibody is connected to the linker via a sulfhydryl group derived from the antibody.

[52-6] An antibody-drug conjugate having a structure represented by the following formula:

[Formula 10]

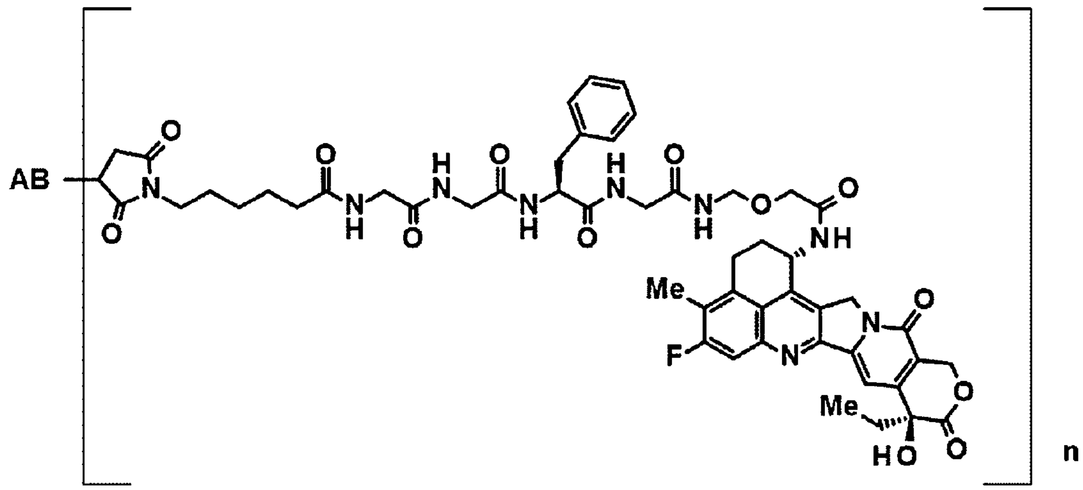

wherein AB represents an antibody comprising a light chain variable region consisting of the amino acid sequence of SEQ ID NO: 18 and a heavy chain variable region consisting of the amino acid sequence of SEQ ID NO: 20, n represents an average number of units of the drug-linker structure conjugated to the antibody per antibody, and the average number is in the range of 7 to 8, and the antibody is connected to the linker via a sulfhydryl group derived from the antibody.

[52-7] An antibody-drug conjugate having a structure represented by the following formula:

[Formula 11]

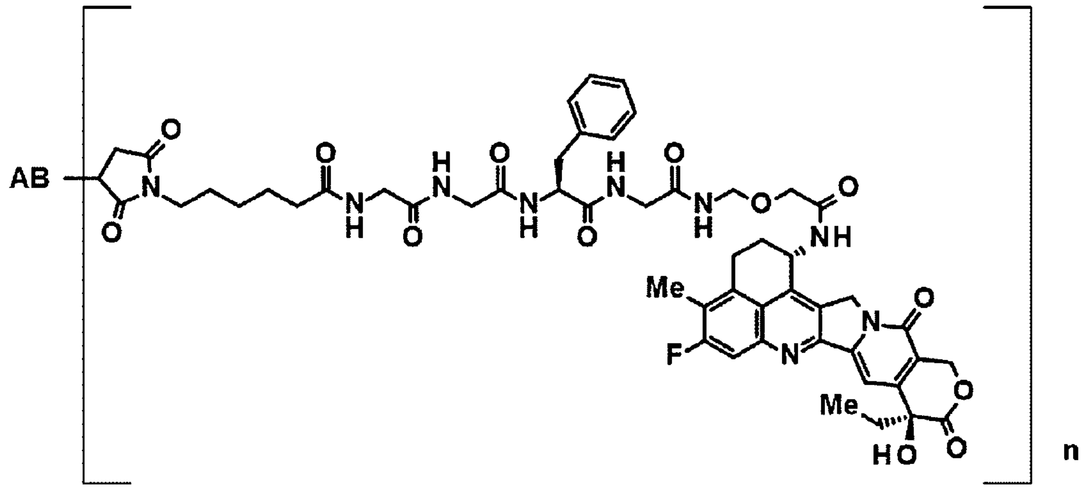

wherein AB represents an antibody comprising a light chain consisting of an amino acid sequence from positions 21 to 232 of SEQ ID NO: 26 and a heavy chain consisting of an amino acid sequence from positions 20 to 476 of SEQ ID NO: 27, n represents an average number of units of the drug-linker structure conjugated to the antibody per antibody, and the average number is in the range of 7 to 8, and the antibody is connected to the linker via a sulfhydryl group derived from the antibody.

[52-8] The antibody-drug conjugate according to any one of [52-2] to [52-7], wherein one amino acid is deleted from each of the carboxyl termini of both of two heavy chains of the antibody.

[52-9] The antibody-drug conjugate according to [52-8], wherein the deleted amino acid is lysine.

[53] A pharmaceutical composition comprising the antibody or the antigen binding fragment of the antibody according to any one of [1] to [15-8] and [24] to [28], the antibody-drug conjugate according to any one of [29] to [52-9], a salt of the component, or a hydrate of the component or the salt.

[54] The pharmaceutical composition according to [53], wherein the pharmaceutical composition is an antitumor drug.

[55] The pharmaceutical composition according to [54], wherein in a subject, tumor cells express CD25, or tumor immunity is suppressed by regulatory T cells.

[56] The pharmaceutical composition according to [55], wherein in a subject, the tumor environment and/or the lymph

node contains regulatory T cells.

[57] The pharmaceutical composition according to any one of [53] to [56], wherein the tumor is blood cancer, breast cancer, small-cell lung cancer, non-small cell lung cancer, head and neck cancer, brain tumor, glioma, eye tumor, esophageal cancer, stomach cancer, colorectal cancer, colon cancer, liver cancer, pancreatic cancer, renal cancer, kidney cancer, urinary bladder cancer, adrenal cortex cancer, prostate cancer, uterine cervical cancer, endometrial cancer, ovary cancer, melanoma, or sarcoma.

[58] A method for treating tumor, comprising administering any member selected from the antibody or the antigen binding fragment of the antibody according to any one of [1] to [15-8] and [24] to [28], the antibody-drug conjugate according to any one of [29] to [52-9], a salt of the component, and a hydrate of the component or the salt to an individual.

[59] The treatment method according to [58], wherein in a subject, tumor cells express CD25 or tumor immunity is suppressed by regulatory T cells.

[60] The treatment method according to [58] or [59], wherein in a subject, the tumor environment and/or the lymph node contains regulatory T cells.

[61] The treatment method according to any one of [58] to [60], wherein the tumor is blood cancer, breast cancer, small cell lung cancer, non-small cell lung cancer, head and neck cancer, brain tumor, glioma, eye tumor, esophageal cancer, stomach cancer, colorectal cancer, colon cancer, liver cancer, pancreatic cancer, renal cancer, kidney cancer, urinary bladder cancer, adrenal cortex cancer, prostate cancer, uterine cervical cancer, endometrial cancer, ovary cancer, melanoma, or sarcoma.

[62] A method for treating tumor, comprising administering a pharmaceutical composition comprising at least one member selected from the antibody or the antigen binding fragment of the antibody according to any one of [1] to [15-8] and [24] to [28], the antibody-drug conjugate according to any one of [29] to [52-9], a salt of the component, and a hydrate of the component or the salt, and at least one antitumor drug to an individual simultaneously, separately, or continuously.

[63] A method for producing an antibody-drug conjugate, comprising the steps of: culturing the host cell according to [21] or [22]; collecting the antibody or the antigen binding fragment of the antibody of interest from the cultures obtained in the preceding step; and reacting the antibody or the antigen binding fragment of the antibody obtained in the preceding step with a drug-linker intermediate compound.

[64] A pharmaceutical composition in which the antibody-drug conjugate according to any one of [29] to [52-9] and an immune checkpoint inhibitor are administered in combination.

[65] The pharmaceutical composition according to [64], wherein the antibody-drug conjugate and the immune checkpoint inhibitor are separately contained as active ingredients in different formulations and administered simultaneously or at different times.

[66] The pharmaceutical composition according to [64], wherein the antibody-drug conjugate and the immune checkpoint inhibitor are contained as active ingredients in the same formulation.

[67] The pharmaceutical composition according to any one of [64] to [66], wherein the immune checkpoint inhibitor is any one or more members selected from the group consisting of an anti-PD-1 antibody, an anti-PD-L1 antibody, and an anti-CTLA-4 antibody.

[68] The pharmaceutical composition according to [67], wherein the immune checkpoint inhibitor is an anti-PD-1 antibody.

[69] The pharmaceutical composition according to [68], wherein the anti-PD-1 antibody is any one or more members selected from the group consisting of nivolumab, pembrolizumab, sintilimab, spartalizumab, dostarlimab, serplulimab, tislelizumab, penpulimab, toripalimab, zimberelimab, camrelizumab, retifanlimab, cemiplimab, prolgolimab, geptanolimab, enlonstobart, QL-1604, pucotenlimab, and finotonlimab.

[69-2] The pharmaceutical composition according to [68], wherein the anti-PD-1 antibody is nivolumab or pembrolizumab.

[70] The pharmaceutical composition according to [67], wherein the immune checkpoint inhibitor is an anti-PD-L1 antibody.

[71] The pharmaceutical composition according to [70], wherein the anti-PD-L1 antibody is any one or more members selected from the group consisting of atezolizumab, durvalumab, cosibelimab, adebrelimab, sugemalimab, avelumab, socazolimab, KL-A167, and envafolimab.

[71-2] The pharmaceutical composition according to [70], wherein the anti-PD-L1 antibody is any one or more members selected from the group consisting of atezolizumab, durvalumab, and avelumab.

[72] The pharmaceutical composition according to [67], wherein the immune checkpoint inhibitor is an anti-CTLA-4 antibody.

[73] The pharmaceutical composition according to [72], wherein the anti-CTLA-4 antibody is any one or more members selected from the group consisting of ipilimumab, botensilimab, and tremelimumab.

[73-2] The pharmaceutical composition according to [72], wherein the anti-CTLA-4 antibody is ipilimumab.

[73-3] The pharmaceutical composition according to any one of [64] to [73-2] for the treatment of a cancer.

[73-4] The pharmaceutical composition according to any one of [64] to [73-2] for the treatment of at least one cancer selected from the group consisting of blood cancer, breast cancer, smallcell lung cancer, non-small cell lung cancer, head and neck cancer, brain tumor, glioma, eye tumor, esophageal cancer, stomach cancer, colorectal cancer, colon cancer, liver cancer, pancreatic cancer, renal cancer, kidney cancer, urinary bladder cancer, adrenal cortex cancer, prostate cancer, uterine cervical cancer, endometrial cancer, ovary cancer, melanoma, and sarcoma.

[74] The pharmaceutical composition according to any one of [64] to [73-2] for the treatment of a cancer that exhibits resistance to the immune checkpoint inhibitor.

[75] A method for treating a disease, comprising administering an antibody-drug conjugate and an immune checkpoint inhibitor in combination to an individual in need of treatment, wherein the antibody-drug conjugate and the immune checkpoint inhibitor are as defined in any one of [29] to [52-9] and [67] to [73-2].

[76] The treatment method according to [75], wherein the disease is as defined in any one of [73-3] to [74].

[77] The treatment method according to [75] or [76], wherein the antibody-drug conjugate and the immune checkpoint inhibitor are separately contained as active ingredients in different formulations and administered simultaneously or at different times.

[78] The treatment method according to [75] or [76], wherein the antibody-drug conjugate and the immune checkpoint inhibitor are contained as active ingredients in the same formulation.

[79] An antibody-drug conjugate for combined use with an immune checkpoint inhibitor for the treatment of a disease, wherein the antibody-drug conjugate and the immune checkpoint inhibitor are as defined in any one of [29] to [52-9] and [67] to [73-2].

[80] The antibody-drug conjugate according to [79], wherein the disease is as defined in any one of [73-3] to [74].

[81] The antibody-drug conjugate according to [79] or [80], wherein the antibody-drug conjugate and the immune checkpoint inhibitor are separately contained as active ingredients in different formulations and administered simultaneously or at different times.

[82] The antibody-drug conjugate according to [79] or [80], wherein the antibody-drug conjugate and the immune checkpoint inhibitor are contained as active ingredients in the same formulation.

[83] Use of an antibody-drug conjugate combined with an immune checkpoint inhibitor in the production of a medicament for the treatment of a disease, wherein the antibody-drug conjugate and the immune checkpoint inhibitor are as defined in any one of [29] to [52-9] and [67] to [73-2].

[84] The use according to [83], wherein the disease is as defined in any one of [73-3] to [74].

[85] The use according to [83] or [84], wherein the antibody-drug conjugate and the immune checkpoint inhibitor are separately contained as active ingredients in different formulations and administered simultaneously or at different times.

[86] The use according to [83] or [84], wherein the antibody-drug conjugate and the immune checkpoint inhibitor are contained as active ingredients in the same formulation.

[87] A pharmaceutical product comprising an antibody-drug conjugate and an immune checkpoint inhibitor for administration in combination, wherein the antibody-drug conjugate and the immune checkpoint inhibitor are as defined in any one of [29] to [52-9] and [67] to [73-2].

[88] The pharmaceutical product according to [87] for the treatment of a disease defined in any one of [73-3] to [74].

[89] The pharmaceutical product according to [87] or [88], wherein the antibody-drug conjugate and the immune checkpoint inhibitor are separately contained as active ingredients in different formulations and administered simultaneously or at different times.

[90] The pharmaceutical product according to [87] or [88], wherein the antibody-drug conjugate and the immune checkpoint inhibitor are contained as active ingredients in the same formulation.

[91] A combination medicament comprising an antibody-drug conjugate and an immune checkpoint inhibitor, wherein the antibody-drug conjugate and the immune checkpoint inhibitor are as defined in any one of [29] to [52-9] and [67] to [73-2].

[92] The combination medicament according to [91] for the treatment of a disease defined in any one of [73-3] to [74].

[93] The combination medicament according to [91] or [92], wherein the antibody-drug conjugate and the immune checkpoint inhibitor are separately contained as active ingredients in different formulations and administered simultaneously or at different times.

[94] The combination medicament according to [91] or [92], wherein the antibody-drug conjugate and the immune checkpoint inhibitor are contained as active ingredients in the same formulation.

[95] A pharmaceutical combination comprising an antibody-drug conjugate and an immune checkpoint inhibitor, wherein the antibody-drug conjugate and the immune checkpoint inhibitor are as defined in any one of [29] to [52-9] and [67] to [73-2].

[96] The pharmaceutical combination according to [95] for the treatment of a disease defined in any one of [73-3] to [74].

[97] The pharmaceutical combination according to [95] or [96], wherein the antibody-drug conjugate and the immune checkpoint inhibitor are separately contained as active ingredients in different formulations and administered simultaneously or at different times.

[97] The pharmaceutical combination according to [95] or [96], wherein the antibody-drug conjugate and the immune checkpoint inhibitor are contained as active ingredients in the same formulation.

[99] Use of an antibody-drug conjugate combined with an immune checkpoint inhibitor for the treatment of a disease, wherein the antibody-drug conjugate and the immune checkpoint inhibitor are as defined in any one of [29] to [52-9] and [67] to [73-2].

[100] The use according to [99], wherein the disease is as defined in any one of [73-3] to [74].

[101] The use according to [99] or [100], wherein the antibody-drug conjugate and the immune checkpoint inhibitor are separately contained as active ingredients in different formulations and administered simultaneously or at different times.

[102] The use according to [99] or [100], wherein the antibody-drug conjugate and the immune checkpoint inhibitor are contained as active ingredients in the same formulation.

[103] A medicament comprising an antibody-drug conjugate and an immune checkpoint inhibitor, wherein the antibody-drug conjugate and the immune checkpoint inhibitor are administered in combination and are as defined in any one of [29] to [52-9] and [67] to [73-2].

[104] The medicament according to [103] for the treatment of a disease defined in any one of [73-3] to [74].

[105] The medicament according to [103] or [104], wherein the antibody-drug conjugate and the immune checkpoint inhibitor are separately contained as active ingredients in different formulations and administered simultaneously or at different times.

[106] The medicament according to [103] or [104], wherein the antibody-drug conjugate and the immune checkpoint inhibitor are contained as active ingredients in the same formulation.

Advantageous Effects of Invention

The present invention provides an antibody that binds to CD25, an antibody-drug conjugate that comprises the antibody and has antitumor activity, a medicament having a therapeutic effect on tumor using the antibody-drug conjugate, and a method for treating tumor using the antibody, the antibody-drug conjugate, or the medicament, etc.

Brief Description of Drawings

[Figure 1] Figure 1 is a diagram showing the binding activity of MAb1 against cells expressing human, cynomolgus monkey, or mouse CD25.

[Figure 2] Figure 2 is a diagram showing change in human pan T cell proliferation by MAb1, a rat IgG2a control antibody, an IL-2 blocking anti-human CD25 antibody basiliximab, or an IL-2 non-blocking anti-human CD25 antibody 7G7B6 in the presence of IL-2. The error bar in the diagram depicts standard error (n = 2 to 24).

[Figure 3] Figure 3 is a diagram showing change in phosphorylated STAT5 in human PBMC cells by MAb1, a rat IgG2a control antibody, basiliximab, or 7G7B6 in the presence of IL-2. The error bar in the diagram depicts standard error (n = 3).

[Figure 4] Figure 4 is a diagram showing the internalization activity of MAb1 or a rat IgG2a control antibody through change in human pan T cell survival rate using an anti-rat IgG reagent conjugated with saporin (Fab-ZAP). The error bar in the diagram depicts standard error (n = 3).

[Figure 5] Figure 5 is a diagram showing the competitiveness of MAb1, a human IgG1 control antibody, or an anti-human CD25 antibody basiliximab with a CD25 antibody M-A251. The error bar in the diagram depicts standard deviation (n = 3).

[Figure 6] Figure 6 is a diagram showing the binding activity of MAb2 against cells expressing human, cynomolgus monkey, or mouse CD25. The error bar in the diagram depicts standard error (n = 3).

[Figure 7] Figure 7 is a diagram showing change in phosphorylated STAT5 in CTLL-2 cells by Mab2, a rat IgG2a control antibody, or an IL-2 blocking anti-mouse CD25 antibody PC61_hIgG1LALA (in the diagram, represented by PC61) in the presence of IL-2. The error bar in the diagram depicts standard error (n = 3).

[Figure 8] Figure 8 is a diagram showing the internalization activity of MAb2, PC61, or a rat IgG2a control antibody through change in mouse pan T cell survival rate using an anti-rat IgG reagent conjugated with saporin (Fab-ZAP). The error bar in the diagram depicts standard error (n = 3).

[Figure 9] Figure 9 is a diagram showing the binding activity of cMAb1_hIgG1LALA against cells expressing human, cynomolgus monkey, or mouse CD25. The error bar in the diagram depicts standard error (n = 3).

[Figure 10] Figure 10 is a diagram showing change in human CD4-positive T cell proliferation by cMAb1_hIgG1LALA, a rat IgG1 control antibody, or basiliximab in the presence of PMA/PHA. The error bar in the diagram depicts standard error (n = 3).

[Figure 11] Figure 11 is a diagram showing change in phosphorylated STAT5 in human PBMC by cMAb1_hIgG1LALA, a human IgG1 control antibody, or basiliximab in the presence of IL-2. The error bar in the diagram depicts standard error (n = 3).

[Figure 12] Figure 12 is a diagram showing the suppressive activity of cMAb1_hIgG1LALA-ADC or human IgG1LALA-ADC on *in vitro* cell proliferation in a CD25-positive human tumor cell line Karpas-299 and in a CD25-negative human tumor cell line Daudi. The error bar in the diagram depicts standard deviation (n = 3).

[Figure 13] Figure 13 is a diagram showing change in the ratio of Treg, CD8-positive T cells, or GZMB-positive and CD8-positive T cells to human immunocytes in tumor tissues of MDA-MB-231 cancer cell-bearing hematopoietic stem cell-transplanted NOG mice given cMAb1_hIgG1LALA-ADC or human IgG1LALA-ADC (n = 5).

[Figure 14] Figure 14 is a diagram showing the binding activity of cMAb2_hIgG1LALA against cells expressing human, cynomolgus monkey, or mouse CD25. The error bar in the diagram depicts standard error (n = 3).

[Figure 15] Figure 15 is a diagram showing the suppressive activity of cMAb2_hIgG1LALA-ADC, human IgG1, or PC61_hIgG1LALA on *in vitro* cell proliferation in a CD25-positive mouse T cell line CTLL-2. The error bar in the diagram depicts standard error (n = 3).

[Figure 16] Figure 16 is a diagram showing the suppressive activity of cMAb2_hIgG1LALA-ADC or human IgG1LALA-ADC on *in vitro* cell proliferation in a CD25-positive mouse T cell line CTLL-2. The error bar in the diagram depicts standard deviation (n = 3).

[Figure 17] Figure 17 is a diagram showing change in tumor volume in CT26 cancer cell-bearing mice given cMAb2_hIgG1-ADC or human IgG1-ADC. The error bar in the diagram depicts standard error (n = 9).

[Figure 18] Figure 18 is a diagram showing change in the numbers of Treg, FoxP3-negative and CD4-positive T cells, CD8-positive T cells, and granzyme-positive and CD8-positive T cells per tissue weight in the tumor of CT26 cancer cell-bearing mice given cMAb2_hIgG1-ADC or human IgG1-ADC. The dots in the diagram depicts each individual, and the diagram is indicated in box-and-whisker plot based thereon (n = 10).

[Figure 19] Figure 19 is a diagram showing the binding activity of hMAb1A and hMAb1B against cells transiently expressing human, cynomolgus monkey, or mouse CD25. The error bar in the diagram depicts standard deviation (n = 3).

[Figure 20] Figure 20 is a diagram showing change in human PBMC proliferation by hMAb1A, hMAb1B, a human IgG1 control antibody, or basiliximab in the presence of IL-2. The error bar in the diagram depicts standard error (n = 3).

[Figure 21] Figure 21 is a diagram showing change in phosphorylated STAT5 in human PBMC by hMAb1A, hMAb1B, a human IgG1 control antibody, or basiliximab in the presence of PMA/PHA. The error bar in the diagram depicts standard error (n = 3).

[Figure 22-1] Figure 22-1 is a diagram showing the suppressive activity of hMAb1A-ADC, hMAb1A, or human IgG1-ADC on *in vitro* cell proliferation in a CD25-positive human tumor cell line Karpas-299 and a CD25-negative human tumor cell line Daudi. The error bar in the diagram depicts standard deviation (n = 3 or 6).

[Figure 22-2] Figure 22-2 is a diagram showing the suppressive activity of hMAb1B-ADC, hMAb1B, or human IgG1LALA-ADC on *in vitro* cell proliferation in a CD25-positive human tumor cell line EoL-1 and a CD25-negative human tumor cell line Daudi. The error bar in the diagram depicts standard deviation (n = 3).

[Figure 23] Figure 23 is a diagram showing change in the ratio of Treg, FoxP3-negative and CD4-positive T cells, CD8-positive T cells, or GZMB-positive and CD8-positive T cells to human CD45-positive cells in tumor tissues of MDA-MB-231 cancer cell-bearing hematopoietic stem cell-transplanted NOG mice given hMAb1A-ADC or ABS (n = 5).

[Figure 24] Figure 24 is a diagram showing change in the ratio of Treg or GZMB-positive and CD8-positive T cells to human CD45-positive cells in tumor tissues of MDA-MB-231 cancer cell-bearing hematopoietic stem cell-transplanted NOG mice given hMAb1A-ADC, hMAb1B-ADC, or human IgG1LALA-ADC (n = 4 or 5).

[Figure 25] Figure 25 is a diagram showing the ADCC activity of RG-6292 against iTreg as target cells.

[Figure 26] Figure 26 is a diagram showing the comparison of cytocidal activity against iTreg between hMAb1A-ADC and RG-6292 at varying ratios of iTreg and NK cells (n = 3).

[Figure 27] Figure 27 is a diagram showing change in the number of GZMB-positive and CD8-positive T cells per mg of tumor tissue in tumor tissues of MDA-MB-231 cancer cell-bearing hematopoietic stem cell-transplanted NOG mice given hMAb1A-ADC, ADCT-301, or ABS (n = 5).

[Figure 28] Figure 28 shows a hMAb1A-L light chain full-length amino acid sequence (amino acid sequence of a signal sequence and a hMAb1A light chain) (SEQ ID NO: 13).

[Figure 29] Figure 29 shows a hMAb1A-H heavy chain full-length amino acid sequence (amino acid sequence of a signal sequence and a hMAb1A heavy chain) (SEQ ID NO: 15).

[Figure 30] Figure 30 shows a hMAb1B-L light chain full-length amino acid sequence (amino acid sequence of a signal sequence and a hMAb1B light chain) (SEQ ID NO: 14).

[Figure 31] Figure 31 shows a hMAb1B-H heavy chain full-length amino acid sequence (amino acid sequence of a signal sequence and a hMAb1B heavy chain) (SEQ ID NO: 16).

[Figure 32] Figure 32 is a diagram showing change in tumor volume in cancer-bearing mice given cMAb2_hIgG1-ADC in combination with an anti-PD-1 antibody or a rat IgG2a control antibody. Results obtained by combining human IgG1-ADC with the anti-PD-1 antibody or the rat IgG2a control antibody are also shown. The error bar in the diagram depicts standard error (n = 3 to 10). The evaluation was conducted using animal models into which CT26.WT cells, EMT6 cells, MC38 cells, RENCA cells, or B16F10 cells were transplanted.

[Figure 33] Figure 33 is a diagram showing change in tumor volume in 3LL cancer cell-bearing mice given cMAb2_hIgG1-ADC in combination with an anti-PD-1 antibody or a rat IgG2a control antibody. Results obtained by combining human IgG1-ADC with the anti-PD-1 antibody or the rat IgG2a control antibody are also shown. The error bar in the diagram depicts standard error (n = 8).

[Figure 34] Figure 34 is a diagram showing change in tumor volume in MC38 cancer cell-bearing mice given cMAb2_hIgG1-ADC in combination with an anti-CTLA-4 antibody or a rat IgG2b control antibody. Results obtained by combining human IgG1-ADC with the anti-CTLA-4 antibody or the rat IgG2b control antibody are also shown. The error bar in the diagram depicts standard error (n = 7 or 8).

Description of Embodiments

Hereinafter, preferable modes for carrying out the present invention will be described. The embodiments described below are given merely for illustrating one example of a typical embodiment of the present invention and are not intended to limit the scope of the present invention.

In the present specification, the term "cancer" is used to have the same meaning as that of the term "tumor".

In the present specification, the term "gene" is used to include not only DNA but also its mRNA and cDNA, and cRNA thereof.

In the present specification, the term "polynucleotide" or "nucleotide" is used to have the same meaning as that of a nucleic acid, and also includes DNA, RNA, a probe, an oligonucleotide, and a primer. In the present specification, the terms "polynucleotide" and "nucleotide" can be used interchangeably with each other unless otherwise specified.

In the present specification, the terms "polypeptide" and "protein" can be used interchangeably with each other.

In the present specification, the term "cell" includes cells in an individual animal, and cultured cells.

In the present specification, the term "CD25" can be used to have the same meaning as that of CD25 protein. In the present specification, human CD25 is also referred to as "hCD25".

In the present specification, the term "cytotoxic activity" means that a pathologic change is caused to cells in any given way. The term not only means a direct trauma, but also means all types of structural or functional damage caused to cells, such as DNA cleavage, formation of a base dimer, chromosomal cleavage, damage to cell mitotic apparatus, and a reduction in the activities of various types of enzymes.

In the present specification, the phrase "exerting toxicity in cells" means that toxicity is exhibited in cells in any given way. The term not only means a direct trauma, but also means all types of structural, functional, or metabolic influences caused to cells, such as DNA cleavage, formation of a base dimer, chromosomal cleavage, damage to cell mitotic apparatus, a reduction in the activities of various types of enzymes, and suppression of effects of cell growth factors.

In the present specification, the term "antibody" may be an antibody derived from any species. Preferable examples thereof can include human-, monkey-, rat-, mouse-, rabbit-, camel-, alpaca-, and llama-derived antibodies. An antibody derived from a non-human species is preferably chimerized or humanized by use of a well-known technique. Thus, in one aspect of the present invention, the antibody is a human antibody, a chimeric antibody, or a humanized antibody, preferably a human antibody or a humanized antibody. The antibody of the present invention may be a polyclonal antibody or a monoclonal antibody and is preferably a monoclonal antibody. The antibody of the present invention may be a monospecific antibody or a multispecific antibody (bispecific antibody, trispecific antibody, etc.).

In the present specification, the term "antigen binding fragment of the antibody" means a fusion polypeptide containing a partial fragment of the antibody having binding activity against an antigen or an antigen binding site (light chain and heavy chain variable regions or CDRs contained in the variable regions, etc.) of the antibody. The antigen binding fragment of the antibody includes, for example, Fab, F(ab')2, Fv, scFv, a VHH (variable domain of heavy chain of heavy-chain antibody) antibody, diabody, a linear antibody, and a fusion protein of scFv linked to a functional domain of another protein and thereby provided with antigen binding activity. The antigen binding fragment of the antibody also encompasses a multispecific antibody formed so as to include the fragment. The antigen binding fragment of the antibody also includes Fab', which is a monovalent fragment of antibody variable regions obtained by treating F(ab')2 under reducing conditions. However, the antigen binding fragment of the antibody is not limited to these molecules as long as the antigen binding fragment has the ability to bind to an antigen. These antigen binding fragments include not only those obtained by treating a full-length molecule of the antibody protein with an appropriate enzyme, but proteins produced in appropriate host cells using a gene encoding a genetically engineered antibody or an antigen binding site thereof.

In the present specification, the term "epitope" means the partial peptide or partial three-dimensional structure of CD25, to which a particular anti-CD25 antibody binds. Such an epitope, which is the above-described partial peptide of CD25, can be determined by a method well known to those skilled in the art, such as an immunoassay. First, various partial structures of an antigen are produced. With regards to the production of such partial structures, a known oligopeptide synthesis technique can be applied. For example, a series of polypeptides, in which CD25 has been successively truncated at an appropriate length from the C-terminus or N-terminus thereof, are produced by a genetic recombination technique well known to those skilled in the art. Thereafter, the reactivity of an antibody to such polypeptides is studied, and recognition sites are roughly determined. Thereafter, further shorter peptides are synthesized, and the reactivity thereof to these peptides can then be studied, so as to determine an epitope. When an antibody binding to a membrane protein having a plurality of extracellular domains is directed to a three-dimensional structure composed of a plurality of domains as an epitope, the domain to which the antibody binds can be determined by modifying the amino acid sequence of a particular extracellular domain, and thereby modifying the three-dimensional structure. The epitope, which is a partial three-dimensional structure of an antigen that binds to a particular antibody, can also be determined by specifying the amino acid residues of an antigen adjacent to the antibody by X-ray structural analysis or the like.

In the present specification, the phrase "antibodies binding to the same epitope" means antibodies that bind to a common epitope. If a second antibody binds to a partial peptide or a partial three-dimensional structure to which a first antibody binds, the first antibody and the second antibody can be determined to bind to the same epitope. Alternatively, by confirming that a second antibody competes with a first antibody for the binding of the first antibody to an antigen (i.e., a second antibody interferes with the binding of a first antibody to an antigen), the first antibody and the second antibody can be determined to bind to the same epitope, even if the specific sequence or structure of the epitope has not been determined. In the present specification, the phrase "binding to the same epitope" refers to the case where the first antibody and the second antibody are determined to bind to a common epitope by any one or both of these determination methods. When a first antibody and a second antibody bind to the same epitope and further, the first antibody has special effects such as antitumor activity or internalization activity, the second antibody can be expected to have the same activity as that of the first antibody.

In the present specification, the term "CDR" means a complementarity determining region. It is known that the heavy chain and light chain of an antibody molecule each have three CDRs. Such CDR is also referred to as a hypervariable region, and is located in the variable regions of the heavy chain and light chain of an antibody. These regions have a primary structure with particularly high variability and are separated into three sites on the primary structure of the polypeptide chain in each of the heavy chain and light chain. In the present specification, with regard to the CDR of an antibody, the CDRs of a heavy chain are referred to as CDRH1, CDRH2 and CDRH3, respectively, from the amino-terminal side of the amino acid sequence of the heavy chain, whereas the CDRs of a light chain are referred to as CDRL1, CDRL2 and CDRL3, respectively, from the amino-terminal side of the amino acid sequence of the light chain. These sites are located close to each other on the three-dimensional structure and determine the specificity of the antibody to an antigen to which the antibody binds. The CDR sequences are defined as well known in the art, for example, defined in accordance with the schemes of IMGT (Lefranc et al., 2003, Dev Comparat Immunol 27: 55-77), Kabat (Kabat et al., 1991, Sequences of Proteins of Immunological Interest, 5th Ed. Public Health Service, National Institutes of Health, Bethesda, Md.) and Chothia (Al-Lazikani et al., 1997, J. Mol. Biol 273: 927-948). In the present invention, the CDR sequences were determined by the definition of IMGT.

In the present specification, the term "several" means 1 to 10, 1 to 9, 1 to 8, 1 to 7, 1 to 6, 1 to 5, 1 to 4, 1 to 3, or 1 or 2.

In the present specification, the term "A to B" refers to a region including A and B.

1. CD25

CD25 (also called IL-2 receptor α chain or Tac antigen) is a type I transmembrane protein extracellularly having two Sushi domains (Non Patent Literatures 13 and 14) and is known as IL-2 receptor α chain. CD25 plays a role in transducing IL-2 signals into cells upon binding to IL-2 (Non Patent Literature 15). It has been reported that CD25 is expressed at a low level in limited types of immunocytes such as activated T cells or B cells (Non Patent Literatures 16 and 17) and is highly expressed in Treg (Non Patent Literature 18). The immunosuppressive activity of Treg has been found to correlate with strong or weak CD25 expression (Non Patent Literature 18). For example, IL-2 signals essential for the survival of Treg (Non Patent Literature 19) and binding to IL-2 and consumption of IL-2 resulting in decreased chance of IL-2 to bind to other immunocytes have been reported as roles of CD25 in Treg (Non Patent Literature 19). Also, CD25 expression in blood cancer cells such as non-Hodgkin's lymphoma has been reported (Non Patent Literature 20).

The CD25 protein used in the present invention can be used by direct purification from CD25-expressing cells of a human or a non-human mammal (rat, mouse, monkey, etc.), or a cell membrane faction of the cells can be prepared and used. Alternatively, the CD25 protein can be obtained by *in vitro* synthesis or by allowing host cells to produce CD25 by genetic manipulation. In the genetic manipulation, specifically, the CD25 protein can be obtained by incorporating CD25

cDNA into a vector capable of expressing the cDNA, and then synthesizing CD25 in a solution containing an enzyme, a substrate, and an energetic material necessary for transcription and translation, or by transforming the host cells of other prokaryotes or eukaryotes so as to express CD25. Also, the CD25-expressing cells based on the genetic manipulation or a cell line expressing CD25 may be used as the CD25 protein. Alternatively, the expression vector containing incorporated CD25 cDNA is administered directly to an animal to be immunized so that CD25 can be expressed in the body of the animal thus immunized.

CD25 also includes a protein that consists of an amino acid sequence derived from the amino acid sequence of the CD25 described above by the substitution, deletion, and/or addition of one or several amino acids and has biological activity equivalent to that of the protein.

The human CD25 protein has the amino acid sequence represented by SEQ ID NO: 17.

2. Anti-CD25 antibody

One example of the anti-CD25 antibody of the present invention can include an anti-CD25 antibody that has no ability to block IL-2 and has internalization activity.

The anti-CD25 antibody of the present invention may be derived from any species. Preferable examples thereof can include human-, monkey-, rat-, mouse-, or rabbit-derived antibodies. An antibody derived from a non-human species is preferably chimerized or humanized by use of a well-known technique. The antibody of the present invention may be a polyclonal antibody or a monoclonal antibody and is preferably a monoclonal antibody.

The anti-CD25 antibody of the present invention is an antibody that can target blood cells and tumor cells. Specifically, the anti-CD25 antibody of the present invention has a characteristic of being capable of recognizing blood cells and tumor cells, a characteristic of being capable of binding to blood cells and cancer cells, and/or a characteristic of being taken up by blood cells and tumor cells for internalization. Thus, the anti-CD25 antibody of the present invention can be conjugated to a drug via a linker to prepare an antibody-drug conjugate.

The binding activity of the antibody against blood cells and tumor cells can be confirmed using flow cytometry.

The internalization of the antibody into blood cells and tumor cells can be confirmed using (1) an assay of visualizing an antibody incorporated in cells under a fluorescence microscope by using a secondary antibody (fluorescently labeled) binding to the therapeutic antibody (Cell Death and Differentiation (2008) 15, 751-761), (2) an assay of measuring a fluorescence intensity incorporated in cells using a secondary antibody (fluorescently labeled) binding to the therapeutic antibody (Molecular Biology of the Cell, Vol. 15, 5268-5282, December 2004), or (3) a Mab-ZAP assay using an immunotoxin binding to the therapeutic antibody wherein the toxin is released upon incorporation into cells to inhibit cell growth (Bio Techniques 28: 162-165, January 2000). A recombinant complex protein of a diphtheria toxin catalytic domain and protein G may be used as the immunotoxin.

In the present specification, the phrases "have no ability to block IL-2" and "not inhibit IL-2 signals" mean that IL-2 signal transduction in the presence of the anti-CD25 antibody or an antigen binding fragment thereof is maintained at at least 70% or more as compared with IL-2 signal transduction in the presence of a control antibody.

The IL-2 signal transduction can be evaluated, for example, by using IL-2-dependent T cell proliferation or protein (e.g., STAT5) phosphorylation as an index. When IL-2-dependent T cell proliferation or protein phosphorylation in the presence of IL-2 and an antibody is at least 70% or more, preferably 80% or more, more preferably 90% or more, of cell proliferation or protein phosphorylation in the presence of IL-2 and a control antibody, the antibody can be determined to "have no ability to block IL-2" and "not inhibit IL-2 signals" (IL-2 non-blocking antibody). The control antibody used can be an antibody known in the art to have no ability to block IL-2, and an antibody used in evaluation in the present Examples can be used. For example, rat IgG2a or human IgG1 may be used. Depending on a measurement system, IL-2-dependent T cell proliferation or protein phosphorylation in the presence of IL-2 and an antibody may exceed 100% of cell proliferation or protein phosphorylation in the presence of IL-2 and a control antibody. In this case as well, the antibody can be determined to be an IL-2 non-blocking antibody.

It has been reported that an anti-CD25 antibody that inhibits IL-2 signals inhibits IL-2-dependent T cell proliferation (Non Patent Literature 21) or suppresses immune response mediated by T cells (Non Patent Literature 22).

It has been reported that the therapeutic effect of an anti-CD25 antibody on tumor is based on both of the removal of Treg from the tumor and the activation of Teff in the tumor, for example, because an anti-CD25 antibody that inhibits IL-2 signals exhibits few antitumor effects in mouse tumor models in which an anti-CD25 antibody that does not inhibit IL-2 signals exhibits a high antitumor effect (Non Patent Literature 22). Hence, such an anti-CD25 antibody that inhibits IL-2 signals presumably inhibits IL-2-dependent Teff proliferation in tumor and produces no sufficient therapeutic effect.

In this respect, the antibody of the present application serving as an antibody that does not inhibit IL-2 signals is expected to exert high antitumor activity without inhibiting Teff proliferation, and considered to have superiority as a therapeutic drug.

In the present specification, the phrase "exhibit the ability to remove human regulatory T cells" means, for example, in the presence of an anti-CD25 antibody or an antigen binding fragment thereof conjugated with the cytotoxic

active compound according to the present invention as compared with in the presence of a non-administration group, a vehicle administration group, or ADC of a control antibody, the ratio of human regulatory T cells to human CD45 cells in the tumor environment, in the lymph node, and/or in blood, or the number of human regulatory T cells per tissue weight can be decreased by inducing the cell death of the human regulatory T cells.

In the present specification, the phrase "exhibit the ability to promote the proliferation of human granzyme-positive and CD8-positive cells" means, for example, in the presence of an anti-CD25 antibody or an antigen binding fragment thereof conjugated with the cytotoxic active compound according to the present invention as compared with in the presence of a non-administration group, a vehicle administration group, or ADC of a control antibody, the ratio of human granzyme-positive and CD8-positive cells to human CD45 cells in the tumor environment, in the lymph node, and/or in blood, or the number of human granzyme-positive and CD8-positive cells per tissue weight can be increased.

For example, a vehicle or ADC of a control antibody used in evaluation in the present Examples can be used for comparison.

In the present specification, the "activity of being internalized in CD25-expressing cells by binding to CD25" can be evaluated, for example, by contacting CD25-expressing cells with a control antibody or a test antibody as a primary antibody and with an antibody that has binding activity against the primary antibody and is conjugated with a cytotoxic material (e.g., saporin) as a secondary antibody, and detecting the cell death of the CD25-expressing cells induced by the cytotoxic material. Specifically, the cell survival rate of the CD25-expressing cells contacted with the control antibody as a primary antibody is defined as 100%. When the cell survival rate of the CD25-expressing cells contacted with the test antibody as a primary antibody is 90% or less, 80% or less, or 70% or less, more preferably 60% or less, 50% or less, or 40% or less, further preferably 30% or less, 20% or less, or 10% or less, the test antibody can be determined to have the activity of being internalized in CD25-expressing cells by binding to CD25. The control antibody used can be an antibody having no binding activity against CD25, and an antibody used in evaluation in the present Examples can be used. For example, rat IgG2a may be used. The "activity of being internalized" may be used interchangeably with "internalization activity" and "internalization effect".

Since the compound conjugated in the antibody-drug conjugate exerts a cytotoxic effect, it is preferred but not essential that the antibody itself should have antibody-dependent cellular cytotoxic activity, antibody-dependent cell-mediated phagocytic activity, and complement-dependent cytotoxic activity. For the purpose of specifically and selectively exerting the cytotoxic activity of the cytotoxic active compound against blood cells and tumor cells, it is important and also preferred that the antibody should have the property of being internalized to migrate into blood cells and into tumor cells.

The anti-CD25 antibody can be obtained by use of a method usually carried out in the art, which involves immunizing animals with an antigenic polypeptide and collecting and purifying antibodies produced *in vivo.* Preferably, CD25 that retains a conformation is preferably used as the antigen.

The origin of the antigen is not limited to humans, and the animals may be immunized with an antigen derived from a non-human animal such as a mouse or a rat. In this case, the cross-reactivity of antibodies binding to the obtained heterologous antigen with human antigens can be tested to screen for an antibody applicable to a human disease.

Antibody-producing cells which produce antibodies against the antigen are fused with myeloma cells in accordance with a method known in the art (e.g., Kohler and Milstein, Nature (1975) 256, p. 495-497; and Kennet, R. ed., Monoclonal Antibodies, p. 365-367, Plenum Press, N.Y. (1980)) to establish hybridomas, from which, in turn, monoclonal antibodies can be obtained.

Hereinafter, the method for obtaining the antibody against CD25 will be specifically described.

(1) Preparation of antigen

The antigen can be obtained by allowing host cells to produce a gene encoding the antigenic protein according to genetic manipulation. Specifically, a vector that permits expression of the antigen gene is prepared, and this vector is transferred to host cells so that the gene can be expressed therein, followed by the purification of the expressed antigen. The antibody can also be obtained by use of a method of immunizing an animal with these genetically manipulated antigen-expressing cells or a cell line expressing the antigen.

cDNA of the antigenic protein may be incorporated into an expression vector without the use of the antigenic protein, and the expression vector is administered to an animal to be immunized so that the antigenic protein can be expressed *in vivo* in the immunized animal to produce an antibody against the antigenic protein. In this way as well, the antibody can be obtained.

(2) Production of anti-CD25 monoclonal antibody

The anti-CD25 antibody or the antigen binding fragment of the antibody used in the present invention is not particularly limited, and, for example, an antibody defined by an amino acid sequence shown in the sequence listing of the present application can be preferably used. The anti-CD25 antibody or the antigen binding fragment of the antibody used in

the present invention desirably has the following characteristics.

(1) An antibody or an antigen binding fragment of the antibody (preferably an antibody) having any one or more of the following characteristics:

(a) specifically binding to CD25,
(b) having no ability to block IL-2,
(c) exhibiting the ability to remove human regulatory T cells and/or the ability to promote the proliferation of human granzyme-positive CD8-positive cells by conjugation to a cytotoxic active compound, and
(d) having activity of being internalized in CD25-expressing cells by binding to CD25

(among the characteristics (a) to (d), preferably (a), (b), (c), or (d), more preferably (a) and (b), (a) and (c), (a) and (d), (b) and (c), (b) and (d), or (c) and (d), still more preferably (a), (b), and (c), (a), (b), and (d), (a), (c), and (d), or (b), (c), and (d), most preferably (a), (b), (c), and (d)).
(2) The antibody according to (1), wherein the CD25 is human CD25, cynomolgus monkey CD25, or mouse CD25 (preferably human CD25 or mouse CD25, more preferably human CD25).

The method for obtaining the antibody against CD25 of the present invention is not particularly limited as long as the anti-CD25 antibody can be obtained. CD25 that retains a higher-order structure is preferably used as the antigen.
The anti-CD25 antibody or the antigen binding fragment of the antibody used in the present invention is preferably an antibody or an antigen binding fragment of the antibody having competitive inhibitory activity, for binding to CD25, against at least any one antibody selected from the group consisting of the following antibodies (1) to (6):

(1) an antibody comprising a light chain consisting of an amino acid sequence from positions 21 to 232 of SEQ ID NO: 26 and a heavy chain consisting of an amino acid sequence from positions 20 to 476 of SEQ ID NO: 27,
(2) an antibody comprising a light chain consisting of an amino acid sequence from positions 21 to 233 of SEQ ID NO: 13 and a heavy chain consisting of an amino acid sequence from positions 20 to 476 of SEQ ID NO: 15,
(3) an antibody comprising a light chain consisting of an amino acid sequence from positions 21 to 233 of SEQ ID NO: 14 and a heavy chain consisting of an amino acid sequence from positions 20 to 476 of SEQ ID NO: 16,
(4) an antibody comprising a light chain consisting of an amino acid sequence from positions 21 to 232 of SEQ ID NO: 30 and a heavy chain consisting of an amino acid sequence from positions 20 to 463 of SEQ ID NO: 31,
(5) an antibody comprising a light chain consisting of an amino acid sequence from positions 21 to 239 of SEQ ID NO: 32 and a heavy chain consisting of an amino acid sequence from positions 20 to 463 of SEQ ID NO: 33, and
(6) an antibody comprising a light chain consisting of an amino acid sequence from positions 21 to 239 of SEQ ID NO: 54 and a heavy chain consisting of an amino acid sequence from positions 20 to 463 of SEQ ID NO: 55,

more preferably an antibody or an antigen binding fragment of the antibody having competitive inhibitory activity, for binding to CD25, against at least any one antibody selected from the group consisting of the following antibodies (1) to (3):

(1) an antibody comprising a light chain consisting of an amino acid sequence from positions 21 to 232 of SEQ ID NO: 26 and a heavy chain consisting of an amino acid sequence from positions 20 to 476 of SEQ ID NO: 27,
(2) an antibody comprising a light chain consisting of an amino acid sequence from positions 21 to 233 of SEQ ID NO: 13 and a heavy chain consisting of an amino acid sequence from positions 20 to 476 of SEQ ID NO: 15, and
(3) an antibody comprising a light chain consisting of an amino acid sequence from positions 21 to 233 of SEQ ID NO: 14 and a heavy chain consisting of an amino acid sequence from positions 20 to 476 of SEQ ID NO: 16,

still more preferably an antibody or an antigen binding fragment of the antibody having competitive inhibitory activity, for binding to CD25, against at least any one of the following antibodies (1) and (2):

(1) an antibody comprising a light chain consisting of an amino acid sequence from positions 21 to 233 of SEQ ID NO: 13 and a heavy chain consisting of an amino acid sequence from positions 20 to 476 of SEQ ID NO: 15, and
(2) an antibody comprising a light chain consisting of an amino acid sequence from positions 21 to 233 of SEQ ID NO: 14 and a heavy chain consisting of an amino acid sequence from positions 20 to 476 of SEQ ID NO: 16.

The anti-CD25 antibody or the antigen binding fragment of the antibody used in the present invention is preferably an antibody comprising

CDRL1, CDRL2, and CDRL3 in any one combination selected from the group consisting of the following combinations

(1) and (2):

(1) CDRL1 consisting of the amino acid sequence represented by SEQ ID NO: 1, CDRL2 consisting of the amino acid sequence represented by SEQ ID NO: 2 (KAS), and CDRL3 consisting of the amino acid sequence represented by SEQ ID NO: 3, and
(2) CDRL1 consisting of the amino acid sequence represented by SEQ ID NO: 4, CDRL2 consisting of the amino acid sequence represented by SEQ ID NO: 5 (FVS), and CDRL3 consisting of the amino acid sequence represented by SEQ ID NO: 6, and

CDRH1, CDRH2, and CDRH3 in any one combination selected from the group consisting of the following combinations (3) and (4):

(3) CDRH1 consisting of the amino acid sequence represented by SEQ ID NO: 7, CDRH2 consisting of the amino acid sequence represented by SEQ ID NO: 8, and CDRH3 consisting of the amino acid sequence represented by SEQ ID NO: 9, and
(4) CDRH1 consisting of the amino acid sequence represented by SEQ ID NO: 10, CDRH2 consisting of the amino acid sequence represented by SEQ ID NO: 11, and CDRH3 consisting of the amino acid sequence represented by SEQ ID NO: 12,

or an antigen binding fragment of the antibody (preferably an antibody),
more preferably an antibody comprising
CDRL1, CDRL2, and CDRL3, and CDRH1, CDRH2, and CDRH3 in any one combination selected from the group consisting of the following combinations (1) and (2):

(1) CDRL1 consisting of the amino acid sequence represented by SEQ ID NO: 1, CDRL2 consisting of the amino acid sequence represented by SEQ ID NO: 2 (KAS), and CDRL3 consisting of the amino acid sequence represented by SEQ ID NO: 3, and CDRH1 consisting of the amino acid sequence represented by SEQ ID NO: 7, CDRH2 consisting of the amino acid sequence represented by SEQ ID NO: 8, and CDRH3 consisting of the amino acid sequence represented by SEQ ID NO: 9, and
(2) CDRL1 consisting of the amino acid sequence represented by SEQ ID NO: 4, CDRL2 consisting of the amino acid sequence represented by SEQ ID NO: 5 (FVS), and CDRL3 consisting of the amino acid sequence represented by SEQ ID NO: 6, and CDRH1 consisting of the amino acid sequence represented by SEQ ID NO: 10, CDRH2 consisting of the amino acid sequence represented by SEQ ID NO: 11, and CDRH3 consisting of the amino acid sequence represented by SEQ ID NO: 12.

or an antigen binding fragment of the antibody (preferably an antibody),
still more preferably antibody comprising CDRL1 consisting of the amino acid sequence represented by SEQ ID NO: 1, CDRL2 consisting of the amino acid sequence represented by SEQ ID NO: 2 (KAS), and CDRL3 consisting of the amino acid sequence represented by SEQ ID NO: 3, and CDRH1 consisting of the amino acid sequence represented by SEQ ID NO: 7, CDRH2 consisting of the amino acid sequence represented by SEQ ID NO: 8, and CDRH3 consisting of the amino acid sequence represented by SEQ ID NO: 9, or an antigen binding fragment of the antibody (preferably an antibody).
The anti-CD25 antibody or the antigen binding fragment of the antibody used in the present invention is preferably an antibody comprising

any one light chain variable region selected from the group consisting of the following light chain variable regions (1) to (4):

(1) a light chain variable region consisting of an amino acid sequence from positions 21 to 127 of SEQ ID NO: 13,
(2) a light chain variable region consisting of an amino acid sequence from positions 21 to 127 of SEQ ID NO: 14,
(3) a light chain variable region consisting of an amino acid sequence having at least 95% or higher sequence identity to the sequences of framework regions except for CDR sequences in each of the amino acid sequences of (1) and (2), and
(4) a light chain variable region consisting of an amino acid sequence derived by the deletion, substitution, or addition of one or several amino acids from the sequences of framework regions except for CDR sequences in each of the amino acid sequences of (1) and (2), and

any one heavy chain variable region selected from the group consisting of the following heavy chain variable regions (5) to (8):

(5) a heavy chain variable region consisting of an amino acid sequence from positions 20 to 146 of SEQ ID NO: 15,
(6) a heavy chain variable region consisting of an amino acid sequence from positions 20 to 146 of SEQ ID NO: 16,
(7) a heavy chain variable region consisting of an amino acid sequence having at least 95% or higher sequence homology to the sequences of framework regions except for CDR sequences in each of the amino acid sequences of (5) and (6), and
(8) a heavy chain variable region consisting of an amino acid sequence derived by the deletion, substitution, or addition of one or several amino acids from the sequences of framework regions except for CDR sequences in each of the amino acid sequences of (5) and (6),

or an antigen binding fragment of the antibody (preferably an antibody),
more preferably an antibody comprising (1) a light chain variable region consisting of an amino acid sequence from positions 21 to 127 of SEQ ID NO: 13 and a heavy chain variable region consisting of an amino acid sequence from positions 20 to 146 of SEQ ID NO: 15, or (2) a light chain variable region consisting of an amino acid sequence from positions 21 to 127 of SEQ ID NO: 14 and a heavy chain variable region consisting of an amino acid sequence from positions 20 to 146 of SEQ ID NO: 16, or an antigen binding fragment of the antibody (preferably an antibody).

The anti-CD25 antibody or the antigen binding fragment of the antibody used in the present invention is preferably an antibody comprising any of the following combinations (1) and (2): (1) a light chain consisting of an amino acid sequence from positions 21 to 233 of SEQ ID NO: 13 and a heavy chain consisting of an amino acid sequence from positions 20 to 476 of SEQ ID NO: 15, and (2) a light chain consisting of an amino acid sequence from positions 21 to 233 of SEQ ID NO: 14 and a heavy chain consisting of an amino acid sequence from positions 20 to 476 of SEQ ID NO: 16, or an antigen binding fragment of the antibody (preferably an antibody), more preferably an antibody or an antigen binding fragment of the antibody (preferably an antibody) in which the heavy chain or the light chain has one or two or more modifications selected from the group consisting of N-linked glycosylation, O-linked glycosylation, N-terminal processing, C-terminal processing, deamidation, isomerization of aspartic acid, oxidation of methionine, addition of a methionine residue to the N terminus, amidation of a proline residue, conversion of N-terminal glutamine or N-terminal glutamic acid to pyroglutamic acid, and deletion of one or two amino acids from the carboxyl terminus,

still more preferably an antibody in which one or two amino acids are deleted from the carboxyl terminus of the heavy chain,
still more preferably an antibody in which one amino acid is deleted from each of the carboxyl termini of both of two such heavy chains,
still more preferably an antibody in which the deleted amino acid in the heavy chains is lysine.

In an embodiment, the anti-CD25 antibody or the antigen binding fragment of the antibody used in the present invention is an anti-CD25 antibody or an antigen binding fragment of the antibody (preferably an antibody) having at least one or more of the following characteristics (a) to (d):

(a) specifically binding to CD25,
(b) having no ability to block IL-2,
(c) exhibiting the ability to remove human regulatory T cells and/or the ability to promote the proliferation of human granzyme-positive CD8-positive cells by conjugation to a cytotoxic active compound, and
(d) having activity of being internalized in CD25-expressing cells by binding to CD25

(among the characteristics (a) to (d), preferably (a), (b), (c), or (d), more preferably (a) and (b), (a) and (c), (a) and (d), (b) and (c), (b) and (d), or (c) and (d), still more preferably (a), (b), and (c), (a), (b), and (d), (a), (c), and (d), or (b), (c), and (d), most preferably (a), (b), (c), and (d)), wherein

the CD25 is human CD25, and
the antibody or the antigen binding fragment of the antibody is an antibody comprising CDRL1 consisting of the amino acid sequence represented by SEQ ID NO: 1, CDRL2 consisting of the amino acid sequence represented by SEQ ID NO: 2 (KAS), and CDRL3 consisting of the amino acid sequence represented by SEQ ID NO: 3, and CDRH1 consisting of the amino acid sequence represented by SEQ ID NO: 7, CDRH2 consisting of the amino acid sequence represented by SEQ ID NO: 8, and CDRH3 consisting of the amino acid sequence represented by SEQ ID NO: 9, or an antigen binding fragment of the antibody.

In an alternative embodiment, the anti-CD25 antibody or the antigen binding fragment of the antibody used in the present invention is an anti-CD25 antibody or an antigen binding fragment of the antibody (preferably an antibody) having

at least one or more of the following characteristics (a) to (d):

(a) specifically binding to CD25,
(b) having no ability to block IL-2,
(c) exhibiting the ability to remove human regulatory T cells and/or the ability to promote the proliferation of human granzyme-positive CD8-positive cells by conjugation to a cytotoxic active compound, and
(d) having activity of being internalized in CD25-expressing cells by binding to CD25

(among the characteristics (a) to (d), preferably (a), (b), (c), or (d), more preferably (a) and (b), (a) and (c), (a) and (d), (b) and (c), (b) and (d), or (c) and (d), still more preferably (a), (b), and (c), (a), (b), and (d), (a), (c), and (d), or (b), (c), and (d), most preferably (a), (b), (c), and (d)), wherein

the CD25 is human CD25, and
the antibody or the antigen binding fragment of the antibody is an antibody comprising a light chain variable region consisting of an amino acid sequence from positions 21 to 127 of SEQ ID NO: 13 and a heavy chain variable region consisting of an amino acid sequence from positions 20 to 146 of SEQ ID NO: 15, or an antigen binding fragment of the antibody.

In an alternative embodiment, the anti-CD25 antibody or the antigen binding fragment of the antibody used in the present invention is an anti-CD25 antibody or an antigen binding fragment of the antibody (preferably an antibody) having at least one or more of the following characteristics (a) to (d):

(a) specifically binding to CD25,
(b) having no ability to block IL-2,
(c) exhibiting the ability to remove human regulatory T cells and/or the ability to promote the proliferation of human granzyme-positive CD8-positive cells by conjugation to a cytotoxic active compound, and
(d) having activity of being internalized in CD25-expressing cells by binding to CD25

(among the characteristics (a) to (d), preferably (a), (b), (c), or (d), more preferably (a) and (b), (a) and (c), (a) and (d), (b) and (c), (b) and (d), or (c) and (d), still more preferably (a), (b), and (c), (a), (b), and (d), (a), (c), and (d), or (b), (c), and (d), most preferably (a), (b), (c), and (d)), wherein

the CD25 is human CD25, and
the antibody or the antigen binding fragment of the antibody is an antibody comprising a light chain variable region consisting of an amino acid sequence from positions 21 to 127 of SEQ ID NO: 14 and a heavy chain variable region consisting of an amino acid sequence from positions 20 to 146 of SEQ ID NO: 16, or an antigen binding fragment of the antibody.

In an alternative embodiment, the anti-CD25 antibody or the antigen binding fragment of the antibody used in the present invention is an anti-CD25 antibody or an antigen binding fragment of the antibody (preferably an antibody) having at least one or more of the following characteristics (a) to (d):

(a) specifically binding to CD25,
(b) having no ability to block IL-2,
(c) exhibiting the ability to remove human regulatory T cells and/or the ability to promote the proliferation of human granzyme-positive CD8-positive cells by conjugation to a cytotoxic active compound, and
(d) having activity of being internalized in CD25-expressing cells by binding to CD25

(among the characteristics (a) to (d), preferably (a), (b), (c), or (d), more preferably (a) and (b), (a) and (c), (a) and (d), (b) and (c), (b) and (d), or (c) and (d), still more preferably (a), (b), and (c), (a), (b), and (d), (a), (c), and (d), or (b), (c), and (d), most preferably (a), (b), (c), and (d)), wherein

the CD25 is human CD25, and
the antibody or the antigen binding fragment of the antibody is an antibody comprising a light chain consisting of an amino acid sequence from positions 21 to 233 of SEQ ID NO: 13 and a heavy chain consisting of an amino acid sequence from positions 20 to 476 of SEQ ID NO: 15, or an antigen binding fragment of the antibody.

In an alternative embodiment, the anti-CD25 antibody or the antigen binding fragment of the antibody used in the

present invention is an anti-CD25 antibody or an antigen binding fragment of the antibody (preferably an antibody) having at least one or more of the following characteristics (a) to (d):

(a) specifically binding to CD25,
(b) having no ability to block IL-2,
(c) exhibiting the ability to remove human regulatory T cells and/or the ability to promote the proliferation of human granzyme-positive CD8-positive cells by conjugation to a cytotoxic active compound, and
(d) having activity of being internalized in CD25-expressing cells by binding to CD25

(among the characteristics (a) to (d), preferably (a), (b), (c), or (d), more preferably (a) and (b), (a) and (c), (a) and (d), (b) and (c), (b) and (d), or (c) and (d), still more preferably (a), (b), and (c), (a), (b), and (d), (a), (c), and (d), or (b), (c), and (d), most preferably (a), (b), (c), and (d)), wherein

the CD25 is human CD25, and
the antibody or the antigen binding fragment of the antibody is an antibody comprising a light chain consisting of an amino acid sequence from positions 21 to 233 of SEQ ID NO: 14 and a heavy chain consisting of an amino acid sequence from positions 20 to 476 of SEQ ID NO: 16, or an antigen binding fragment of the antibody.

In an alternative embodiment, the anti-CD25 antibody or the antigen binding fragment of the antibody used in the present invention is an anti-CD25 antibody having at least one or more of the following characteristics (a) to (d):

(a) specifically binding to CD25,
(b) having no ability to block IL-2,
(c) exhibiting the ability to remove human regulatory T cells and/or the ability to promote the proliferation of human granzyme-positive CD8-positive cells by conjugation to a cytotoxic active compound, and
(d) having activity of being internalized in CD25-expressing cells by binding to CD25

(among the characteristics (a) to (d), preferably (a), (b), (c), or (d), more preferably (a) and (b), (a) and (c), (a) and (d), (b) and (c), (b) and (d), or (c) and (d), still more preferably (a), (b), and (c), (a), (b), and (d), (a), (c), and (d), or (b), (c), and (d), most preferably (a), (b), (c), and (d)), wherein

the CD25 is human CD25, and
the antibody is an antibody comprising a light chain consisting of an amino acid sequence from positions 21 to 233 of SEQ ID NO: 13 and a heavy chain consisting of an amino acid sequence from positions 20 to 476 of SEQ ID NO: 15, and
one or two amino acids are deleted from the carboxyl terminus of the heavy chain.

In an alternative embodiment, the anti-CD25 antibody or the antigen binding fragment of the antibody used in the present invention is an anti-CD25 antibody having at least one or more of the following characteristics (a) to (d):

(a) specifically binding to CD25,
(b) having no ability to block IL-2,
(c) exhibiting the ability to remove human regulatory T cells and/or the ability to promote the proliferation of human granzyme-positive CD8-positive cells by conjugation to a cytotoxic active compound, and
(d) having activity of being internalized in CD25-expressing cells by binding to CD25

(among the characteristics (a) to (d), preferably (a), (b), (c), or (d), more preferably (a) and (b), (a) and (c), (a) and (d), (b) and (c), (b) and (d), or (c) and (d), still more preferably (a), (b), and (c), (a), (b), and (d), (a), (c), and (d), or (b), (c), and (d), most preferably (a), (b), (c), and (d)), wherein

the CD25 is human CD25, and
the antibody is an antibody comprising a light chain consisting of an amino acid sequence from positions 21 to 233 of SEQ ID NO: 13 and a heavy chain consisting of an amino acid sequence from positions 20 to 476 of SEQ ID NO: 15, and
one amino acid is deleted from each of the carboxyl termini of both of two such heavy chains.

In an alternative embodiment, the anti-CD25 antibody or the antigen binding fragment of the antibody used in the present invention is an anti-CD25 antibody having at least one or more of the following characteristics (a) to (d):

(a) specifically binding to CD25,
(b) having no ability to block IL-2,
(c) exhibiting the ability to remove human regulatory T cells and/or the ability to promote the proliferation of human granzyme-positive CD8-positive cells by conjugation to a cytotoxic active compound, and
(d) having activity of being internalized in CD25-expressing cells by binding to CD25

(among the characteristics (a) to (d), preferably (a), (b), (c), or (d), more preferably (a) and (b), (a) and (c), (a) and (d), (b) and (c), (b) and (d), or (c) and (d), still more preferably (a), (b), and (c), (a), (b), and (d), (a), (c), and (d), or (b), (c), and (d), most preferably (a), (b), (c), and (d)), wherein
the CD25 is human CD25, and
the antibody is an antibody comprising a light chain consisting of an amino acid sequence from positions 21 to 233 of SEQ ID NO: 13 and a heavy chain consisting of an amino acid sequence from positions 20 to 476 of SEQ ID NO: 15, and one lysine is deleted from each of the carboxyl termini of both of two such heavy chains.

In an alternative embodiment, the anti-CD25 antibody or the antigen binding fragment of the antibody used in the present invention is an anti-CD25 antibody having at least one or more of the following characteristics (a) to (d):

(a) specifically binding to CD25,
(b) having no ability to block IL-2,
(c) exhibiting the ability to remove human regulatory T cells and/or the ability to promote the proliferation of human granzyme-positive CD8-positive cells by conjugation to a cytotoxic active compound, and
(d) having activity of being internalized in CD25-expressing cells by binding to CD25

(among the characteristics (a) to (d), preferably (a), (b), (c), or (d), more preferably (a) and (b), (a) and (c), (a) and (d), (b) and (c), (b) and (d), or (c) and (d), still more preferably (a), (b), and (c), (a), (b), and (d), (a), (c), and (d), or (b), (c), and (d), most preferably (a), (b), (c), and (d)), wherein

the CD25 is human CD25, and
the antibody is an antibody comprising a light chain consisting of an amino acid sequence from positions 21 to 233 of SEQ ID NO: 14 and a heavy chain consisting of an amino acid sequence from positions 20 to 476 of SEQ ID NO: 16, and
one or two amino acids are deleted from the carboxyl terminus of the heavy chain.

In an alternative embodiment, the anti-CD25 antibody or the antigen binding fragment of the antibody used in the present invention is an anti-CD25 antibody having at least one or more of the following characteristics (a) to (d):

(a) specifically binding to CD25,
(b) having no ability to block IL-2,
(c) exhibiting the ability to remove human regulatory T cells and/or the ability to promote the proliferation of human granzyme-positive CD8-positive cells by conjugation to a cytotoxic active compound, and
(d) having activity of being internalized in CD25-expressing cells by binding to CD25

(among the characteristics (a) to (d), preferably (a), (b), (c), or (d), more preferably (a) and (b), (a) and (c), (a) and (d), (b) and (c), (b) and (d), or (c) and (d), still more preferably (a), (b), and (c), (a), (b), and (d), (a), (c), and (d), or (b), (c), and (d), most preferably (a), (b), (c), and (d)), wherein

the CD25 is human CD25, and
the antibody is an antibody comprising a light chain consisting of an amino acid sequence from positions 21 to 233 of SEQ ID NO: 14 and a heavy chain consisting of an amino acid sequence from positions 20 to 476 of SEQ ID NO: 16, and
one amino acid is deleted from each of the carboxyl termini of both of two such heavy chains.

In an alternative embodiment, the anti-CD25 antibody or the antigen binding fragment of the antibody used in the present invention is an anti-CD25 antibody having at least one or more of the following characteristics (a) to (d):

(a) specifically binding to CD25,
(b) having no ability to block IL-2,
(c) exhibiting the ability to remove human regulatory T cells and/or the ability to promote the proliferation of human granzyme-positive CD8-positive cells by conjugation to a cytotoxic active compound, and

(d) having activity of being internalized in CD25-expressing cells by binding to CD25

(among the characteristics (a) to (d), preferably (a), (b), (c), or (d), more preferably (a) and (b), (a) and (c), (a) and (d), (b) and (c), (b) and (d), or (c) and (d), still more preferably (a), (b), and (c), (a), (b), and (d), (a), (c), and (d), or (b), (c), and (d), most preferably (a), (b), (c), and (d)), wherein

the CD25 is human CD25, and
the antibody is an antibody comprising a light chain consisting of an amino acid sequence from positions 21 to 233 of SEQ ID NO: 14 and a heavy chain consisting of an amino acid sequence from positions 20 to 476 of SEQ ID NO: 16, and
one lysine is deleted from each of the carboxyl termini of both of two such heavy chains.

The antibody-drug conjugate used in the present invention may comprise the anti-CD25 antibody or the antigen binding fragment according to any embodiment described above. An anti-CD25 antibody-drug conjugate in which the anti-CD25 antibody, etc. is conjugated with a drug that exerts toxicity in cells via a linker having a particular structure is capable of exhibiting stronger ability to remove Treg and/or activity of inducing granzyme (GZMB)-positive and CD8-positive T cells than that of a conventional anti-CD25 antibody and anti-CD25 antibody-drug complex.
Specific examples of how the monoclonal antibody can be obtained can include the following procedures:

(a) purification of a biopolymer for use as the antigen;
(b) induction of immune response by administering recombinant CD25 and an adjuvant directly to an animal to be immunized (e.g., a rat or a mouse) (this administration may be performed one or more times, if necessary for enhancing an antibody titer);
(c) collection of a tissue (e.g., the lymph node) containing antibody-producing cells from the aforementioned animal in which the immune response has been induced;
(d) preparation of myeloma cells (hereinafter, referred to as "myelomas") (e.g., mouse myeloma SP2/0-ag14 cells);
(e) cell fusion between the antibody-producing cells and the myelomas;
(f) selection of a hybridoma group producing the antibody of interest;
(g) division into single cell clones (cloning);
(h) optionally, the culture of hybridomas for the mass production of monoclonal antibodies, or the raising of animals into which the hybridomas are transplanted; and/or
(i) study on the physiological activity (internalization activity) and binding specificity of the monoclonal antibody thus produced, or examination of the characteristics of the antibody as a labeling reagent.

Examples of the method for measuring the antibody titer used herein can include, but are not limited to, flow cytometry and Cell-ELISA.
Examples of the hybridoma line thus established can include anti-CD25 antibody-producing hybridomas MAb1 and MAb2. In the present specification, an antibody produced by the anti-CD25 antibody-producing hybridoma MAb1 is referred to as "MAb1 antibody" or simply as "MAb1", and an antibody produced by the hybridoma MAb2 is referred to as "MAb2 antibody" or simply as "MAb2".
The light chain variable region of the MAb1 antibody consists of the amino acid sequence represented by SEQ ID NO: 18. The amino acid sequence of the light chain variable region of the MAb1 antibody is encoded by the nucleotide sequence represented by SEQ ID NO: 19. The light chain variable region of the MAb1 antibody has CDRL1 consisting of the amino acid sequence represented by SEQ ID NO: 1, CDRL2 consisting of the amino acid sequence represented by SEQ ID NO: 2, and CDRL3 consisting of the amino acid sequence represented by SEQ ID NO: 3. The heavy chain variable region of the MAb1 antibody consists of the amino acid sequence represented by SEQ ID NO: 20. The amino acid sequence of the heavy chain variable region of the MAb1 antibody is encoded by the nucleotide sequence represented by SEQ ID NO: 21. The heavy chain variable region of the MAb1 antibody has CDRH1 consisting of the amino acid sequence represented by SEQ ID NO: 7, CDRH2 consisting of the amino acid sequence represented by SEQ ID NO: 8, and CDRH3 consisting of the amino acid sequence represented by SEQ ID NO: 9. The sequence of the MAb1 antibody is shown in Table 1.

[Table 1]

| | |
|---|---|
| Light chain variable region Amino acid sequence (SEQ ID NO: 18) | DTVLTQSPALAVSPGERVTISCKASESVSTRMH<br>WYQQKPGQQPKLLIYKASNLASGVPARFSGSGS<br>GTDFTLTIDPVEADDTATYFCQQSWNGWAFGGG<br>TKLELKR |
| Light chain variable region Nucleotide sequence (SEQ ID NO: 19) | GACACTGTGCTGACCCAGTCTCCTGCTTTGGCT<br>GTGTCTCCAGGAGAGAGGGTTACCATCTCCTGT<br>AAGGCCAGTGAAAGTGTCAGTACACGTATGCAC<br>TGGTACCAACAGAAACCAGGACAGCAACCCAAA<br>CTCCTCATCTATAAAGCATCAAACCTAGCATCT<br>GGGGTCCCTGCCAGGTTCAGTGGCAGTGGGTCT<br>GGGACAGACTTCACCCTCACCATTGATCCTGTG<br>GAGGCTGATGACACTGCAACCTATTTCTGTCAG<br>CAGAGTTGGAATGGTTGGGCGTTCGGTGGAGGC<br>ACCAAGCTGGAATTGAAACGG |
| CDRL1 (SEQ ID NO: 1) | ESVSTR |
| CDRL2 (SEQ ID NO: 2) | KAS |
| CDRL3 (SEQ ID NO: 3) | QQSWNGWA |
| Heavy chain variable region Amino acid sequence (SEQ ID NO: 20) | EVQLVESGGGLVQPGRSLKLSCAASGFTFNNYY<br>MVWVRQAPTKGLEWVAYISTGGGTTYYRESVKG<br>RFTISRDNAKSTLYLQMDSLRSEDTATYYCTTS<br>VYFSDGSSHYDGEWFAYWGQGTLVTVSS |
| Heavy chain variable region Nucleotide sequence (SEQ ID NO: 21) | GAGGTGCAGCTGGTGGAATCTGGGGGAGGCTTA<br>GTGCAGCCTGGAAGGTCCCTGAAACTCTCCTGT<br>GCAGCCTCAGGATTCACTTTCAATAACTATTAC<br>ATGGTCTGGGTCCGCCAGGCTCCAACGAAGGGT<br>CTGGAGTGGGTCGCATACATTAGTACTGGTGGT<br>GGTACCACTTACTATCGAGAGTCCGTGAAGGGC<br>CGGTTCACTATCTCCAGAGATAATGCAAAAAGC<br>ACCCTATACCTGCAGATGGACAGTCTGAGGTCT<br>GAAGACACGGCCACTTATTACTGTACAACGAGT<br>GTTTATTTTTCTGATGGTAGTTCTCACTACGAT<br>GGGGAGTGGTTTGCTTACTGGGGCCAAGGCACT<br>CTGGTCACTGTCTCTTCA |
| CDRH1 (SEQ ID NO: 7) | GFTFNNYY |
| CDRH2 (SEQ ID NO: 8) | ISTGGGTT |
| CDRH3 (SEQ ID NO: 9) | TTSVYFSDGSSHYDGEWFAY |

The light chain variable region of the MAb2 antibody consists of the amino acid sequence represented by SEQ ID NO: 22. The amino acid sequence of the light chain variable region of the MAb2 antibody is encoded by the nucleotide sequence represented by SEQ ID NO: 23. The light chain variable region of the MAb2 antibody has CDRL1 consisting of

the amino acid sequence represented by SEQ ID NO: 4, CDRL2 consisting of the amino acid sequence represented by SEQ ID NO: 5, and CDRL3 consisting of the amino acid sequence represented by SEQ ID NO: 6. The heavy chain variable region of the MAb2 antibody consists of the amino acid sequence represented by SEQ ID NO: 24. The amino acid sequence of the heavy chain variable region of the MAb2 antibody is encoded by the nucleotide sequence represented by SEQ ID NO: 25. The heavy chain variable region of the MAb2 antibody has CDRH1 consisting of the amino acid sequence represented by SEQ ID NO: 10, CDRH2 consisting of the amino acid sequence represented by SEQ ID NO: 11, and CDRH3 consisting of the amino acid sequence represented by SEQ ID NO: 12. The sequence of the MAb2 antibody is shown in Table 2.

[Table 2]

| | |
|---|---|
| Light chain variable region Amino acid sequence (SEQ ID NO: 22) | DVVLTQTPPTLSATIGQSVSISCRSSQSLSHT<br>NGNTYLYWLLQRPGQPPQLLIYFVSRLESGVP<br>NRFSGSGSGTDFTLKISGVEAEDLGLYYCVQS<br>THVPNTFGAGTKLELKR |
| Light chain variable region Nucleotide sequence (SEQ ID NO: 23) | GATGTTGTGCTGACCCAGACTCCACCCACTTT<br>ATCGGCTACCATTGGACAATCAGTCTCCATCT<br>CTTGCAGGTCAAGTCAGAGTCTCTCACATACT<br>AATGGAAACACCTATTTATATTGGTTGCTACA<br>GAGGCCAGGCCAACCTCCACAACTTCTAATTT<br>ATTTCGTGTCCAGGCTTGAATCTGGGGTCCCC<br>AACAGGTTCAGTGGCAGTGGGTCAGGAACTGA<br>TTTCACACTCAAAATCAGTGGAGTAGAGGCTG<br>AGGATTTGGGACTTTATTACTGCGTGCAAAGT<br>ACCCATGTTCCGAACACGTTTGGAGCTGGGAC<br>CAAGCTGGAACTGAAACGG |
| CDRL1 (SEQ ID NO: 4) | QSLSHTNGNTY |
| CDRL2 (SEQ ID NO: 5) | FVS |
| CDRL3 (SEQ ID NO: 6) | VQSTHVPNT |
| Heavy chain variable region Amino acid sequence (SEQ ID NO: 24) | QVKLLQSGAALEKPGASVKMSCKASGYTFTDY<br>WVIWVKQSHGKSLEWIGEIYPNSGATNFNAKF<br>KGKATLTVDKSTSTAYMELSRLTSEDSAIYYC<br>TTYDFDYWGQGVMVTVSS |
| Heavy chain variable region Nucleotide sequence (SEQ ID NO: 25) | CAGGTCAAGTTGCTGCAGTCTGGGGCTGCACT<br>GGAGAAGCCTGGAGCCTCTGTGAAGATGTCTT<br>GCAAAGCTTCTGGTTATACATTCACTGACTAC<br>TGGGTGATCTGGGTGAAGCAGAGTCATGGAAA<br>GAGCCTTGAGTGGATTGGGGAAATTTATCCTA<br>ACAGTGGTGCTACTAACTTCAATGCAAAGTTC<br>AAGGGCAAGGCCACATTGACTGTAGACAAATC<br>CACCAGCACAGCCTATATGGAGCTCAGCAGAT<br>TGACATCTGAGGACTCTGCAATCTATTACTGT<br>ACAACATACGACTTTGATTATTGGGGCCAAGG<br>AGTCATGGTCACAGTCTCCTCA |

(continued)

| | |
|---|---|
| CDRH1 (SEQ ID NO: 10) | GYTFTDYW |
| CDRH2 (SEQ ID NO: 11) | IYPNSGAT |
| CDRH3 (SEQ ID NO: 12) | TTYDFDY |

Also in the case of carrying out again the steps (a) to (h) in "2. Production of anti-CD25 antibody" to independently obtain a monoclonal antibody separately and in the case of obtaining a monoclonal antibody separately by other methods, an antibody having internalization activity equivalent to that of the MAb1 antibody or the MAb2 antibody can be obtained. One example of such an antibody can include an antibody that binds to the same epitope as that for the MAb1 antibody or the MAb2 antibody. Provided that a newly prepared monoclonal antibody binds to a partial peptide or a partial conformation to which the MAb1 antibody or the MAb2 antibody binds, the monoclonal antibody can be determined to bind to the same epitope as that for the MAb1 antibody or the MAb2 antibody. Alternatively, if the specific sequence or structure of an epitope is not determined, the monoclonal antibody can be determined to bind to the same epitope as that for the anti-CD25 antibody by confirming that the monoclonal antibody competes with the MAb1 antibody or the MAb2 antibody for the binding of the MAb1 antibody or the MAb2 antibody to CD25 (i.e., the monoclonal antibody interferes with the binding of the MAb1 antibody or the MAb2 antibody to CD25). The monoclonal antibody thus confirmed to bind to the same epitope is strongly expected to have the ability to bind to the antigen, biological activity, and/or internalization activity equivalent to that of the MAb1 antibody or the MAb2 antibody.

(3) Other antibodies

The antibody of the present invention includes, in addition to the monoclonal antibody against CD25, a recombinant antibody obtained by artificial modification for the purpose of decreasing heterologous antigenicity to humans, for example, a chimeric antibody, a humanized antibody, or a human antibody. These antibodies can be produced by use of a known method.

Examples of the chimeric antibody can include an antibody in which antibody variable and constant regions are derived from different species, for example, a chimeric antibody in which a mouse- or rat-derived antibody variable region is connected to a human-derived antibody constant region (see Proc. Natl. Acad. Sci. U.S.A., 81, 6851-6855, (1984)).

Examples of the chimeric antibody derived from the rat anti-human CD25 antibody include an antibody consisting of a light chain comprising the light chain variable region of each rat anti-human CD25 antibody (e.g., the MAb1 antibody) described in the present specification and a human-derived constant region, and a heavy chain comprising the heavy chain variable region of this rat anti-human CD25 antibody and a human-derived constant region.

Other examples of the chimeric antibody derived from the rat anti-human CD25 antibody include an antibody consisting of a light chain comprising a light chain variable region having the substitution of one to several residues, 1 to 3 residues, 1 or 2 residues, preferably 1 residue, of amino acids in the light chain variable region of each rat anti-human CD25 antibody (e.g., the MAb1 antibody) described in the present specification by other amino acid residues, and a heavy chain comprising a heavy chain variable region having the substitution of one to several residues, 1 to 3 residues, 1 or 2 residues, preferably 1 residue, of amino acids in the heavy chain variable region thereof by other amino acid residues. This antibody may have an arbitrary human-derived constant region.

Other examples of the chimeric antibody derived from the rat anti-human CD25 antibody include an antibody consisting of a light chain comprising a light chain variable region having the substitution of 1 or 2 residues, preferably 1 residue, of amino acids in any one to three CDRs in the light chain variable region of each rat anti-human CD25 antibody (e.g., the MAb1 antibody) described in the present specification by other amino acid residues, and a heavy chain comprising a heavy chain variable region having the substitution of 1 or 2 residues, preferably 1 residue, of amino acids in any one to three CDRs in the heavy chain variable region thereof by other amino acid residues. This antibody may have an arbitrary human-derived constant region.

Examples of the chimeric antibody derived from the MAb1 antibody include an antibody consisting of a light chain comprising a light chain variable region consisting of an amino acid sequence from positions 21 to 126 of SEQ ID NO: 26, and a heavy chain comprising a heavy chain variable region consisting of an amino acid sequence from positions 20 to 146 of SEQ ID NO: 27. This antibody may have an arbitrary human-derived constant region.

Other examples of the chimeric antibody derived from the MAb1 antibody include an antibody consisting of a light chain comprising a light chain variable region having the substitution of one to several residues, 1 to 3 residues, 1 or 2 residues, preferably 1 residue, of amino acids in the light chain variable region consisting of an amino acid sequence from positions 21 to 126 of SEQ ID NO: 26 by other amino acid residues, and a heavy chain comprising a heavy chain variable region having the substitution of one to several residues, 1 to 3 residues, 1 or 2 residues, preferably 1 residue, of amino acids in the heavy chain variable region consisting of an amino acid sequence from positions 20 to 146 of SEQ ID NO: 27 by

other amino acid residues. This antibody may have an arbitrary human-derived constant region.

Other examples of the chimeric antibody derived from the MAb1 antibody include an antibody consisting of a light chain comprising a light chain variable region having the substitution of 1 or 2 residues (preferably 1 residue) of amino acids in any one to three CDRs in the light chain variable region consisting of an amino acid sequence from positions 21 to 126 of SEQ ID NO: 26 by other amino acid residues, and a heavy chain comprising a heavy chain variable region having the substitution of 1 or 2 residues (preferably 1 residue) of amino acids in any one to three CDRs in the heavy chain variable region consisting of an amino acid sequence from positions 20 to 146 of SEQ ID NO: 27 by other amino acid residues. This antibody may have an arbitrary human-derived constant region.

Specific examples of the chimeric antibody derived from the MAb1 antibody include an antibody consisting of a light chain consisting of an amino acid sequence from positions 21 to 232 of SEQ ID NO: 26 and a heavy chain consisting of an amino acid sequence from positions 20 to 476 of SEQ ID NO: 27. In the present specification, this chimeric anti-human CD25 antibody is referred to as "cMAb1_hIgG1LALA antibody" or "cMAb1_hIgG1LALA". The light chain amino acid sequence of the cMAb1_hIgG1LALA antibody is encoded by a nucleotide sequence from positions 61 to 696 of SEQ ID NO: 28, and the heavy chain amino acid sequence of the cMAb1_hIgG1LALA antibody is encoded by a nucleotide sequence from positions 58 to 1428 of SEQ ID NO: 29.

The light chain variable region amino acid sequence of the cMAb1_hIgG1LALA antibody is identical to the light chain variable region amino acid sequence of the MAb1 antibody and consists of the amino acid sequence represented by SEQ ID NO: 18. The light chain of the cMAb1_hIgG1LALA antibody has CDRL1 consisting of the amino acid sequence represented by SEQ ID NO: 1, CDRL2 consisting of the amino acid sequence represented by SEQ ID NO: 2, and CDRL3 consisting of the amino acid sequence represented by SEQ ID NO: 3, which are identical to CDRL1, CDRL2, and CDRL3, respectively, of the light chain of MAb1. The light chain variable region amino acids of the cMAb1_hIgG1LALA antibody are encoded by the nucleotide sequence represented by SEQ ID NO: 19.

The heavy chain variable region amino acid sequence of the cMAb1_hIgG1LALA antibody is identical to the heavy chain variable region amino acid sequence of the MAb1 antibody and consists of the amino acid sequence represented by SEQ ID NO: 20. The heavy chain of the cMAb1_hIgG1LALA antibody has CDRH1 consisting of the amino acid sequence represented by SEQ ID NO: 7, CDRH2 consisting of the amino acid sequence represented by SEQ ID NO: 8, and CDRH3 consisting of the amino acid sequence represented by SEQ ID NO: 9, which are identical to CDRH1, CDRH2, and CDRH3, respectively, of the heavy chain of MAb1. The heavy chain variable region amino acid sequence of the cMAb1_hIgG1LALA antibody is encoded by the nucleotide sequence represented by SEQ ID NO: 21. The sequence of the cMAb1_hIgG1LALA antibody is shown in Table 3.

[Table 3]

| | |
|---|---|
| Light chain variable region Amino acid sequence (SEQ ID NO: 18) | DTVLTQSPALAVSPGERVTISCKASESVSTRMH<br>WYQQKPGQQPKLLIYKASNLASGVPARFSGSGS<br>GTDFTLTIDPVEADDTATYFCQQSWNGWAFGGG<br>TKLELKR |
| Light chain variable region Nucleotide sequence (SEQ ID NO: 19) | GACACTGTGCTGACCCAGTCTCCTGCTTTGGCT<br>GTGTCTCCAGGAGAGAGGGTTACCATCTCCTGT<br>AAGGCCAGTGAAAGTGTCAGTACACGTATGCAC<br>TGGTACCAACAGAAACCAGGACAGCAACCCAAA<br>CTCCTCATCTATAAAGCATCAAACCTAGCATCT<br>GGGGTCCCTGCCAGGTTCAGTGGCAGTGGGTCT<br>GGGACAGACTTCACCCTCACCATTGATCCTGTG<br>GAGGCTGATGACACTGCAACCTATTTCTGTCAG<br>CAGAGTTGGAATGGTTGGGCGTTCGGTGGAGGC<br>ACCAAGCTGGAATTGAAACGG |
| CDRL1 (SEQ ID NO: 1) | ESVSTR |
| CDRL2 (SEQ ID NO: 2) | KAS |
| CDRL3 (SEQ ID NO: 3) | QQSWNGWA |

(continued)

| | |
|---|---|
| Heavy chain variable region Amino acid sequence (SEQ ID NO: 20) | EVQLVESGGGLVQPGRSLKLSCAASGFTFNNYY<br>MVWVRQAPTKGLEWVAYISTGGGTTYYRESVKG<br>RFTISRDNAKSTLYLQMDSLRSEDTATYYCTTS<br>VYFSDGSSHYDGEWFAYWGQGTLVTVSS |
| Heavy chain variable region Nucleotide sequence (SEQ ID NO: 21) | GAGGTGCAGCTGGTGGAATCTGGGGGAGGCTTA<br>GTGCAGCCTGGAAGGTCCCTGAAACTCTCCTGT<br>GCAGCCTCAGGATTCACTTTCAATAACTATTAC<br>ATGGTCTGGGTCCGCCAGGCTCCAACGAAGGGT<br>CTGGAGTGGGTCGCATACATTAGTACTGGTGGT<br>GGTACCACTTACTATCGAGAGTCCGTGAAGGGC<br>CGGTTCACTATCTCCAGAGATAATGCAAAAAGC<br>ACCCTATACCTGCAGATGGACAGTCTGAGGTCT<br>GAAGACACGGCCACTTATTACTGTACAACGAGT<br>GTTTATTTTTCTGATGGTAGTTCTCACTACGAT<br>GGGGAGTGGTTTGCTTACTGGGGCCAAGGCACT<br>CTGGTCACTGTCTCTTCA |
| CDRH1 (SEQ ID NO: 7) | GFTFNNYY |
| CDRH2 (SEQ ID NO: 8) | ISTGGGTT |
| CDRH3 (SEQ ID NO: 9) | TTSVYFSDGSSHYDGEWFAY |

Examples of the chimeric antibody derived from the rat anti-mouse CD25 antibody include an antibody consisting of a light chain comprising the light chain variable region of each rat anti-mouse CD25 antibody (e.g., the MAb2 antibody) described in the present specification and a human-derived constant region, and a heavy chain comprising the heavy chain variable region of this rat anti-mouse CD25 antibody and a human-derived constant region.

Other examples of the chimeric antibody derived from the rat anti-mouse CD25 antibody include an antibody consisting of a light chain comprising a light chain variable region having the substitution of one to several residues, 1 to 3 residues, 1 or 2 residues, preferably 1 residue, of amino acids in the light chain variable region of each rat anti-mouse CD25 antibody (e.g., the MAb2 antibody) described in the present specification by other amino acid residues, and a heavy chain comprising a heavy chain variable region having the substitution of one to several residues, 1 to 3 residues, 1 or 2 residues, preferably 1 residue, of amino acids in the heavy chain variable region thereof by other amino acid residues. This antibody may have an arbitrary human-derived constant region.

Other examples of the chimeric antibody derived from the rat anti-mouse CD25 antibody include an antibody consisting of a light chain comprising a light chain variable region having the substitution of 1 or 2 residues, preferably 1 residue, of amino acids in any one to three CDRs in the light chain variable region of each rat anti-mouse CD25 antibody (e.g., the MAb2 antibody) described in the present specification by other amino acid residues, and a heavy chain comprising a heavy chain variable region having the substitution of 1 or 2 residues, preferably 1 residue, of amino acids in any one to three CDRs in the heavy chain variable region thereof by other amino acid residues. This antibody may have an arbitrary human-derived constant region.

Examples of the chimeric antibody derived from the MAb2 antibody include an antibody consisting of a light chain comprising a light chain variable region consisting of an amino acid sequence from positions 21 to 133 of SEQ ID NO: 30, and a heavy chain comprising a heavy chain variable region consisting of an amino acid sequence from positions 20 to 133 of SEQ ID NO: 31. This antibody may have an arbitrary human-derived constant region.

Other examples of the chimeric antibody derived from the MAb2 antibody include an antibody consisting of a light chain comprising a light chain variable region having the substitution of one to several residues, 1 to 3 residues, 1 or 2 residues, preferably 1 residue, of amino acids in the light chain variable region consisting of an amino acid sequence from positions 21 to 133 of SEQ ID NO: 30 by other amino acid residues, and a heavy chain comprising a heavy chain variable region having the substitution of one to several residues, 1 to 3 residues, 1 or 2 residues, preferably 1 residue, of amino

acids in the heavy chain variable region consisting of an amino acid sequence from positions 20 to 133 of SEQ ID NO: 31 by other amino acid residues. This antibody may have an arbitrary human-derived constant region.

Other examples of the chimeric antibody derived from the MAb2 antibody include an antibody consisting of a light chain comprising a light chain variable region having the substitution of 1 or 2 residues (preferably 1 residue) of amino acids in any one to three CDRs in the light chain variable region consisting of an amino acid sequence from positions 21 to 133 of SEQ ID NO: 30 by other amino acid residues, and a heavy chain comprising a heavy chain variable region having the substitution of 1 or 2 residues (preferably 1 residue) of amino acids in any one to three CDRs in the heavy chain variable region consisting of an amino acid sequence from positions 20 to 133 of SEQ ID NO: 31 by other amino acid residues. This antibody may have an arbitrary human-derived constant region.

Specific examples of the chimeric antibody derived from the MAb2 antibody include an antibody consisting of a light chain consisting of an amino acid sequence from positions 21 to 232 of SEQ ID NO: 30 and a heavy chain consisting of an amino acid sequence from positions 20 to 463 of SEQ ID NO: 31. In the present specification, this chimeric anti-mouse CD25 antibody is referred to as "cMAb2_hIgG1LALA antibody" or "cMAb2_hIgG1LALA". The light chain amino acid sequence of the cMAb2_hIgG1LALA antibody is encoded by a nucleotide sequence from positions 61 to 717 of SEQ ID NO: 34, and the heavy chain amino acid sequence of the cMAb2_hIgG1LALA antibody is encoded by a nucleotide sequence from positions 58 to 1389 of SEQ ID NO: 35.

Other specific examples of the chimeric antibody derived from the MAb2 antibody include an antibody consisting of a light chain consisting of an amino acid sequence from positions 21 to 239 of SEQ ID NO: 32 and a heavy chain consisting of an amino acid sequence from positions 20 to 463 of SEQ ID NO: 33. In the present specification, this chimeric anti-mouse CD25 antibody is referred to as "cMAb2_hIgG1 antibody" or "cMAb2_hIgG1". The light chain amino acid sequence of the cMAb2_hIgG1 antibody is encoded by a nucleotide sequence from positions 61 to 717 of SEQ ID NO: 36, and the heavy chain amino acid sequence of the cMAb2_hIgG1 antibody is encoded by a nucleotide sequence from positions 58 to 1389 of SEQ ID NO: 37.

Alternative examples of the chimeric antibody derived from the MAb2 antibody include an antibody consisting of a light chain comprising a light chain variable region consisting of an amino acid sequence from positions 21 to 133 of SEQ ID NO: 54, and a heavy chain comprising a heavy chain variable region consisting of an amino acid sequence from positions 20 to 133 of SEQ ID NO: 55. This antibody may have an arbitrary human-derived constant region.

Other examples of the chimeric antibody derived from the MAb2 antibody include an antibody consisting of a light chain comprising a light chain variable region having the substitution of one to several residues, 1 to 3 residues, 1 or 2 residues, preferably 1 residue, of amino acids in the light chain variable region consisting of an amino acid sequence from positions 21 to 133 of SEQ ID NO: 54 by other amino acid residues, and a heavy chain comprising a heavy chain variable region having the substitution of one to several residues, 1 to 3 residues, 1 or 2 residues, preferably 1 residue, of amino acids in the heavy chain variable region consisting of an amino acid sequence from positions 20 to 133 of SEQ ID NO: 55 by other amino acid residues. This antibody may have an arbitrary human-derived constant region.

Other examples of the chimeric antibody derived from the MAb2 antibody include an antibody consisting of a light chain comprising a light chain variable region having the substitution of 1 or 2 residues (preferably 1 residue) of amino acids in any one to three CDRs in the light chain variable region consisting of an amino acid sequence from positions 21 to 133 of SEQ ID NO: 54 by other amino acid residues, and a heavy chain comprising a heavy chain variable region having the substitution of 1 or 2 residues (preferably 1 residue) of amino acids in any one to three CDRs in the heavy chain variable region consisting of an amino acid sequence from positions 20 to 133 of SEQ ID NO: 55 by other amino acid residues. This antibody may have an arbitrary human-derived constant region.

Specific examples of the chimeric antibody derived from the MAb2 antibody include an antibody consisting of a light chain consisting of an amino acid sequence from positions 21 to 239 of SEQ ID NO: 54 and a heavy chain consisting of an amino acid sequence from positions 20 to 463 of SEQ ID NO: 55. In the present specification, this chimeric anti-mouse CD25 antibody is referred to as "cMAb2_hIgG1LALA-PA antibody" or "cMAb2_hIgG1LALA-PA". The light chain amino acid sequence of the cMAb2_hIgG1LALA-PA antibody is encoded by a nucleotide sequence from positions 61 to 717 of SEQ ID NO: 56, and the heavy chain amino acid sequence of the cMAb2_hIgG1LALA-PA antibody is encoded by a nucleotide sequence from positions 58 to 1389 of SEQ ID NO: 57.

The light chain variable region amino acid sequences of the cMAb2_hIgG1LALA antibody, the cMAb2_hIgG1 antibody, and the cMAb2_hIgG1LALA-PA antibody are identical to the light chain variable region amino acid sequence of the MAb2 antibody and consists of the amino acid sequence represented by SEQ ID NO: 22. The light chains of the cMAb2_hIgG1LALA antibody, the cMAb2_hIgG1 antibody, and the cMAb2_hIgG1LALA-PA antibody have CDRL1 consisting of the amino acid sequence represented by SEQ ID NO: 4, CDRL2 consisting of the amino acid sequence represented by SEQ ID NO: 5, and CDRL3 consisting of the amino acid sequence represented by SEQ ID NO: 6, which are identical to CDRL1, CDRL2, and CDRL3, respectively, of the light chain of MAb2. The light chain variable region amino acids of the cMAb2_hIgG1LALA antibody, the cMAb2_hIgG1 antibody, and the cMAb2_hIgG1LALA-PA antibody are encoded by the nucleotide sequence represented by SEQ ID NO: 23.

The heavy chain variable region amino acid sequences of the cMAb2_hIgG1LALA antibody, the cMAb2_hIgG1

antibody, and the cMAb2_hIgG1LALA-PA antibody are identical to the heavy chain variable region amino acid sequence of the MAb2 antibody and consists of the amino acid sequence represented by SEQ ID NO: 24. The heavy chains of the cMAb2_hIgG1LALA antibody, the cMAb2_hIgG1 antibody, and the cMAb2_hIgG1LALA-PA antibody have CDRH1 consisting of the amino acid sequence represented by SEQ ID NO: 10, CDRH2 consisting of the amino acid sequence represented by SEQ ID NO: 11, and CDRH3 consisting of the amino acid sequence represented by SEQ ID NO: 12, which are identical to CDRH1, CDRH2, and CDRH3, respectively, of the heavy chain of MAb2. The heavy chain variable region amino acid sequences of the cMAb2_hIgG1LALA antibody, the cMAb2_hIgG1 antibody, and the cMAb2_hIgG1LALA-PA antibody are encoded by the nucleotide sequence represented by SEQ ID NO: 25. The sequences of the cMAb2_hIgG1LALA antibody, the cMAb2_hIgG1 antibody, and the cMAb2_hIgG1LALA-PA antibody are shown in Table 4.

[Table 4]

| | |
|---|---|
| Light chain variable region Amino acid sequence (SEQ ID NO: 22) | DVVLTQTPPTLSATIGQSVSISCRSSQSLSHT NGNTYLYWLLQRPGQPPQLLIYFVSRLESGVP NRFSGSGSGTDFTLKISGVEAEDLGLYYCVQS THVPNTFGAGTKLELKR |
| Light chain variable region Nucleotide sequence (SEQ ID NO: 23) | GATGTTGTGCTGACCCAGACTCCACCCACTTT ATCGGCTACCATTGGACAATCAGTCTCCATCT CTTGCAGGTCAAGTCAGAGTCTCTCACATACT AATGGAAACACCTATTTATATTGGTTGCTACA GAGGCCAGGCCAACCTCCACAACTTCTAATTT ATTTCGTGTCCAGGCTTGAATCTGGGGTCCCC AACAGGTTCAGTGGCAGTGGGTCAGGAACTGA TTTCACACTCAAAATCAGTGGAGTAGAGGCTG AGGATTTGGGACTTTATTACTGCGTGCAAAGT ACCCATGTTCCGAACACGTTTGGAGCTGGGAC CAAGCTGGAACTGAAACGG |
| CDRL1 (SEQ ID NO: 4) | QSLSHTNGNTY |
| CDRL2 (SEQ ID NO: 5) | FVS |
| CDRL3 (SEQ ID NO: 6) | VQSTHVPNT |
| Heavy chain variable region Amino acid sequence (SEQ ID NO: 24) | QVKLLQSGAALEKPGASVKMSCKASGYTFTDY WVIWVKQSHGKSLEWIGEIYPNSGATNFNAKF KGKATLTVDKSTSTAYMELSRLTSEDSAIYYC TTYDFDYWGQGVMVTVSS |

(continued)

| | |
|---|---|
| Heavy chain variable region Nucleotide sequence (SEQ ID NO: 25) | CAGGTCAAGTTGCTGCAGTCTGGGGCTGCACT<br>GGAGAAGCCTGGAGCCTCTGTGAAGATGTCTT<br>GCAAAGCTTCTGGTTATACATTCACTGACTAC<br>TGGGTGATCTGGGTGAAGCAGAGTCATGGAAA<br>GAGCCTTGAGTGGATTGGGGAAATTTATCCTA<br>ACAGTGGTGCTACTAACTTCAATGCAAAGTTC<br>AAGGGCAAGGCCACATTGACTGTAGACAAATC<br>CACCAGCACAGCCTATATGGAGCTCAGCAGAT<br>TGACATCTGAGGACTCTGCAATCTATTACTGT<br>ACAACATACGACTTTGATTATTGGGGCCAAGG<br>AGTCATGGTCACAGTCTCCTCA |
| CDRH1 (SEQ ID NO: 10) | GYTFTDYW |
| CDRH2 (SEQ ID NO: 11) | IYPNSGAT |
| CDRH3 (SEQ ID NO: 12) | TTYDFDY |

Examples of the humanized antibody can include an antibody obtained by integrating only complementarity determining regions (CDRs) into a human-derived antibody (see Nature (1986) 321, pp. 522-525), and an antibody obtained by grafting the CDR sequences as well as some framework amino acid residues to a human antibody by a CDR grafting method (International Publication No. WO 90/07861), and an antibody obtained by engineering some amino acid sequences of CDRs while maintaining the ability to bind to an antigen.

In the present specification, the humanized antibody derived from the MAb1 antibody is not limited to a particular humanized antibody as long as the humanized antibody retains all six CDR sequences unique to the MAb1 antibody and has internalization activity. Some amino acid sequences of CDRs of this humanized antibody may be further engineered as long as the engineered humanized antibody has internalization activity.

Concrete examples of the humanized antibody of the MAb1 antibody can include arbitrary combinations of a light chain comprising a light chain variable region having any one amino acid sequence selected from the group consisting of (1) an amino acid sequence from positions 21 to 127 of SEQ ID NO: 13 or positions 21 to 127 of SEQ ID NO: 14 (hMAb1A-L or hMAb1B-L), (2) an amino acid sequence having at least 95% or higher sequence identity to the amino acid sequence (1) (preferably an amino acid sequence having at least 95% or higher sequence identity to the sequences of framework regions except for CDR sequences), and (3) an amino acid sequence derived from the amino acid sequence (1) by the deletion, substitution, or addition of one or several amino acids, and a heavy chain comprising a heavy chain variable region having any one amino acid sequence selected from the group consisting of (4) an amino acid sequence from positions 20 to 146 of SEQ ID NO: 15 or positions 20 to 146 of SEQ ID NO: 16 (hMAb1A-H or hMAb1B-H), (5) an amino acid sequence having at least 95% or higher sequence identity to the amino acid sequence (4) (preferably an amino acid sequence having at least 95% or higher sequence identity to the sequences of framework regions except for CDR sequences), and (6) an amino acid sequence derived from the amino acid sequence (4) by the deletion, substitution, or addition of one or several amino acids.

In the present specification, the amino acid substitution is preferably a conservative amino acid substitution. The conservative amino acid substitution is a substitution occurring within an amino acid group associated with amino acid side chains. Preferred amino acid groups are as follows: acidic group = aspartic acid and glutamic acid; basic group = lysine, arginine, and histidine; non-polar group = alanine, valine, leucine, isoleucine, proline, phenylalanine, methionine, and tryptophan; and uncharged polar family = glycine, asparagine, glutamine, cysteine, serine, threonine, and tyrosine. Other preferable amino acid groups are as follows: aliphatic hydroxy group = serine and threonine; amide-containing group = asparagine and glutamine; aliphatic group = alanine, valine, leucine and isoleucine; and aromatic group = phenylalanine, tryptophan and tyrosine. Such an amino acid substitution is preferably performed without reducing the properties of a substance having the original amino acid sequence.

Examples of the antibody having a preferable combination of the light chains and the heavy chains described above include an antibody consisting of a light chain having a light chain variable region amino acid sequence from positions 21 to 127 of SEQ ID NO: 13 (in the present specification, also referred to as hMAb1A-L light chain variable region amino acid sequence) or a light chain having a light chain variable region amino acid sequence from positions 21 to 127 of

SEQ ID NO: 14 (in the present specification, also referred to as hMAb1B-L light chain variable region amino acid sequence), and a heavy chain having a heavy chain variable region amino acid sequence from positions 20 to 146 of SEQ ID NO: 15 (in the present specification, also referred to as hMAblA-H heavy chain variable region amino acid sequence) or a heavy chain having a heavy chain variable region amino acid sequence from positions 20 to 146 of SEQ ID NO: 16 (in the present specification, also referred to as hMAb1B-H heavy chain variable region amino acid sequence).

Preferred examples thereof include: an antibody consisting of a light chain having a light chain variable region amino acid sequence from positions 21 to 127 of SEQ ID NO: 13 and a heavy chain having a heavy chain variable region amino acid sequence from positions 20 to 146 of SEQ ID NO: 15; and an antibody consisting of a light chain having a light chain variable region amino acid sequence from positions 21 to 127 of SEQ ID NO: 14 and a heavy chain having a heavy chain variable region amino acid sequence from positions 20 to 146 of SEQ ID NO: 16.

Other examples of the antibody having a preferable combination of the light chains and the heavy chains described above include an antibody consisting of a light chain consisting of an amino acid sequence from positions 21 to 233 of the amino acid sequence represented by SEQ ID NO: 13 (in the present specification, also referred to as hMAb1A-L light chain full-length amino acid sequence) or a light chain consisting of an amino acid sequence from positions 21 to 233 of the amino acid sequence represented by SEQ ID NO: 14 (in the present specification, also referred to as hMAb1B-L light chain full-length amino acid sequence), and a heavy chain consisting of an amino acid sequence from positions 20 to 476 of the amino acid sequence represented by SEQ ID NO: 15 (in the present specification, also referred to as hMAb1A-H heavy chain full-length amino acid sequence) or a heavy chain consisting of an amino acid sequence from positions 20 to 476 of the amino acid sequence represented by SEQ ID NO: 16 (in the present specification, also referred to as hMAb1B-H heavy chain full-length amino acid sequence).

Preferred examples thereof include: an antibody consisting of a light chain consisting of an amino acid sequence from positions 21 to 233 of the amino acid sequence represented by SEQ ID NO: 13 and a heavy chain consisting of an amino acid sequence from positions 20 to 476 of the amino acid sequence represented by SEQ ID NO: 15; and an antibody consisting of a light chain consisting of an amino acid sequence from positions 21 to 233 of the amino acid sequence represented by SEQ ID NO: 14 and a heavy chain consisting of an amino acid sequence from positions 20 to 476 of the amino acid sequence represented by SEQ ID NO: 16 (in the present specification, also referred to as "hMAb1A antibody" or "hMAb1B antibody").

More preferable examples thereof include an antibody consisting of a light chain consisting of an amino acid sequence from positions 21 to 233 of the amino acid sequence represented by SEQ ID NO: 13 and a heavy chain consisting of an amino acid sequence from positions 20 to 476 of the amino acid sequence represented by SEQ ID NO: 15. The sequences of the hMAb1A antibody and the hMAb1B antibody are shown in Tables 5-1 to 5-4.

[Table 5-1]

| | |
|---|---|
| Amino acid sequence of signal sequence and hMAb1A light chain (SEQ ID NO: 13) | MVLQTQVFISLLLWISGAYGDIVMTQSPDSLAVSLGERATINCKASESVSTRMHW<br>YQQKPGQPPKLLIYKASNLASGVPARFSGSGSGTDFTLTISSLQAEDVAVYYCQQS<br>WNGWAFGQGTKVEIKRTVAAPSVFIFPPSDEQLKSGTASVVCLLNNFYPREAKVQ<br>WKVDNALQSGNSQESVTEQDSKDSTYSLSSTLTLSKADYEKHKVYACEVTHQGL<br>SSPVTKSFNRGEC |
| Nucleotide sequence encoding signal sequence and hMAb1A light chain (SEQ ID NO: 38) | ATGGTGCTGCAGACCCAGGTGTTCATCTCCCTGCTGCTGTGGATCTCCGGC<br>GCGTACGGCGACATCGTGATGACACAGAGCCCTGATAGCCTGGCCGTGTCT<br>CTGGGAGAGAGAGCCACCATCAATTGCAAGGCCAGCGAGAGCGTGTCCACC<br>AGAATGCACTGGTATCAGCAGAAGCCCGGCCAGCCTCCTAAGCTGCTGATC<br>TACAAGGCCTCCAATCTGGCCAGCGGAGTGCCTGCCAGATTTTCTGGCTCT<br>GGCAGCGGCACCGATTTCACCCTGACAATCAGTTCCCTGCAGGCCGAGGAT<br>GTGGCCGTGTACTACTGTCAGCAGAGCTGGAATGGCTGGGCCTTTGGCCAG<br>GGCACCAAGGTGGAAATCAAGCGTACGGTGGCCGCCCCCTCCGTGTTCATC<br>TTCCCCCCCTCCGACGAGCAGCTGAAGTCCGGCACCGCCTCCGTGGTGTGC<br>CTGCTGAATAACTTCTACCCCAGAGAGGCCAAGGTGCAGTGGAAGGTGGAC<br>AACGCCCTGCAGTCCGGGAACTCCCAGGAGAGCGTGACCGAGCAGGACAGC<br>AAGGACAGCACCTACAGCCTGAGCAGCACCCTGACCCTGAGCAAAGCCGACT<br>ACGAGAAGCACAAGGTGTACGCCTGCGAGGTGACCCACCAGGGCCTGAGCT<br>CCCCCGTCACCAAGAGCTTCAACAGGGGGGAGTGT |

[Table 5-2]

| | |
|---|---|
| Amino acid sequence of signal sequence and hMAb1A heavy chain (SEQ ID NO: 15) | MKHLWFFLLLVAAPRWVLSEVQLVESGGGLVQPGGSLRLSCAASGFTFNNYYMV<br>WVRQAPGKGLEWVAYISTGGGTTYYRESVKGRFTISRDNSKSTLYLQMNSLRAE<br>DTAVYYCTTSVYFSDGSSHYDGEWFAYWGQGTLVTVSSASTKGPSVFPLAPSSKS<br>TSGGTAALGCLVKDYFPEPVTVSWNSGALTSGVHTFPAVLQSSGLYSLSSVVTVP<br>SSSLGTQTYICNVNHKPSNTKVDKKVEPKSCDKTHTCPPCPAPELLGGPSVFLFP<br>PKPKDTLMISRTPEVTCVVVDVSHEDPEVKFNWYVDGVEVHNAKTKPREEQYNS<br>TYRVVSVLTVLHQDWLNGKEYKCKVSNKALPAPIEKTISKAKGQPREPQVYTLPP<br>SREEMTKNQVSLTCLVKGFYPSDIAVEWESNGQPENNYKTTPPVLDSDGSFFLYS<br>KLTVDKSRWQQGNVFSCSVMHEALHNHYTQKSLSLSPGK |
| Nucleotide sequence encoding signal sequence and hMAb1A heavy chain (SEQ ID NO: 40) | ATGAAACACCTGTGGTTCTTCCTCCTGCTGGTGGCAGCTCCCAGATGGGTG<br>CTGAGCGAGGTGCAGCTGGTTGAATCTGGCGGAGGACTGGTTCAGCCTGG<br>CGGATCTCTGAGACTGTCTTGTGCCGCCAGCGGCTTCACCTTCAACAACTAC<br>TACATGGTCTGGGTCCGACAGGCCCCTGGCAAAGGACTTGAGTGGGTCGCC<br>TACATCTCTACCGGCGGAGGCACCACCTACTACCGCGAGTCTGTGAAGGGC<br>AGATTCACCATCAGCCGGGACAACAGCAAGAGCACCCTGTACCTGCAGATGA<br>ACAGCCTGAGAGCCGAGGACACCGCCGTGTACTACTGTACCACCAGCGTGT<br>ACTTCAGCGACGGCAGCAGCCACTATGACGGCGAGTGGTTCGCTTATTGGG<br>GCCAGGGCACACTGGTCACAGTTAGCTCAGCCTCCACCAAGGGCCCAAGCG<br>TCTTCCCCCTGGCACCCTCCTCCAAGAGCACCTCTGGCGGCACAGCCGCCCT<br>GGGCTGCCTGGTCAAGGACTACTTCCCCGAACCCGTGACCGTGAGCTGGAA<br>CTCAGGCGCCCTGACCAGCGGCGTGCACACCTTCCCCGCTGTCCTGCAGTC<br>CTCAGGACTCTACTCCCTCAGCAGCGTGGTGACCGTGCCCTCCAGCAGCTT<br>GGGCACCCAGACCTACATCTGCAACGTGAATCACAAGCCCAGCAACACCAAG<br>GTGGACAAGAAGGTTGAGCCCAAATCTTGTGACAAAACTCACACATGCCCAC<br>CCTGCCCAGCACCTGAACTCCTGGGGGGACCCTCAGTCTTCCTCTTCCCCCC<br>AAAACCCAAGGACACCCTCATGATCTCCCGGACCCCTGAGGTCACATGCGTG<br>GTGGTGGACGTGAGCCACGAAGACCCTGAGGTCAAGTTCAACTGGTACGTG<br>GACGGCGTGGAGGTGCATAATGCCAAGACAAAGCCCCGGGAGGAGCAGTAC<br>AACAGCACGTACCGGGTGGTCAGCGTCCTCACCGTCCTGCACCAGGACTGG<br>CTGAATGGCAAGGAGTACAAGTGCAAGGTCTCCAACAAAGCCCTCCCAGCCC<br>CCATCGAGAAAACCATCTCCAAAGCCAAAGGCCAGCCCCGGGAACCACAGGT<br>GTACACCCTGCCCCCATCCCGGGAGGAGATGACCAAGAACCAGGTCAGCCT<br>GACCTGCCTGGTCAAAGGCTTCTATCCCAGCGACATCGCCGTGGAGTGGGA<br>GAGCAATGGCCAGCCCGAGAACAACTACAAGACCACCCCTCCCGTGCTGGAC<br>TCCGACGGCTCCTTCTTCCTCTACAGCAAGCTCACCGTGGACAAGAGCAGG<br>TGGCAGCAGGGCAACGTCTTCTCATGCTCCGTGATGCATGAGGCTCTGCAC<br>AACCACTACACCCAGAAGAGCCTCTCCCTGTCTCCCGGCAAA |

[Table 5-3]

| | |
|---|---|
| Amino acid sequence of signal sequence and hMAb1B light chain (SEQ ID NO: 14) | MVLQTQVFISLLLWISGAYGEIVLTQSPGTLSLSPGERATLSCKASESVSTRMHW<br>YQQKPGQAPRLLIYKASNLASGVPARFSGSGSGTDFTLTISRLEPEDFAVYFCQQ<br>SWNGWAFGQGTKVEIKRTVAAPSVFIFPPSDEQLKSGTASVVCLLNNFYPREAK<br>VQWKVDNALQSGNSQESVTEQDSKDSTYSLSSTLTLSKADYEKHKVYACEVTH<br>QGLSSPVTKSFNRGEC |

(continued)

| | |
|---|---|
| Nucleotide sequence encoding signal sequence and hMAb1B light chain (SEQ ID NO: 39) | ATGGTGCTGCAGACCCAGGTGTTCATCTCCCTGCTGCTGTGGATCTCCGG CGCGTACGGCGAGATCGTGCTGACACAGAGCCCTGGCACACTGTCACTGT CTCCAGGCGAAAGAGCCACACTGAGCTGCAAGGCCAGCGAGAGCGTGTCC ACACGGATGCACTGGTATCAGCAGAAGCCTGGACAGGCCCCTCGGCTGCT GATCTACAAGGCCTCTAATCTGGCCAGCGGCGTGCCAGCCAGATTTTCTG GTTCTGGCAGCGGCACCGACTTCACCCTGACAATCAGCAGACTGGAACCC GAGGACTTCGCCGTGTACTTCTGTCAGCAGTCCTGGAACGGCTGGGCCTT TGGCCAGGGAACAAAGGTGGAAATCAAGCGTACGGTGGCCGCCCCCTCCG TGTTCATCTTCCCCCCCTCCGACGAGCAGCTGAAGTCCGGCACCGCCTCCG TGGTGTGCCTGCTGAATAACTTCTACCCCAGAGAGGCCAAGGTGCAGTGG AAGGTGGACAACGCCCTGCAGTCCGGGAACTCCCAGGAGAGCGTGACCGA GCAGGACAGCAAGGACAGCACCTACAGCCTGAGCAGCACCCTGACCCTGA GCAAAGCCGACTACGAGAAGCACAAGGTGTACGCCTGCGAGGTGACCCAC CAGGGCCTGAGCTCCCCCGTCACCAAGAGCTTCAACAGGGGGGAGTGT |

[Table 5-4]

| | |
|---|---|
| Amino acid sequence of signal sequence and hMAb1B heavy chain (SEQ ID NO: 16) | MKHLWFFLLLVAAPRWVLSEVQLVESGGGLVQPGGSLRLSCAASGFTFNNYYMV WVRQAPGKGLEWVAYISTGGGTTYYADSVKGRFTISRDNSKSTLYLQMNSLRAE DTAVYYCTTSVYFSDGSSHYDGEWFAYWGQGTLVTVSSASTKGPSVFPLAPSSKS TSGGTAALGCLVKDYFPEPVTVSWNSGALTSGVHTFPAVLQSSGLYSLSSVVTVP SSSLGTQTYICNVNHKPSNTKVDKKVEPKSCDKTHTCPPCPAPELLGGPSVFLFP PKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQVYTLPP SREEMTKNQVSLTCLVKGFYPSDIAVEWESNGQPENNYKTTPPVLDSDGSFFLYS KLTVDKSRWQQGNVFSCSVMHEALHNHYTQKSLSLSPGK |

(continued)

| | |
|---|---|
| Nucleotide sequence encoding signal sequence and hMAb1B heavy chain SEQ ID NO: 41) | ATGAAACACCTGTGGTTCTTCCTCCTGCTGGTGGCAGCTCCCAGATGGGTG<br>CTGAGCGAGGTGCAGCTGGTTGAATCTGGCGGAGGACTGGTTCAGCCTGG<br>CGGATCTCTGAGACTGTCTTGTGCCGCCAGCGGCTTCACCTTCAACAACTAC<br>TACATGGTCTGGGTCCGACAGGCCCCTGGCAAAGGACTTGAGTGGGTCGCC<br>TACATCTCTACCGGCGGAGGCACCACCTACTACGCCGATTCTGTGAAGGGC<br>AGATTCACCATCAGCCGGGACAACAGCAAGAGCACCCTGTACCTGCAGATGA<br>ACAGCCTGAGAGCCGAGGACACCGCCGTGTACTACTGTACCACCAGCGTGT<br>ACTTCAGCGACGGCAGCAGCCACTATGACGGCGAGTGGTTCGCTTATTGGG<br>GCCAGGGCACACTGGTCACAGTTAGCTCAGCCTCCACCAAGGGCCCAAGCG<br>TCTTCCCCCTGGCACCCTCCTCCAAGAGCACCTCTGGCGGCACAGCCGCCCT<br>GGGCTGCCTGGTCAAGGACTACTTCCCCGAACCCGTGACCGTGAGCTGGAA<br>CTCAGGCGCCCTGACCAGCGGCGTGCACACCTTCCCCGCTGTCCTGCAGTC<br>CTCAGGACTCTACTCCCTCAGCAGCGTGGTGACCGTGCCCTCCAGCAGCTT<br>GGGCACCCAGACCTACATCTGCAACGTGAATCACAAGCCCAGCAACACCAAG<br>GTGGACAAGAAGGTTGAGCCCAAATCTTGTGACAAAACTCACACATGCCCAC<br>CCTGCCCAGCACCTGAACTCCTGGGGGGACCCTCAGTCTTCCTCTTCCCCCC<br>AAAACCCAAGGACACCCTCATGATCTCCCGGACCCCTGAGGTCACATGCGTG<br>GTGGTGGACGTGAGCCACGAAGACCCTGAGGTCAAGTTCAACTGGTACGTG<br>GACGGCGTGGAGGTGCATAATGCCAAGACAAAGCCCCGGGAGGAGCAGTAC<br>AACAGCACGTACCGGGTGGTCAGCGTCCTCACCGTCCTGCACCAGGACTGG<br>CTGAATGGCAAGGAGTACAAGTGCAAGGTCTCCAACAAAGCCCTCCCAGCCC<br>CCATCGAGAAAACCATCTCCAAAGCCAAAGGCCAGCCCCGGGAACCACAGGT<br>GTACACCCTGCCCCCATCCCGGGAGGAGATGACCAAGAACCAGGTCAGCCT<br>GACCTGCCTGGTCAAAGGCTTCTATCCCAGCGACATCGCCGTGGAGTGGGA<br>GAGCAATGGCCAGCCCGAGAACAACTACAAGACCACCCCTCCCGTGCTGGAC<br>TCCGACGGCTCCTTCTTCCTCTACAGCAAGCTCACCGTGGACAAGAGCAGG<br>TGGCAGCAGGGCAACGTCTTCTCATGCTCCGTGATGCATGAGGCTCTGCAC<br>AACCACTACACCCAGAAGAGCCTCTCCCTGTCTCCCGGCAAA |

An antibody having biological activity equivalent to that of each of the antibodies described above can be selected by combining sequences that exhibit high sequence identity to the heavy chain amino acid sequences and the light chain amino acid sequences described above. Such sequence identity is generally 80% or higher sequence identity, preferably 90% or higher sequence identity, more preferably 95% or higher sequence identity, most preferably 99% or higher sequence identity. Alternatively, an antibody having biological activity equivalent to that of each of the antibodies described above may be selected by combining amino acid sequences derived from the heavy chain or light chain amino acid sequence by the substitution, deletion, or addition of one or several amino acid residues.

In one aspect of the present invention, the antibody of the present invention or the antigen binding activity fragment of the antibody comprises CDRL1, CDRL2, and CDRL3, and CDRH1, CDRH2, and CDRH3 in the following combination (a) or (b):

(a) CDRL1 consisting of an amino acid sequence having 80% or higher, preferably 85%, 86%, 87%, 88%, 89%, 90%, 91%, 92%, 93%, or 94% or higher, more preferably 95%, 96%, 97%, or 98% or higher, still more preferably 99% or higher sequence identity to the amino acid sequence represented by SEQ ID NO: 1, CDRL2 consisting of an amino acid sequence having 80% or higher, preferably 85%, 86%, 87%, 88%, 89%, 90%, 91%, 92%, 93%, or 94% or higher, more preferably 95%, 96%, 97%, or 98% or higher, still more preferably 99% or higher sequence identity to the amino acid sequence represented by SEQ ID NO: 2 (KAS), and CDRL3 consisting of an amino acid sequence having 80% or higher, preferably 85%, 86%, 87%, 88%, 89%, 90%, 91%, 92%, 93%, or 94% or higher, more preferably 95%, 96%, 97%, or 98% or higher, still more preferably 99% or higher sequence identity to the amino acid sequence represented by SEQ ID NO: 3, and CDRH1 consisting of an amino acid sequence having 80% or higher, preferably 85%, 86%, 87%, 88%, 89%, 90%, 91%, 92%, 93%, or 94% or higher, more preferably 95%, 96%, 97%, or 98% or higher, still more preferably 99% or higher sequence identity to the amino acid sequence represented by SEQ ID NO: 7, CDRH2

consisting of an amino acid sequence having 80% or higher, preferably 85%, 86%, 87%, 88%, 89%, 90%, 91%, 92%, 93%, or 94% or higher, more preferably 95%, 96%, 97%, or 98% or higher, still more preferably 99% or higher sequence identity to the amino acid sequence represented by SEQ ID NO: 8, and CDRH3 consisting of an amino acid sequence having 80% or higher, preferably 85%, 86%, 87%, 88%, 89%, 90%, 91%, 92%, 93%, or 94% or higher, more preferably 95%, 96%, 97%, or 98% or higher, still more preferably 99% or higher sequence identity to the amino acid sequence represented by SEQ ID NO: 9, and

(b) CDRL1 consisting of an amino acid sequence having 80% or higher, preferably 85%, 86%, 87%, 88%, 89%, 90%, 91%, 92%, 93%, or 94% or higher, more preferably 95%, 96%, 97%, or 98% or higher, still more preferably 99% or higher sequence identity to the amino acid sequence represented by SEQ ID NO: 4, CDRL2 consisting of an amino acid sequence having 80% or higher, preferably 85%, 86%, 87%, 88%, 89%, 90%, 91%, 92%, 93%, or 94% or higher, more preferably 95%, 96%, 97%, or 98% or higher, still more preferably 99% or higher sequence identity to the amino acid sequence represented by SEQ ID NO: 5 (FVS), and CDRL3 consisting of an amino acid sequence having 80% or higher, preferably 85%, 86%, 87%, 88%, 89%, 90%, 91%, 92%, 93%, or 94% or higher, more preferably 95%, 96%, 97%, or 98% or higher, still more preferably 99% or higher sequence identity to the amino acid sequence represented by SEQ ID NO: 6, and CDRH1 consisting of an amino acid sequence having 80% or higher, preferably 85%, 86%, 87%, 88%, 89%, 90%, 91%, 92%, 93%, or 94% or higher, more preferably 95%, 96%, 97%, or 98% or higher, still more preferably 99% or higher sequence identity to the amino acid sequence represented by SEQ ID NO: 10, CDRH2 consisting of an amino acid sequence having 80% or higher, preferably 85%, 86%, 87%, 88%, 89%, 90%, 91%, 92%, 93%, or 94% or higher, more preferably 95%, 96%, 97%, or 98% or higher, still more preferably 99% or higher sequence identity to the amino acid sequence represented by SEQ ID NO: 11, and CDRH3 consisting of an amino acid sequence having 80% or higher, preferably 85%, 86%, 87%, 88%, 89%, 90%, 91%, 92%, 93%, or 94% or higher, more preferably 95%, 96%, 97%, or 98% or higher, still more preferably 99% or higher sequence identity to the amino acid sequence represented by SEQ ID NO: 12.

In one aspect of the present invention, the antibody of the present invention or the antigen binding activity fragment of the antibody comprises a heavy chain variable region and a light chain variable region in the following combination (a) or (b):

(a) a light chain variable region consisting of an amino acid sequence having 80% or higher, preferably 85%, 86%, 87%, 88%, 89%, 90%, 91%, 92%, 93%, or 94% or higher, more preferably 95%, 96%, 97%, or 98% or higher, still more preferably 99% or higher sequence identity to an amino acid sequence from positions 21 to 127 of SEQ ID NO: 13, and a heavy chain variable region consisting of an amino acid sequence having 80% or higher, preferably 85%, 86%, 87%, 88%, 89%, 90%, 91%, 92%, 93%, or 94% or higher, more preferably 95%, 96%, 97%, or 98% or higher, still more preferably 99% or higher sequence identity to an amino acid sequence from positions 20 to 146 of SEQ ID NO: 15, and

(b) a light chain variable region consisting of an amino acid sequence having 80% or higher, preferably 85%, 86%, 87%, 88%, 89%, 90%, 91%, 92%, 93%, or 94% or higher, more preferably 95%, 96%, 97%, or 98% or higher, still more preferably 99% or higher sequence identity to an amino acid sequence from positions 21 to 127 of SEQ ID NO: 14, and a heavy chain variable region consisting of an amino acid sequence having 80% or higher, preferably 85%, 86%, 87%, 88%, 89%, 90%, 91%, 92%, 93%, or 94% or higher, more preferably 95%, 96%, 97%, or 98% or higher, still more preferably 99% or higher sequence identity to an amino acid sequence from positions 20 to 146 of SEQ ID NO: 16.

In an alternative aspect of the present invention, the antibody of the present invention or the antigen binding activity fragment of the antibody comprises a heavy chain variable region and a light chain variable region in the following combination (a) or (b):

(a) a light chain variable region consisting of an amino acid sequence that comprises CDRs having 100% sequence identity to CDR sequences in an amino acid sequence from positions 21 to 127 of SEQ ID NO: 13 and has 80% or higher, preferably 85%, 86%, 87%, 88%, 89%, 90%, 91%, 92%, 93%, or 94% or higher, more preferably 95%, 96%, 97%, or 98% or higher, still more preferably 99% or higher sequence identity to the sequences of framework regions except for the CDR sequences, and a heavy chain variable region consisting of an amino acid sequence that comprises CDRs having 100% sequence identity to CDR sequences in an amino acid sequence from positions 20 to 146 of SEQ ID NO: 15 and has 80% or higher, preferably 85%, 86%, 87%, 88%, 89%, 90%, 91%, 92%, 93%, or 94% or higher, more preferably 95%, 96%, 97%, or 98% or higher, still more preferably 99% or higher sequence identity to the sequences of framework regions except for the CDR sequences, and

(b) a light chain variable region consisting of an amino acid sequence that comprises CDRs having 100% sequence identity to CDR sequences in an amino acid sequence from positions 21 to 127 of SEQ ID NO: 14 and has 80% or higher, preferably 85%, 86%, 87%, 88%, 89%, 90%, 91%, 92%, 93%, or 94% or higher, more preferably 95%, 96%, 97%, or 98% or higher, still more preferably 99% or higher sequence identity to the sequences of framework regions

except for the CDR sequences, and a heavy chain variable region consisting of an amino acid sequence that comprises CDRs having 100% sequence identity to CDR sequences in an amino acid sequence from positions 20 to 146 of SEQ ID NO: 16 and has 80% or higher, preferably 85%, 86%, 87%, 88%, 89%, 90%, 91%, 92%, 93%, or 94% or higher, more preferably 95%, 96%, 97%, or 98% or higher, still more preferably 99% or higher sequence identity to the sequences of framework regions except for the CDR sequences.

In an alternative aspect of the present invention, the antibody of the present invention or the antigen binding activity fragment of the antibody comprises a heavy chain and a light chain in the following combination (a) or (b):

(a) a light chain consisting of an amino acid sequence having 80% or higher, preferably 85%, 86%, 87%, 88%, 89%, 90%, 91%, 92%, 93%, or 94% or higher, more preferably 95%, 96%, 97%, or 98% or higher, still more preferably 99% or higher sequence identity to an amino acid sequence from positions 21 to 233 of SEQ ID NO: 13, and a heavy chain consisting of an amino acid sequence having 80% or higher, preferably 85%, 86%, 87%, 88%, 89%, 90%, 91%, 92%, 93%, or 94% or higher, more preferably 95%, 96%, 97%, or 98% or higher, still more preferably 99% or higher sequence identity to an amino acid sequence from positions 20 to 476 of SEQ ID NO: 15, and
(b) a light chain consisting of an amino acid sequence having 80% or higher, preferably 85%, 86%, 87%, 88%, 89%, 90%, 91%, 92%, 93%, or 94% or higher, more preferably 95%, 96%, 97%, or 98% or higher, still more preferably 99% or higher sequence identity to an amino acid sequence from positions 21 to 233 of SEQ ID NO: 14, and a heavy chain consisting of an amino acid sequence having 80% or higher, preferably 85%, 86%, 87%, 88%, 89%, 90%, 91%, 92%, 93%, or 94% or higher, more preferably 95%, 96%, 97%, or 98% or higher, still more preferably 99% or higher sequence identity to an amino acid sequence from positions 20 to 476 of SEQ ID NO: 16.

In an alternative aspect of the present invention, the antibody of the present invention or the antigen binding activity fragment of the antibody comprises a heavy chain a light chain in the following combination (a) or (b):

(a) a light chain consisting of an amino acid sequence that comprises CDRs having 100% sequence identity to CDR sequences in an amino acid sequence from positions 21 to 233 of SEQ ID NO: 13 and has 80% or higher, preferably 85%, 86%, 87%, 88%, 89%, 90%, 91%, 92%, 93%, or 94% or higher, more preferably 95%, 96%, 97%, or 98% or higher, still more preferably 99% or higher sequence identity to sequences except for the CDR sequences, and a heavy chain consisting of an amino acid sequence that comprises CDRs having 100% sequence identity to CDR sequences in an amino acid sequence from positions 20 to 476 of SEQ ID NO: 15 and has 80% or higher, preferably 85%, 86%, 87%, 88%, 89%, 90%, 91%, 92%, 93%, or 94% or higher, more preferably 95%, 96%, 97%, or 98% or higher, still more preferably 99% or higher sequence identity to sequences except for the CDR sequences, and
(b) a light chain consisting of an amino acid sequence that comprises CDRs having 100% sequence identity to CDR sequences in an amino acid sequence from positions 21 to 233 of SEQ ID NO: 14 and has 80% or higher, preferably 85%, 86%, 87%, 88%, 89%, 90%, 91%, 92%, 93%, or 94% or higher, more preferably 95%, 96%, 97%, or 98% or higher, still more preferably 99% or higher sequence identity to sequences except for the CDR sequences, and a heavy chain consisting of an amino acid sequence that comprises CDRs having 100% sequence identity to CDR sequences in an amino acid sequence from positions 20 to 476 of SEQ ID NO: 16 and has 80% or higher, preferably 85%, 86%, 87%, 88%, 89%, 90%, 91%, 92%, 93%, or 94% or higher, more preferably 95%, 96%, 97%, or 98% or higher, still more preferably 99% or higher sequence identity to sequences except for the CDR sequences.

The sequence identity between two types of amino acid sequences can be determined, for example, by using default parameters of Clustal W version 2 (Larkin MA, Blackshields G, Brown NP, Chenna R, McGettigan PA, McWilliam H, Valentin F, Wallace IM, Wilm A, Lopez R, Thompson JD, Gibson TJ and Higgins DG(2007), "Clustal W and Clustal X version 2.0", Bioinformatics.23(21): 2947-2948) and aligning the sequences. The determination method is not limited thereto as long as the method is used by those skilled in the art.
In this context, the "identity" between amino acid sequences refers to a value in percentage obtained by aligning two amino acid sequences to be compared so as to maximize the number of matched residues between the amino acid sequences, and dividing the number of matched residues by the total number of residues. For the alignment, a gap is appropriately inserted, if necessary, to one or both of the two sequences to be compared. Such alignment of the sequences can be performed using a well-known program, for example, BLAST, FASTA, or CLUSTAL W. In the case of inserting a gap, the total number of residues is the number of residues calculated by counting one gap as one residue. When the total number of residues thus counted differs between the two sequences to be compared, the identity (%) is calculated by dividing the number of matched residues by the total number of residues in the longer sequence. The same holds true for the identity between nucleotide sequences.
In the present specification, the amino acid sequence identity is calculated using sequence analysis software GENETYX-SV/RC (manufactured by Genetyx Corp.), and this algorithm is usually used in the art.

In the hMAb1A-L light chain full-length amino acid sequence represented by SEQ ID NO: 13, the amino acid sequence consisting of amino acid residues 1 to 20 is a signal sequence, the amino acid sequence consisting of amino acid residues 21 to 127 is a variable region, and the amino acid sequence consisting of amino acid residues 128 to 233 is a constant region. In the hMAb1A-L light chain full-length nucleotide sequence represented by SEQ ID NO: 38, the nucleotide sequence consisting of nucleotides 1 to 60 encodes the signal sequence, the nucleotide sequence consisting of nucleotides 61 to 381 encodes the variable region, and the nucleotide sequence consisting of nucleotides 382 to 699 encodes the constant region.

In the hMAb1B-L light chain full-length amino acid sequence represented by SEQ ID NO: 14, the amino acid sequence consisting of amino acid residues 1 to 20 is a signal sequence, the amino acid sequence consisting of amino acid residues 21 to 127 is a variable region, and the amino acid sequence consisting of amino acid residues 128 to 233 is a constant region. In the hMAb1B-L light chain full-length nucleotide sequence represented by SEQ ID NO: 39, the nucleotide sequence consisting of nucleotides 1 to 60 encodes the signal sequence, the nucleotide sequence consisting of nucleotides 61 to 381 encodes the variable region, and the nucleotide sequence consisting of nucleotides 382 to 699 encodes the constant region.

In the hMAblA-H heavy chain full-length amino acid sequence represented by SEQ ID NO: 15, the amino acid sequence consisting of amino acid residues 1 to 19 is a signal sequence, the amino acid sequence consisting of amino acid residues 20 to 146 is a variable region, and the amino acid sequence consisting of amino acid residues 147 to 476 is a constant region. In the hMAblA-H heavy chain full-length nucleotide sequence represented by SEQ ID NO: 40, the nucleotide sequence consisting of nucleotides 1 to 57 encodes the signal sequence, the nucleotide sequence consisting of nucleotides 58 to 438 encodes the variable region, and the nucleotide sequence consisting of nucleotides 439 to 1428 encodes the constant region.

In the hMAb1B-H heavy chain full-length amino acid sequence represented by SEQ ID NO: 16, the amino acid sequence consisting of amino acid residues 1 to 19 is a signal sequence, the amino acid sequence consisting of amino acid residues 20 to 146 is a variable region, and the amino acid sequence consisting of amino acid residues 147 to 476 is a constant region. In the hMAb1B-H heavy chain full-length nucleotide sequence represented by SEQ ID NO: 41, the nucleotide sequence consisting of nucleotides 1 to 57 encodes the signal sequence, the nucleotide sequence consisting of nucleotides 58 to 438 encodes the variable region, and the nucleotide sequence consisting of nucleotides 439 to 1428 encodes the constant region.

Further examples of the antibody of the present invention can include a human antibody that binds to CD25. The anti-CD25 human antibody means a human antibody having only the gene sequence of an antibody derived from human chromosomes. The anti-CD25 human antibody can be obtained by a method using a human antibody-producing mouse having a human chromosomal fragment comprising the heavy chain and light chain genes of a human antibody (see Tomizuka, K. et al., Nature Genetics (1997) 16, p. 133-143; Kuroiwa, Y. et al., Nucl. Acids Res. (1998) 26, p. 3447-3448; Yoshida, H. et al., Animal Cell Technology: Basic and Applied Aspects vol. 10, p. 69-73 (Kitagawa, Y., Matsuda, T. and Iijima, S. eds.), Kluwer Academic Publishers, 1999; Tomizuka, K. et al., Proc. Natl. Acad. Sci. USA (2000) 97, p. 722-727; etc.).

Such a human antibody-producing mouse can be specifically produced by using a genetically modified animal in which the gene loci of endogenous immunoglobulin heavy chain and light chain of which have been disrupted and instead the gene loci of human immunoglobulin heavy chain and light chain have been then introduced via a yeast artificial chromosome (YAC) vector or the like, preparing a knock-out animal and a transgenic animal as the genetically modified animal, and breeding these animals each other.

Alternatively, the anti-CD25 human antibody may be obtained by transforming eukaryotic cells with cDNA encoding each of the heavy chain and light chain of such a human antibody, preferably with a vector comprising the cDNA, by a gene recombination technique, and culturing the transformed cells producing a genetically recombinant human monoclonal antibody so that the antibody can be obtained from the culture supernatant.

In this context, for example, eukaryotic cells, preferably mammalian cells such as CHO cells, lymphocytes or myelomas can be used as a host.

A method for obtaining a phage display-derived human antibody selected from a human antibody library (see Wormstone, I.M. et al., Investigative Ophthalmology & Visual Science. (2002) 43 (7), p. 2301-2308; Carmen, S. et al., Briefings in Functional Genomics and Proteomics (2002), 1 (2), p. 189-203; Siriwardena, D. et al., Ophthalmology (2002) 109 (3), p. 427-431; etc.) is also known.

For example, a phage display method which involves expressing the variable regions of a human antibody as a single chain antibody (scFv) on the surface of phages, and selecting a phage binding to an antigen (Nature Biotechnology (2005), 23, (9), p. 1105-1116) can be used.

The phage gene selected because of its binding to the antigen can be analyzed to determine DNA sequences encoding the variable regions of a human antibody binding to the antigen.

Once the DNA sequence of scFv binding to the antigen is determined, an expression vector having this sequence can be prepared and transferred to an appropriate host so that a human antibody can be expressed therein and thus

obtained (International Publication Nos. WO92/01047, WO92/20791, WO93/06213, WO93/11236, WO93/19172, WO95/01438, and WO95/15388; Annu. Rev. Immunol (1994) 12, p. 433-455; and Nature Biotechnology (2005) 23 (9), p. 1105-1116).

Provided that a newly prepared human antibody binds to a partial peptide or a partial conformation to which at least any one rat anti-CD25 antibody, chimeric anti-CD25 antibody, or humanized anti-human CD25 antibody (e.g., the MAb1 antibody, the MAb2 antibody, the cMAb1_hIgG1LALA antibody, the cMAb2_hIgG1LALA antibody, the cMAb2_hIgG1 antibody, the cMAb2_hIgG1LALA-PA antibody, the hMAb1A antibody, or the hMAb1B antibody) described in the present specification binds, the human antibody can be determined to bind to the same epitope as that for the rat anti-CD25 antibody, the chimeric anti-CD25 antibody, or the humanized anti-human CD25 antibody. Alternatively, if the specific sequence or structure of an epitope is not determined, the human antibody can be determined to bind to the same epitope as that for the rat anti-CD25 antibody, the chimeric anti-CD25 antibody, or the humanized anti-human CD25 antibody described in the present specification by confirming that the human antibody competes with the rat anti-CD25 antibody, the chimeric anti-CD25 antibody, or the humanized anti-human CD25 antibody (e.g., the MAb1 antibody, the MAb2 antibody, the cMAb1_hIgG1LALA antibody, the cMAb2_hIgG1LALA antibody, the cMAb2_hIgG1 antibody, the cMAb2_hIgG1LALA-PA antibody, the hMAb1A antibody, or the hMAb1B antibody) described in the present specification for the binding of the rat anti-CD25 antibody, the chimeric anti-CD25 antibody, or the humanized anti-human CD25 antibody to CD25 (e.g., the human antibody interferes with the binding of the MAb1 antibody, the MAb2 antibody, the cMAb1_hIgG1LALA antibody, the cMAb2_hIgG1LALA antibody, the cMAb2_hIgG1 antibody, the cMAb2_hIgG1LALA-PA antibody, the hMAb1A antibody, or the hMAb1B antibody to CD25). In the present specification, the newly prepared human antibody determined to "bind to the same epitope" by at least any one of these determination methods is regarded as "binding to the same epitope" as that for the rat anti-CD25 antibody, the chimeric anti-CD25 antibody, or the humanized anti-human CD25 antibody described in the present specification. When the epitope is confirmed to be the same, the human antibody is expected to have biological activity equivalent to that of the rat anti-CD25 antibody, the chimeric anti-CD25 antibody, or the humanized anti-human CD25 antibody (e.g., the MAb1 antibody, the MAb2 antibody, the cMAb1_hIgG1LALA antibody, the cMAb2_hIgG1LALA antibody, the cMAb2_hIgG1 antibody, the cMAb2_hIgG1LALA-PA antibody, the hMAb1A antibody, or the hMAb1B antibody).

The chimeric antibodies, the humanized antibodies, or the human antibodies obtained by the methods described above are evaluated for their binding activity against the antigen according to a method known in the art, etc. so that a preferable antibody can be selected.

The antibody of the present invention also includes a modified variant of the antibody. The modified variant refers to a variant obtained by subjecting the antibody of the present invention to chemical or biological modification. Examples of the chemically modified variant include chemically modified variants including a linkage of a chemical moiety to an amino acid skeleton or an N-linked or O-linked carbohydrate chain, etc. Examples of the biologically modified variant include variants obtained by post-translational modification (e.g., N-linked or O-linked glycosylation, N- or C-terminal processing, deamidation, isomerization of aspartic acid, oxidation of methionine, or conversion of N-terminal glutamine or N-terminal glutamic acid to pyroglutamic acid), and variants with a methionine residue added to the N terminus through expression in prokaryotic host cells. Further, such a modified variant is also meant to include an antibody labeled so as to permit detection or isolation of the antibody of the present invention or the antigen, for example, an enzyme-labeled antibody, a fluorescence-labeled antibody, and an affinity-labeled antibody. Such a modified variant of the antibody of the present invention is useful for improving the stability and blood retention of the antibody, reducing the antigenicity thereof, detecting or isolating the antibody or the antigen, and so on.

Further, antibody-dependent cellular cytotoxic activity can be enhanced by regulating the modification of a glycan linked to the antibody of the present invention (glycosylation, defucosylation, etc.). The technique for regulating the modification of a glycan in antibodies is known by International Publication Nos. WO 1999/54342, WO 2000/61739, WO 2002/31140, WO 2013/120066, etc., though the technique is not limited thereto. The antibody of the present invention also includes an antibody in which the modification of a glycan is regulated.

In the case of temporarily isolating an antibody gene and transferring the gene to an appropriate host to prepare an antibody, an appropriate combination of a host and an expression vector can be used. Specific examples of the antibody gene can include combinations of genes encoding the heavy chain and light chain sequences of the antibodies described in the present specification. For the transformation of host cells, such a heavy chain sequence gene and a light chain sequence gene may be inserted into the same expression vector or may be inserted into separate expression vectors.

In the case of using eukaryotic cells as the host, animal cells, plant cells, or eukaryotic microbes can be used. Particularly, examples of the animal cells can include mammalian cells, for example, COS cells which are monkey cells (Gluzman, Y., Cell (1981) 23, p. 175-182, ATCC CRL-1650), mouse fibroblasts NIH3T3 (ATCC No. CRL-1658), a dihydrofolate reductase-deficient cell line of Chinese hamster ovary cells (CHO cells, ATCC CCL-61) (Urlaub, G. and Chasin, L. A. Proc. Natl. Acad. Sci. U.S.A. (1980) 77, p. 4126-4220), and FreeStyle 293F cells (Invitrogen Corp.).

In the case of using prokaryotic cells, examples thereof can include *E. coli* and *Bacillus subtilis.*

The antibody gene of interest is transferred to these cells by transformation, and the transformed cells are

cultured *in vitro* to obtain an antibody. In this culture, yields may differ depending on the sequence of the antibody. Thus, an antibody that is easily produced as a medicament can be selected from among antibodies having equivalent binding activity by using the yield as an index. Accordingly, the antibody of the present invention also includes an antibody obtainable by a method for producing the antibody, comprising the steps of: culturing the transformed host cells; and collecting the antibody of interest or an antigen binding fragment of the antibody from the cultures obtained in the preceding step.

It is known that a lysine residue at the carboxyl terminus of the heavy chain of an antibody produced in a cultured mammalian cell is deleted (Journal of Chromatography A, 705: 129-134 (1995)), and it is also known that two amino acid residues (glycine and lysine) at the carboxyl terminus of the heavy chain of an antibody produced in a cultured mammalian cell are deleted and a proline residue newly located at the carboxyl terminus is amidated (Analytical Biochemistry, 360: 75-83 (2007)). However, such deletion and modification of the heavy chain sequence do not affect the antigen-binding affinity and the effector function (the activation of complement, antibody-dependent cellular cytotoxicity, etc.) of the antibody. Therefore, in the antibody according to the present invention, antibodies subjected to such modification and antigen binding fragments of the antibody are also included, and deletion variants in which one or two amino acids have been deleted at the carboxyl terminus of the heavy chain, variants obtained by amidation of deletion variants (for example, a heavy chain in which the carboxyl terminal proline residue has been amidated), and the like are also included. The type of deletion variant having a deletion at the carboxyl terminus of the heavy chain of the antibody according to the present invention is not limited to the above variants as long as the antigen-binding affinity and the effector function are conserved. The two heavy chains constituting the antibody according to the present invention may be of one type selected from the group consisting of a full-length heavy chain and the above-described deletion variant, or may be of two types in combination selected therefrom. The ratio of the amount of each deletion variant can be affected by the type of cultured mammalian cells which produce the antibody according to the present invention and the culture conditions. Examples of the main component of the antibody according to the present invention can include two heavy chains in which one amino acid is deleted from each of the carboxyl termini of both the heavy chains.

Examples of the isotype of the antibody of the present invention can include IgG (IgG1, IgG2, IgG3, and IgG4) and can preferably include IgG1 and IgG4. In the case of using IgG1 as the isotype of the antibody of the present invention, the IgG1 antibody may have a mutation. Its effector functions may be adjusted by the partial substitution of constant region amino acid residues (see WO88/007089, WO94/28027, and WO94/29351). Examples of the IgG1 mutant having attenuated effector functions include IgG1 LALA (IgG1-L234A and -L235A) and IgG1 LALA-PA (IgG1-L234A, -L235A, and -P329A).

Examples of the biological activity of the antibody can generally include antigen binding activity, activity of being internalized in antigen-expressing cells by binding to the antigen, activity of neutralizing the activity of an antigen, activity of enhancing the activity of an antigen, antibody-dependent cellular cytotoxic (ADCC) activity, complement-dependent cytotoxic (CDC) activity, and antibody-dependent cell-mediated phagocytosis (ADCP). The function of the antibody according to the present invention is binding activity to CD25. Preferably, the antibody of the present invention has no ability to block IL-2 and has activity of being internalized in CD25-expressing cells by binding to CD25. Further, the antibody of the present invention may have ADCC activity, CDC activity, and/or ADCP activity in addition to the cell internalization activity.

The obtained antibody can be purified until homogenous. The antibody can be separated and purified by use of protein separation and purification methods usually used. For example, column chromatography, filtration, ultrafiltration, salting-out, dialysis, preparative polyacrylamide gel electrophoresis, and isoelectric focusing can be appropriately selected or combined to separate and purify the antibody (Strategies for Protein Purification and Characterization: A Laboratory Course Manual, Daniel R.Marshak et al. eds., Cold Spring Harbor Laboratory Press (1996); Antibodies: A Laboratory Manual. Ed Harlow and David Lane, Cold Spring Harbor Laboratory (1988)) though the separation and purification methods are not limited thereto.

Examples of the chromatography can include affinity chromatography, ion exchange chromatography, hydrophobic chromatography, gel filtration chromatography, reverse phase chromatography, and adsorption chromatography.

These chromatographic techniques can be performed using liquid chromatography such as HPLC or FPLC.

Examples of the column for use in the affinity chromatography can include protein A columns and protein G columns. Examples of the column using the protein A column can include Hyper D, POROS, and Sepharose F. F. (Pharmacia).

Alternatively, the antibody may be purified by using an antigen-immobilized carrier and exploiting the binding activity of the antibody against the antigen.

3. Anti-CD25 antibody-drug conjugate

(1) Drug

The anti-CD25 antibody obtained in "2. Production of anti-CD25 antibody" described above can be conjugated to a drug via a linker structure to prepare an anti-CD25 antibody-drug conjugate. The drug is not particularly limited as long as the drug has a substituent or a partial structure that can be connected to a linker structure. The anti-CD25 antibody-drug conjugate can be used for various purposes depending on the drug to be conjugated. Examples of such a drug can include substances having cytotoxic activity (including cytotoxic active compounds), substances having antitumor activity, chemotherapeutics, molecular targeting drugs, immunoactivators, immunosuppressants, toxins, photosensitive substances, drugs for diagnosis, proteins, peptides, amino acids, nucleic acids, antigens, vitamins, hormones, substances effective for blood diseases, substances effective for autoimmune diseases, antiinflammatory substances, antibacterial substances, antifungal substances, antiparasitic substances, antiviral substances, and anti-anesthetic substances.

(1)-1 Cytotoxic active compound

An example using a cytotoxic active compound as the compound to be conjugated in the anti-CD25 antibody-drug conjugate of the present invention will be described below. An anti-CD25 antibody-drug conjugate comprising the anti-CD25 antibody of the present invention conjugated to a drug that exerts toxicity in cells via a linker having a particular structure is capable of exhibiting an effect of directly killing cancer cells expressing CD25. The cytotoxic active compound is not particularly limited as long as the compound has a cytotoxic effect and has a substituent or a partial structure that can be connected to a linker structure. The cytotoxic effect of the cytotoxic active compound is exerted by the cleavage of a portion or the whole of the linker in blood cells and in tumor cells, through which the cytotoxic active compound (which may contain a portion of the linker) is then released. It is preferred that the linker should be cleaved at a connecting position with the drug because the cytotoxic active compound is released in its original structure to exert its original cytotoxic effect.

The anti-CD25 antibody obtained in "2. Production of anti-CD25 antibody" described above can be conjugated to the cytotoxic active compound via a linker structure to prepare an anti-CD25 antibody-drug conjugate.

As one example of the cytotoxic active compound used in the present invention, exatecan, a camptothecin derivative ((1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione represented by the following formula) can be preferably used.

[Formula 12]

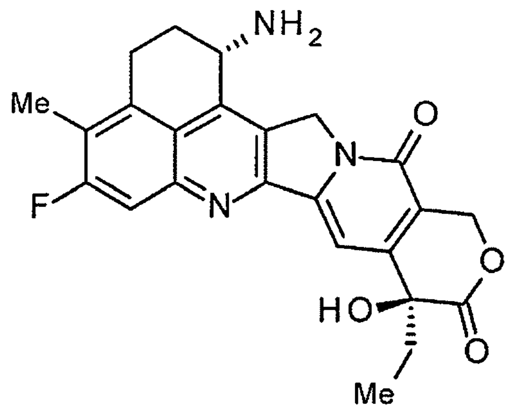

The compound can be easily obtained by, for example, a method described in U.S. Patent Publication No. US2016/0297890 or other known methods, and the amino group at the 1-position can be preferably used as a connecting position to the linker structure. Further, exatecan may be released in cells while a portion of the linker is still attached thereto. However, the compound exerts an excellent cytotoxic effect even in such a state.

Since exatecan has a camptothecin structure, it is known that the equilibrium shifts to a structure with a formed lactone ring (closed ring) in an acidic aqueous medium (e.g., on the order of pH 3) whereas the equilibrium shifts to a structure with an opened lactone ring (open ring) in a basic aqueous medium (e.g., on the order of pH 10). A drug conjugate into which exatecan residues corresponding to such a closed ring structure and an open ring structure have been introduced is also expected to have an equivalent cytotoxic effect, and it is needless to say that any of such drug conjugates is included in the scope of the present invention.

Other examples of the cytotoxic active compound can include antitumor compounds described in the literature (Pharmacological Reviews, 68, p. 3-19, 2016). Specific examples thereof can include doxorubicin, calicheamicin, dolastatin 10, auristatins such as monomethyl auristatin E (MMAE) and monomethyl auristatin F (MMAF), maytansinoids

such as DM1 and DM4, a pyrrolobenzodiazepine dimer SG2000 (SJG-136), a camptothecin derivative SN-38, duocarmycins such as CC-1065, amanitin, daunorubicin, mitomycin C, bleomycin, cyclocytidine, vincristine, vinblastine, methotrexate, platinum-based antitumor agents (cisplatin and derivatives thereof), and Taxol and derivatives thereof.

In the antibody-drug conjugate, the number of conjugated drug molecules per antibody molecule is a key factor having an influence on the efficacy and safety thereof. The production of the antibody-drug conjugate is carried out by specifying reaction conditions such as the amounts of starting materials and reagents used for reaction, so as to attain a constant number of conjugated drug molecules. Unlike the chemical reaction of a low-molecular-weight compound, a mixture containing different numbers of conjugated drug molecules is usually obtained. The number of conjugated drug molecules per antibody molecule is defined and indicated as an average value, i.e., the average number of conjugated drug molecules. Unless otherwise specified, i.e., except in the case of representing an antibody-drug conjugate having a particular number of conjugated drug molecules that is included in an antibody-drug conjugate mixture having different numbers of conjugated drug molecules, the number of conjugated drug molecules according to the present invention also means an average value as a rule. The number of exatecan molecules conjugated to an antibody molecule is controllable, and as an average number of conjugated drug molecules per antibody, approximately 1 to 10 exatecan molecules can be conjugated. The number of exatecan molecules is preferably 2 to 8, 3 to 8, 4 to 8, 5 to 8, 6 to 8, or 7 to 8, more preferably 5 to 8, further preferably 7 to 8, still further preferably 8. Those skilled in the art can design a reaction for conjugating a required number of drug molecules to an antibody molecule based on the description of Examples of the present application, and can obtain an antibody-drug conjugate with a controlled number of conjugated exatecan molecules.

(2) Linker structure

The linker structure which conjugates the drug to the anti-CD25 antibody in the anti-CD25 antibody-drug conjugate of the present invention will be described.

In the antibody-drug conjugate of the present application, the linker structure which conjugates the anti-CD25 antibody to the drug is not particularly limited as long as the resulting antibody-drug conjugate can be used. The linker structure may be appropriately selected and used according to the purpose of use. One example of the linker structure can include a linker described in a known literature (Pharmacol Rev 68: 3-19, January 2016, Protein Cell DOI 10.1007/s13238-016-0323-0, etc.). Further specific examples thereof can include VC (valine-citrulline), MC (maleimidocaproyl), SMCC (succinimidyl 4-(N-maleimidomethyl) cyclohexane-1-carboxylate), SPP (N-succinimidyl 4-(2-pyridyldithio)pentanoate, SS (disulfide), SPDB (N-succinimidyl 4-(2-pyridyldithio)butyrate, SS/hydrazone, hydrazone, and carbonate.

Another example thereof can include a linker structure described in U.S. Patent Publication No. US2016/0297890 (as one example, those described in the paragraphs [0260] to [0289]). Any linker structure given below can be preferably used. The left end of the structure shown below is a connecting position to the antibody, and the right end thereof is a connecting position to the drug. Furthermore, GGFG in the linker structures given below represents an amino acid sequence consisting of glycine-glycine-phenylalanine-glycine (GGFG) linked through peptide bonds.

- (Succinimid-3-yl-N)-$CH_2CH_2$-C(=O)-GGFG-NH-$CH_2CH_2CH_2$-C(=O)-,

- (Succinimid-3-yl-N)-$CH_2CH_2CH_2CH_2CH_2$-C(=O)-GGFG-NH-$CH_2CH_2CH_2$-C(=O) -,

- (Succinimid-3-yl-N)-$CH_2CH_2CH_2CH_2CH_2$-C(=O) -GGFG-NH-$CH_2$-O-$CH_2$-C(=O) -,

- (Succinimid-3-yl-N)-$CH_2CH_2CH_2CH_2CH_2$-C(=O)-GGFG-NH-$CH_2CH_2$-O-$CH_2$-C(=O) -,

- (Succinimid-3-yl-N)-$CH_2CH_2$-C(=O) -NH-$CH_2CH_2O$-$CH_2CH_2O$-$CH_2CH_2$-C(=O)-GGFG-NH-$CH_2CH_2CH_2$-C(=O)-,

- (Succinimid-3-yl-N)-$CH_2CH_2$-C(=O) -NH-$CH_2CH_2O$-$CH_2CH_2O$-$CH_2CH_2O$-$CH_2CH_2O$-$CH_2CH_2$-C(=O) -GGFG-NH-$CH_2CH_2CH_2$-C(=O)-

More preferred are the following:

- (Succinimid-3-yl-N)-$CH_2CH_2CH_2CH_2CH_2$-C(=O) -GGFG-NH-$CH_2$-O-$CH_2$-C(=O) -,

- (Succinimid-3-yl-N)-$CH_2CH_2CH_2CH_2CH_2$-C(=O) -GGFG-NH-$CH_2CH_2$-O-$CH_2$-C(=O) -,

- (Succinimid-3-yl-N)-$CH_2CH_2$-C(=O) -NH-$CH_2CH_2O$-$CH_2CH_2O$-$CH_2CH_2$-C(=O)-GGFG-NH-$CH_2CH_2CH_2$-C

(=O)-

Still more preferred are the following:

- (Succinimid-3-yl-N)-$CH_2CH_2CH_2CH_2CH_2$-C(=O) -GGFG-NH-$CH_2$-O-$CH_2$-C(=O)-,

- (Succinimid-3-yl-N)-$CH_2CH_2$-C(=O) -NH-$CH_2CH_2O$-$CH_2CH_2O$-$CH_2CH_2$-C(=O)-GGFG-NH-$CH_2CH_2CH_2$-C(=O)-.

The antibody is connected to the end of - (Succinimid-3-yl-N)(e.g., an end opposite (left end) to the end to which (-$CH_2CH_2CH_2CH_2CH_2$- is connected in "-(Succinimid-3-yl-N)-$CH_2CH_2CH_2CH_2CH_2$-C(=O) -GGFG-NH-$CH_2$-O-$CH_2$-C(=O)-") is connected), and the cytotoxic active compound is connected to an end opposite to the end to which -(Succinimid-3-yl-N) is connected (the carbonyl group of $CH_2$-O-$CH_2$-C(=O)- at the right end in the example described above. "-(Succinimid-3-yl-N)-" has a structure represented by the following formula:

[Formula 13]

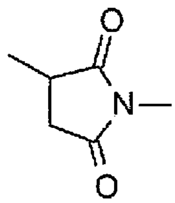

The 3-position of this partial structure is the connecting position to the anti-CD25 antibody. This connection to the antibody at the 3-position is characterized by forming a thioether bond. The nitrogen atom at the 1-position of this structure moiety is connected to the carbon atom of methylene which is present within the linker including the structure.

In the antibody-drug conjugate of the present invention having exatecan as the drug, a drug-linker structure moiety having any structure given below is preferred for the conjugation to the antibody or the antigen binding fragment of the antibody (preferably the antibody). For these drug-linker structure moieties, the average conjugated number per antibody or per antigen binding fragment of the antibody may be 1 to 10 and is preferably 2 to 8, more preferably 5 to 8, further preferably 7 to 8, still further preferably 8.

- (Succinimid-3-yl-N)-$CH_2CH_2$-C(=O) -GGFG-NH-$CH_2CH_2CH_2$-C(=O)-(NH-DX),

- (Succinimid-3-yl-N)-$CH_2CH_2CH_2CH_2CH_2$-C(=O) -GGFG-NH-$CH_2CH_2CH_2$-C(=O) - (NH-DX),

- (Succinimid-3-yl-N)-$CH_2CH_2CH_2CH_2CH_2$-C(=O) -GGFG-NH-$CH_2$-O-$CH_2$-C(=O) - (NH-DX),

- (Succinimid-3-yl-N)-$CH_2CH_2CH_2CH_2CH_2$-C(=O) -GGFG-NH-$CH_2CH_2$-O-$CH_2$-C(=O)-(NH-DX),

- (Succinimid-3-yl-N)-$CH_2CH_2$-C(=O) -NH-$CH_2CH_2O$-$CH_2CH_2O$-$CH_2CH_2$-C(=O) -GGFG-NH-$CH_2CH_2CH_2$-C(=O) - (NH-DX),

- (Succinimid-3-yl-N)-$CH_2CH_2$-C(=O) -NH-$CH_2CH_2O$-$CH_2CH_2O$-$CH_2CH_2O$-$CH_2CH_2O$-$CH_2CH_2$-C(=O) -GGFG-NH-$CH_2CH_2CH_2$-C(=O)-(NH-DX)

More preferred are the following:

- (Succinimid-3-yl-N)-$CH_2CH_2CH_2CH_2CH_2$-C(=O) -GGFG-NH-$CH_2$-O-$CH_2$-C(=O) - (NH-DX),

- (Succinimid-3-yl-N)-$CH_2CH_2CH_2CH_2CH_2$-C(=O) -GGFG-NH-$CH_2CH_2$-O-$CH_2$-C(=O)-(NH-DX),

- (Succinimid-3-yl-N)-$CH_2CH_2$-C(=O) -NH-$CH_2CH_2O$-$CH_2CH_2O$-$CH_2CH_2$-C(=O) -GGFG-NH-$CH_2CH_2CH_2$-C(=O)-(NH-DX)

Further preferred is the following:

- (Succinimid-3-yl-N)-$CH_2CH_2CH_2CH_2CH_2$-C(=O) -GGFG-NH-$CH_2$-O-$CH_2$-C(=O) - (NH-DX),

- (Succinimid-3-yl-N)-$CH_2CH_2$-C(=O) -NH-$CH_2CH_2O$-$CH_2CH_2O$-$CH_2CH_2$-C(=O) -GGFG-NH-$CH_2CH_2CH_2$-C(=O)-(NH-DX)

-(NH-DX) has a structure represented by the following formula:

[Formula 14]

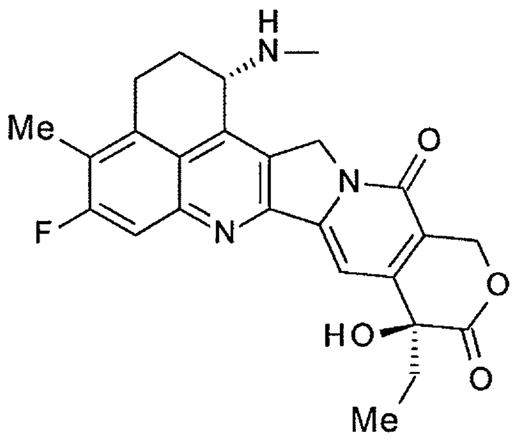

and represents a group that is derived by removing one hydrogen atom of the amino group at the 1-position of exatecan.

In an embodiment, the linker structure which conjugates the anti-CD25 antibody to the drug in the antibody-drug conjugate of the present application may have the following structure.

-(Succinimid-3-yl-N)-$(CH_2)n^1$-C(=O)-L-GGFG-NH-X-C(=O)-

wherein $n^1$ represents an integer of 2 to 8,

L represents -NH-$(CH_2$-$CH_2$-O)$n^2$-$CH_2$-$CH_2$- or a single bond,
$n^2$ represents an integer of 1 to 6, and
X represents a linear or branched alkylene group having 1 to 20 carbon atoms (preferably 1 to 10 carbon atoms) (one or two or more methylene groups in the chain are optionally replaced with an oxygen atom or a sulfur atom), the alkylene group optionally having a cyclic saturated hydrocarbon group having 1 to 6 carbon atoms (e.g., a cyclopropyl group, a cyclobutyl group, a cyclopentanyl group, a cyclohexanyl group, a cis-1,3-cyclobutylene group, a trans-1,3-cyclobutylene group, a cis-1,2-cyclopropylene group, a trans-1,2-cyclopropylene group, a cis-1,4-cyclohexylene group, or a trans-1,4-cyclohexylene group) as a portion of the structure thereof.

The connecting position to the antibody, the connecting position to the drug, GGFG, and "-(Succinimid-3-yl-N)-" are as described above.

In an embodiment, the average number of conjugated drug linker molecules per antibody molecule in the anti-CD25 antibody-drug conjugate used in the present invention is preferably 1 to 10, more preferably 2 to 8, still more preferably 5 to 8, still more preferably 7 to 8, still more preferably 7.5 to 8, still more preferably approximately 8.

In an alternative embodiment, the number of conjugated drug molecules or drug linker molecules per antibody molecule in the anti-CD25 antibody-drug conjugate used in the present invention is preferably an integer within the range of 2 to 8, more preferably 2, 4, 6, or 8, still more preferably 8.

(3) Method for producing antibody-drug conjugate

The antibody that can be used in the antibody-drug conjugate of the present invention is not particularly limited as long as the antibody is the anti-CD25 antibody or the antigen binding fragment of the antibody having internalization activity, described in the above section "2. Production of anti-CD25 antibody" and Examples.

Next, typical methods for producing the antibody-drug conjugate of the present invention will be described. In the

description below, compound No. shown in each reaction scheme is used to represent a compound. Specifically, each compound is referred to as a "compound of the formula (1)", "compound (1)", or the like. The same holds true for the other compound Nos.

(3)-1 Production method 1

The antibody-drug conjugate represented by the formula (1) given below in which the anti-CD25 antibody is connected to the linker structure via thioether can be produced by reacting an antibody having a sulfhydryl group converted from a disulfide bond by the reduction of the anti-CD25 antibody, with the compound (2) obtainable by a known method (e.g., obtainable by a method described in the patent publication literature US2016/297890 (e.g., a method described in the paragraphs [0336] to [0374])). This antibody-drug conjugate can be produced by the following method, for example.

[Expression 1]

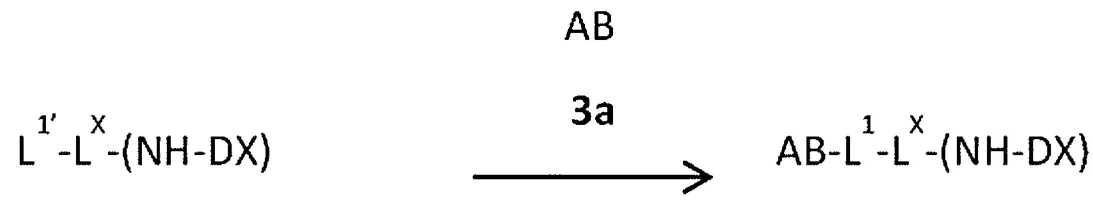


(2) (1)

wherein AB represents an antibody with a sulfhydryl group.

In this context, $L^1$ has a structure represented by -(Succinimid-3-yl-N)-.

$L^{1'}$ represents a maleimidyl group represented by the following formula:

[Formula 15]

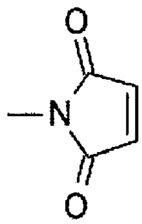

$L^1$-$L^X$ has a structure represented by any of the following formulas:

- (Succinimid-3-yl-N)-$CH_2CH_2$-C(=O) -GGFG-NH-$CH_2CH_2CH_2$- C(=O)-,

- (Succinimid-3-yl-N)-$CH_2CH_2CH_2CH_2CH_2$-C(=O) -GGFG-NH-$CH_2CH_2CH_2$-C(=O) -,

- (Succinimid-3-yl-N)-$CH_2CH_2CH_2CH_2CH_2$-C(=O) -GGFG-NH-$CH_2$-O-$CH_2$-C(=O) -,

- (Succinimid-3-yl-N)-$CH_2CH_2CH_2CH_2CH_2$-C(=O) -GGFG-NH-$CH_2CH_2$-O-$CH_2$-C(=O) -,

- (Succinimid-3-yl-N)-$CH_2CH_2$-C(=O) -NH-$CH_2CH_2O$-$CH_2CH_2O$-$CH_2CH_2$-C(=O)-GGFG-NH-$CH_2CH_2CH_2$-C(=O)-,

- (Succinimid-3-yl-N)-$CH_2CH_2$-C(=O) -NH-$CH_2CH_2O$-$CH_2CH_2O$-$CH_2CH_2O$-$CH_2CH_2O$-$CH_2CH_2$-C(=O) -GGFG-NH-$CH_2CH_2CH_2$-C(=O)-

Among them, more preferred are the following:

- (Succinimid-3-yl-N)-$CH_2CH_2CH_2CH_2CH_2$-C(=O) -GGFG-NH-$CH_2$-O-$CH_2$-C(=O) -,

- (Succinimid-3-yl-N)-$CH_2CH_2CH_2CH_2CH_2$-C(=O) -GGFG-NH-$CH_2CH_2$-O-$CH_2$-C(=O) -,

- (Succinimid-3-yl-N)-$CH_2CH_2$-C(=O) -NH-$CH_2CH_2$O-$CH_2CH_2$O-$CH_2CH_2$-C(=O)-GGFG-NH-$CH_2CH_2CH_2$-C(=O)-

Further preferred are the following:

- (Succinimid-3-yl-N)-$CH_2CH_2CH_2CH_2CH_2$-C(=O) -GGFG-NH-$CH_2$-O-$CH_2$-C(=O) -,

- (Succinimid-3-yl-N)-$CH_2CH_2$-C(=O) -NH-$CH_2CH_2$O-$CH_2CH_2$O-$CH_2CH_2$-C(=O)-GGFG-NH-$CH_2CH_2CH_2$-C(=O)-

In the reaction scheme described above, the antibody-drug conjugate (1) can be interpreted as having a structure where one structure moiety from the drug to the linker end is connected to one antibody. However, this description is given for the sake of convenience, and there are actually many cases in which a plurality of the aforementioned structure moieties is connected to one antibody molecule. The same holds true for the explanation of the production method described below.

Specifically, the antibody-drug conjugate (1) can be produced by reacting the compound (2) obtainable by a known method (e.g., obtainable by a method described in the patent publication literature US2016/297890 (e.g., a method described in the paragraphs [0336] to [0374])), with the antibody (3a) having a sulfhydryl group.

The antibody (3a) having a sulfhydryl group can be obtained by a method well known to those skilled in the art (Hermanson, G.T, Bioconjugate Techniques, pp.56-136, pp.456-493, Academic Press (1996)) Examples of the method can include, but are not limited to: Traut's reagent is reacted with the amino group of the antibody; N-succinimidyl S-acetylthioalkanoates are reacted with the amino group of the antibody followed by reaction with hydroxylamine; N-succinimidyl 3-(pyridyldithio)propionate is reacted with the antibody, followed by reaction with a reducing agent; the antibody is reacted with a reducing agent such as dithiothreitol, 2-mercaptoethanol, or tris(2-carboxyethyl)phosphine hydrochloride (TCEP) to reduce the interchain disulfide bond in the antibody, so as to form a sulfhydryl group.

Specifically, an antibody with interchain disulfide bonds partially or completely reduced can be obtained by using 0.3 to 3 molar equivalents of TCEP as a reducing agent per interchain disulfide bond in the antibody, and reacting the reducing agent with the antibody in a buffer solution containing a chelating agent. Examples of the chelating agent can include ethylenediaminetetraacetic acid (EDTA) and diethylenetriaminepentaacetic acid (DTPA). The chelating agent can be used at a concentration of 1 mM to 20 mM. A solution of sodium phosphate, sodium borate, sodium acetate, or the like can be used as the buffer solution. As a specific example, the antibody (3a) having partially or completely reduced sulfhydryl groups can be obtained by reacting the antibody with TCEP at 4°C to 37°C for 1 to 4 hours.

By carrying out an addition reaction of a sulfhydryl group to a drug-linker moiety, the drug-linker moiety can be conjugated through a thioether bond.

Then, using 2 to 20 molar equivalents of the compound (2) per antibody (3a) having a sulfhydryl group, the antibody-drug conjugate (1) in which 2 to 8 drug molecules are conjugated per antibody can be produced. Specifically, a solution containing the compound (2) dissolved therein may be added to a buffer solution containing the antibody (3a) having a sulfhydryl group for the reaction. In this context, a sodium acetate solution, sodium phosphate, sodium borate, or the like can be used as the buffer solution. pH for the reaction is 5 to 9, and more preferably, the reaction may be performed near pH 7. An organic solvent such as dimethyl sulfoxide (DMSO), dimethylformamide (DMF), dimethylacetamide (DMA), or N-methyl-2-pyrrolidone (NMP) can be used as a solvent for dissolving the compound (2). The reaction may be performed by adding the solution containing the compound (2) dissolved in the organic solvent at 1 to 20% v/v to a buffer solution containing the antibody (3a) having a sulfhydryl group. The reaction temperature is 0 to 37°C, more preferably 10 to 25°C, and the reaction time is 0.5 to 2 hours. The reaction can be terminated by deactivating the reactivity of unreacted compound (2) with a thiol-containing reagent. The thiol-containing reagent is, for example, cysteine or N-acetyl-L-cysteine (NAC). More specifically, the reaction can be terminated by adding 1 to 2 molar equivalents of NAC to the compound (2) used, and incubating the obtained mixture at room temperature for 10 to 30 minutes.

(4) Identification of antibody-drug conjugate

The produced antibody-drug conjugate can be subjected to concentration, buffer exchange, purification, and measurement of antibody concentration and an average number of conjugated drug molecules per antibody molecule in accordance with common procedures described below, to identify the antibody-drug conjugate (1).

(4)-1 Common procedure A: Concentration of aqueous solution of antibody or antibody-drug conjugate

A solution of an antibody or an antibody-drug conjugate is added to Amicon Ultra (50,000 MWCO, Millipore Corporation) container, and the solution of the antibody or the antibody-drug conjugate is concentrated by centrifugation (centrifugation at 2000 G to 3800 G for 5 to 20 minutes) using a centrifuge (Allegra X-15R, Beckman Coulter, Inc.).

(4)-2 Common procedure B: Measurement of antibody concentration

Using a UV detector (Nanodrop 1000, Thermo Fisher Scientific Inc.), measurement of the antibody concentration is carried out in accordance with the method defined by the manufacturer. In this respect, 280 nm absorption coefficient differing among antibodies (1.3 $mLmg^{-1}cm^{-1}$ to 1.8 $mLmg^{-1}cm^{-1}$) is used.

(4)-3 Common procedure C: Buffer exchange for antibody

NAP-25 column (Cat. No. 17-0852-02, GE Healthcare Japan Corporation) using Sephadex G-25 carrier is equilibrated with a phosphate buffer (50 mM, pH 6.0) (in the present specification, referred to as PBS6.0/EDTA) containing sodium chloride (50 mM) and EDTA (2 mM) in accordance with the method defined by the manufacturer. An aqueous solution of the antibody is applied in an amount of 2.5 mL per NAP-25 column, and a fraction (3.5 mL) eluted with 3.5 mL of PBS6.0/EDTA is then collected. This fraction was concentrated by the common procedure A. After measurement of the concentration of the antibody using the common procedure B, the antibody concentration is adjusted to 20 mg/mL using PBS6.0/EDTA.

(4)-4 Common procedure D: Purification of antibody-drug conjugate

NAP-25 column is equilibrated with any commercially available buffer solution such as an acetate buffer containing sorbitol (5%) (10 mM, pH 5.5; in the present specification, referred to as ABS). An aqueous reaction solution of the antibody-drug conjugate (approximately 2.5 mL) is applied to the NAP-25 column, and elution is then carried out with the buffer solution in an amount defined by the manufacturer, so as to collect an antibody fraction. A gel filtration purification process of applying the collected fraction again to the NAP-25 column, and performing elution with the buffer solution is repeated a total of 2 or 3 times to obtain the antibody-drug conjugate excluding non-conjugated drug linker and low-molecular-weight compounds (tris(2-carboxyethyl)phosphine hydrochloride (TCEP), N-acetyl-L-cysteine (NAC), and dimethyl sulfoxide).

(4)-5 Common procedure E: Measurement of antibody concentration in antibody-drug conjugate and average number of conjugated drug molecules per antibody molecule

The conjugated drug concentration in the antibody-drug conjugate can be calculated by measuring UV absorbance of an aqueous solution of the antibody-drug conjugate at two wavelengths of 280 nm and 370 nm, and then performing a calculation shown below.

The total absorbance at any given wavelength is equal to the sum of the absorbance of all light-absorbing chemical species that are present in a system [additivity of absorbance]. Therefore, based on the hypothesis that the molar absorption coefficients of the antibody and the drug do not vary between before and after conjugation between the antibody and the drug, the antibody concentration and the drug concentration in the antibody-drug conjugate are represented by the following equations.

$$A_{280} = A_{D,280} + A_{A,280} = \varepsilon_{D,280}C_D + \varepsilon_{A,280}C_A \quad \text{Equation (1)}$$

$$A_{370} = A_{D,370} + A_{A,370} = \varepsilon_{D,370}C_D + \varepsilon_{A,370}C_A \quad \text{Equation (2)}$$

In this context, $A_{280}$ represents the absorbance of an aqueous solution of the antibody-drug conjugate at 280 nm, $A_{370}$ represents the absorbance of an aqueous solution of the antibody-drug conjugate at 370 nm, $A_{A,280}$ represents the absorbance of the antibody at 280 nm, $A_{A,370}$ represents the absorbance of the antibody at 370 nm, $A_{D,280}$ represents the absorbance of a conjugate precursor at 280 nm, $A_{D,370}$ represents the absorbance of a conjugate precursor at 370 nm, $\varepsilon_{A,280}$ represents the molar absorption coefficient of the antibody at 280 nm, $\varepsilon_{A,370}$ represents the molar absorption coefficient of the antibody at 370 nm, $\varepsilon_{D,280}$ represents the molar absorption coefficient of a conjugate precursor at 280 nm, $\varepsilon_{D,370}$ represents the molar absorption coefficient of a conjugate precursor at 370 nm, $C_A$ represents the antibody concentration in the antibody-drug conjugate, and $C_D$ represent the drug concentration in the antibody-drug conjugate.

In this context, with regard to $\varepsilon_{A,280}$, $\varepsilon_{A,370}$, $\varepsilon_{D,280}$, and $\varepsilon_{D,370}$, preliminarily prepared values (estimated values based on calculation or measurement values obtained by UV measurement of the compound) are used. For example, $\varepsilon_{A,280}$ can be estimated from the amino acid sequence of the antibody by a known calculation method (Protein Science, 1995, vol. 4, 2411-2423). $\varepsilon_{A,370}$ is generally zero. $\varepsilon_{D,280}$ and $\varepsilon_{D,370}$ can be obtained according to the Lambert-Beer's law

(Absorbance = Molar concentration × Molar absorption coefficient × Cell path length) by measuring the absorbance of a solution in which the conjugate precursor used is dissolved at a certain molar concentration. $C_A$ and $C_D$ can be determined by measuring $A_{280}$ and $A_{370}$ of an aqueous solution of the antibody-drug conjugate, and then solving the simultaneous equations (1) and (2) by substitution of these values. Further, by diving $C_D$ by $C_A$, the average number of conjugated drug molecules per antibody can be determined.

(4)-6 Common procedure F: Measurement of average number of conjugated drug molecules per antibody molecule in antibody-drug conjugate - (2)

The average number of conjugated drug molecules per antibody molecule in the antibody-drug conjugate can also be determined by high-performance liquid chromatography (HPLC) analysis using the following method, in addition to "(4)-5 Common procedure E" mentioned above. Hereinafter, the method for measuring the average number of conjugated drug molecules by HPLC when the antibody is conjugated to the drug linker by a disulfide bond will be described. Those skilled in the art are capable of appropriately measuring the average number of conjugated drug molecules by HPLC, depending on the connecting manner between the antibody and the drug linker, with reference to this method.

F-1. Preparation of sample for HPLC analysis

(Reduction of antibody-drug conjugate)

An antibody-drug conjugate solution (approximately 1 mg/mL, 60 μL) is mixed with an aqueous solution of dithiothreitol (DTT) (100 mM, 15 μL). By incubating the mixture at 37°C for 30 minutes, the disulfide bond between the light chain and heavy chain of the antibody-drug conjugate is cleaved. The resulting sample is used in HPLC analysis.

F-2. HPLC analysis

The HPLC analysis is conducted under the following measurement conditions.

HPLC system: Agilent 1290 HPLC system (Agilent Technologies, Inc.)
Detector: Ultraviolet absorption spectrometer (measurement wavelength: 280 nm)
Column: ACQUITY UPLC BEH Phenyl (2.1 × 50 mm, 1.7 μm, 130 angstroms; Waters Corp., P/N 186002884)
Column temperature: 80°C
Mobile phase A: Aqueous solution containing 0.10% trifluoroacetic acid (TFA) and 15% 2-propanol
Mobile phase B: Acetonitrile solution containing 0.075% TFA and 15% 2-propanol
Gradient program: 14%-36% (0 min-15 min), 36%-80% (15 min-17 min), 80%-14% (17 min-17.01 min.), and 14% (17.01 min-25 min)
Sample injection: 10 μL

F-3. Data analysis

F-3-1. Compared with non-conjugated antibody light (L0) and heavy (H0) chains, a light chain bound to drug molecule(s) (light chain bound to i drug molecule(s): $L_i$) and a heavy chain bound to drug molecule(s) (heavy chain bound to i drug molecule(s): $H_i$) exhibit higher hydrophobicity in proportion to the number of conjugated drug molecules and thus have a longer retention time. These chains are therefore eluted in the order of, for example, L0 and L1 or H0, H1, H2, and H3. Detection peaks can be assigned to any of L0, L1, H0, H1, H2, and H3 by the comparison of retention times with L0 and H0. The number of conjugated drug molecules can be defined by those skilled in the art, but is preferably L0, L1, H0, H1, H2, and H3.

F-3-2. Since the drug linker has UV absorption, peak area values are corrected in response to the number of conjugated drug linker molecules according to the following expression using the molar absorption coefficients of the light chain or heavy chain and the drug linker.

[Expression 2]

$$\text{Corrected value of peak area of light chain bound to i drug molecule(s) } (A_{Li}) = \text{Peak area} \times \frac{\text{Molar absorption coefficient of light chain}}{\text{Molar absorption coefficient of light chain} + \text{The number of conjugated drug molecules} \times \text{Molar absorption coefficient of drug linker}}$$

[Expression 3]

$$\text{Corrected value of peak area of heavy chain bound to i drug molecule s } (A_{Hi}) = \text{Peak area} \times \frac{\text{Molar absorption coefficient of heavy chain}}{\text{Molar absorption coefficient of heavy chain} + \text{The number of conjugated drug molecules} \times \text{Molar absorption coefficient of drug linker}}$$

In this context, a value estimated from the amino acid sequence of the light chain or heavy chain of each antibody by a known calculation method (Protein Science, 1995, vol. 4, 2411-2423) can be used as the molar absorption coefficient (280 nm) of the light chain or heavy chain of the antibody. In the case of cMAb1_hIGg1LALA, a molar absorption coefficient of 32742 and a molar absorption coefficient of 84458 can be used as estimated values for the light chain and heavy chain, respectively, according to the amino acid sequence of the antibody. The actually measured molar absorption coefficient (280 nm) of a compound in which the maleimide group has been converted to succinimide thioether by the reaction of each drug linker with mercaptoethanol or N-acetylcysteine can be used as the molar absorption coefficient (280 nm) of the drug linker. The wavelength for absorbance measurement can be appropriately set by a person skilled in the art, but is preferably a wavelength at which the peak of the antibody can be measured, and more preferably 280 nm.

F-3-3 The peak area ratio (%) of each chain is calculated for the total of the corrected values of peak areas according to the following expression.

[Expression 4]

$$\text{Peak area ratio of light chain bound to i drug molecules} = \frac{A_{Li}}{A_{L0} + A_{L1}} \times 100$$

$$\text{Peak area ratio of heavy chain bound to i drug molecule(s)} = \frac{A_{Hi}}{A_{H0} + A_{H1} + A_{H2} + A_{H3}} \times 100$$

$A_{Li}$, $A_{Hi}$ : $L_i$, Corrected values of peak areas of $L_i$ and $H_i$, respectively

F-3-4 The average number of conjugated drug molecules per antibody molecule in the antibody-drug conjugate is calculated according to the following expression.

Average number of conjugated drug molecules = ($L_0$ peak area ratio × 0 + $L_1$ peak area ratio × 1 + $H_0$ peak area ratio × 0 + $H_1$ peak area ratio × 1 + $H_2$ peak area ratio × 2 + $H_3$ peak area ratio × 3) / 100 × 2

In order to secure the amount of the antibody-drug conjugate, a plurality of antibody-drug conjugates having almost the same average number of conjugated drug molecules (e.g., on the order of ±1), which have been produced under similar conditions, can be mixed to prepare a new lot. In this case, the average number of drug molecules falls between the average numbers of drug molecules before the mixing.

One specific example of the antibody-drug conjugate of the present invention can include an antibody-drug conjugate having a structure represented by the following formula:

[Formula 16]

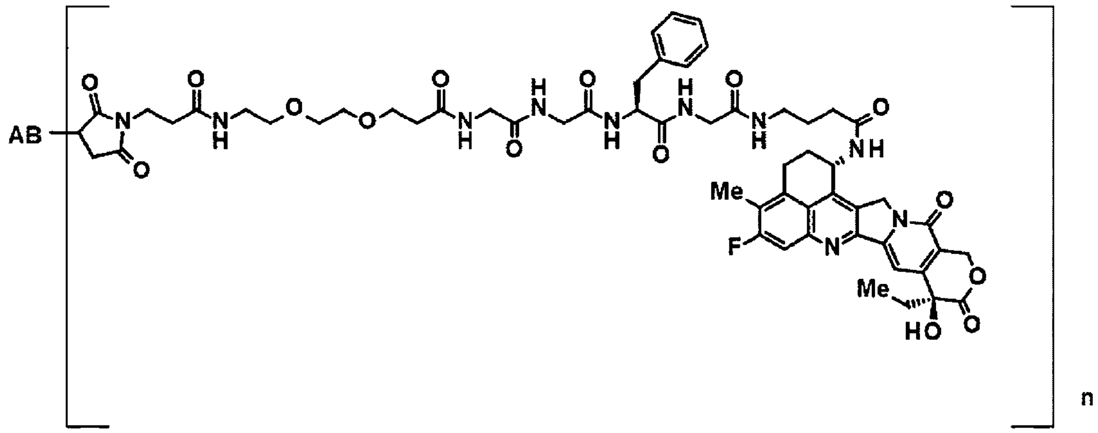

or the following formula:

[Formula 17]

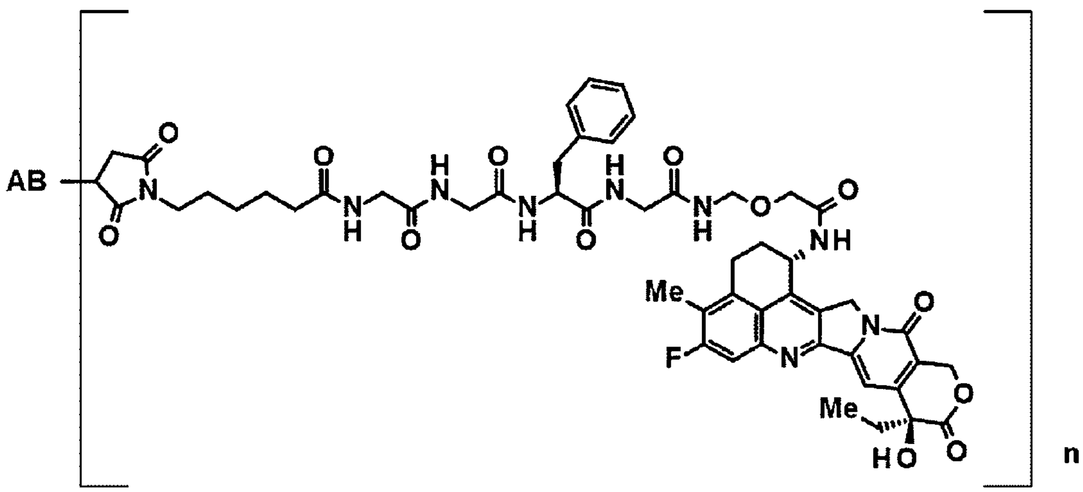

In this context, AB represents the anti-CD25 antibody disclosed in the present specification, and the antibody is conjugated to the drug linker via a sulfhydryl group derived from the antibody. In this context, n has the same meaning as that of so-called DAR (Drug-to-Antibody Ratio), and represents a drug-to-antibody ratio per antibody. Specifically, n represents the number of conjugated drug molecules per antibody molecule, which is a numeric value defined and indicated as an average value, i.e., the average number of conjugated drug molecules. AB may be the antigen binding fragment of the antibody. In this case, n represents the average number of units of the drug-linker structure conjugated to the antigen binding fragment of the antibody per antigen binding fragment of the antibody. In the case of the antibody-drug conjugate represented by [Formula 16] or [Formula 17] of the present invention, n can be 2 to 8 and is preferably 5 to 8, more preferably 7 to 8, still more preferably 8, in measurement by common procedure F.

One example of the antibody-drug conjugate of the present invention can include an antibody-drug conjugate having a structure represented by the above formula [Formula 16] or [Formula 17] wherein the antibody represented by AB comprises an antibody having a heavy chain and a light chain in any one combination selected from the group consisting of the following combinations (a) to (c), or an antigen binding fragment of the antibody, or a pharmaceutically acceptable salt of the antibody-drug conjugate:

(a) an antibody consisting of a light chain consisting of an amino acid sequence from positions 21 to 233 of SEQ ID NO: 13 and a heavy chain consisting of an amino acid sequence from positions 20 to 476 of SEQ ID NO: 15;

(b) an antibody consisting of a light chain consisting of an amino acid sequence from positions 21 to 233 of SEQ ID NO: 14 and a heavy chain consisting of an amino acid sequence from positions 20 to 476 of SEQ ID NO: 16; and
(c) the antibody according to (a) or (b) wherein the heavy chain or the light chain comprises one or two or more modifications selected from the group consisting of posttranslational modifications typified by N-linked glycosylation, O-linked glycosylation, N-terminal processing, C-terminal processing, deamidation, isomerization of aspartic acid, oxidation of methionine, addition of a methionine residue to the N terminus, amidation of a proline residue, and conversion of N-terminal glutamine or N-terminal glutamic acid to pyroglutamic acid, and deletion of one or two amino acids from the carboxyl terminus.

Another example of the antibody-drug conjugate of the present invention can include an antibody-drug conjugate having a structure represented by the above formula [Formula 16] or [Formula 17] wherein the antibody represented by AB comprises an antibody having a heavy chain variable region and a light chain variable region in any one combination selected from the group consisting of the following combinations (a) to (c), or an antigen binding fragment of the antibody, or a pharmaceutically acceptable salt of the antibody-drug conjugate:

(a) a light chain variable region consisting of an amino acid sequence from positions 21 to 127 of SEQ ID NO: 13 and a heavy chain variable region consisting of an amino acid sequence from positions 20 to 146 of SEQ ID NO: 15;
(b) a light chain variable region consisting of an amino acid sequence from positions 21 to 127 of SEQ ID NO: 14 and a heavy chain variable region consisting of an amino acid sequence from positions 20 to 146 of SEQ ID NO: 16; and
(c) the antibody according to (a) or (b) wherein the heavy chain or the light chain comprises one or two or more modifications selected from the group consisting of posttranslational modifications typified by N-linked glycosylation, O-linked glycosylation, N-terminal processing, C-terminal processing, deamidation, isomerization of aspartic acid, oxidation of methionine, addition of a methionine residue to the N terminus, amidation of a proline residue, and conversion of N-terminal glutamine or N-terminal glutamic acid to pyroglutamic acid, and deletion of one or two amino acids from the carboxyl terminus.

4. Medicament

Since the anti-CD25 antibody of the present invention and the antigen binding fragment of the antibody described in the above section "2. Production of anti-CD25 antibody" and Examples bind to CD25 on cell surface and have internalization activity, the antibody or the antigen binding fragment can be used as a medicament, particularly, as a therapeutic agent for a cancer, either singly or in combination with an additional drug.
The anti-CD25 antibody of the present invention or the antigen binding fragment of the antibody can also be used in the detection of cells expressing CD25.
Further, the anti-CD25 antibody of the present invention or the antigen binding fragment of the antibody can be applied to an antibody for an antibody-drug conjugate because of having internalization activity.
The anti-CD25 antibody-drug conjugate of the present invention described in the above section "3. Anti-CD25 antibody-drug conjugate" and Examples, when a drug having cytotoxic activity y is used as the drug, is a conjugate of the anti-CD25 antibody and/or the antigen binding fragment of the antibody having internalization activity, and the drug having cytotoxic activity. This anti-CD25 antibody-drug conjugate exhibits cytotoxic activity against Treg and cancer cells expressing CD25 and as such, can be used as a medicament, particularly, as a therapeutic agent and/or a prophylactic agent for a cancer. The anti-CD25 antibody-drug conjugate is capable of exhibiting an effect of directly killing cancer cells expressing CD25. In addition, the anti-CD25 antibody-drug conjugate is capable of exhibiting stronger ability to remove Treg and/or activity of inducing granzyme (GZMB)-positive and CD8-positive T cells than that of a conventional anti-CD25 antibody and anti-CD25 antibody-drug complex. Thus, the anti-CD25 antibody-drug conjugate can be expected to exert antitumor activity based on a Treg suppressive effect, and can be expected to achieve an excellent antitumor effect and safety by administration to patients having cancer cells expressing CD25 and cancer patients with tumor immunity suppressed by Treg.
The anti-CD25 antibody-drug conjugate of the present invention may absorb moisture or have adsorption water, for example, to turn into a hydrate when the conjugate is left in air or subjected to recrystallization or purification procedures. Such a compound and a pharmaceutically acceptable salt containing water are each also included as an embodiment of the present invention in the present invention.
The anti-CD25 antibody-drug conjugate of the present invention, when having a basic group such as an amino group, can form a pharmaceutically acceptable acid-addition salt, if desired. Examples of such an acid-addition salt can include: hydrohalides such as hydrofluoride, hydrochloride, hydrobromide, and hydroiodide; inorganic acid salts such as nitrate, perchlorate, sulfate, and phosphate; lower alkanesulfonates such as methanesulfonate, trifluoromethanesulfonate, and ethanesulfonate; arylsulfonates such as benzenesulfonate and p-toluenesulfonate; organic acid salts such as formate, acetate, trifluoroacetate, malate, fumarate, succinate, citrate, tartrate, oxalate, and maleate; and amino acid salts

such as ornithine salt, glutamate, and aspartate.

The anti-CD25 antibody-drug conjugate of the present invention, when having an acidic group such as a carboxy group, can form a pharmaceutically acceptable base-addition salt, if desired. Examples of such a base-addition salt can include: alkali metal salts such as sodium salt, potassium salt, and lithium salt; alkaline earth metal salts such as calcium salt and magnesium salt; inorganic salts such as ammonium salt; and organic amine salts such as dibenzylamine salt, morpholine salt, phenylglycine alkyl ester salt, ethylenediamine salt, N-methylglucamine salt, diethylamine salt, triethylamine salt, cyclohexylamine salt, dicyclohexylamine salt, N,N'-dibenzylethylenediamine salt, diethanolamine salt, N-benzyl-N-(2-phenylethoxy)amine salt, piperazine salt, tetramethylammonium salt, and tris(hydroxymethyl)aminomethane salt.

The present invention may also encompass an anti-CD25 antibody or an antigen binding fragment of the antibody, or an anti-CD25 antibody-drug conjugate in which one or more atoms constituting the anti-CD25 antibody or the antigen binding fragment of the antibody, or the antibody-drug conjugate of the present invention are replaced with isotopes of the atoms. There exist two types of isotopes: radioisotopes and stable isotopes. Examples of the isotope can include isotypes of hydrogen (2H and 3H), isotopes of carbon (11C, 13C, and 14C), isotopes of nitrogen (13N and 15N), isotopes of oxygen (15O, 17O, and 18O), and isotopes of fluorine (18F). A composition comprising the anti-CD25 antibody or the antigen binding fragment of the antibody, or the antibody-drug conjugate labeled with such an isotope is useful as, for example, a therapeutic agent, a prophylactic agent, a research reagent, an assay reagent, a diagnostic agent, and an *in vivo* diagnostic imaging agent. The present invention also encompasses all of each anti-CD25 antibody or antigen binding fragment of the antibody, or antibody-drug conjugate labeled with an isotope, and mixtures of anti-CD25 antibodies or antigen binding fragments of the antibodies, or antibody-drug conjugates labeled with an isotope at any given ratio. The anti-CD25 antibody or the antigen binding fragment of the antibody, or the antibody-drug conjugate labeled with an isotope can be produced, for example, by using a starting material labeled with an isotope instead of a starting material for the production method of the present invention mentioned later according to a method known in the art.

*In vitro* cytocidal activity can be measured, for example, on the basis of cell proliferation suppressive activity. For example, a cancer cell line overexpressing CD25 is cultured, and the anti-CD25 antibody or the antigen binding fragment of the antibody, or the anti-CD25 antibody-drug conjugate of the present invention is added at varying concentrations to the culture system. Thereafter, its suppressive activity against focus activity, colony formation, and spheroid growth can be measured. In this context, cell proliferation suppressive activity against non-Hodgkin's lymphoma and acute myeloid leukemia can be examined by using a non-Hodgkin's lymphoma-derived cancer cell line and an acute myeloid leukemia-derived cancer cell line. Further, cell proliferation suppressive activity against Treg can be examined by using Treg isolated from a human healthy donor and/or a patient or *in vitro* induced Treg (iTreg).

An *In vivo* therapeutic effect on a cancer in a laboratory animal can be evaluated, for example, by administering the anti-CD25 antibody or the antigen binding fragment of the antibody, or the anti-CD25 antibody-drug conjugate of the present invention to a tumor cell line-transplanted syngeneic mouse, and measuring change in the cancer cells (e.g., change in tumor volume). In this respect, change in immunocytes (e.g., change in the number of immunocytes) in tumor can also be measured. Further, change in immunocytes (e.g., change in the number of immunocytes) in tumor can also be measured by administering the anti-CD25 antibody or the antigen binding fragment of the antibody, or the anti-CD25 antibody-drug conjugate of the present invention to a tumor cell line-transplanted NOG mouse engrafted with human umbilical cord blood-derived CD34+ hematopoietic stem cells (huNOG mouse). In this way, the therapeutic effect on a cancer can be measured.

The type of the cancer to which the anti-CD25 antibody of the present invention or the antigen binding fragment of the antibody, or the anti-CD25 antibody-drug conjugate of the present invention is applied is not particularly limited as long as the cancer expresses CD25 in cancer cells to be treated or tumor immunity is suppressed by Treg. Examples thereof can include blood cancer (B cell lymphoma, T/NK cell lymphoma, Hodgkin's lymphoma, non-Hodgkin's lymphoma, acute myeloid leukemia, and chronic myeloid leukemia), and solid tumor (breast cancer, small-cell lung cancer, non-small cell lung cancer, head and neck cancer, brain tumor, glioma, eye tumor, esophageal cancer, stomach cancer, colorectal cancer, colon cancer, liver cancer, pancreatic cancer, renal cancer, kidney cancer, urinary bladder cancer, adrenal cortex cancer, prostate cancer, uterine cervical cancer, endometrial cancer, ovary cancer, melanoma, and sarcoma). Further examples thereof can include cancers defined by having a biomarker such as microsatellite instability-high (MSI-H), mismatch repair deficient (dMMR), or tumor mutation burden-high (TMB-H). More preferable examples of the cancer can include non-small cell lung cancer, head and neck cancer, esophageal cancer, stomach cancer, and melanoma.

The antibody of the present invention, the antigen binding fragment of the antibody, or the anti-CD25 antibody-drug conjugate of the present invention (in this paragraph, also including the drug released by the cleavage of the linker of the anti-CD25 antibody-drug conjugate) has excellent properties from one or more viewpoints of binding activity against CD25, the ability to block IL-2, an effector function, the ability to remove regulatory T cells, the ability to promote the proliferation of FoxP3-negative and CD4-positive cells and CD8-positive cells and/or granzyme-positive and CD8-positive cells, binding specificity, the ability to bind to the antigen, internalization activity, antitumor activity, solubility, cell membrane permeability, a concentration in blood, metabolic stability, tissue migration, bioavailability, *in vitro* activity, *in vivo* activity,

rapid onset of drug efficacy, sustained drug efficacy, physical stability, drug interaction, toxicity, and the like, and is useful as a medicament. The phrase "useful as a medicament" described in this paragraph includes, for example, not only the case of having excellent properties based on various abilities, effects, actions, or properties described above which are "present", "high", "large", "many", or "positive" and being thus useful as a medicament, but the case of having excellent properties based on various abilities, effects, actions, or properties described above which are "absent", "low", "small", "few", or "negative" and being thus useful as a medicament (e.g., toxicity is low).

The antibody of the present invention or the antigen binding fragment of the antibody, or the anti-CD25 antibody-drug conjugate of the present invention can be preferably administered to a mammal, more preferably a human.

A substance for use in a pharmaceutical composition comprising the antibody of the present invention or the antigen binding fragment of the antibody, or the anti-CD25 antibody-drug conjugate of the present invention can be appropriately selected and used from pharmaceutical additives and others usually used in this field according to a dose or an administration concentration.

The antibody of the present invention or the antigen binding fragment of the antibody, or the anti-CD25 antibody-drug conjugate of the present invention can be administered as a pharmaceutical composition comprising one or more pharmaceutically compatible components. For example, the pharmaceutical composition typically comprises one or more pharmaceutical carriers (e.g., sterilized liquids (e.g., water and oil (including petroleum oil and oil of animal origin, plant origin, or synthetic origin (e.g., peanut oil, soybean oil, mineral oil, and sesame oil))). Water is a more typical carrier when the pharmaceutical composition is intravenously administered. An aqueous saline solution, an aqueous dextrose solution, and an aqueous glycerol solution can also be used as a liquid carrier, in particular, for an injection solution. A suitable pharmaceutical vehicle is known in the art. If desired, the composition may also comprise a trace amount of a moisturizing agent, an emulsifying agent, or a pH buffering agent. Examples of suitable pharmaceutical carriers are disclosed in "Remington's Pharmaceutical Sciences" by E. W. Martin. The prescription corresponds to an administration mode.

Various delivery systems are known, and they can be used for administering the anti-CD25 antibody of the present invention or the antigen binding fragment of the antibody, or the anti-CD25 antibody-drug conjugate of the present invention. Examples of the administration route can include, but are not limited to, intradermal, intramuscular, intraperitoneal, intravenous, and subcutaneous routes. The administration can be made by injection or bolus injection, for example. In a particular preferable embodiment, the administration of the above-described anti-CD25 antibody or antigen binding fragment of the antibody, or antibody-drug conjugate is performed by injection. Parenteral administration is a preferable administration route.

In a representative embodiment, the pharmaceutical composition is prescribed, as a pharmaceutical composition suitable for intravenous administration to a human, in accordance with the conventional procedures. The composition for intravenous administration is typically a solution in a sterile and isotonic aqueous buffer solution. If necessary, the medicament may also contain a solubilizing agent and a local anesthetic to alleviate pain at an injection area (e.g., lignocaine). In general, the components described above are provided, either separately or together in a mixture in a unit dosage form, as a freeze-dried powder or an anhydrous concentrate contained in a container which is obtained by sealing in (e.g., an ampoule or a sachet indicating the amount of the active agent. When the medicament is to be administered by injection, it may be administered using, for example, an injection bottle containing water or saline of sterile pharmaceutical grade. When the medicament is to be administered by injection, an ampoule of sterile water or saline for injection may be provided such that the components described above are admixed with each other before administration.

The pharmaceutical composition of the present invention may be a pharmaceutical composition comprising only the anti-CD25 antibody of the present application or the antigen binding fragment of the antibody, or the anti-CD25 antibody-drug conjugate of the present application, or may be a pharmaceutical composition comprising the anti-CD25 antibody or the antigen binding fragment of the antibody, or the anti-CD25 antibody-drug conjugate, and at least one of other therapeutic agents for cancers. The anti-CD25 antibody of the present invention or the antigen binding fragment of the antibody, or the anti-CD25 antibody-drug conjugate of the present invention may be administered together with an additional therapeutic agent for a cancer, and can thereby enhance an anticancer effect. The additional anticancer agent used for such a purpose and the anti-CD25 antibody of the present invention or the antigen binding fragment of the antibody, or the antibody-drug conjugate may be administered to an individual simultaneously, separately, or continuously, or may be administered in a staggered manner. Examples of such a therapeutic agent for a cancer can include platinum formulations including cisplatin, oxaliplatin, and carboplatin, taxane compounds including paclitaxel and docetaxel, antimetabolites such as 5-fluorouracil and gemcitabine, anti-estrogen such as tamoxifen, aromatase inhibitors such as letrozole, androgen receptor antagonists such as enzalutamide, tyrosine kinase inhibitors including imatinib, sunitinib, and lenvatinib, CDK4/6 inhibitors including palbociclib, PARP inhibitors including olaparib, HSP90 inhibitors including pimitespib, MEK inhibitors including cabozantinib, BRAF inhibitors including dabrafenib, KRAS inhibitors including adagrasib, FGFR inhibitors including futibatinib, cytokines including IL-2, IFN-y, and G-CSF, angiogenesis inhibitors including bevacizumab, tumor antigen- or marker antigen-targeting monoclonal antibodies such as trastuzumab, rituximab, and panitumumab, antibody-drug conjugates including trastuzumab deruxtecan, and immune checkpoint inhibitors including ipilimumab and relatlimab. The therapeutic agent for a cancer is not limited as long as the drug has

antitumor activity.

Such a pharmaceutical composition can be prepared as a formulation having selected composition and a necessary purity in the form of a freeze-dried formulation or a liquid formulation. The freeze-dried formulation thus prepared may be a formulation containing an appropriate pharmaceutical additive used in this field. Likewise, the liquid formulation can be prepared as a liquid formulation containing various pharmaceutical additives used in this field.

The composition and concentration of the pharmaceutical composition also vary depending on the administration method. As for the affinity of the anti-CD25 antibody or the antigen binding fragment of the antibody, or the anti-CD25 antibody-drug conjugate contained in the pharmaceutical composition of the present invention for the antigen, i.e., the dissociation constant (Kd value) thereof to the antigen, the anti-CD25 antibody or the antigen binding fragment of the antibody, or the antibody-drug conjugate having higher affinity (lower Kd value) can exert drug efficacy even at a smaller dose. Thus, the dose of the anti-CD25 antibody or the antigen binding fragment of the antibody, or the antibody-drug conjugate of the present application may be determined by setting the dose based on the status of the affinity of the anti-CD25 antibody or the antigen binding fragment of the antibody, or the antibody-drug conjugate for the antigen. When the anti-CD25 antibody or the antigen binding fragment of the antibody, or the antibody-drug conjugate of the present invention is administered to a human, it can be administered at a dose of, for example, approximately 0.001 to 100 mg/kg once or a plurality of times at intervals of 1 to 180 days.

Preferable examples of the additional therapeutic agent for a cancer that can be administered together with the anti-CD25 antibody of the present invention or the antigen binding fragment of the antibody, or the anti-CD25 antibody-drug conjugate of the present invention can include immune checkpoint inhibitors.

In one aspect of the present invention, the anti-CD25 antibody of the present invention or the antigen binding fragment of the antibody, or the anti-CD25 antibody-drug conjugate of the present invention and the immune checkpoint inhibitor may be separately contained as active ingredients in different formulations and administered simultaneously or at different times, or the antibody-drug conjugate and the immune checkpoint inhibitor may be contained as active ingredients in one formulation and administered.

In one aspect of the present invention, the anti-CD25 antibody of the present invention or the antigen binding fragment of the antibody, or the anti-CD25 antibody-drug conjugate of the present invention and the immune checkpoint inhibitor may be separately administered through different routes.

The anti-CD25 antibody of the present invention or the antigen binding fragment of the antibody, or the anti-CD25 antibody-drug conjugate of the present invention may be contained as active ingredients in one formulation and administered for the treatment of a disease that is improved by an effect of activating antitumor immunity.

In the present invention, the "immune checkpoint inhibitor" refers to a drug that activates tumor immunity by binding to an immune checkpoint molecule or its ligand, inhibiting immunosuppressive signal transduction, and thereby canceling T cell activation suppressive signals.

The immune checkpoint inhibitor is an anti-PD-1 antibody, an anti-PD-L1 antibody, an anti-CTLA-4 antibody, or the like, and is not particularly limited as long as the drug has been clinically confirmed to have effectiveness and safety or has the possibility of clinical application through the mechanism of action equivalent thereto. Preferable examples thereof can include anti-PD-1 antibodies, anti-PD-L1 antibodies, and anti-CTLA-4 antibodies. These drugs can be used singly or in combination of two or more thereof.

In the present invention, the "anti-PD-1 antibody" refers to an antibody having an effect of specifically binding to PD-1 (programmed cell death-1; CD279; PDCD1) and thereby reducing, inhibiting, and/or interfering with signal transduction resulting from the interaction between PD-1 and its binding partner PD-L1 or PD-L2.

The anti-PD-1 antibody is not particularly limited as long as the antibody has been clinically confirmed to have effectiveness and safety. Preferable examples thereof can include nivolumab (International Publication No. WO 2006/121168, etc.), pembrolizumab (International Publication No. WO 2008/156712, etc.), sintilimab, spartalizumab, dostarlimab, serplulimab, tislelizumab, penpulimab, toripalimab, zimberelimab, camrelizumab, retifanlimab, cemiplimab, prolgolimab, geptanolimab, enlonstobart, QL-1604, pucotenlimab, and finotonlimab, more preferably nivolumab and pembrolizumab. These antibodies can be used singly or in combination of two or more thereof.

For example, a commercially available anti-PD-1 antibody for research (e.g., clone RMP1-14) may be used for the purpose of confirming a combined effect with the antibody-drug conjugate used in the present invention in preclinical study.

In the present invention, the "anti-PD-L1 antibody" refers to an antibody having an effect of specifically binding to PD-L1 (programmed cell death ligand 1; CD274; B7-H1) and thereby reducing, inhibiting, and/or interfering with signal transduction resulting from the interaction between PD-L1 and its binding partner PD-1 or B7.1 (CD80).

The anti-PD-L1 antibody is not particularly limited as long as the antibody has been clinically confirmed to have effectiveness and safety. Preferable examples thereof can include atezolizumab (International Publication No. WO 2010/077634, etc.), durvalumab (International Publication No. WO 2011/066389, etc.), cosibelimab, adebrelimab, sugemalimab, avelumab (International Publication No. WO 2013/079174, etc.), socazolimab, KL-A167, and envafolimab, more preferably atezolizumab, durvalumab, and avelumab. These antibodies can be used singly or in combination of two

or more thereof.

For example, a commercially available anti-PD-L1 antibody for research (e.g., clone 10F.9G2) may be used for the purpose of confirming a combined effect with the antibody-drug conjugate used in the present invention in preclinical study.

In the present invention, the "anti-CTLA-4 antibody" refers to an antibody having an effect of specifically binding to CTLA-4 (cytotoxic T-lymphocyte-associated protein 4; CD152) and thereby reducing, inhibiting, and/or interfering with signal transduction resulting from the interaction between CTLA-4 and its binding partner B7.1 (CD80) or B7.2 (CD86).

The anti-CTLA-4 antibody is not particularly limited as long as the antibody has been clinically confirmed to have effectiveness and safety. Preferable examples thereof can include ipilimumab (International Publication No. WO 2001/014424, etc.), botensilimab, and tremelimumab (International Publication No. WO 2000/037504, etc.), more preferably ipilimumab. These antibodies can be used singly or in combination of two or more thereof.

For example, a commercially available anti-CTLA-4 antibody for research (e.g., clone 9H10) may be used for the purpose of confirming a combined effect with the antibody-drug conjugate used in the present invention in preclinical study.

The immune checkpoint inhibitor includes a multispecific molecule. The multispecific molecule includes an IgG-type multispecific molecule, a multispecific molecule having two or more types of variable regions, for example, antibody fragments such as tandem scFv, single-chain diabody, diabody, and triabody, and antibody fragments linked through a covalent or noncovalent bond, though the multispecific molecule is not limited thereto. The multispecific molecule may include Fc.

Specific examples of the multispecific molecule can include, but are not limited to, multispecific antibodies comprising one or more antibodies or antigen binding fragments thereof selected from the group consisting of an anti-PD-1 antibody or an antigen binding fragment thereof, an anti-PD-L1 antibody or an antigen binding fragment thereof, an anti-PD-L2 antibody or an antigen binding fragment thereof, and an anti-CTLA-4 antibody or an antigen binding fragment thereof, more preferably bispecific antibodies comprising an anti-PD-1 antibody or an antigen binding fragment thereof or an anti-CTLA4 antibody or an antigen binding fragment thereof. Such a multispecific molecule is not particularly limited as long as the multispecific molecule has been clinically confirmed to have effectiveness and safety. Preferable examples thereof can include cadonilimab and ivonescimab.

The present invention includes a method for treating a disease, comprising administering the anti-CD25 antibody of the present application or the antigen binding fragment of the antibody, or the anti-CD25 antibody-drug conjugate of the present application to an individual in need of treatment. The present invention includes the anti-CD25 antibody of the present application or the antigen binding fragment of the antibody, or the anti-CD25 antibody-drug conjugate of the present application for use in the treatment of a disease. The present invention includes use of the anti-CD25 antibody of the present application or the antigen binding fragment of the antibody, or the anti-CD25 antibody-drug conjugate of the present application for producing a medicament for the treatment of a disease. The anti-CD25 antibody of the present invention or the antigen binding fragment of the antibody, or the anti-CD25 antibody-drug conjugate of the present invention may be administered together with an additional therapeutic agent for a cancer, and can thereby enhance an anticancer effect.

In the present invention, the phrase "cancer that exhibits resistance to an immune checkpoint inhibitor" refers to a cancer confirmed to exhibit resistance to the immune checkpoint inhibitor, or a cancer that can be rationally recognized or predicted to exhibit resistance to the immune checkpoint inhibitor. The "cancer that exhibits resistance to an immune checkpoint inhibitor" is preferably a "cancer that exhibits resistance to an existing immune checkpoint inhibitor". In the present invention, the "cancer that exhibits resistance to an existing immune checkpoint inhibitor" refers to a cancer confirmed to exhibit resistance to the existing immune checkpoint inhibitor, or a cancer that can be rationally recognized or predicted to exhibit resistance to the existing immune checkpoint inhibitor.

In the present invention, the "existing immune checkpoint inhibitor" refers to a clinically used immune checkpoint inhibitor.

In the present invention, the "resistance" refers to a property of having non-response to treatment with an anticancer agent, and can also be expressed as "refractoriness", "non-responsiveness", or "unresponsiveness". The term can also be expressed as "intolerance" because tumor growth cannot be prevented due to the non-response. In the present invention, the term "resistance" includes the case where a cancer in a patient is low sensitive to treatment with an anticancer agent, the case where cancer cells neither disappear nor decrease in size, the case where neither complete response (CR) nor partial response (PR) is obtained, and/or the case where cancer progresses at an early stage (e.g., within or shorter than 6 months for ovarian cancer), after treatment with the anticancer agent.

The "resistance" of the present invention may be "resistance acquired by treatment with an existing immune checkpoint inhibitor" or may be "originally possessed resistance irrespective of treatment with an existing immune checkpoint inhibitor".

Examples

The present invention will be specifically described with reference to Examples given below. The present invention is not limited by these examples. Furthermore, these examples should not be construed in a limited manner by any means. In the following examples, unless otherwise specified, individual operations regarding genetic manipulation were performed by the method described in "Molecular Cloning" (Sambrook, J., Fritsch, E. F. and Maniatis, T., published by Cold Spring Harbor Laboratory Press in 1989) or other methods described in experimental manuals used by those skilled in the art, or when commercially available reagents or kits were used, the examples were performed in accordance with the instructions included in the commercially available products. In the present specification, reagents, solvents, and starting materials are readily available from commercially available sources, unless otherwise specified.

(Example 1) Preparation of expression vector

1)-1 Human CD25 expression vector

The human CD25 expression vector (hereinafter, referred to as "pCMV3-hCD25") used was CD25/IL2R alpha cDNA ORF Clone, Human, untagged (Sino Biological Inc.). The polynucleotide sequence of the human CD25 gene cloned in this vector is shown in SEQ ID NO: 42 of the sequence listing, and the amino acid sequence of human CD25 is shown in SEQ ID NO: 17 of the sequence listing.

1)-2 Mouse CD25 expression vector

The mouse CD25 expression vector (hereinafter, referred to as "pCMV3-mCD25") used was CD25/IL2R alpha cDNA ORF Clone, Mouse, untagged (Sino Biological Inc.). The polynucleotide sequence of an ORF site of the mouse CD25 gene cloned in this vector is shown in SEQ ID NO: 43 of the sequence listing, and the amino acid sequence of mouse CD25 is shown in SEQ ID NO: 44 of the sequence listing.

1)-3 Preparation of cynomolgus monkey CD25 expression vector

PCR reaction was performed with cDNA (prepared using an oligo dT primer from mRNA prepared from cynomolgus monkey PBMC) as a template using the following primer set: Primer 1F: 5'-ggtaccgccatggatccatacctgctc atgtggg-3' (SEQ ID NO: 45) and
Primer 1R: 5'-ctcgagctagattgttcttctattcttcctctg-3' (SEQ ID NO: 46).

The obtained PCR product was incorporated into pCR4 Blunt-TOPO vector using Zero Blunt TOPO PCR Cloning Kit for Sequencing (Thermo Fisher Scientific Inc.) to prepare a cloning vector containing cynomolgus monkey CD25 cDNA (the description about a mutagenesis step is omitted). Cynomolgus monkey CD25 cDNA was excised from the cloning vector with Kpn I and Xho I, and this cDNA was incorporated between Kpn I and Xho I of pCI vector to prepare a cynomolgus monkey CD25 expression vector (hereinafter, referred to as "pCI-cCD25"). The polynucleotide sequence of an ORF site of the cynomolgus monkey CD25 gene cloned in this vector is shown in SEQ ID NO: 47 of the sequence listing, and the amino acid sequence of cynomolgus monkey CD25 is shown in SEQ ID NO: 48 of the sequence listing.

(Example 2) Preparation of monoclonal antibody and screening

2)-1 Immunization

A female WKY/Izm rat (Japan SLC, Inc.) was used in immunization. Recombinant Human IL-2 Receptor Subunit $\alpha$/IL-2RA/CD25 (C-6His) (Novoprotein Scientific Inc.) and Recombinant Mouse IL-2R $\alpha$/IL-2RA/CD25 (C-6His) (Novoprotein Scientific Inc.) and Freund's Complete Adjuvant (FUJIFILM Wako Pure Chemical Corp.) were administered to the tail root of the rat, and the lymph node and the spleen were collected from the rat and used in hybridoma preparation.

2)-2 Preparation of hybridoma

Lymph node cells or spleen cells and mouse myeloma SP2/0-ag14 cells (ATCC: CRL-1581) were subjected to electrical cell fusion using LF301-Cell Fusion Unit (BEX Co., Ltd.), diluted with ClonaCell-HY Selection Medium D (STEMCELL Technologies Inc.), and cultured. Hybridoma colonies that appeared were recovered to prepare monoclonal hybridomas. Each of the recovered hybridoma colonies was cultured, and anti-CD25 antibody-producing hybridomas were screened for using the obtained hybridoma culture supernatant or an antibody purified from the hybridoma culture.

2)-3 Antibody screening

2)-3-1 Measurement of antigen binding activity

2)-3-1-1 Preparation of antigen gene-expressing cell

CHO-K1 cells subcultured in Ham's F-12K (Kaighn's) medium (Thermo Fisher Scientific Inc.) containing 10% FBS were adjusted to $4.5 \times 10^5$ cells/mL in Ham's F-12K (Kaighn's) medium containing 10% FBS. 15 μg of pCMV3-hCD25, pCMV3-mCD25, or pCI-cCD25 was transferred to 10 mL of the cell suspension using FuGene6 Transfection Reagent (Promega Corp.), and 100 μL of the cells was inoculated to each well of Collagen Type I-coated 96-well plate (AGC TECHNO GLASS Co., Ltd.) and cultured overnight under conditions of 37°C and 5% $CO_2$. The obtained transfected cells were used in an adherent state in Cell-ELISA.

2)-3-1-2 Cell-ELISA

After removal of a supernatant from the expression vector-transfected CHO-K1 cells prepared in 2)-3-1-1, 50 μL of the hybridoma culture supernatant or the purified antibody was added to each well of the CHO-K1 cells harboring pCMV3-hCD25, pCMV3-mCD25, or pCI-cCD25 and left standing at 4°C for 1 hour. The cells in the well were washed with PBS containing 5% FBS, and 50 μL of ECL anti-Rat IgG, HRP linked (GE Healthcare Bio-Sciences Corp.) diluted 500-fold with PBS containing 5% FBS was then added thereto and left standing at 4°C for 1 hour. The cells in the well were washed with PBS containing 5% FBS, and 100 μL of TMB Microwell Peroxidase Substrate (Kirkegaard & Perry Laboratories Inc.) was then added thereto. Chromogenic reaction was performed for 10 minutes with stirring at room temperature under light shielding and terminated by the addition of STOP Solution (Kirkegaard & Perry Laboratories Inc.) at 100 μL/well, followed by the measurement of absorbance at 450 nm using a plate reader (SpectraMax: Molecular Devices, LLC) (in the drawings, A450 represents absorbance at 450 nm). In order to select hybridomas producing an antibody capable of specifically binding to CD25 expressed on cell membrane surface, hybridomas that produced a culture supernatant with absorbance elevated in an added antibody concentration-dependent manner were selected as anti-CD25 antibody production-positive hybridomas.

2)-3-2 Purification of anti-CD25 antibody

A clone that exhibited binding activity against human and monkey CD25 and a clone that exhibited binding activity against mouse CD25 were selected from among the rat anti-CD25 antibody-producing hybridomas selected in 2)-3-1, and each antibody was purified from the hybridoma culture supernatant using Spin column based Antibody Purification Kit (Cosmo Bio Co., Ltd.). 500 μL of Binding Buffer was added to Monolithic silica-based Spin column and centrifuged under conditions of 6000 rpm, 30 seconds, and 4°C using a centrifuge MX-160 (Tomy Seiko Co., Ltd.) to remove a flow-through fraction. 500 μL of the hybridoma culture supernatant was added to the column and centrifuged under conditions of 6000 rpm, 30 seconds, and 4°C using a centrifuge MX-160 (Tomy Seiko Co., Ltd.) to remove a flow-through fraction. 500 μL of PBS (FUJIFILM Wako Pure Chemical Corp.) was added to the column and centrifuged under conditions of 6000 rpm, 30 seconds, and 4°C using a centrifuge MX-160 (Tomy Seiko Co., Ltd.) to remove a flow-through fraction. 20 μL of Neutralization Buffer was added to a 1.5 mL microtube, and Spin column was placed therein. 200 μL of Elution Buffer was added to the Spin column and centrifuged under conditions of 6000 rpm, 30 seconds, and 4°C using a centrifuge MX-160 (Tomy Seiko Co., Ltd.) to obtain a purified antibody.

2)-3-3 Measurement of antibody concentration

2)-3-3-1 Isotyping of antibody

A clone that exhibited binding activity against human and monkey CD25 and a clone that exhibited binding activity against mouse CD25 were selected from among the rat anti-CD25 antibody-producing hybridomas selected in 2)-3-1, and each antibody was isotyped. The heavy chain subclass and light chain type of the antibody were identified using Rat Ig Isotyping Ready-Set-Go (Thermo Fisher Scientific Inc.).

Capture Antibody diluted 250-fold with PBS was added at 100 μL/well to MaxiSorp flat-bottom 96 well plate (Thermo Fisher Scientific Inc.) and left standing at 4°C for 3 days for immobilization. The plate was washed twice with Wash Buffer (20 × Wash Buffer of BD OptEIA Set A (BD Biosciences Pharmingen) diluted 20-fold with MilliQ) and then blocked using Blocking Buffer (PBS containing 0.1% Tween-20 (Bio-Rad Laboratories, Inc.) and 1% BSA (FUJIFILM Wako Pure Chemical Corp.)). After washing twice with Wash Buffer, Assay Buffer (PBS containing 0.05% Tween-20 (Bio-Rad Laboratories, Inc.) and 0.5% BSA (FUJIFILM Wako Pure Chemical Corp.)) was added at 50 μL/well. Further, the culture

supernatant diluted with PBS was added at 50 μL/well, and the plate was incubated at room temperature for 2 hours. After washing four times with Wash Buffer, Detection Antibody (diluted 500-fold with Assay Buffer) was added. After washing four times with Wash Buffer, TMB Substrate Solution (Substrate Reagents A and B of BD OptEIA Set A (BD Biosciences Pharmingen) mixed in equal amounts) was added at 100 μL/well, and the plate was incubated for 10 minutes with stirring at room temperature under light shielding. Stop Solution was added at 100 μL/well (BD OptEIA Set A (BD Biosciences Pharmingen), and absorbance was measured at 450 nm and 570 nm using a plate reader (Spectra Max, Molecular Devices, LLC).

2)-3-3-2 Concentration measurement

The antibody concentration was measured using Rat IgG ELISA Quantitation Set (Bethyl Laboratories, Inc.). A standard substance was set for each clone on the basis of the isotype information determined in 2)-3-3-1, and Rat IgG1 Isotype Control (R&D Systems, Inc.), Rat IgG2A Isotype Control (R&D Systems, Inc.), or Rat IgG2B Isotype Control (R&D Systems, Inc.) was used.

2)-3-4 Influence on IL-2-dependent cell proliferation using human pan T cell

2)-3-4-1 Preparation of human pan T cell and serum starvation

Frozen human PBMC (Cellular Technology Limited) was thawed in accordance with C.T.L. Protocol. Then, pan T cells were separated from PBMC using Pan T Cell isolation kit (Miltenyi Biotec). The separated pan T cells were suspended at $5.0 \times 10^5$ cells/mL in RPMI (Life Technologies Corp.) medium supplemented with 10% FBS (Hyclone, Global Life Sciences Technologies Japan K.K.) and 1% Penicillin-Streptomycin (Life Technologies Corp.). Then, PHA (Sigma-Aldrich Co., LLC) was added at 3 μg/mL to this cell suspension, which was then cultured for 3 days under conditions of 37°C and 5% $CO_2$. Cells recovered after culture were washed twice with PBS, and the number of cells was counted. Then, the cells were suspended at $2.0 \times 10^5$ cells/mL in RPMI medium supplemented with 1% FBS and 1% Penicillin-Streptomycin and added to a culture flask. The cells were further cultured for 24 hours under conditions of 37°C and 5% $CO_2$ for serum starvation.

2)-3-4-2 Preparation of antibody solution and addition

Anti-human CD25 antibodies basiliximab (Novartis Pharma K.K.) and 7G7B6 (Bio X Cell) or a control antibody InVivoMab Rat IgG2a Isotype control (rat IgG2a) (Bio X Cell) was diluted into 10 μg/mL, 25 μg/mL, and 50 μg/mL with ClonaCell-HY Cloning Medium E (Medium E) (STEMCELL Technologies Inc.) and used.

5 μL of basiliximab, 7G7B6, the control antibody, or the hybridoma culture supernatant with each concentration was added to each well of a 96-well plate, and 15 μL of RPMI medium supplemented with 1% FBS and 1% Penicillin-Streptomycin was further added to the well.

2)-3-4-3 Addition of human pan T cell and IL-2 solution

The serum-starved pan T cells were recovered and suspended at $1.25 \times 10^5$ cells/mL by the addition of RPMI medium supplemented with 1% FBS and 1% Penicillin-Streptomycin. The cell suspension was added at 40 μL/well to the wells supplemented with the solution in 2)-3-5-2. Further, the plate was incubated for 15 minutes under conditions of 5% $CO_2$ and 37°C.

Recombinant human IL-2 (PeproTech, Inc.) was dissolved in 50 μL of 0.1 M acetic acid and then diluted with 0.45 mL of PBS containing 0.1% BSA to prepare a 100 μg/mL solution. Further, the solution was diluted into 3.75 ng/mL with RPMI medium containing 1% FBS and 1% Penicillin-Streptomycin. The dilution was added at 40 μL/well to wells of an IL-2 addition group. RPMI medium containing 1% FBS and 1% Penicillin-Streptomycin was added at 40 μL/well to wells of an IL-2 non-addition group. Then, the plate was incubated for 48 hours under conditions of 5% $CO_2$ and 37°C.

2)-3-4-4 Measurement of cell proliferation

The plate thus incubated was centrifuged at 300 × g for 5 minutes, and 50 μL of a supernatant was removed from each well. 50 μL of Celltiter-Glo Reagent of CellTiter-Glo luminescent Cell Viability Assay (Promega Corp.) kit was added to each well, and the plate was stirred for 2 minutes using a plate shaker. A 50 μL aliquot per well was transferred to OptiPlate-96 (PerkinElmer, Inc.), and luminescence intensity (relative light unit (RLU)) was measured using a plate reader (Ensight: PerkinElmer, Inc.) and used as an index for the number of cells.

2)-3-5 Influence on phosphorylated STAT5 (pSTAT5) whose level is increased by IL-2 stimulation in human pan T cell

2)-3-5-1 Preparation of human pan T cell and serum starvation

Frozen human PBMC (Cellular Technology Limited) was thawed in accordance with C.T.L. Protocol. Then, pan T cells were separated from PBMC using Pan T Cell isolation kit (Miltenyi Biotec). The separated pan T cells were suspended at $5.0 \times 10^5$ cells/mL in RPMI (Life Technologies Corp.) medium supplemented with 10% FBS (Hyclone, Global Life Sciences Technologies Japan K.K.) and 1% Penicillin-Streptomycin (Life Technologies Corp.). Then, PHA (Sigma-Aldrich Co., LLC) was added at 3 μg/mL to this cell suspension, which was then cultured for 3 days under conditions of 37°C and 5% $CO_2$. Cells recovered after culture were washed twice with PBS, and the number of cells was counted. Then, the cells were suspended at $2.0 \times 10^5$ cells/mL in RPMI medium supplemented with 1% FBS and 1% Penicillin-Streptomycin and added to a culture flask. The cells were further cultured for 24 hours under conditions of 37°C and 5% $CO_2$ for serum starvation.

2)-3-5-2 Preparation of antibody solution and addition

Anti-human CD25 antibodies basiliximab (Novartis Pharma K.K.) and 7G7B6 (Bio X Cell) or a control antibody InVivoMab Rat IgG2a Isotype control (rat IgG2a) (Bio X Cell) was diluted into 10 μg/mL, 25 μg/mL, and 50 μg/mL with ClonaCell-HY Cloning Medium E (Medium E) (STEMCELL Technologies Inc.) and used.

20 μL of basiliximab, 7G7B6, the control antibody, or the hybridoma culture supernatant with each concentration was added to each well of a 96-well plate.

2)-3-5-3 Addition of human pan T cell and IL-2 solution

The serum-starved pan T cells were recovered and suspended at $1.25 \times 10^5$ cells/mL by the addition of RPMI medium supplemented with 1% FBS and 1% Penicillin-Streptomycin. The cell suspension was added at 40 μL/well to the wells supplemented with the solution in 2)-3-5-2. Further, the plate was incubated for 15 minutes under conditions of 5% $CO_2$ and 37°C.

Recombinant human IL-2 (PeproTech, Inc.) was dissolved in 50 μL of 0.1 M acetic acid and then diluted with 0.45 mL of PBS containing 0.1% BSA to prepare a 100 μg/mL solution. Further, the solution was diluted to 3.75 ng/mL with RPMI medium containing 1% FBS and 1% Penicillin-Streptomycin. The dilution was added at 40 μL/well to wells of an IL-2 addition group. RPMI medium containing 1% FBS and 1% Penicillin-Streptomycin was added at 40 μL/well to wells of an IL-2 non-addition group. Then, the plate was incubated for 15 minutes under conditions of 5% $CO_2$ and 37°C.

2)-3-5-4 pSTAT5 measurement

$5 \times$ lysis buffer included in AlphaLISA SureFire Ultra phosho-STAT5 (Tyr694/699) kit (PerkinElmer, Inc.) was adjusted to $1 \times$ lysis buffer with $H_2O$.

The plate incubated for 15 minutes was centrifuged at $300 \times g$ for 5 minutes, and a supernatant was removed by suction from each well. Then, the $1 \times$ lysis buffer was added at 50 μL/well to each well. The plate was stirred for 10 minutes using a plate shaker to prepare a cell lysate.

The cell lysate of each well was added at 5 μL/well to 384-well SW plate for AlphaLISA (PerkinElmer, Inc.), and Acceptor Mix was added at 2.5 μL/well, followed by stirring for 1 hour using a plate shaker. After incubation overnight at 4°C under light shielding, Donor Mix was added at 2.5 μL/well, and the plate was stirred for 2 minutes using a shaker and then further incubated at room temperature for 2 hours under light shielding. Then, Alpha signals were measured using a plate reader (Ensight: PerkinElmer, Inc.).

2)-3-6 Measurement of cell internalization activity using human pan T cell (Fab-ZAP method)

2)-3-6-1 Immobilization of anti-human CD3 antibody and anti-human CD28 antibody onto well of 6-well plate

PBS containing 20 μg/mL anti-human CD3 antibody (EXBIO Praha) and anti-human CD28 antibody (Becton, Dickinson and Company) was added at 2 mL/well to each well of a 6-well plate (Corning Inc.), and the plate was then mixed using a plate mixer and incubated at 37°C for 2 hours. A supernatant was removed from the plate thus incubated, and the wells were washed once with PBS and used.

2)-3-6-2 Isolation of pan T cell from thawed human PBMC and culture of pan T cell

Frozen PBMC (Cellular Technology Limited) was thawed in a water bath of 37°C, added to RPMI (Life Technologies Corp.) medium containing 10% FBS, 1% Penicillin-streptomycin, and Anti-Aggregate Wash (1/20 addition), and then centrifuged at 310 × g for 10 minutes. After removal of a supernatant, the cells were resuspended in a fresh medium. Pan T cells were isolated from the resuspended PBMC using Pan T Cell isolation kit (Miltenyi Biotec). The isolated pan T cells were suspended at 1.07 × $10^6$ cells/mL in IMDM (Thermo Fisher Scientific Inc.) medium containing 10% CTS Immune Cell SR (Thermo Fisher Scientific Inc.) to prepare a cell suspension. The cell suspension was inoculated at 4 mL/well to the 6-well plate provided in 2)-3-5-1, and cultured for 4 days under conditions of 5% $CO_2$ and 37°C. On culture day 2, IMDM medium containing 1.5 ng/mL Recombinant Human IL-2 (PeproTech, Inc.) and 10% CTS Immune Cell SR was added at 2 mL/well.

2)-3-6-3 ZAP assay

Fab-ZAP rat (Advanced Targeting Systems, Inc.) was diluted with RPMI medium supplemented with 1% Penicillin-Streptomycin to prepare 40 μg/mL, 8 μg/mL, 1.6 μg/mL, and 0.32 μg/mL Fab-ZAP rat solutions. A control antibody rat IgG2a and the rat hybridoma culture supernatant or the purified antibody were diluted with RPMI medium supplemented with 1% Penicillin-Streptomycin such that the rat IgG concentration was 3000 ng/mL, 600 ng/mL, 120 ng/mL, and 24 ng/mL. Each solution was added to a 96-well plate such that the concentration ratio between Fab-ZAP rat and the anti-CD25 antibody in wells was 2.67:1. RPMI medium supplemented with 1% Penicillin-Streptomycin was added to wells containing Fab-ZAP and no anti-CD25 antibody (antibody concentration: 0 ng/mL). In order to obtain results in the absence of Fab-ZAP rat, a 96-well plate supplemented with only the rat hybridoma culture supernatant or the purified antibody solution was also provided.

The pan T cells cultured in the 6-well plate were recovered and centrifuged at 300 × g for 10 minutes. After removal of a supernatant, RPMI medium supplemented with 1% Penicillin-Streptomycin was added to the cells so as to attain 2.5 × $10^5$ cells/mL. The resulting solution was added at 40 μL/well to the 96-well plate. The cells were cultured for 3 days under conditions of 5% $CO_2$ and 37°C, and the number of cells was then measured using CellTiter-Glo luminescent Cell Viability Assay (Promega Corp.). The % luminescence intensity of each well was calculated when the luminescence intensity of the well having an antibody concentration of 0 ng/mL was defined as 100%.

2)-3-7 Cross-competition assay of anti-human CD25 antibody and M-A251

2)-3-7-1 Preparation of CHO-1 cell expressing human CD25

CHO-K1 cells subcultured in Ham's F-12K (Kaighn's) medium (Thermo Fisher Scientific Inc.) containing 10% FBS and 1% Penicillin-streptomycin were adjusted to 4.5 × $10^5$ cells/mL in Ham's F-12K (Kaighn's) medium containing 10% FBS and 1% Penicillin-streptomycin. 15 μg of pCMV3-hCD25 was transferred to 10 mL of the cell suspension using FuGene6 Transfection Reagent (Promega Corp.), and 100 μL of the cells was inoculated to each well of Collagen Type I-coated 96-well plate (AGC TECHNO GLASS Co., Ltd.) and cultured overnight to for 4 days under conditions of 37°C and 5% $CO_2$. The obtained transfected cells were used in an adherent state in assay.

2)-3-7-2 Cross-competition assay

0.5 mg/mL Biotin anti-human CD25 Antibody (clone; MA251, BioLegend, Inc.) was diluted into 0.1 mg/mL with PBS containing 5% FBS. Basiliximab (Novartis Pharma K.K.), M-A251 (BD Biosciences), the obtained antibody, or IgG1, κ from human myeloma plasma (hIgG1, Merck KGaA) used as a control antibody was diluted into 1 μg/mL, 3 μg/mL, and 10 μg/mL with PBS containing 5% FBS and then mixed with the Biotin M-A251 antibody solution at a ratio of 1:1 (mixed solution 1). For an antibody non-addition group (0 μg/mL), PBS containing 5% FBS and the Biotin MA251 antibody solution were mixed at a ratio of 1:1 (mixed solution 2). 0.5 mg/mL HRP Streptavidin (BioLegend, Inc.) was diluted 1000-fold with PBS containing 5% FBS. TMB Peroxidase Substrate and Peroxidase Substrate Solution B of TMB Microwell Peroxidase Substrate (2-Component System) (SeraCare Life Sciences, Inc.) kit were mixed at a ratio of 1:1 to prepare a substrate solution. A culture supernatant was removed from the wells of the 96-well plate, and each well was washed once with 0.2 mL of PBS containing 5% FBS. After removal of PBS containing 5% FBS from each well, the mixed solutions 1 and 2 were added at 50 μL/well and incubated at 4°C for 1 hour. After removal of the mixed solutions from the wells, each well was washed three times with 0.2 mL of PBS containing 5% FBS. After removal of PBS containing 5% FBS from each well, the substrate solution was added at 0.1 mL/well to each well. Chromogenic reaction was performed for 5 minutes with stirring at room temperature under light shielding and terminated by the addition of STOP Solution (SeraCare Life Sciences, Inc.) at 0.1 mL/well, followed by the measurement of absorbance at 450 nm using a plate reader (SpectraMax: Molecular

Devices, LLC).

2)-3-8 Preparation of anti-mouse CD25 antibodies PC61_hIgG1LALA and PC61G_hIgG1LALA

Anti-mouse CD25 antibodies PC61_hIgG1LALA and PC61G_hIgG1LALA were prepared in accordance with a method known from the literature (Huss et al., 2016. Immunology. 148 (3): 276-286) and the patent (WO2017/174331).

2)-3-9 Influence on phosphorylated STAT5 (pSTAT5) whose level is increased by IL-2 stimulation in mouse T cell line CTLL-2 cell

2)-3-9-1 Culture of CTLL-2 cell and serum starvation

A CD25-positive mouse tumor cell line CTLL-2 was subcultured under conditions of 37°C and 5% $CO_2$ in RPMI1640 (Thermo Fisher Scientific Inc.) medium containing 1 mM Sodium Pyruvate (Thermo Fisher Scientific Inc.), 1 × MEM Non-Essential Amino Acids Solution (Thermo Fisher Scientific Inc.), and 10% T-STIM with ConA.

The cultured CTLL-2 cells were recovered, centrifuged at 300 × g for 5 minutes, and then suspended at $2.0 \times 10^6$ cells/mL in RPMI1640 medium. The cell suspension was added at 50 μL/well to a 96-well plate and cultured for 5 hours under conditions of 37°C and 5% $CO_2$.

2)-3-9-2 Preparation of antibody solution and mouse IL-2 solution and addition

The purified antibody, the anti-mouse CD25 antibody PC61G_hIgGLALA which inhibits the binding of IL-2 to mouse CD25, or a control antibody rat IgG2a was diluted into 20 μg/mL with RPMI1640 medium. Each antibody solution was added to the wells inoculated with the cells, and the plate was then incubated for 15 minutes under conditions of 37°C and 5% $CO_2$.

2)-3-9-3 pSTAT5 measurement

The measurement was carried out in accordance with the method of 2)-3-5-4.

2)-3-10 Measurement of cell internalization activity using mouse pan T cell (Fab-ZAP method)

2)-3-10-1 Immobilization of anti-mouse CD3 antibody and anti-mouse CD28 antibody onto well

1 mg/mL solutions of Ultra-LEAF Purified anti-mouse CD3 Antibody (anti-mouse CD3 antibody: BioLegend, Inc.) and LEAF Purified anti-mouse CD28 Antibody (anti-mouse CD28 antibody: BioLegend, Inc.) were mixed and diluted with PBS to prepare a 10 μg/mL mixed solution. 2 mL of this mixed solution was added to each well of a 6-well plate, mixed using a plate mixer, and then incubated at 37°C for 2 hours. The plate was washed three times with PBS and used in experiments.

2)-3-10-2 Preparation of mouse pan T cell from mouse spleen cell and activation

Mouse pan T cells were separated and recovered from spleen cells prepared from a Balb/c mouse using Pan T Cell Isolation Kit II, mouse (Miltenyi Biotec). 8 mL of RPMI medium containing 0.5% penicillin/streptomycin, 5 mM HEPES, 1 mM sodium pyruvate, 1 × MEM-NEAA, and 10% FBS was added to the separated and recovered mouse pan T cells to prepare a cell suspension of $1.31 \times 10^6$ cells/mL. 50 μM 2-mercaptoethanol and 10 ng/mL mouse IL-2 were added to the cell suspension, and 4 mL of the cells was inoculated to each well of the 6-well plate obtained by immobilization in 2)-3-10-1, and incubated at 37°C for 3 days.

2)-3-10-3 ZAP assay

Fab-ZAP rat (Advanced Targeting Systems, Inc.) was diluted with RPMI medium supplemented with 1% Penicillin-Streptomycin to prepare 40 μg/mL, 8 μg/mL, 1.6 μg/mL, and 0.32 μg/mL Fab-ZAP rat solutions. A control antibody rat IgG2a, Purified NA/LE Rat Anti-Mouse CD25 (PC61 antibody) (Becton, Dickinson and Company), and the rat hybridoma culture supernatant or the purified antibody were diluted with RPMI medium supplemented with 1% Penicillin-Streptomycin such that the rat IgG concentration was 1500 ng/mL, 300 ng/mL, 60 ng/mL, and 12 ng/mL. Each solution was added to a 96-well plate such that the concentration ratio between Fab-ZAP rat and the anti-CD25 antibody in wells was 0.53:1. RPMI medium supplemented with 1% Penicillin-Streptomycin was added to wells containing Fab-ZAP and no anti-CD25 antibody (antibody concentration: 0 ng/mL). In order to obtain results in the absence of Fab-ZAP rat, a 96-well plate

supplemented with only the rat hybridoma culture supernatant or the purified antibody solution was also provided.

The pan T cells cultured in the 6-well plate were recovered and centrifuged at 310 × g for 10 minutes. After removal of a supernatant, RPMI medium supplemented with 1% Penicillin-Streptomycin was added to the cells so as to attain 2.5 × $10^5$ cells/mL. The resulting solution was added at 40 μL/well to the 96-well plate. The cells were cultured for 3 days under conditions of 5% $CO_2$ and 37°C, and the number of cells was then measured using CellTiter-Glo luminescent Cell Viability Assay (Promega Corp.). The % luminescence intensity of each well was calculated when the luminescence intensity of the well having an antibody concentration of 0 ng/mL was defined as 100%.

Approximately 2000 clones prepared in 2)-2 were evaluated, and further analysis was pursued on selected MAb1 and MAb2.

(Example 3) Property of MAb1

3)-1 Antigen binding activity

The test was carried out in accordance with the method of 2)-3-1. MAb1 was added at final antibody concentrations of 0.02 μg/mL, 0.16 μg/mL, 1.25 μg/mL, and 10 μg/mL using a purified antibody. 5% FBS in PBS was added to antibody non-addition wells (0 μg/mL). As a result, MAb1 bound to CHO-K1 cells harboring pCMV3-hCD25 or pCI-cCD25 in an antibody concentration-dependent manner, and did not bind to CHO-K1 cells harboring pCMV3-mCD25 (Figure 1). This demonstrated that MAb1 binds to human CD25 and cynomolgus monkey CD25.

3)-2 Influence on IL-2-dependent proliferation of human pan T cell

The test was carried out in accordance with the method of 2)-3-4. MAb1 was added at a final antibody concentration of 11.1 μg/mL using a hybridoma culture supernatant (n = 2). As a result, when the number of T cells in a group supplemented with IL-2 and a control antibody rat IgG2a (2.5 μg/mL) was defined as 100%, the number of T cells in a group supplemented with IL-2 and an IL-2 blocking antibody basiliximab (2.5 μg/mL) was 52%; the number of T cells in a group supplemented with IL-2 and an IL-2 non-blocking antibody 7G7B6 (2.5 μg/mL) was 94%: and the number of T cells in a group supplemented with IL-2 and MAb1 was 109% (Figure 2). The number of T cells increased by the addition of IL-2 in the MAb1 addition group was at the same level as in the control antibody rat IgG2a addition group, and the number of T cells increased by the addition of IL-2 in the IL-2 blocking antibody basiliximab addition group was decreased as compared with the rat IgG2a addition group whereas the number of T cells increased by the addition of IL-2 in the IL-2 non-blocking antibody 7G7B6 addition group was not decreased, demonstrating that MAb1 is an IL-2 non-blocking antibody that does not influence IL-2-dependent promotion of human T cell proliferation.

3)-3 Influence on phosphorylated STAT5 (pSTAT5) whose level is increased by IL-2 stimulation

3)-3-1 Immobilization of anti-human CD3 antibody onto well of 6-well plate

PBS containing 10 μg/mL anti-human CD3 antibody (EXBIO Praha) was added at 2 mL/well to each well of a 6-well plate (Corning Inc.), and the plate was then mixed using a plate mixer and incubated at 4°C. A supernatant was removed from the plate thus incubated, and the wells were washed three times with PBS and used.

3)-3-2 Preparation of human PBMC and serum starvation

Frozen human PBMC was thawed in accordance with C.T.L. Protocol. The thawed PBMC was suspended in IMDM (Thermo Fisher Scientific Inc.) medium containing 10% CTS Immune Cell SR (Thermo Fisher Scientific Inc.) and inoculated at 4 mL/well to the 6-well plate provided in 3)-3-1. The plate was incubated for 5 days under conditions of 37°C and 5% $CO_2$.

3)-3-3 Preparation of antibody solution and addition

MAb1 was added at a final antibody concentration of 20 μg/mL using a purified antibody (n = 3). Rat IgG2a was used as a control antibody, basiliximab was used as an IL-2 blocking antibody, and 7G7B6 was used as an IL-2 non-blocking antibody. These antibodies were added at a concentration of 20 μg/mL (n = 3).

3)-3-4 Addition of human PBMC and IL-2 solution

The serum-starved PBMC was recovered and adjusted to 2 × $10^6$ cells/mL with IMDM medium (Thermo Fisher

Scientific Inc.). The cell suspension was added at 50 μL/well to the plate supplemented with the antibody solution in 7)-3-2. Further, the plate was incubated for 15 minutes under conditions of 5% $CO_2$ and 37°C.

Recombinant human IL-2 (PeproTech, Inc.) was dissolved in 50 μL of 0.1 M acetic acid and then diluted with 0.45 mL of PBS containing 0.1% BSA to prepare a 100 μg/mL solution. Further, the solution was diluted into 4 ng/mL with IMDM medium (Thermo Fisher Scientific Inc.) and added at 25 μL/well to wells of an IL-2 addition group. IMDM medium (Thermo Fisher Scientific Inc.) was added at 25 μL/well to wells of an IL-2 non-addition group. Then, the plate was incubated for 15 minutes under conditions of 5% $CO_2$ and 37°C.

3)-3-5 pSTAT5 measurement

5 × lysis buffer included in AlphaLISA SureFire Ultra phosho-STAT5 (Tyr694/699) kit (PerkinElmer, Inc.) was adjusted to 1 × lysis buffer with $H_2O$.

The plate incubated for 15 minutes was centrifuged at 377 g for 5 minutes, and a supernatant was removed by suction from each well.

Then, the 1 × lysis buffer was added at 50 μL/well to each well. The plate was stirred for 10 minutes using a plate shaker to prepare a cell lysate. The cell lysate of each well was added at 5 μL/well to 384-well SW plate for AlphaLISA (PerkinElmer, Inc.), and Acceptor Mix was added at 4 μL/well, followed by stirring for 2 minutes using a plate shaker. Then, the plate was incubated for 1 hour under light shielding.

Donor Mix was added at 2.5 μL/well, and the plate was stirred for 2 minutes using a plate shaker and then further incubated overnight under light shielding. Then, Alpha signals were measured using a plate reader (Ensight: PerkinElmer, Inc.).

As a result, when the amount of pSTAT5 in the group supplemented with IL-2 and the control antibody rat IgG2a was defined as 100%, the amount of pSTAT5 was 1% in the group supplemented with IL-2 and the IL-2 blocking antibody basiliximab, 103% in the group supplemented with IL-2 and the IL-2 non-blocking antibody 7G7B6, and 108% in the group supplemented with IL-2 and MAb1 (Figure 3). The amount of pSTAT5 increased by the addition of IL-2 was decreased in the basiliximab addition as compared with the rat IgG2a addition group, and the amount of pSTAT5 increased by the addition of IL-2 was not decreased in the 7G7B6 addition, demonstrating that MAb1 is an IL-2 non-blocking antibody that does not inhibit IL-2 signals of T cells.

3)-4 Measurement of cell internalization activity using human pan T cell (Fab-ZAP method)

The test was carried out in accordance with the method of 2)-3-6. MAb1 was added at a concentration of 1500 ng/mL, 300 ng/mL, 60 ng/mL, and 12 ng/mL to each well using a purified antibody. When a cell survival rate at each concentration of a control antibody is defined as 100%, the cell survival rate of MAb1 was 58% in the 12 ng/mL addition group, 53% in the 60 ng/mL addition group, 44% in the 300 ng/mL addition group, and 47% in the 1500 ng/mL addition group (n = 3) (Figure 4). In a Fab-ZAP non-addition group, no change in survival rate was found in the control antibody rat IgG2a or MAb1 addition group at any concentration. This demonstrated that MAb1 is an antibody that is internalized in CD25-positive cells.

3)-5 Cross-competition assay of anti-human CD25 antibody and M-A251

The test was carried out in accordance with the method of 2)-3-7. As a result, the binding of Biotin MA251 to CHO-1 cells expressing human CD25 was not suppressed by the addition of human IgG1 and was suppressed by the addition of M-A251, confirming the validity of the evaluation system (Figure 5). In this evaluation system, the binding of Biotin M-A251 to CHO-1 cells expressing human CD25 was not suppressed by the addition of MAb1. This demonstrated that MAb1 and M-A251 bind to different sites of human CD25 (Figure 5).

(Example 4) Property of MAb2

4)-1 Antigen binding activity

The test was carried out in accordance with the method of 2)-3-1. MAb2 was added at final antibody concentrations of 0.02 μg/mL, 0.16 μg/mL, 1.25 μg/mL, and 10 μg/mL using a purified antibody. PBS containing 5% FBS was added instead of an antibody solution to antibody non-addition wells (0 μg/mL). As a result, MAb2 bound to CHO-K1 cells harboring pCMV3-mCD25 and did not bind to CHO-K1 cells harboring pCMV3-hCD25 or pCI-cCD25 (Figure 6). This demonstrated that MAb2 binds to mouse CD25.

4)-2 Influence on phosphorylated STAT5 (pSTAT5) whose level is increased by IL-2 stimulation in mouse CTLL-2 cell.

The test was carried out in accordance with the method of 2)-3-9. When the amount of pSTAT5 in the group supplemented with IL-2 and the control antibody rat IgG2a was defined as 100%, the amount of pSTAT5 was 48% in the group supplemented with IL-2 and PC61_hIgG1LALA and 96.5% in the group supplemented with IL-2 and MAb2 (Figure 7). The amount of pSTAT5 increased by the addition of IL-2 was decreased in the IL-2 blocking antibody PC61_hIgG1LALA addition, demonstrating that MAb2 is an antibody that does not block IL-2 signals.

4)-3 Internalization activity measurement

The test was carried out in accordance with the method of 2)-3-10. MAb2 was added at a concentration of 7500 ng/mL, 1500 ng/mL, 300 ng/mL, and 60 ng/mL to each well using a purified antibody (n = 3). When a cell survival rate at each concentration of a control antibody is defined as 100%, the cell survival rates of MAb2 and PC61 were 6% and 59%, respectively, in the 60 ng/mL addition group, 4% and 39%, respectively, in the 300 ng/mL addition group, 4% and 31%, respectively, in the 1500 ng/mL addition group, and 5% and 29%, respectively, in the 7500 ng/mL addition group (Figure 8). In a Fab-ZAP non-addition group, no change in survival rate was found in the control antibody rat IgG2a, PC61, or MAb2 addition group at any concentration. This demonstrated that MAb2 is an antibody that is internalized in CD25-positive cells.

(Example 5) Determination of amino acid sequences of MAb1 and MAb2

5)-1 Preparation of total RNA from MAb1- or MAb2-producing hybridoma

In order to amplify cDNA of each variable region of MAb1 or MAb2, total RNA was prepared from a MAb1- or MAb2-producing hybridoma using a RNA purification kit Direct-zol RNA Miniprep kit (Zymo Research Corp.).

5)-2 Amplification of cDNA encoding light chain variable region of MAb1 or MAb2 by 5'-RACE PCR, and sequencing

cDNA of the light chain variable region was amplified using approximately 1 μg of the total RNA prepared in 5)-1 and SMARTer RACE 5'/3' Kit (manufactured by Thermo Fisher Scientific Inc.) The primers used for amplifying the cDNA of the light chain variable region of MAb1 or MAb2 by PCR were UPM (Universal Primer A Mix: included in SMARTer RACE 5'/3' Kit) and a primer designed from the sequence of a rat light chain constant region known in the art.

The cDNA of the light chain variable region amplified by 5'-RACE PCR was cloned into a plasmid. Subsequently, sequencing analysis was carried out on the nucleotide sequence of the cDNA of the light chain variable region.

The determined cDNA nucleotide sequences of the light chain variable regions of MAb1 and MAb2 are shown in SEQ ID NOs: 19 and 23, respectively, and their amino acid sequences are shown in SEQ ID NOs: 18 and 22, respectively.

5)-3 Amplification of cDNA encoding heavy chain variable region of MAb1 or MAb2 by 5'-RACE PCR, and sequencing

cDNA of the heavy chain variable region was amplified using approximately 1 μg of the total RNA prepared in 5)-1 and SMARTer RACE 5'/3' Kit (manufactured by Thermo Fisher Scientific Inc.) The primers used for amplifying the cDNA of the heavy chain variable region of MAb1 or MAb2 by PCR were UPM (Universal Primer A Mix: included in SMARTer RACE 5'/3' Kit) and a primer designed from the sequence of a rat heavy chain constant region known in the art.

The cDNA of the heavy chain variable region amplified by 5'-RACE PCR was cloned into a plasmid. Subsequently, sequencing analysis was carried out on the nucleotide sequence of the cDNA of the heavy chain variable region.

The determined cDNA nucleotide sequences of the heavy chain variable regions of MAb1 and MAb2 are shown in SEQ ID NOs: 21 and 25, respectively, and their amino acid sequences are shown in SEQ ID NOs: 20 and 24, respectively.

(Example 6) Preparation of human IgG1LALA chimeric MAb1 (cMAb1_hIgG1LALA) and human IgG1LALA chimeric MAb2 (cMAb2_hIgG1LALA)

6)-1 Construction of light chain expression vector pCMA-LK

A fragment of approximately 5.4 kb obtained by the digestion of a plasmid pcDNA3.3-TOPO/LacZ (manufactured by Thermo Fisher Scientific Inc.) with restriction enzymes XbaI and PmeI, and a DNA fragment comprising a nucleotide sequence (shown in SEQ ID NO: 51) encoding a human light chain signal sequence and a human κ chain constant region were ligated using In-Fusion HD PCR cloning kit (manufactured by Thermo Fisher Scientific Inc.) to prepare pcDNA3.3/LK.

A neomycin expression unit was removed from pcDNA3.3/LK to construct pCMA-LK.

6)-2 Construction of heavy chain expression vector pCMA-G1LALA

A DNA fragment that lost the nucleotide sequence encoding the light chain signal sequence and the human κ chain constant region by the digestion of pCMA-LK with XbaI and PmeI, and a DNA fragment comprising a nucleotide sequence (shown in SEQ ID NO: 52) encoding a human heavy chain signal sequence and a human IgG1LALA constant region were ligated using In-Fusion HD PCR cloning kit (manufactured by Thermo Fisher Scientific Inc.) to construct pCMA-G1LALA.

6)-3 Construction of cMAb1_hIgG1LALA and cMAb2_hIgG1LALA light chain expression vectors

A DNA fragment represented by nucleotide positions 61 to 381 of the cMAb1_hIgG1LALA light chain nucleotide sequence represented by SEQ ID NO: 28 and a DNA fragment represented by nucleotide positions 61 to 402 of the cMAb2_hIgG1LALA light chain nucleotide sequence represented by SEQ ID NO: 34 were synthesized (manufactured by Thermo Fisher Scientific Inc.). Each synthesized DNA fragment was inserted into a cleavage site of pCMA-LK with a restriction enzyme BsiWI using In-Fusion HD PCR cloning kit (manufactured by Thermo Fisher Scientific Inc.) to construct cMAb1_hIgG1LALA and cMAb2_hIgG1LALA light chain expression vectors. The amino acid sequence of the cMAb1_hIgG1LALA light chain is shown in SEQ ID NO: 26, and the amino acid sequence of the cMAb2_hIgG1LALA light chain is shown in SEQ ID NO: 30.

6)-4 Construction of cMAb1_hIgG1LALA and cMAb2_hIgG1LALA heavy chain expression vectors

A DNA fragment represented by nucleotide positions 58 to 438 of the cMAb1_hIgG1LALA heavy chain nucleotide sequence represented by SEQ ID NO: 29 and a DNA fragment represented by nucleotide positions 58 to 399 of the cMAb2_hIgG1LALA heavy chain nucleotide sequence represented by SEQ ID NO: 35 were synthesized (manufactured by Thermo Fisher Scientific Inc.). Each synthesized DNA fragment was inserted into a cleavage site of pCMA-G1LALA with a restriction enzyme BlpI using In-Fusion HD PCR cloning kit (manufactured by Thermo Fisher Scientific Inc.) to construct cMAb1_hIgG1LALA and cMAb2_hIgG1LALA heavy chain expression vectors. The amino acid sequence of the cMAb1_hIgG1LALA heavy chain is shown in SEQ ID NO: 27, and the amino acid sequence of the cMAb2_hIgG1LALA heavy chain is shown in SEQ ID NO: 31.

6)-5 Preparation of cMAb1_hIgG1LALA and cMAb2_hIgG1LALA

6)-5-1 Production of cMAb1_hIgG1LALA and cMAb2_hIgG1LALA

FreeStyle 293F cells (manufactured by Thermo Fisher Scientific Inc.) were passaged and cultured in accordance with the manual. $1.2 \times 10^9$ FreeStyle 293F cells (manufactured by Thermo Fisher Scientific Inc.) in the logarithmic growth phase were inoculated to 3-L Fernbach Erlenmeyer Flask (manufactured by Corning Inc.) and diluted into $2.0 \times 10^6$ cells/mL with FreeStyle 293 expression medium (manufactured by Thermo Fisher Scientific Inc.). 1.8 mg of Polyethyleneimine (manufactured by Polyscience) was added to 20 mL of Opti-Pro SFM medium (manufactured by Thermo Fisher Scientific Inc.). Subsequently, 0.24 mg of the heavy chain expression vector and 0.36 mg of the light chain expression vector were added to 20 mL of Opti-Pro SFM medium (manufactured by Thermo Fisher Scientific Inc.). The expression vector/Opti-Pro SFM mixed solution was added to the Polyethyleneimine/Opti-Pro SFM mixed solution, and the mixture was gently stirred, further left for 5 minutes, and then added to the FreeStyle 293F cells. The cells were shake-cultured at 90 rpm in an 8% $CO_2$ incubator at 37°C for 4 hours. Then, 600 mL of EX-CELL VPRO medium (manufactured by SAFC Biosciences Inc.), 18 mL of GlutaMAX I (manufactured by Gibco/Thermo Fisher Scientific Inc.), and 30 mL of Yeastolate Ultrafiltrate (manufactured by Gibco/Thermo Fisher Scientific Inc.) were added thereto, and the cells were shake-cultured at 90 rpm in an 8% $CO_2$ incubator at 37°C for 7 days. The obtained culture supernatant was filtrated through Disposable Capsule Filter (manufactured by Advantec).

6)-5-2 Purification of cMAb1_hIgG1LALA

From the culture supernatant obtained in 6)-5-1, the antibody was purified by two steps using rProtein A affinity chromatography and ceramic hydroxyapatite. The culture supernatant was applied to a column (manufactured by Cytiva) packed with MabSelectSuRe equilibrated with PBS, and the column was then washed with PBS in an amount of two or more times the volume of the column. Subsequently, the antibody was eluted with a 2 M arginine hydrochloride solution (pH 4.0). The fraction containing the antibody was dialyzed (manufactured by Thermo Fisher Scientific Inc., Slide-A-Lyzer Dialysis Cassette) for buffer replacement with PBS, diluted 5-fold with a buffer of 5 mM sodium phosphate/50 mM MES/pH 7.0, and was then applied to a ceramic hydroxyapatite column (manufactured by Bio-Rad Laboratories, Inc., Bio-Scale

CHT Type-1 Hydroxyapatite Column) equilibrated with a buffer of 5 mM NaPi/50 mM MES/30 mM NaCl/pH 7.0. Elution was carried out on a linear concentration gradient of sodium chloride to collect a fraction containing the antibody. The fraction was dialyzed (manufactured by Thermo Fisher Scientific Inc., Slide-A-Lyzer Dialysis Cassette) for buffer replacement with HBSor (25 mM histidine/5% sorbitol, pH 6.0). Finally, the antibody solution was filtered through Minisart-Plus filter (manufactured by Sartorius Stedim Biotech SA) to prepare a purified sample.

6)-5-3 Purification of cMAb2_hIgG1LALA

From the culture supernatant obtained in 6)-5-1, the antibody was purified by rProtein A affinity chromatography. The culture supernatant was applied to a column (manufactured by Cytiva) packed with MabSelectSuRe equilibrated with PBS, and the column was then washed with PBS in an amount of two or more times the volume of the column. Subsequently, the antibody was eluted with a 2 M arginine hydrochloride solution (pH 4.0). The eluted fraction was dialyzed (manufactured by Thermo Fisher Scientific Inc., Slide-A-Lyzer Dialysis Cassette) for buffer replacement with HBSor (25 mM histidine/5% sorbitol, pH 6.0) (concentration was not adjusted). Finally, the antibody solution was filtered through Minisart-Plus filter (manufactured by Sartorius Stedim Biotech SA) to prepare a purified sample.

(Example 7) Property of cMAb1_hIgG1LALA

7)-1 Measurement of antigen binding activity

The test was carried out in accordance with the method of 2)-3-1. cMAb1_hIgG1LALA to be added to CHO-K1 cells was added in the range of 80 to 0.001 μg/mL. The secondary antibody used was Peroxidase-AffiniPure F(ab')2 Fragment Goat Anti-Human IgG (H + L) (Jackson ImmunoResearch Laboratories, Inc.) diluted 1000-fold with PBS containing 5% FBS. TMB Microwell Peroxidase Substrate (2-Component System) (SeraCare Life Sciences, Inc.) and TMB Stop Solution (SeraCare Life Sciences, Inc.) were used in color development and its termination, respectively. As a result, cMAb1_hIgG1LALA bound to CHO-K1 cells harboring pCMV3-hCD25 or pCI-cCD25 in an antibody concentration-dependent manner, and did not bind to CHO-K1 cells harboring pCMV3-mCD25 (Figure 9). This demonstrated that cMAb1_hIgG1LALA binds to human CD25 and cynomolgus monkey CD25.

7)-2 Influence on IL-2-dependent cell proliferation using human CD4-positive T cell

7)-2-1 Preparation of human CD4-positive T cell and serum starvation

Frozen human PBMC (Cellular Technology Limited) was thawed in accordance with C.T.L. Protocol. Then, CD4-positive T cells were separated from PBMC using CD4+ T Cell isolation kit, human (Miltenyi Biotec). The separated CD4-positive T cells were suspended at $2 \times 10^5$ cells/mL in IMDM (Thermo Fisher Scientific Inc.) medium containing 10% CTS Immune Cell SR (Thermo Fisher Scientific Inc.) to prepare a cell suspension. 25 μL of the cell suspension was inoculated to each well of a 96-well plate (U-bottom), and PHA (Sigma-Aldrich Co., LLC) at 2 μg/mL and Phorbol 12-myristate 13-acetate (PMA) (Sigma-Aldrich Co., LLC) at 0.2 μg/mL were added. Further, human IgG1, basiliximab, or cMAb1_hIgG1LALA was added at a final concentration of 15 μg/mL. The plate was incubated for 5 days under conditions of 37°C and 5% $CO_2$. The plate thus incubated was brought back to room temperature. 100 μL of Celltiter-Glo Reagent of CellTiter-Glo luminescent Cell Viability Assay (Promega Corp.) kit was added to each well, and the plate was stirred for 10 minutes using a plate shaker. 100 μL/well of the reaction solution was transferred to 96-well White Flat Bottom plate, and luminescence intensity (relative light unit (RLU)) was measured using a plate reader (Ensight: PerkinElmer, Inc.) and used as an index for the number of cells. As a result, when the number of cells in the human IgG1 addition group was defined as 100%, the number of cells was 34% in the IL-2 blocking antibody basiliximab addition group and 92% in the cMAb1_hIgG1LALA addition group (Figure 10). The number of T cells in the IL-2 blocking antibody basiliximab addition group was decreased as compared with the human IgG1 addition group whereas the number of T cells in the cMAb1_hIgG1LALA addition group was not changed. This demonstrated that cMAb1_hIgG1LALA is an IL-2 non-blocking antibody that does not influence the proliferation of human CD4-positive T cells.

7)-3 Influence on phosphorylated STAT5 (pSTAT5) whose level is increased by IL-2 stimulation in human PBMC

7)-3-1 Immobilization of anti-human CD3 antibody onto well of 6-well plate

PBS containing 10 μg/mL anti-human CD3 antibody (EXBIO Praha) was added at 2 mL/well to each well of a 6-well plate (Corning Inc.), and the plate was then mixed using a plate mixer and incubated at 4°C. A supernatant was removed from the plate thus incubated, and the wells were washed three times with PBS and used.

7)-3-2 Preparation of human PBMC and serum starvation

Frozen human PBMC was thawed in accordance with C.T.L. Protocol. The thawed PBMC was suspended in IMDM (Thermo Fisher Scientific Inc.) medium containing 10% CTS Immune Cell SR (Thermo Fisher Scientific Inc.) and inoculated at 4 mL/well to the 6-well plate provided in 7)-3-1. The plate was incubated for 3 days under conditions of 37°C and 5% $CO_2$.

7)-3-3 Preparation of antibody solution and addition

A control antibody human IgG1, an anti-human CD25 antibody basiliximab (Novartis Pharma K.K.), or cMAb1_hIgG1LALA was diluted into 80 μg/mL with IMDM medium (Thermo Fisher Scientific Inc.), and 25 μL of the dilution was added to each well of a V-bottom 96-well plate (Corning Inc.). 25 μL of IMDM medium (Thermo Fisher Scientific Inc.) was added to each of antibody non-addition wells.

7)-3-4 Addition of human PBMC and IL-2 solution

The serum-starved PBMC was recovered and adjusted to $2 \times 10^6$ cells/mL with IMDM medium (Thermo Fisher Scientific Inc.). The cell suspension was added at 50 μL/well to the plate supplemented with the antibody solution in 7)-3-3. Further, the plate was incubated for 15 minutes under conditions of 5% $CO_2$ and 37°C.

Recombinant human IL-2 (PeproTech, Inc.) was dissolved in 50 μL of 0.1 M acetic acid and then diluted with 0.45 mL of PBS containing 0.1% BSA to prepare a 100 μg/mL solution. Further, the solution was diluted into 4 ng/mL with IMDM medium (Thermo Fisher Scientific Inc.) and added at 25 μL/well to wells of an IL-2 addition group. IMDM medium (Thermo Fisher Scientific Inc.) was added at 25 μL/well to wells of an IL-2 non-addition group. Then, the plate was incubated for 15 minutes under conditions of 5% $CO_2$ and 37°C.

7)-3-5 pSTAT5 measurement

5 × lysis buffer included in AlphaLISA SureFire Ultra phosho-STAT5 (Tyr694/699) kit (PerkinElmer, Inc.) was adjusted to 1 × lysis buffer with $H_2O$.

The plate incubated for 15 minutes was centrifuged at 340 × g for 5 minutes, and a supernatant was removed by suction from each well. Then, PBS was added to each well, and the plate was centrifuged in the same manner as above, followed by the removal of a supernatant by suction.

The 1 × lysis buffer was added at 50 μL/well to each well. The plate was stirred for 10 minutes using a plate shaker to prepare a cell lysate. The cell lysate of each well was added at 8 μL/well to 384-well SW plate for AlphaLISA (PerkinElmer, Inc.), and Acceptor Mix was added at 4 μL/well, followed by stirring for 2 minutes using a plate shaker. Then, the plate was incubated for 1 hour under light shielding.

Donor Mix was added at 4 μL/well, and the plate was stirred for 2 minutes using a plate shaker and then further incubated overnight under light shielding. Then, Alpha signals were measured using a plate reader (Ensight: PerkinElmer, Inc.). As a result, when the amount of pSTAT5 in the group supplemented with IL-2 and the control antibody human IgG1 was defined as 100%, the amount of pSTAT5 was 1.2% in the group supplemented with IL-2 and the IL-2 blocking antibody basiliximab and 83.2% in the group supplemented with IL-2 and cMAb1_hIgG1LALA (Figure 11). The amount of pSTAT5 increased by the addition of IL-2 was decreased in the IL-2 blocking antibody basiliximab addition group and was not changed by the addition of cMAb1_hIgG1LALA. This demonstrated that cMAb1_hIgG1LALA is an antibody that does not block IL-2 signals.

(Example 8) Preparation of cMAb1_hIgG1LALA-ADC, cMAb1_hIgG1LALA-ADC2, cMAb2_hIgG1LALA-ADC, and cMAb2_hIgG1LALA-ADC2

cMAb1_hIgG1LALA-ADC, cMAb1_hIgG1LALA-ADC2, cMAb2_hIgG1LALA-ADC, and cMAb2_hIgG1LALA-ADC2 were prepared by a method known in the art (e.g., the method of WO2014/057687) using cMAb1_hIgG1LALA and cMAb2_hIgG1LALA prepared in Example 6.

The cMAb1_hIgG1LALA antibody comprises a heavy chain comprising an amino acid sequence from positions 20 to 146 of SEQ ID NO: 27 and a light chain comprising an amino acid sequence from positions 21 to 126 of SEQ ID NO: 26. The cMAb2_hIgG1LALA antibody comprises a heavy chain comprising an amino acid sequence from positions 20 to 133 of SEQ ID NO: 31 and a light chain comprising an amino acid sequence from positions 21 to 133 of SEQ ID NO: 30. The cMAb1_hIgG1LALA antibody and the cMAb2_hIgG1LALA antibody were each linked to a compound represented by the following formula through a linker.

[Formula 18]

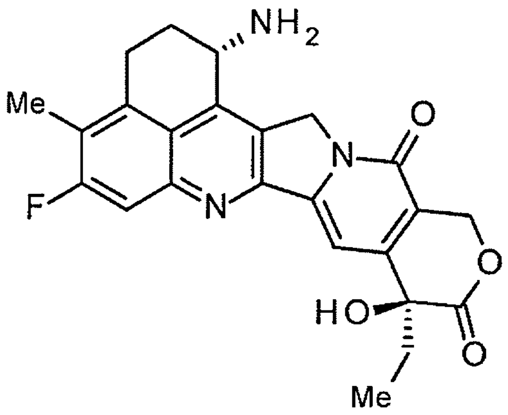

Such cMAb1_hIgG1LALA-ADC and cMAb2_hIgG1LALA-ADC have a structure represented by the following formula (n: the number of drug molecules conjugated per antibody molecule is 4 to 8, i.e., the average number (n) of drug molecules conjugated per antibody molecule: approximately 8), and AB represents cMAb1_hIgG1LALA or cMAb2_hIgG1LALA.

[Formula 19]

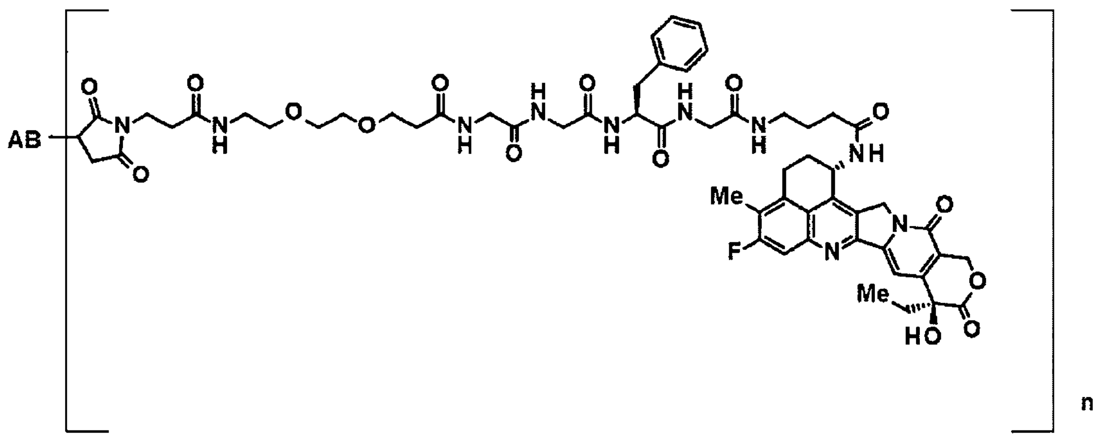

cMAb1_hIgG1LALA-ADC2 and cMAb2_hIgG1LALA-ADC2 have a structure represented by the following formula (n: the number of drug molecules conjugated per antibody molecule is 4 to 8, i.e., the average number (n) of drug molecules conjugated per antibody molecule: approximately 8), and AB represents cMAb1_hIgG1LALA or cMAb2_hIgG1LALA.

[Formula 20]

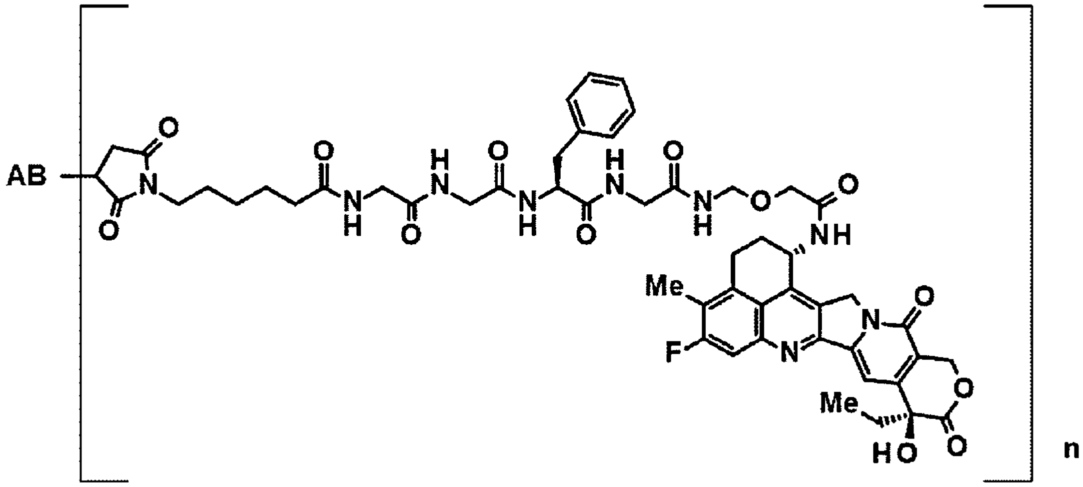

Control hIgG-ADC is constituted by the same drug-linker as in cMAb1_hIgG1LALA-ADC and cMAb2_hIgG1LALA-ADC using a humanized IgG1 isotype control monoclonal antibody that does not bind to mammalian cells. The drug linker was prepared by a method known in the art (e.g., the method of WO2015/115091).

8)-1 Preparation of antibody-drug conjugate cMAb1_hIgG1LALA-ADC

[Formula 21]

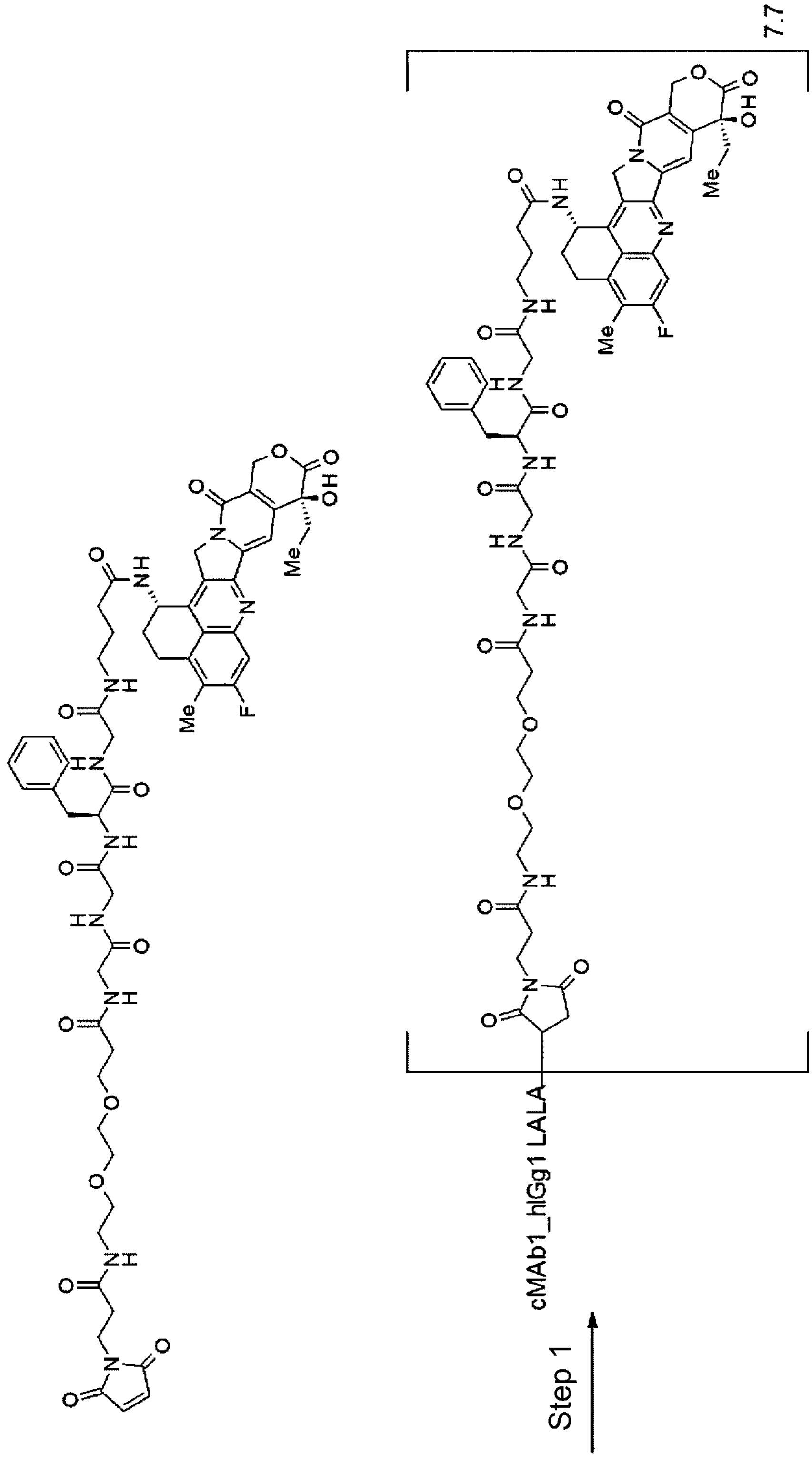


Reduction of antibody: cMAb1_hIgG1LALA prepared in Example 6 was adjusted to 10.3 mg/mL with PBS6.0/EDTA by use of common procedures B (using 1.60 $mLmg^{-1}cm^{-1}$ as 280 nm absorption coefficient) and C described in production method 1. To this solution (4.0 mL), an aqueous solution of 10 mM TCEP (Tokyo Chemical Industry Co., Ltd.) (0.169 mL; 6.0 equivalents per antibody molecule) and a 1 M aqueous dipotassium hydrogen phosphate solution (Nacalai Tesque, Inc.; 0.0600 mL) were added. After confirming that the solution had a pH within 7.0 $\pm$ 0.1, the interchain disulfide bond in the antibody was reduced by incubating the solution at 37°C for 2 hours.

Conjugation between antibody and drug linker: The solution was incubated at 15°C for 10 minutes. Subsequently, a 10 mM solution of N-{3-[2-(2-{[3-(2,5-dioxo-2,5-dihydro-1H-pyrrol-1-yl)propanoyl]amino}ethoxy)ethoxy]propanoyl}glycylglycyl-L-phenylalanyl-N-(4-{[(1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl]amino}-4-oxobutyl)glycinamide (WO2015/115091) in dimethyl sulfoxide (0.282 mL; 10 equivalents per antibody molecule) was added thereto, and the mixture was incubated at 15°C for 1 hour to conjugate the drug linker to the antibody. Subsequently, an aqueous solution of 100 mM NAC (Sigma-Aldrich Co. LLC) (0.0282 mL; 10 equivalents per antibody molecule) was added thereto, and the mixture was further stirred at room temperature for 20 minutes to terminate the reaction of the drug linker.

Purification: The solution was purified by common procedure D described in production method 1 to obtain 15.0 mL of a solution containing the title antibody-drug conjugate "cMAb1_hIgG1LALA-ADC".

Characterization: Using common procedure E (using $\varepsilon_{D,280}$ = 7642 and $\varepsilon_{D,370}$ = 24111) described in production method 1, the following characteristic values were obtained.

Antibody concentration: 2.36 mg/mL, antibody yield: 35.4 mg (86%), average number of conjugated drug molecules (n) per antibody molecule measured by common procedure E: 5.1; average number of conjugated drug molecules (n) per antibody molecule measured by common procedure F: 7.7.

8)-2 Preparation of antibody-drug conjugate cMAb1_hIgG1LALA-ADC2

[Formula 22]

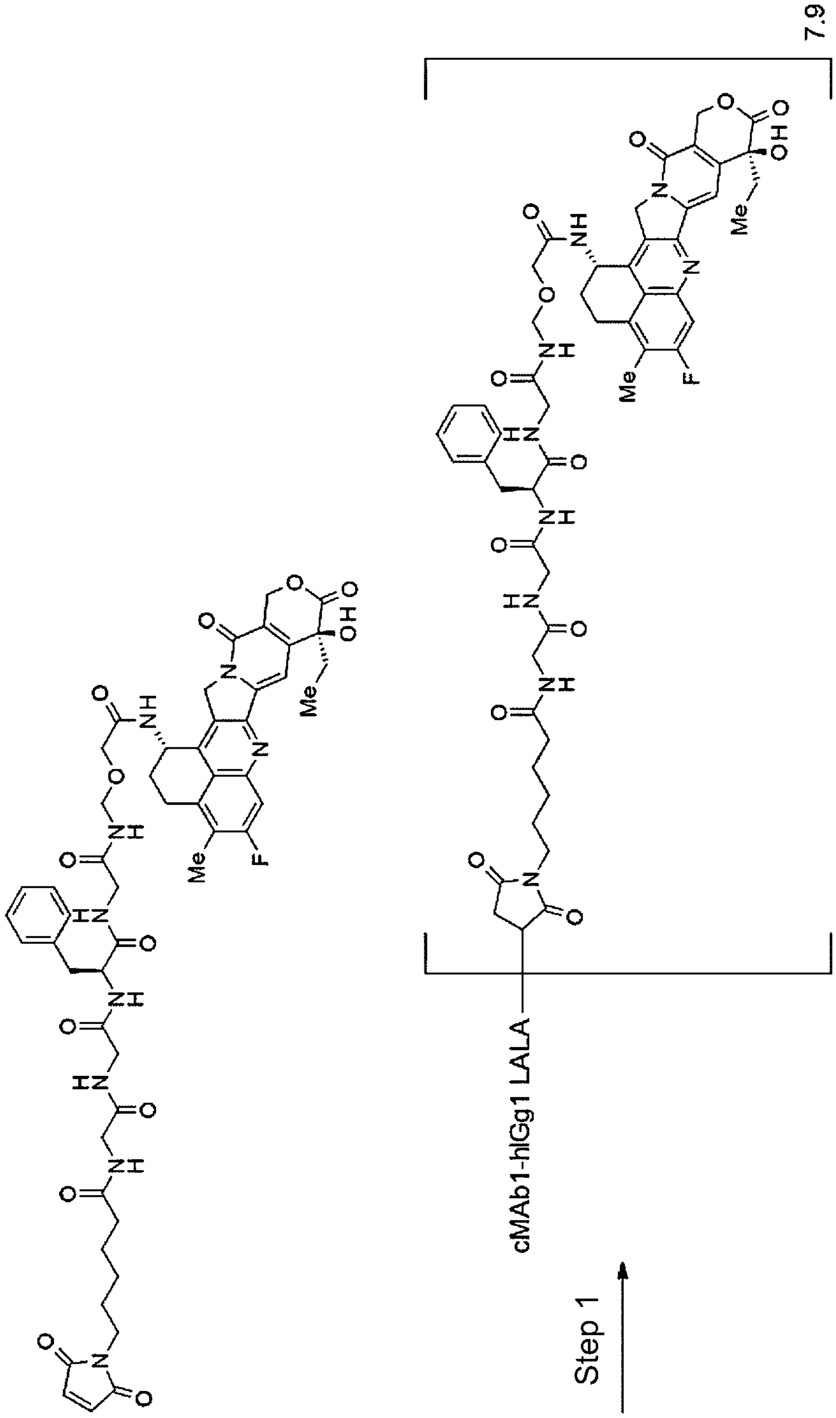


Reduction of antibody: cMAb1_hIgG1LALA prepared in Example 6 was adjusted to 10.3 mg/mL with PBS6.0/EDTA by use of common procedures B (using 1.60 $mLmg^{-1}cm^{-1}$ as 280 nm absorption coefficient) and C described in production method 1. To this solution (4.0 mL), an aqueous solution of 10 mM TCEP (Tokyo Chemical Industry Co., Ltd.) (0.169 mL; 6.0 equivalents per antibody molecule) and a 1 M aqueous dipotassium hydrogen phosphate solution (Nacalai Tesque, Inc.; 0.0600 mL) were added. After confirming that the solution had a pH within 7.0 $\pm$ 0.1, the interchain disulfide bond in the antibody was reduced by incubating the solution at 37°C for 2 hours.

Conjugation between antibody and drug linker: The solution was incubated at 15°C for 10 minutes. Subsequently, a 10 mM solution of N-[6-(2,5-dioxo-2,5-dihydro-1H-pyrrol-1-yl)hexanoyl]glycylglycyl-L-phenylalanyl-N-[(2-{[(1S,9S)-9-ethyl-5-fluoro-9-hidroxy-4-methyl-10,13-dioxo-2,3,9,10,13,15-hexahydro-1H,12H-benzo[de]pyrano [3',4':6,7]indolizino[1,2-b]quinolin-1-yl]amino}-2-oxoethoxy)methyl]glycinamide (WO2015/115091) in dimethyl sulfoxide (0.282 mL; 10 equivalents per antibody molecule) was added thereto, and the mixture was incubated at 15°C for 1 hour to conjugate the drug linker to the antibody. Subsequently, an aqueous solution of 100 mM NAC (Sigma-Aldrich Co. LLC)

(0.0282 mL; 10 equivalents per antibody molecule) was added thereto, and the mixture was further stirred at room temperature for 20 minutes to terminate the reaction of the drug linker.

Purification: The solution was purified by common procedure D described in production method 1 to obtain 15.0 mL of a solution containing the title antibody-drug conjugate "cMAb1_hIgG1LALA-ADC2".

Characterization: Using common procedure E (using $\varepsilon_{D,280}$ = 5440 and $\varepsilon_{D,370}$ = 21240) described in production method 1, the following characteristic values were obtained.

Antibody concentration: 1.74 mg/mL, antibody yield: 26.2 mg (63%), average number of conjugated drug molecules (n) per antibody molecule measured by common procedure E: 6.2; average number of conjugated drug molecules (n) per antibody molecule measured by common procedure F: 7.9.

8)-3 Preparation of antibody-drug conjugate cMAb2_hIgG1LALA-ADC

[Formula 23]

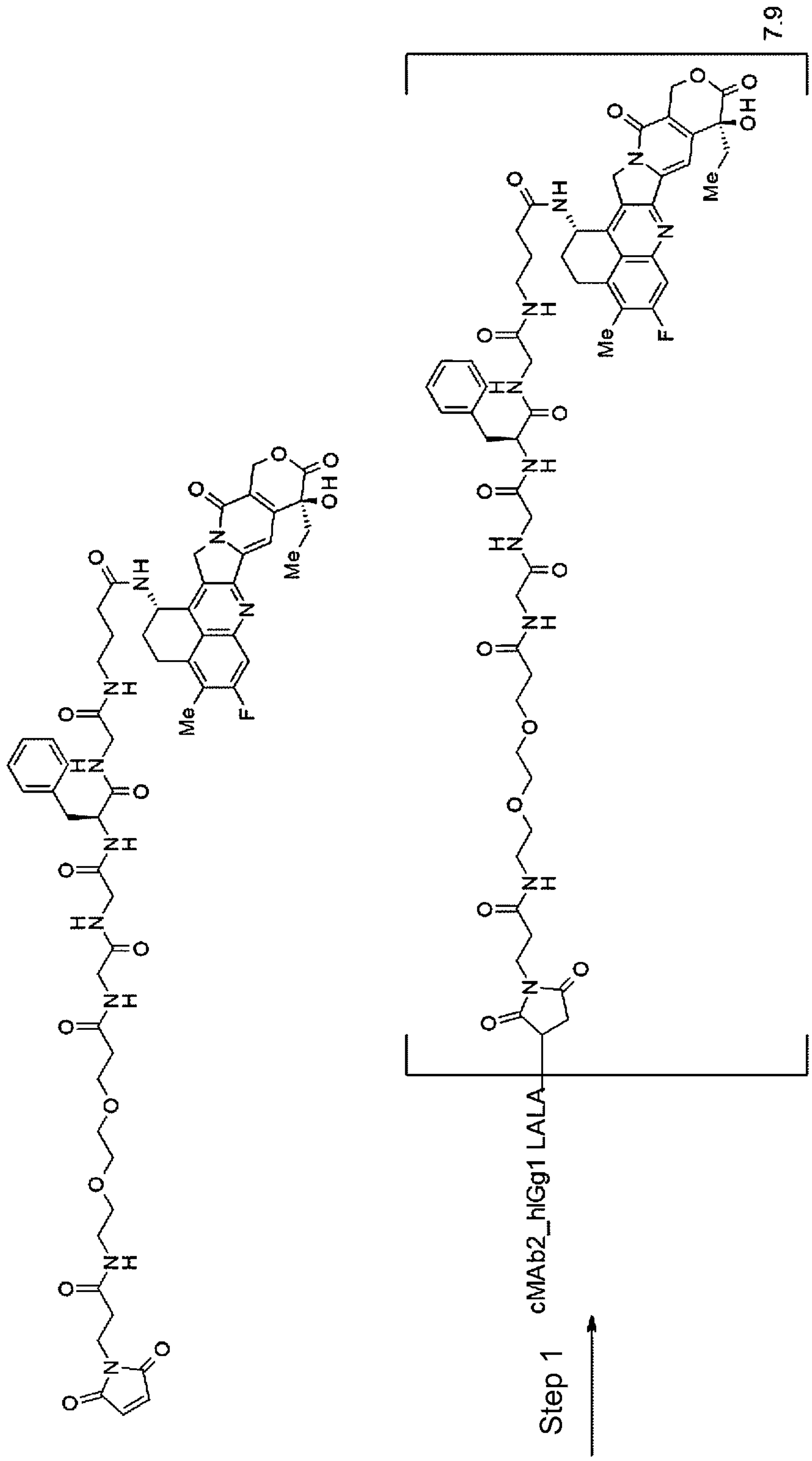


Reduction of antibody: cMAb2_hIgG1LALA prepared in Example 6 was adjusted to 10.1 mg/mL with PBS6.0/EDTA by use of common procedures B (using 1.45 $mLmg^{-1}cm^{-1}$ as 280 nm absorption coefficient) and C described in production method 1. To this solution (1.0 mL), an aqueous solution of 10 mM TCEP (Tokyo Chemical Industry Co., Ltd.) (0.0419 mL; 6.0 equivalents per antibody molecule) and a 1 M aqueous dipotassium hydrogen phosphate solution (Nacalai Tesque, Inc.; 0.0150 mL) were added. After confirming that the solution had a pH within 7.0 ± 0.1, the interchain disulfide bond in the antibody was reduced by incubating the solution at 37°C for 2 hours.

Conjugation between antibody and drug linker: The solution was incubated at 15°C for 10 minutes. Subsequently, a 10 mM solution of N-{3-[2-(2-{[3-(2,5-dioxo2,5-dihydro-1H-pyrrol-1-yl)propanoyl]amino}ethoxy)ethoxy]propanoyl}glycylglycyl-L-phenylalanyl-N-(4-{[(1S,9S)-9-ethyl-5-fluoro-9-hidroxy-4-methyl-10,13-dioxo-2,3,9,10,13,15-hexahydro-1H,12H-benzo[de]pyrano[3',4':6,7]indolizino[1,2-b]quinolin-1-yl]amino}-4-oxobutyl)glycinamide in dimethyl sulfoxide (0.0698 mL; 10 equivalents per antibody molecule) was added thereto, and the mixture was incubated at 15°C for 1 hour to conjugate the drug linker to the antibody. Subsequently, an aqueous solution of 100 mM NAC (Sigma-Aldrich Co. LLC) (0.0070 mL; 10 equivalents per antibody molecule) was added thereto, and the mixture was further stirred at room temperature for 20 minutes to terminate the reaction of the drug linker.

Purification: The solution was purified by common procedure D described in production method 1 to obtain 6.0 mL of a solution containing the title antibody-drug conjugate "cMAb2_hIgG1LALA-ADC".

Characterization: Using common procedure E (using $\varepsilon_{D,280}$ = 7642 and $\varepsilon_{D,370}$ = 24111) described in production method 1, the following characteristic values were obtained.

Antibody concentration: 1.38 mg/mL, antibody yield: 8.30 mg (82%), average number of conjugated drug molecules (n) per antibody molecule measured by common procedure E: 5.5; average number of conjugated drug molecules (n) per antibody molecule measured by common procedure F: 7.9.

8)-4 Preparation of antibody-drug conjugate cMAb2_hIgG1LALA-ADC2

[Formula 24]

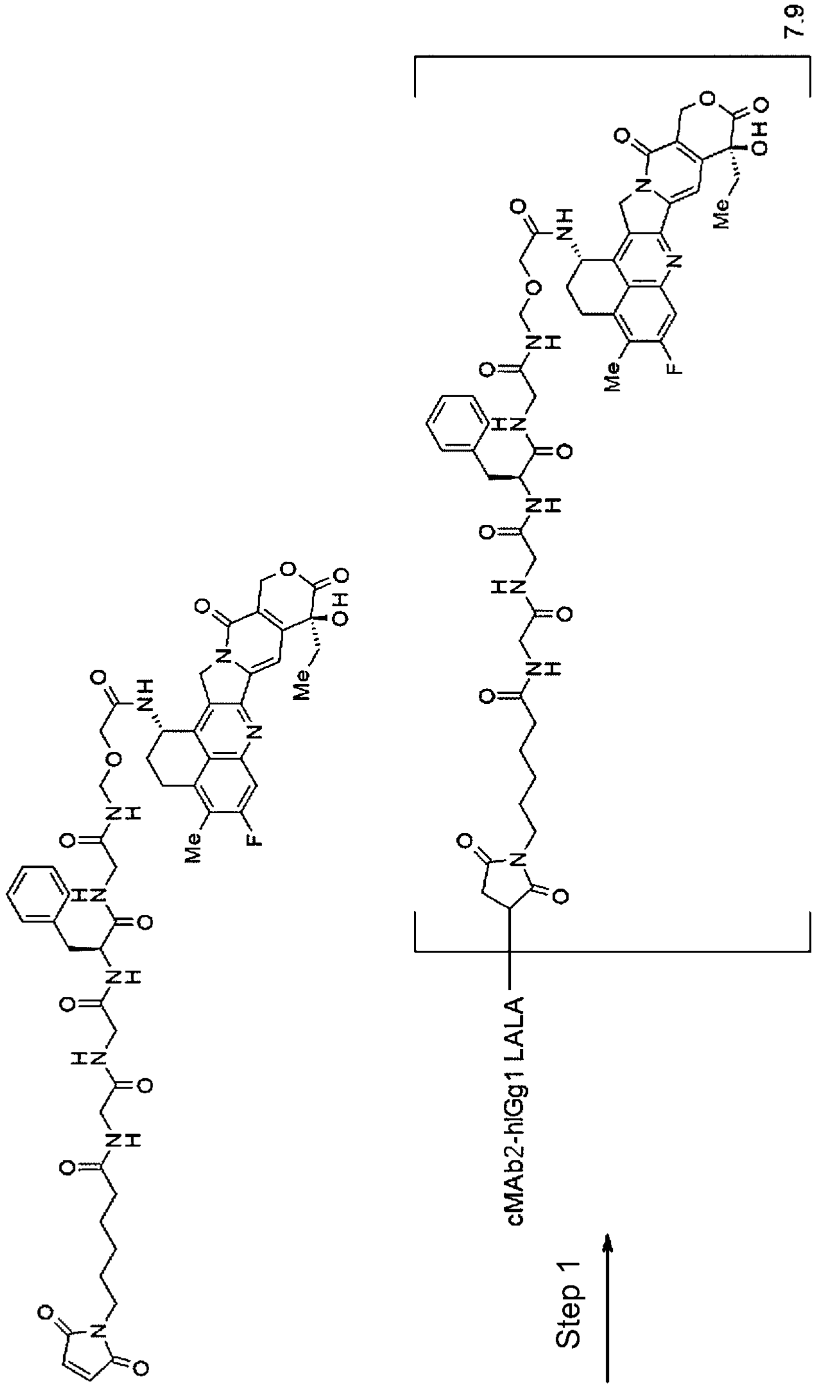


Reduction of antibody: cMAb2_hIgG1LALA prepared in Example 6 was adjusted to 10.1 mg/mL with PBS6.0/EDTA by use of common procedures B (using 1.45 $mLmg^{-1}cm^{-1}$ as 280 nm absorption coefficient) and C described in production method 1. To this solution (4.0 mL), an aqueous solution of 10 mM TCEP (Tokyo Chemical Industry Co., Ltd.) (0.168 mL; 6.0 equivalents per antibody molecule) and a 1 M aqueous dipotassium hydrogen phosphate solution (Nacalai Tesque, Inc.; 0.0600 mL) were added. After confirming that the solution had a pH within 7.0 ± 0.1, the interchain disulfide bond in the antibody was reduced by incubating the solution at 37°C for 2 hours.

Conjugation between antibody and drug linker: The solution was incubated at 15°C for 10 minutes. Subsequently, a 10 mM solution of N-[6-(2,5-dioxo-2,5-dihydro-1H-pyrrol-1-yl)hexanoyl]glycylglycyl-L-phenylalanyl-N-[(2-{[(1S,9S)-9-ethyl-5-fluoro-9-hidroxy-4-methyl-10,13-dioxo-2,3,9,10,13,15-hexahydro-1H,12H-benzo[de]pyrano [3',4':6,7]indolizino[1,2-b]quinolin-1-yl]amino}-2-oxoethoxy)methyl]glycinamide in dimethyl sulfoxide (0.278 mL; 10 equivalents per antibody molecule) was added thereto, and the mixture was incubated at 15°C for 1 hour to conjugate the drug linker to the antibody. Subsequently, an aqueous solution of 100 mM NAC (Sigma-Aldrich Co. LLC) (0.0278 mL; 10 equivalents per antibody molecule) was added thereto, and the mixture was further stirred at room temperature for 20

minutes to terminate the reaction of the drug linker.

Purification: The solution was purified by common procedure D described in production method 1 to obtain 17.5 mL of a solution containing the title antibody-drug conjugate "cMAb2_hIgG1LALA-ADC2".

Characterization: Using common procedure E (using $\varepsilon_{D,280}$ = 5440 and $\varepsilon_{D,370}$ = 21240) described in production method 1, the following characteristic values were obtained.

Antibody concentration: 2.06 mg/mL, antibody yield: 36.1 mg (90%), average number of conjugated drug molecules (n) per antibody molecule measured by common procedure E: 6.0; average number of conjugated drug molecules (n) per antibody molecule measured by common procedure F: 7.9.

(Example 9) Property of cMAb1_hIgG1LALA-ADC

9)-1 Cell proliferation suppressive activity of cMAb1_hIgG1LALA-ADC

9)-1-1 Cell culture

A CD25-positive human tumor cell line Karpas-299 was subcultured in RPMI1640 (Thermo Fisher Scientific Inc.) medium containing 2 mM glutamine (Life Technologies Corp.) and 20% FBS under conditions of 37°C and 5% $CO_2$. A CD25-negative human tumor cell line Daudi was subcultured in RPMI1640 medium containing 10% FBS under conditions of 37°C and 5% $CO_2$.

9)-1-2 Cell proliferation suppression assay

The cultured Karpas-299 was inoculated at $1 \times 10^3$ cells/50 μL/well in RPMI1640 medium containing 2 mM glutamine and 20% FBS to 96 well clear U bottom microplate (Corning Inc.). cMAb1_hIgG1LALA was added at 0.015 μg/mL to 15 μg/mL, and control hIgG1LALA-ADC and cMAb1_hIgG1LALA-ADC were added at 0.0015 μg/mL to 1.5 μg/mL. The cells were cultured for 6 days under conditions of 37°C and 5% $CO_2$. The incubated plate was left standing at room temperature for 30 minutes. Reagent solution of CellTiter-Glo(TM) Luminescent Cell Viability Assay (Promega Corp.) was added at 100μL/well, and the plate was mixed at room temperature for 10 minutes under light shielding using a plate mixer (Taitec Corp.). 100 μL of the solution of each well was added to each well of 96 well white flat bottom microplate (Corning Inc.), and luminescence intensity (relative light unit (RLU)) was measured using a plate reader (Ensight: PerkinElmer, Inc.) and used as an index for the number of cells.

Cell proliferation suppressive activity against the CD25 expression-negative human tumor cell line DauDi was measured in the same manner as above using RPMI1640 medium (Thermo Fisher Scientific Inc.) containing 10% FBS (GE Healthcare Bio-Sciences Corp.). Daudi was inoculated at $1 \times 10^4$ cells/50 μL/well.

As a result, cMAb1_hIgG1LALA-ADC exhibited cell proliferation suppressive activity at a lower concentration than that of human IgG1LALA-ADC only against the CD25-positive human tumor cell line Karpas-299 (Figure 12). The addition of cMAb1_hIgG1LALA exhibited no cell proliferation suppressive activity. This demonstrated that cMAb1_hIgG1LALA-ADC has proliferation suppressive activity against CD25-positive cells. cMAb1_hIgG1LALA-ADC2 exhibited similar effects. The suppression of proliferation of CD25-positive cells by these antibody-drug conjugates was able to be presumed to be based on the induction of cell death through the internalization of cMAb1_hIgG1LALA-ADC and cMAb1_hIgG1LALA-ADC2 to CD25-positive cells.

9)-2 *In vivo* immunoenhancing effect of cMAb1_hIgG1LALA-ADC

9)-2-1 Harvest of tumor tissue and preparation of cell suspension

$5 \times 10^6$ human breast adenocarcinoma-derived MDA-MB-231 cells (American Type Culture Collection) subcultured in Leibovitz's L-15 medium containing 10% FBS were suspended in Matrigel and subcutaneously transplanted to each female immune-humanized NOG mouse (huNOG mouse) engrafted with human CD34 cells. The mice were grouped on the basis of their tumor volumes. 7 days after transplantation, 1 mg/kg control hIgG1LALA-ADC or 0.1 mg/kg, 0.3 mg/kg, or 1 mg/kg cMAb1_hIgG1LALA-ADC was intravenously administered to each mouse (n = 5). 28 days after ADC administration, tumor was harvested. The harvested tumor mass was treated with gentleMACS (Miltenyi Biotec) to recover cells. The cells were washed with PBS and resuspended therein. Then, the cell suspension was used as a sample for flow cytometry. The amount of PBS in resuspending the cells was the following amount depending on a tumor weight for addition: 0.6 mL for a tumor weight up to 50 mg; 0.9 mL for a tumor weight of 50 to 100 mg; 1.2 mL for a tumor weight of 100 to 150 mg; 1.8 mL for a tumor weight of 150 to 200 mg; and 2.4 mL for a tumor weight of 200 to 300 mg.

9)-2-2 Preparation of staining solution for flow cytometry analysis

Zombie NIR dye solution was prepared by diluting Zombie NIR dye solution attached to Zombie NIR Fixable Viability Kit (BioLegend, Inc.) 1000-fold with PBS. Fc Block solution was prepared by adding and mixing Mouse BD Fc Block (BD Biosciences) solution, Human BD Fc Block solution, and autoMACS Rinsing Solution (Miltenyi Biotec) containing 0.5% BSA (hereinafter, referred to as "MACS buffer") at a ratio of 1:1:48. Foxp3 Fixation/Permeabilization buffer was prepared by mixing 3.5 mL of Concentrate and 10.5 mL of Diluent of Foxp3 Fixation/Permeabilization Concentrate and Diluent (eBioscience, Inc.). Perm wash buffer was prepared by diluting Intracellular Staining Perm Wash Buffer (BioLegend, Inc.) 10-fold with $H_2O$. A cell surface marker antibody cocktail solution was used by adding 2 μL of FITC anti-human CD25 Antibody (BioLegend, Inc.), 1 μL of APC anti-human CD8 Antibody (BioLegend, Inc.), 3 μL of Brilliant Violet 421 anti-human CD4 Antibody (BioLegend, Inc.), 2.5 μL of PE/Cyanine7 anti-human CD45RA Antibody (BioLegend, Inc.), 2 μL of Alexa Fluor 700 anti-mouse CD45 Antibody, 2 μL of Brilliant Violet 605 anti-human CD45 Antibody (BD Biosciences), and Brilliant Violet 650 anti-human CD3 Antibody, and adjusting the whole amount to 50 μL with MACS buffer. An intracellular marker antibody cocktail solution was used by adding 3 μL of PE Mouse anti-Human FoxP3 (BD Biosciences), 3 μL of PerCP/Cyanine5.5 anti-human/mouse Granzyme B Recombinant Antibody (BioLegend, Inc.), 1 μL of Mouse BD Fc Block (BD Biosciences), and 1 μL of Human BD Fc Block, and adjusting the whole amount to 100 μL with Perm wash buffer.

9)-2-3 Antibody staining of sample for flow cytometry analysis

0.1 mL of Zombie NIR dye diluting solution was added to 0.2 mL of the sample for flow cytometry prepared in 9)-2-1, and the mixture was incubated at 4°C for 30 minutes. 0.1 mL of MACS buffer was added thereto, and the mixture was centrifuged, followed by the removal of a supernatant by suction. 0.1 mL of Fc Block solution was added to the remaining cells, which were then incubated at 4°C for 5 minutes. 0.1 mL of the cell surface marker antibody cocktail solution prepared in 9)-2-2 was added to the cells, which were then incubated at 4°C for 30 minutes. Subsequently, 0.1 mL of MACS buffer was added thereto, and the mixture was centrifuged, followed by the removal of a supernatant by suction. 0.2 mL of MACS buffer was added to the remaining cells, and the mixture was further centrifuged to remove a supernatant. This operation was further carried out twice for the washing of the cells. 0.2 mL of Foxp3 Fixation/Permeabilization buffer was added to the cells, which were then incubated at 4°C for 1 hour. 0.2 mL of the intracellular marker antibody cocktail solution was added to the washed cells, which were then incubated at 4°C for 1 hour and then washed twice with Perm wash buffer. A 4% paraformaldehyde solution was added to the cells, which were then incubated at 4°C for 15 minutes. The cells were washed twice with MACS buffer, and 0.18 mL of MACS buffer was then added to the cells. The sample was incubated overnight at 4°C, followed by flow cytometry analysis.

9)-2-4 Flow cytometry analysis

The sample prepared in 9)-2-3 was applied to a flow cytometer (BD LSR Fortessa X-20 cell analyzer: BD Biosciences) to measure the numbers of human CD45-positive cells, human FoxP3-, CD4-, CD3-, and CD45-positive cells (human Treg cells), human CD8-, CD3-, and CD45-positive cells as CD8-positive T cells (human CD8-positive T cells), and human granzyme-positive CD8T cells (human GZMB-positive and CD8-positive T cells) in a Zombie NIR dye-negative fraction. Then, the respective numbers of human Treg cells, human CD8-positive T cells, and human GZMB-positive and CD8-positive T cells were divided by the number of human CD45-positive cells to calculate the ratios of the numbers of human Treg cells, human CD8-positive T cells, and human GZMB-positive and CD8-positive T cells to the number of human CD45 cells (% of hCD45). As a result, in the cMAb1_hIgG1LALA-ADC administration group compared with the control hIgG1LALA-ADC administration group, the ratio of the number of human Treg cells to the number of human CD45 cells was decreased in a dose-dependent manner, and increase in the ratios of the numbers of human CD8-positive T cells and human GZMB-positive and CD8-positive T cells was observed (Figure 13). These results demonstrated that cMAb1_hIgG1LALA-ADC activates human CD8-positive T cells by decreasing the number of human Treg. cMAb1_hIgG1LALA-ADC2 exhibited similar effects.

(Example 10) Property of cMAb2_hIgG1LALA

10)-1 Measurement of antigen binding activity

The test was carried out in accordance with the method of 2)-3-1. cMAb2_hIgG1LALA to be added to CHO-K1 cells was added in the range of 80 to 0.001 μg/mL. The secondary antibody used was Peroxidase-AffiniPure F(ab')2 Fragment Goat Anti-Human IgG (H + L) (Jackson ImmunoResearch Laboratories, Inc.) diluted 1000-fold with PBS containing 5% FBS. TMB Microwell Peroxidase Substrate (2-Component System) (SeraCare Life Sciences, Inc.) and

TMB Stop Solution (SeraCare Life Sciences, Inc.) were used in color development and its termination, respectively. As a result, cMAb2_hIgG1LALA bound to CHO-K1 cells harboring pCMV3-mCD25 in an antibody concentration-dependent manner, and did not bind to CHO-K1 cells harboring pCMV3-hCD25 or pCI-cCD25 (Figure 14). This demonstrated that cMAb2_hIgG1LALA binds to mouse CD25.

10)-2 Influence on IL-2-dependent cell proliferation using mouse CTLL-2

Cells subcultured in accordance with 2)-3-9-1 were used for the culture of mouse CTLL-2 cells.

The CD25-positive mouse tumor cell line CTLL-2 was inoculated at $5 \times 10^2$ cells/50 μL/well in RPMI1640 (Thermo Fisher Scientific Inc.) medium containing 1 mM Sodium Pyruvate (Thermo Fisher Scientific Inc.), 1 × MEM Non-Essential Amino Acids Solution (Thermo Fisher Scientific Inc.), and 10% T-STIM with ConA to 96 well clear U bottom microplate (Corning Inc.). Further, cMAb2_hIgG1LALA, control hIgG1, and PC61_hIgG1LALA were added at 15 μg/mL. The cells were cultured for 4 days under conditions of 37°C and 5% $CO_2$. The incubated plate was left standing at room temperature for 30 minutes. Reagent solution of CellTiter-Glo(TM) Luminescent Cell Viability Assay (Promega Corp.) was added at 100μL/well, and the plate was then mixed at room temperature for 10 minutes under light shielding using a plate mixer (Taitec Corp.). 100 μL of the solution of each well was added to each well of 96 well white flat bottom microplate (Corning Inc.), and luminescence intensity (relative light unit (RLU)) was measured using a plate reader (EnSight: PerkinElmer, Inc.) and used as an index for the number of cells. As a result, when the number of cells in the control antibody human IgG1 addition group was defined as 100%, the number of cells in the IL-2 blocking antibody PC61_hIgG1LALA addition group was 55% and the number of cells in the cMAb2_hIgG1LALA addition group was 104% (Figure 15). The number of cells in the IL-2 blocking antibody PC61_hIgG1LALA addition group was decreased as compared with the control antibody human IgG1 addition group whereas the number of cells in the cMAb2_hIgG1LALA addition group was not changed, demonstrating that cMAb2_hIgG1LALA is an IL-2 non-blocking antibody that does not influence the IL-2-dependent promotion of cell proliferation.

(Example 11) Property of cMAb2_hIgG1LALA-ADC

11)-1 Cell proliferation suppressive activity of cMAb2_hIgG1LALA-ADC

11)-1-1 Cell culture

A CD25-positive mouse tumor cell line CTLL-2 was subcultured in RPMI1640 (Thermo Fisher Scientific Inc.) medium containing 1 mM Sodium Pyruvate (Thermo Fisher Scientific Inc.), 1 × MEM Non-Essential Amino Acids Solution (Thermo Fisher Scientific Inc.), and 10% T-STIM with ConA under conditions of 37°C and 5% $CO_2$.

11)-1-2 Cell proliferation suppression assay

The CD25-positive mouse T cell line CTLL-2 was inoculated at $5 \times 10^2$ cells/50 μL/well in RPMI1640 (Thermo Fisher Scientific Inc.) medium containing 1 mM Sodium Pyruvate (Thermo Fisher Scientific Inc.), 1 × MEM Non-Essential Amino Acids Solution (Thermo Fisher Scientific Inc.), and 10% T-STIM with ConA to 96 well clear U bottom microplate (Corning Inc.). Further, cMAb2_hIgG1LALA, control hIgG1LALA-ADC, and cMAb2_hIgG1LALA-ADC were added at 0.015 μg/mL to 15 μg/mL. The cells were cultured for 4 days under conditions of 37°C and 5% $CO_2$. The incubated plate was left standing at room temperature for 30 minutes. Reagent solution of CellTiter-Glo(TM) Luminescent Cell Viability Assay (Promega Corp.) was added at 100μL/well, and the plate was then mixed at room temperature for 10 minutes under light shielding using a plate mixer (Taitec Corp.). 100 μL of the solution of each well was added to each well of 96 well white flat bottom microplate (Corning Inc.), and luminescence intensity (relative light unit (RLU)) was measured using a plate reader (EnSight: PerkinElmer, Inc.) and used as an index for the number of cells. As a result, the addition of human IgG1LALA-ADC and cMAb2_hIgG1LALA exhibited no cell proliferation suppressive activity. On the other hand, cMAb2_hIgG1LALA-ADC exhibited cell proliferation suppressive activity as compared with these two types of addition groups (Figure 16). This demonstrated that cMAb2_hIgG1LALA-ADC has proliferation suppressive activity against mouse CD25 expression-positive cells. cMAb2_hIgG1LALA-ADC2 exhibited similar effects.

(Example 12) Preparation of cMAb2_hIgG1

12)-1 Construction of light chain expression vector pCMA-LK

The expression vector was constructed in accordance with the method of 6)-1.

12)-2 Construction of heavy chain expression vector pCMA-G1'

A DNA fragment comprising a nucleotide sequence (shown in SEQ ID NO: 53) encoding a heavy chain signal sequence and a human heavy chain G1 constant region was synthesized (manufactured by Eurofins Genomics K.K.). This DNA fragment was cleaved with restriction enzymes XbaI and PmeI, and a DNA fragment of 1.1 kb was then excised by agarose gel electrophoresis and purified using Wizard SV Gel and PCR Clean-Up System (manufactured by Promega Corp.). A fragment of approximately 3.4 kb obtained by the digestion of pCMA-G1LALA with restriction enzymes XbaI and PmeI and the DNA fragment of 1.1 kb were ligated using Ligation High (manufactured by Toyobo Co., Ltd.) to construct pCMA-G1'.

12)-3 Construction of cMAb2_hIgG1 expression vector

12)-3-1 Construction of cMAb2_hIgG1 light chain expression vector

The cMAb2_hIgG1 light chain expression vector was constructed in accordance with 6)-3. The nucleotide sequence of the antibody light chain in the cMAb2_hIgG1 light chain expression vector is shown in SEQ ID NO: 36. Its amino acid sequence is shown in SEQ ID NO: 32.

12)-3-2 Construction of cMAb2_hIgG1 heavy chain expression vector

A fragment comprising the nucleotide sequence of a variable region was amplified by PCR with the cMAb2_hIgG1-LALA heavy chain expression vector constructed in 6)-4 as a template using primer 2F (AGCTCCCA GATGGGTGCTGAGC: SEQ ID NO: 49) and primer 2R (GGGCCCTTGGTGGAGGCTGAGC: SEQ ID NO: 50). Then, the fragment was inserted into the BlpI site of pCMA-G1' using In-Fusion HD PCR cloning kit to construct a cMAb2_hIgG1 heavy chain expression vector. The nucleotide sequence of the antibody heavy chain in the cMAb2_hIgG1 heavy chain expression vector is shown in SEQ ID NO: 37. Its amino acid sequence is shown in SEQ ID NO: 33.

12)-4 Production of cMAb2_hIgG1

FreeStyle 293F cells (manufactured by Thermo Fisher Scientific Inc.) were passaged and cultured in accordance with the manual. The FreeStyle 293F cells in the logarithmic growth phase were diluted into $2.0 \times 10^6$ cells/mL with FreeStyle 293 expression medium (manufactured by Thermo Fisher Scientific Inc.), and 1.2 L of the dilution was inoculated to 5 L Optimum Growth Flask (manufactured by Thomson Instrument Company). 3.6 mg of Polyethyleneimine (manufactured by Polyscience) was added to 40 mL of Opti-Pro SFM medium (manufactured by Thermo Fisher Scientific Inc.). Subsequently, 600 μg of the heavy chain expression vector and 600 μg of the light chain expression vector were added to 40 mL of Opti-Pro SFM medium. The expression vector/Opti-Pro SFM mixed solution was added to the Polyethyleneimine/Opti-Pro SFM mixed solution, and the mixture was gently stirred, further left standing for 5 minutes, and then added to the FreeStyle 293F cells. The cells were shake-cultured at 105 rpm in an 8% $CO_2$ incubator at 37°C for 4 hours. Then, 1.2 L of EX-CELL VPRO medium (manufactured by SAFC Biosciences Inc.) and 60 mL of 43.4 g/L BD Recharge CD (manufactured by BD Biosciences) were added thereto, and the cells were shake-cultured at 105 rpm in an 8% $CO_2$ incubator at 37°C for 6 days. The obtained culture supernatant was centrifuged and then filtrated through a filter having a pore size of 0.2 μm (manufactured by Pall Corp.).

12)-5 Purification of cMAb2_hIgG1

From the culture supernatant obtained in 12)-4, the antibody was purified by one step using rProtein A affinity chromatography. The culture supernatant was applied to a column (manufactured by Cytiva) packed with MabSelectSuRe equilibrated with PBS, and the column was then washed with PBS in an amount of two or more times the volume of the column. Subsequently, the antibody was eluted with a 2 M arginine hydrochloride solution (pH 4.0). A fraction containing the antibody was dialyzed using Slide-A-Lyzer Dialysis Cassette (manufactured by Thermo Fisher Scientific Inc.) for buffer replacement with HBSor (25 mM histidine/5% sorbitol, pH 6.0). The antibody was concentrated using VIVASPIN 20 (molecular weight cutoff: UF10K, manufactured by Sartorius Stedim Biotech SA) to adjust an IgG concentration to approximately 2 mg/mL. Finally, the antibody solution was filtered through Minisart-Plus (manufactured by Sartorius Stedim Biotech SA) to prepare a purified sample.

(Example 13) Preparation of cMAb2_hIgG1-ADC

cMAb2_hIgG1-ADC was prepared in accordance with Example 8 using cMAb2_hIgG1.

13)-1 Preparation of antibody-drug conjugate cMAb2_hIgG1-ADC

[Formula 25]

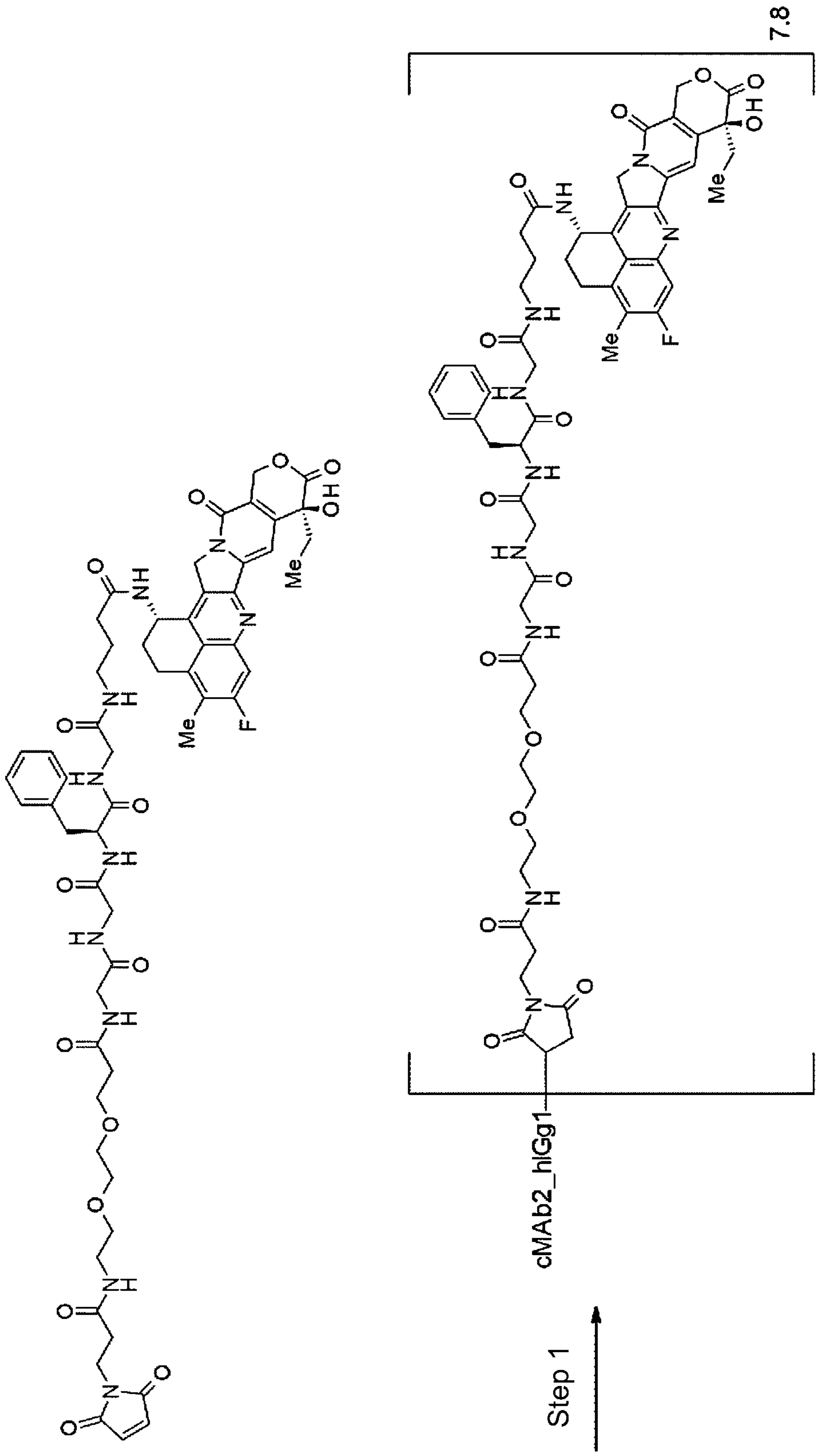


Reduction of antibody: cMAb2_hIgG1 prepared in Example 12 was adjusted to 10.8 mg/mL with PBS6.0/EDTA by use of common procedures B (using 1.45 $mLmg^{-1}cm^{-1}$ as 280 nm absorption coefficient) and C described in production method 1. To this solution (7.7 mL), an aqueous solution of 10 mM TCEP (Tokyo Chemical Industry Co., Ltd.) (0.344 mL; 6.0 equivalents per antibody molecule) and a 1 M aqueous dipotassium hydrogen phosphate solution (Nacalai Tesque, Inc.; 0.116 mL) were added. After confirming that the solution had a pH within 7.0 ± 0.1, the interchain disulfide bond in the

antibody was reduced by incubating the solution at 37°C for 2 hours.

Conjugation between antibody and drug linker: The solution was incubated at 15°C for 10 minutes. Subsequently, a 10 mM solution of N-{3-[2-(2-{[3-(2,5-dioxo-2,5-dihydro-1H-pyrrol-1-yl)propanoyl]amino}ethoxy)ethoxy]propanoyl}glycylglycyl-L-phenylalanyl-N-(4-{[(1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl]amino}-4-oxobutyl)glycinamide in dimethyl sulfoxide (0.573 mL; 10 equivalents per antibody molecule) was added thereto, and the mixture was incubated at 15°C for 1 hour to conjugate the drug linker to the antibody. Subsequently, an aqueous solution of 100 mM NAC (Sigma-Aldrich Co. LLC) (0.0573 mL; 10 equivalents per antibody molecule) was added thereto, and the mixture was further stirred at room temperature for 20 minutes to terminate the reaction of the drug linker.

Purification: The solution was purified by common procedure D described in production method 1 to obtain 28.0 mL of a solution containing the title antibody-drug conjugate "cMAb2_hIgG1-ADC".

Characterization: Using common procedure E (using $\varepsilon_{D,280}$ = 7642 and $\varepsilon_{D,370}$ = 24111) described in production method 1, the following characteristic values were obtained.

Antibody concentration: 2.82 mg/mL, antibody yield: 79.1 mg (95%), average number of conjugated drug molecules (n) per antibody molecule measured by common procedure E: 5.9; average number of conjugated drug molecules (n) per antibody molecule measured by common procedure F: 7.8.

(Example 14) Antitumor activity of cMAb2_hIgG1-ADC and change in immunocyte in tumor

14)-1 Antitumor activity evaluation in CT26 model

2 $\times$ $10^5$ mouse colorectal cancer-derived CT26.WT cells (American Type Culture Collection) subcultured in RPMI1640 containing 10% FBS were subcutaneously transplanted to each female BALB/C mouse. The mice were grouped on the basis of their tumor volumes. 5 days after transplantation, 5 mg/kg human IgG1-ADC or 5 mg/kg cMAb2_hIgG1-ADC was intravenously administered to each mouse (n = 9). The major axis and minor axis of the transplanted tumor were measured two or three times a week using electronic digital calipers (manufactured by Mitutoyo Corp.), and the tumor volume was calculated according to the following expression.

Tumor volume ($mm^3$) = 1/2 $\times$ Minor axis (mm) $\times$ Minor axis (mm) $\times$ Major axis (mm)

As a result, delayed tumor growth and tumor regression were found in the cMAb2_hIgG1-ADC administration group as compared with human IgG1-ADC (Figure 17). This demonstrated that cMAb2_hIgG1-ADC has antitumor activity.

14)-2 *In vivo* immunoenhancing effect of cMAb2_hIgG1-ADC

14)-2-1 Harvest of tumor tissue and preparation of cell suspension

2 $\times$ $10^5$ mouse colorectal cancer-derived CT26.WT cells (American Type Culture Collection) subcultured in RPMI1640 medium (Thermo Fisher Scientific Inc.) containing 10% FBS were subcutaneously transplanted to each female BALB/C mouse. The mice were grouped on the basis of their tumor volumes. 5 days after transplantation, 10 mg/kg human IgG1-ADC or 10 mg/kg cMAb2_hIgG1-ADC was intravenously administered to each mouse (n = 20). 7 and 14 days after ADC administration, tumor was harvested from 10 individuals per group. The harvested tumor mass was treated with gentleMACS (Miltenyi Biotec) to recover cells. The cells were washed with PBS and resuspended therein. Then, the cell suspension was used as a sample for flow cytometry. The amount of PBS in resuspending the cells was the following amount depending on a tumor weight for addition: 0.8 mL for a tumor weight up to 50 mg; 1.6 mL for a tumor weight of 50 to 100 mg; 2.0 mL for a tumor weight of 100 to 150 mg; 3.0 mL for a tumor weight of 150 to 200 mg; and 4.0 mL for a tumor weight of 200 to 250 mg.

14)-2-2 Preparation of staining solution for flow cytometry analysis

Zombie NIR dye solution was prepared by diluting Zombie NIR dye solution attached to Zombie NIR Fixable Viability Kit (BioLegend, Inc.) 1000-fold with PBS. Fc Block solution was prepared by adding and mixing Mouse BD Fc Block (BD Biosciences) solution and MACS buffer at a ratio of 1:49. Foxp3 Fixation/Permeabilization buffer was prepared by mixing 2.5 mL of Concentrate and 7.5 mL of Diluent of Foxp3 Fixation/Permeabilization Concentrate and Diluent (eBioscience, Inc.). Perm wash buffer was prepared by diluting Intracellular Staining Perm Wash Buffer (BioLegend, Inc.) 10-fold with $H_2O$. A cell surface marker antibody cocktail solution was used by adding 2 $\mu$L of FITC anti-mouse CD25 Antibody (BioLegend, Inc.), 2 $\mu$L of Brilliant Violet 605 anti-mouse CD45 Antibody (BioLegend, Inc.), 2 $\mu$L of PE/Cyanine7

anti-mouse CD4 Antibody (BD Biosciences), 3 μL of Brilliant Violet 650 anti-mouse CD3 Antibody (BioLegend, Inc.), and 2 μL of APC anti-mouse CD8 Antibody (BioLegend, Inc.), and adjusting the whole amount to 50 μL with MACS buffer. An intracellular marker antibody cocktail solution was used by adding 3 μL of PE Mouse anti-mouse FoxP3 (Invitrogen Corp.), 3 μL of Brilliant Violet 421 anti-human Granzyme B Antibody (BD Biosciences), and 1 μL of Mouse BD Fc Block (BD Biosciences), and adjusting the whole amount to 100 μL with Perm wash buffer.

14)-2-3 Antibody staining of sample for flow cytometry analysis

0.1 mL of Zombie NIR dye diluting solution was added to 0.2 mL of the sample for flow cytometry prepared in 14)-2-1, and the mixture was incubated at 4°C for 30 minutes. 0.1 mL of MACS buffer was added thereto, and the mixture was centrifuged, followed by the removal of a supernatant by suction. 0.05 mL of Fc Block solution was added to the remaining cells, which were then incubated at 4°C for 5 minutes. 0.05 mL of the cell surface marker antibody cocktail solution prepared in 14)-2-2 was added to the cells, which were then incubated at 4°C for 30 minutes. Subsequently, 0.1 mL of MACS buffer was added thereto, and the mixture was centrifuged, followed by the removal of a supernatant by suction. 0.2 mL of MACS buffer was added to the remaining cells, and the mixture was further centrifuged to remove a supernatant. This operation was further carried out twice for the washing of the cells. 0.2 mL of Foxp3 Fixation/Permeabilization buffer was added to the cells, which were then left standing overnight at 4°C. The cells thus left standing were centrifuged to remove a supernatant, and 0.2 mL of Perm wash buffer was added to the cells, which were then centrifuged to remove a supernatant. This operation was carried out two more times for the washing of the cells. 0.2 mL of the intracellular marker antibody cocktail solution was added to the washed cells, which were then incubated at 4°C for 1 hour and then washed twice with Perm wash buffer. 0.18 mL of MACS buffer was added to the cells, followed by flow cytometry analysis.

14)-2-4 Flow cytometry analysis

The sample prepared in 14)-2-3 was applied to a flow cytometer (BD LSR Fortessa X-20 cell analyzer: BD Biosciences) to measure 0.15 mL of the sample. The numbers of mouse CD45-positive cells, mouse FoxP3-, CD4-, CD3-, and CD45-positive cells (mouse Treg cells), mouse FoxP3-negative and CD4-, CD3-, and CD45-positive cells (mouse FoxP3-negative and CD4-positive T cells), mouse CD8-, CD3-, and CD45-positive cells as CD8-positive T cells (mouse CD8-positive T cells), and mouse granzyme-positive CD8T cells (mouse GZMB-positive CD8-positive T cells) in a Zombie NIR dye-negative fraction were measured. Then, the respective numbers of mouse Treg cells, mouse CD8-positive T cells, and mouse GZMB-positive and CD8-positive T cells were assigned to the following expression to calculate the numbers of mouse Treg cells, mouse CD8-positive T cells, and mouse GZMB-positive and CD8-positive T cells per mg of the tumor tissue.

[Expression 5]

$$\text{The number of cells measured} \times \frac{\text{Total amount of the sample measured in the flow cytometer}}{\text{Amount of the solution measured in the flow cytometer}} \times \frac{\text{Total amount of the cell suspension obtained from the tumor tissue}}{0.2} \times \frac{1}{\text{Weight of the tumor tissue}}$$

As a result, in the cMAb2_hIgG1-ADC administration group compared with the control hIgG1-ADC administration group, the number of mouse Treg cells per mg of the tumor tissue was decreased on day 7, and increase in the numbers of mouse FoxP3-negative and CD4-positive T cells, mouse CD8-positive T cells, and mouse GZMB-positive and CD8-positive T cells per mg of the tumor tissue was observed on day 14 (Figure 18). These results demonstrated that cMAb2_hIgG1-ADC activates mouse FoxP3-negative and CD4-positive T cells and mouse CD8-positive T cells by decreasing the number of mouse Treg.

(Example 15) Preparation of humanized antibody

15)-1 Design of humanized antibody of MAb1

15)-1-1 Molecular modeling of variable region

A method known in the art as homology modeling (Methods in Enzymology, 203, 121-153 (1991)) was exploited. Structures registered in Protein Data Bank (Nuc. Acid Res. 35, D301-D303 (2007)) were searched for sequences having high sequence identity to the variable regions using a commercially available protein conformation analysis program Discovery Studio (manufactured by Dassault Systems). The identified heavy chain, light chain and heavy chain-light chain interface structures were used as a template to prepare a three-dimensional model.

15)-1-2 Method for designing humanized antibody

The antibody was humanized by CDR grafting (Proc. Natl. Acad. Sci. USA 86, 10029-10033 (1989)). The framework regions of cMAb1 have high homology to human kappa chain subgroup 3 and 4 consensus sequences and a human gamma chain subgroup 3 consensus sequence defined by Kabat et al. (Sequences of Proteins of Immunological Interest, 5th Ed. Public Health Service National Institutes of Health, Bethesda, MD. (1991)). Therefore, these sequences were respectively selected as acceptors for the light chain and heavy chain of cMAb1. Donor residues to be grafted onto the acceptors were selected by analyzing the three-dimensional model with reference to the criteria, etc. provided by Queen et al. (Proc. Natl. Acad. Sci. USA 86, 10029-10033 (1989)).

15)-1-3 Humanization of MAb1 light chain

The variable regions of humanized antibody light chains hMAb1A and hMAb1B were designed on the basis of the cMAb1 light chain variable region.

The designed variable regions were connected to the human κ chain constant region to design humanized antibody light chains, which were respectively designated as hMAb1A-L and hMAb1B-L. The full-length amino acid sequence of the light chains hMAb1A-L and hMAb1B-L are described in SEQ ID NOs: 13 and 14, respectively. The nucleotide sequences encoding the amino acid sequences of SEQ ID NOs: 13 and 14 are described in SEQ ID NOs: 38 and 39, respectively.

15)-1-4 Humanization of MAb1 heavy chain

The variable regions of humanized antibody heavy chains hMAb1A and hMAb1B were designed on the basis of the cMAb1 heavy chain variable region.

The designed variable regions were connected to the gamma chain constant region of human IgG1 to design humanized antibody heavy chains, which were respectively designated as hMAb1A-H and hMAb1B-H. The full-length amino acid sequence of hMAb1A-H and hMAb1B-H are described in SEQ ID NOs: 15 and 16, respectively. The nucleotide sequences encoding the amino acid sequences of SEQ ID NOs: 15 and 16 are described in SEQ ID NOs: 40 and 41, respectively.

15)-2 Construction of humanized antibody expression vector

15)-2-1 Construction of humanized antibody hMAb1A light chain expression vector

A DNA fragment represented by nucleotide positions 37 to 399 of the humanized antibody hMAb1 light chain variable region-encoding nucleotide sequence represented by SEQ ID NO: 38 was synthesized (manufactured by Thermo Fisher Scientific Inc.). The synthesized DNA fragment was inserted into a cleavage site of pCMA-LK with a restriction enzyme BsiWI using In-Fusion HD PCR cloning kit to construct a humanized antibody hMAb1 light chain expression vector. The nucleotide sequence of the humanized antibody hMAb1A light chain comprising a signal sequence in the humanized antibody hMAb1A light chain expression vector is shown in SEQ ID NO: 38. Its amino acid sequence is shown in SEQ ID NO: 13.

15)-2-2 Construction of humanized antibody hMAb1B light chain expression vector

A DNA fragment represented by nucleotide positions 37 to 399 of the humanized antibody hMAb1B light chain variable region-encoding nucleotide sequence represented by SEQ ID NO: 39 was synthesized (manufactured by Thermo Fisher Scientific Inc.). The synthesized DNA fragment was inserted into a cleavage site of pCMA-LK with a restriction enzyme BsiWI using In-Fusion HD PCR cloning kit to construct a humanized antibody hMAb1B light chain expression vector. The nucleotide sequence of the humanized antibody hMAb1B light chain comprising a signal sequence in the humanized antibody hMAb1B light chain expression vector is shown in SEQ ID NO: 39. Its amino acid sequence is shown

in SEQ ID NO: 14.

15)-2-3 Construction of humanized antibody hMAb1A heavy chain expression vector

A DNA fragment represented by nucleotide positions 36 to 455 of the humanized antibody hMAb1A heavy chain variable region-encoding nucleotide sequence represented by SEQ ID NO: 40 was synthesized (manufactured by Thermo Fisher Scientific Inc.). The synthesized DNA fragment was inserted into a cleavage site of pCMA-G1' constructed in 12)-2 with a restriction enzyme BlpI using In-Fusion HD PCR cloning kit to construct a humanized antibody hMAb1A heavy chain expression vector. The nucleotide sequence of the humanized antibody hMAb1A heavy chain comprising a signal sequence in the humanized antibody hMAb1A heavy chain expression vector is shown in SEQ ID NO: 40. Its amino acid sequence is shown in SEQ ID NO: 15.

15)-2-4 Construction of humanized antibody hMAb1B heavy chain expression vector

A DNA fragment represented by nucleotide positions 36 to 455 of the humanized antibody hMAb1B heavy chain variable region-encoding nucleotide sequence represented by SEQ ID NO: 41 was synthesized (manufactured by Thermo Fisher Scientific Inc.). The synthesized DNA fragment was inserted into a cleavage site of pCMA-G1' constructed in 12)-2 with a restriction enzyme BlpI using In-Fusion HD PCR cloning kit to construct a humanized antibody hMAb1B heavy chain expression vector. The nucleotide sequence of the humanized antibody hMAb1B heavy chain comprising a signal sequence in the humanized antibody hMAb1B heavy chain expression vector is shown in SEQ ID NO: 41. Its amino acid sequence is shown in SEQ ID NO: 16.

15)-3 Preparation of humanized antibody hMAb1

15)-3-1 Preparation of humanized antibody hMAb1A

FreeStyle 293F cells (manufactured by Thermo Fisher Scientific Inc.) were passaged and cultured at 37°C in 8% $CO_2$ in a spinner flask using a BCP-type animal cell culture apparatus (manufactured by Biott Corp.). Subsequent culture was carried out using WAVE BIOREACTOR (manufactured by GE Healthcare Bio-Sciences Corp.) in accordance with the manual. The FreeStyle 293F cells in the logarithmic growth phase were diluted into 2.0 to $2.4 \times 10^6$ cells/mL with FreeStyle 293 expression medium (manufactured by Thermo Fisher Scientific Inc.), and 6 L of the dilution was transferred to 50 L WAVE bag for culture (manufactured by Cytiva). Then, 6 L of FreeStyle 293 expression medium was also transferred thereto, and the cells were shake-cultured overnight at 37°C in 8% $CO_2$. 37.5 mg of Polyethyleneimine (manufactured by Polyscience) was added to 400 mL of Opti-Pro SFM medium (manufactured by Thermo Fisher Scientific Inc.). Subsequently, 6.25 mg of the humanized antibody hMAb1 heavy chain expression vector and 6.25 mg of the humanized antibody hMAb1 light chain expression vector were added to 400 mL of Opti-Pro SFM medium. The expression vector/Opti-Pro SFM mixed solution was added to the Polyethyleneimine/Opti-Pro SFM mixed solution, and the mixture was gently stirred, further left standing for 5 minutes, and then added in the whole amount to the 50 L WAVE bag for culture where the FreeStyle 293F cells were cultured. The cells were shake-cultured at 37°C in 8% $CO_2$ for 4 hours. Then, 10 L of Balan CD HEK293 medium (FUJIFILM Wako Pure Chemical Corp.) containing 1% GlutaMAX (manufactured by Thermo Fisher Scientific Inc.) and 2.5 L of Balan CD HEK293 Feed medium (FUJIFILM Wako Pure Chemical Corp.) containing 1% GlutaMAX were added thereto. The cells were shake-cultured at 37°C in 8% $CO_2$ for 6 days. The obtained culture supernatant was passed through a depth filter (pore size: 5 μm, manufactured by GE Healthcare Bio-Sciences Corp.) and then filtered through a capsule cartridge filter (pore size: 0.45 μm, manufactured by Advantec Toyo Kaisha, Ltd.).

15)-3-2 Preparation of humanized antibody hMAb1B

The humanized antibody was prepared by transient expression in accordance with the method described in 15)-3-1 using 1.25 mg of the humanized antibody hMAb1B light chain expression vector and 1.25 mg of the humanized antibody hMAb1B heavy chain expression vector and using FreeStyle 293F cells (manufactured by Thermo Fisher Scientific Inc.).

15)-4 Purification of humanized antibodies hMAb1A and hMAb1B

From each of the culture supernatants obtained in 15)-3-1 and 15)-3-2, the antibody was purified by two steps using rProtein A affinity chromatography and ceramic hydroxyapatite. The culture supernatant was applied to a column (manufactured by GE Healthcare Bio-Sciences Corp.) packed with MabSelectSuRe equilibrated with PBS, and the column was then washed with PBS in an amount of two or more times the volume of the column. Subsequently, the

antibody was eluted with a 2 M arginine hydrochloride solution (pH 4.0). The fraction containing the antibody was dialyzed (Thermo Fisher Scientific Inc., Slide-A-Lyzer Dialysis Cassette) for buffer replacement with PBS, diluted 5-fold with a buffer of 5 mM sodium phosphate/50 mM MES/pH 7.0, and then was applied to a ceramic hydroxyapatite column (Bio-Rad Laboratories, Inc., Bio-Scale CHT Type-1 Hydroxyapatite Column) equilibrated with a buffer of 5 mM NaPi/50 mM MES/30 mM NaCl/pH 7.0. Elution was carried out on a linear concentration gradient of sodium chloride to collect a fraction containing the antibody. The fraction was dialyzed (Thermo Fisher Scientific Inc., Slide-A-Lyzer Dialysis Cassette) for buffer replacement with HBSor (25 mM histidine/5% sorbitol, pH 6.0). The antibody was concentrated using Centrifugal UF Filter Device VIVASPIN 20 (molecular weight cutoff: UF10K, Sartorius Stedim Biotech SA) to adjust an IgG concentration to approximately 20 mg/mL. Finally, the antibody solution was filtered through Minisart-Plus filter (Sartorius Stedim Biotech SA) to prepare a purified sample.

(Example 16) Property of hMAb1 antibody

16)-1 Evaluation of ability of humanized antibody to bind to CD25

The dissociation constants of the humanized anti-human CD25 antibodies hMAb1A and hMAb1R prepared in Example 15 were measured by the capture method of using Biacore T200 (manufactured by GE Healthcare Bio-Sciences Corp.), capturing each antibody as a ligand onto Anti-Human IgG (Fc) antibody immobilized using Human Antibody Capture Kit (manufactured by GE Healthcare Bio-Sciences Corp.), and measuring the antigen as an analyte. The running buffer used was HBS-EP+ (manufactured by GE Healthcare Bio-Sciences Corp.), and the sensor chip used was CM5 (manufactured by GE Healthcare Bio-Sciences Corp.). 1 μg/mL humanized antibody was added at 10 μL/min onto the sensor chip for 60 seconds. Then, serial dilution solutions of the antigen (0.0625 to 1 μg/mL for hMAb1 used in Example 5 were each added at a flow rate of 30 μL/min for 120 seconds. Subsequently, the dissociation phase was monitored for 300 seconds. 3 M magnesium chloride (manufactured by GE Healthcare Bio-Sciences Corp.) was added as a regenerating solution at a flow rate of 20 μL/min for 30 seconds. The data was analyzed using a 1:1 binding model to calculate association rate constant ka, dissociation rate constant kd, and dissociation constant KD (KD = kd / ka). The results are shown in Table 6.

[Table 6]

| Name | KD (nM) |
|---|---|
| hMAb1A | 2.9 |
| hMAb1B | 5.7 |

16)-2 Species cross-reactivity

The test was carried out in accordance with the method of 2)-3-1. The secondary antibody used was Peroxidase-AffiniPure F(ab')2 Fragment Goat Anti-Human IgG (H + L) (Jackson ImmunoResearch Laboratories, Inc.) diluted 1000-fold with PBS containing 5% FBS. TMB Microwell Peroxidase Substrate (2-Component System) (SeraCare Life Sciences, Inc.) and TMB Stop Solution (SeraCare Life Sciences, Inc.) were used in color development and its termination, respectively. hMAb1A and hMAb1B were added at final antibody concentrations of 20 to 0.001 μg/mL using a purified antibody. PBS containing 5% FBS was added instead of an antibody solution to antibody non-addition wells (0 μg/mL). The results are shown in Figure 19. These results demonstrated that: hMAb1A binds to human CD25, cynomolgus monkey CD25 and does not bind to mouse CD25; and hMAb1B binds to human CD25 and cynomolgus monkey CD25 (Figure 19).

16)-3 Influence on IL-2-dependent cell proliferation using human CD4-positive T cell or human PBMC

For hMAb1A, the test was carried out in accordance with the method of 7)-2. CD4-positive Tcells were adjusted to $8 \times 10^5$ cells/mL. The antibody was added at an antibody concentration of 100 μg/mL. For hMAb1B, the test was carried out using PBMC of a healthy individual. PBMC of a healthy individual was separated from blood using Ficoll-Paque PLUS (Cytiva). The separated PBMC was suspended at $2 \times 10^5$ cells/mL in IMDM (Thermo Fisher Scientific Inc.) medium containing 10% CTS Immune Cell SR (Thermo Fisher Scientific Inc.) to prepare a cell suspension. 25 μL of the cell suspension was inoculated to each well of a 96-well plate (U-bottom), and PHA (Sigma-Aldrich Co., LLC) at 2 μg/mL and Phorbol 12-myristate 13-acetate (PMA) (Sigma-Aldrich Co., LLC) at 0.2 μg/mL were added. Further, human IgG1, basiliximab, or hMAb1B was added at a final concentration of 20 μg/mL. The plate was incubated for 5 days under conditions of 37°C and 5% $CO_2$. The plate thus incubated was brought back to room temperature. 100 μL of Celltiter-Glo Reagent of CellTiter-Glo luminescent Cell Viability Assay (Promega Corp.) kit was added to each well, and the plate was

stirred for 10 minutes using a plate shaker. 100 μL/well of the reaction solution was transferred to 96-well White Flat Bottom plate, and luminescence intensity (relative light unit (RLU)) was measured using a plate reader (Ensight: PerkinElmer, Inc.) and used as an index for the number of cells. As a result, when the number of cells in the human IgG1 addition group was defined as 100%, the number of cells was 42% or 65% in the IL-2 blocking antibody basiliximab addition group and 92% and 95% in the hMAb1A and hMAb1B addition groups, respectively (Figure 20). The number of T cells in the IL-2 blocking antibody basiliximab addition group was decreased as compared with the human IgG1 addition group whereas the number of T cells in the hMAb1A and hMAb1B addition groups was not changed, demonstrating that hMAb1A and hMAb1B are IL-2 non-blocking antibodies that do not influence the proliferation of human CD4-positive T cells and human PBMC, respectively.

16)-4 Influence on phosphorylated STAT5 (pSTAT5) whose level is increased by IL-2 stimulation in human PBMC

The test was carried out in accordance with the method of 7)-3. As a result, when the amount of pSTAT5 in the group supplemented with IL-2 and the control antibody hIgG1 was defined as 100%, the amount of pSTAT5 was 0.01% in the group supplemented with IL-2 and the IL-2 blocking antibody basiliximab and 94% and 93% in the groups supplemented with hMAb1 and hMAb1B, respectively (Figure 21). The amount of pSTAT5 increased by the addition of IL-2 was not changed by the addition of hMAb1A or hMAb1B compared with the addition of the control antibody hIgG1 and was decreased by the addition of the IL-2 blocking antibody basiliximab, demonstrating that hMAb1A and hMAb1B are antibodies that do not block IL-2 signals.

(Example 17) Preparation of hMAb1-ADC

17)-1-1 Preparation of hMAb1A-ADC

hMAb1A-ADC was prepared in accordance with Example 8 using hMAb1A.

[Formula 26]

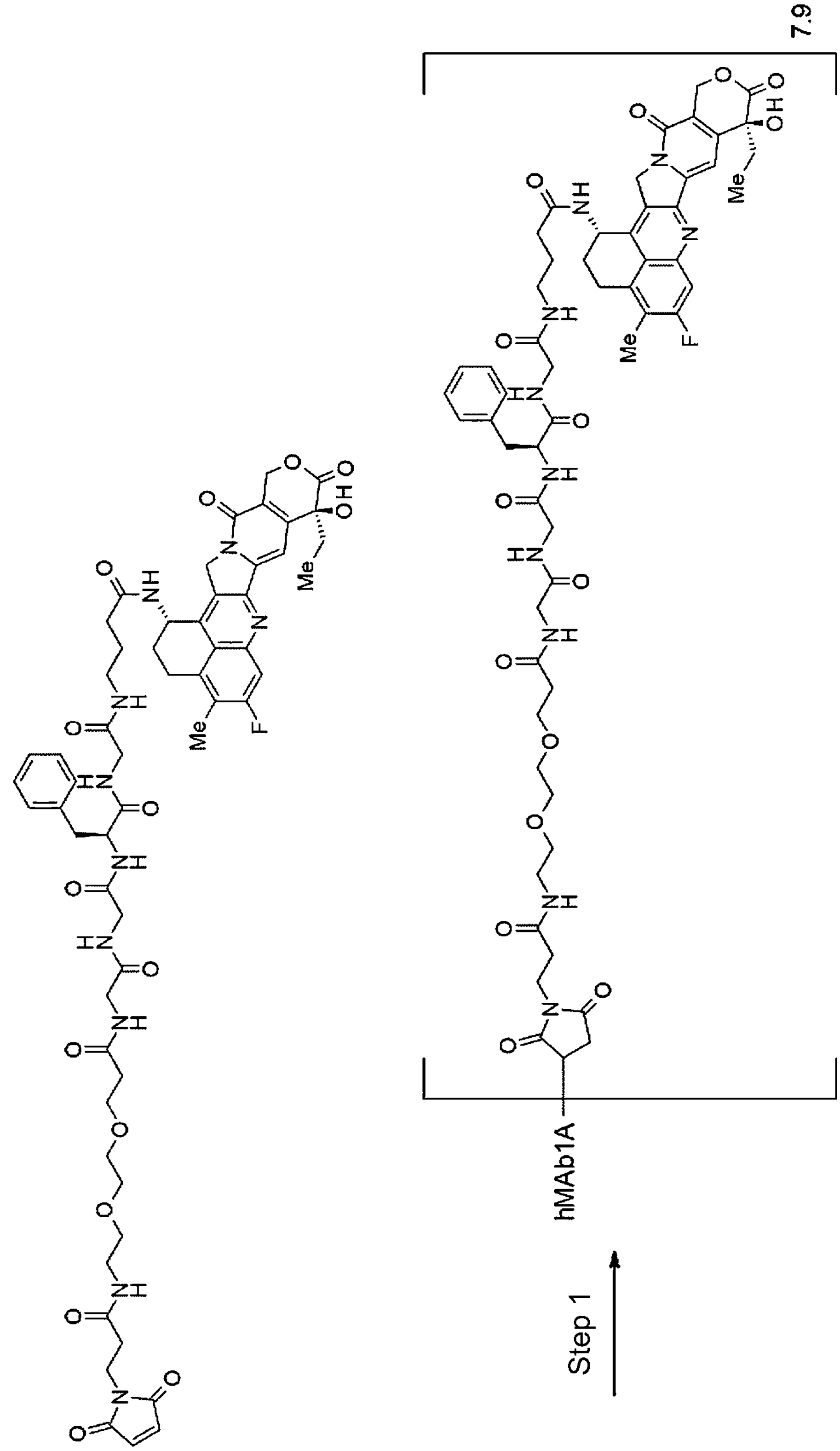


Reduction of antibody: hMAb1A prepared in Example 15 was adjusted to 10.0 mg/mL with PBS6.0/EDTA by use of common procedures B (using 1.62 $mLmg^{-1}cm^{-1}$ as 280 nm absorption coefficient) and C described in production method 1. To this solution (15.0 mL), an aqueous solution of 10 mM TCEP (Tokyo Chemical Industry Co., Ltd.) (0.617 mL; 6.0 equivalents per antibody molecule) and a 1 M aqueous dipotassium hydrogen phosphate solution (Nacalai Tesque, Inc.; 0.225 mL) were added. After confirming that the solution had a pH within 7.0 $\pm$ 0.1, the interchain disulfide bond in the antibody was reduced by incubating the solution at 37°C for 2 hours.

Conjugation between antibody and drug linker: The solution was incubated at 15°C for 10 minutes. Subsequently, a 10 mM solution of N-{3-[2-(2-{[3-(2,5-dioxo2,5-dihydro-1H-pyrrol-1-yl)propanoyl]amino}ethoxy)ethoxy]propanoyl}glycylglycyl-L-phenylalanyl-N-(4-{[(1S,9S)-9-ethyl-5-fluoro-9-hidroxy-4-methyl-10,13-dioxo-2,3,9,10,13,15-hexahydro-1H,12H-benzo[de]pyrano[3',4':6,7]indolizino[1,2-b]quinolin-1-yl]amino}-4-oxobutyl)glycinamide in dimethyl sulfoxide (1.03 mL; 10 equivalents per antibody molecule) was added thereto, and the mixture was incubated at 15°C for 1 hour to conjugate the drug linker to the antibody. Subsequently, an aqueous solution of 100 mM NAC (Sigma-Aldrich Co. LLC) (0.103 mL; 10 equivalents per antibody molecule) was added thereto, and the mixture was further stirred at room temperature for 20 minutes to terminate the reaction of the drug linker.

Purification: The solution was purified by common procedure D described in production method 1 to obtain 49.0 mL of a solution containing the title antibody-drug conjugate "hMAb1A-ADC".

Characterization: Using common procedure E (using $\varepsilon_{D,280}$ = 7642 and $\varepsilon_{D,370}$ = 24111) described in production method 1, the following characteristic values were obtained.

Antibody concentration: 2.62 mg/mL, antibody yield: 128 mg (85%), average number of conjugated drug molecules (n) per antibody molecule measured by common procedure E: 4.7; average number of conjugated drug molecules (n) per antibody molecule measured by common procedure F: 7.9.

17)-1-2 Preparation of hMAb1B-ADC

hMAb1B-ADC was prepared in accordance with Example 8 using hMAb1B.

[Formula 27]

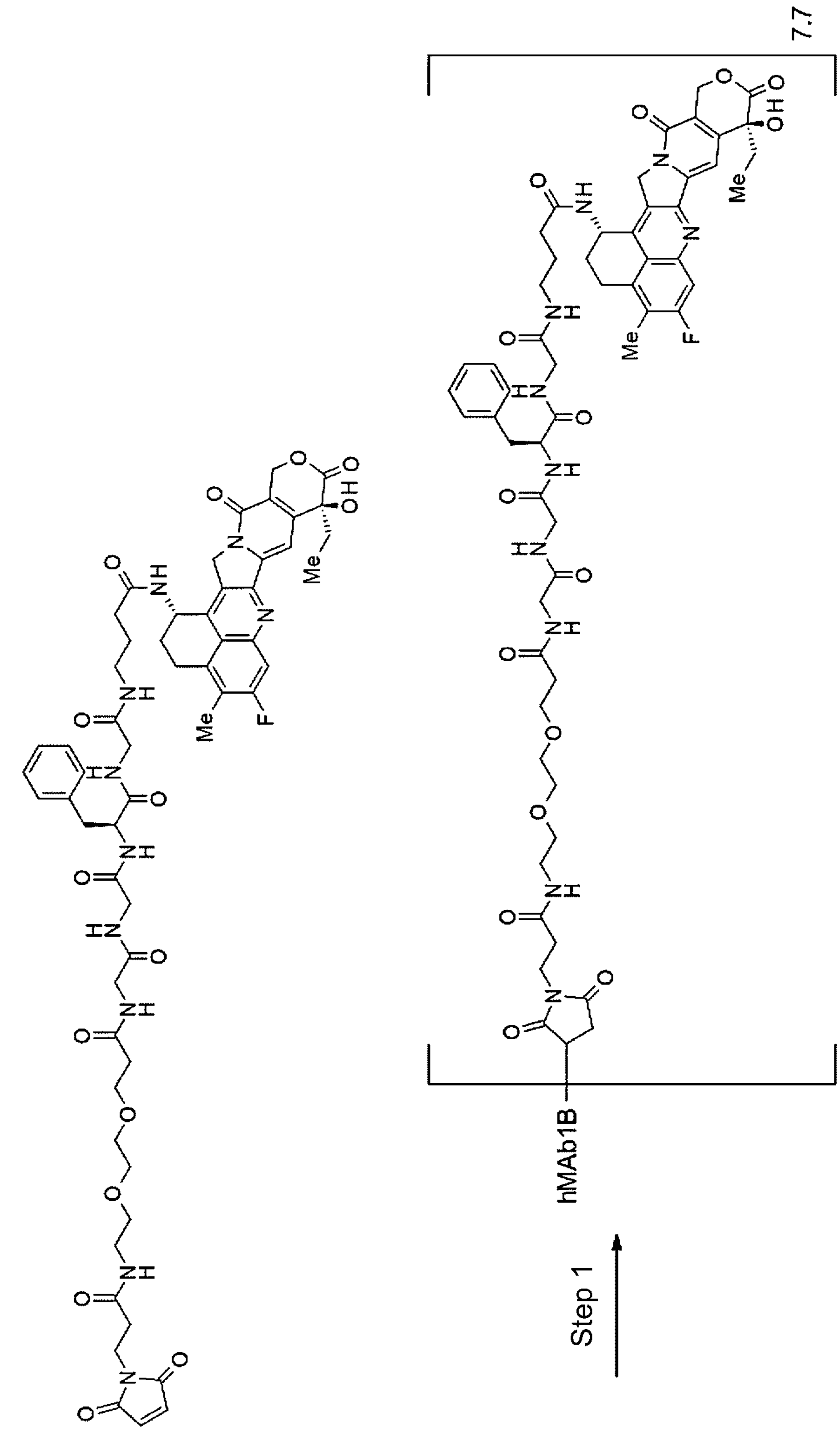


Reduction of antibody: hMAb1B prepared in Example 15 was adjusted to 10.0 mg/mL with PBS6.0/EDTA by use of common procedures B (using 1.60 $mLmg^{-1}cm^{-1}$ as 280 nm absorption coefficient) and C described in production method 1. To this solution (16.5 mL), an aqueous solution of 10 mM TCEP (Tokyo Chemical Industry Co., Ltd.) (0.676 mL; 6.0 equivalents per antibody molecule) and a 1 M aqueous dipotassium hydrogen phosphate solution (Nacalai Tesque, Inc.; 0.248 mL) were added. After confirming that the solution had a pH within 7.0 ± 0.1, the interchain disulfide bond in the antibody was reduced by incubating the solution at 37°C for 2 hours.

Conjugation between antibody and drug linker: The solution was incubated at 15°C for 10 minutes. Subsequently, a 10 mM solution of N-{3-[2-(2-{[3-(2,5-dioxo2,5-dihydro-1H-pyrrol-1-yl)propanoyl]amino}ethoxy)ethoxy]propanoyl}glycylglycyl-L-phenylalanyl-N-(4-{[(1S,9S)-9-ethyl-5-fluoro-9-hidroxy-4-methyl-10,13-dioxo-2,3,9,10,13,15-hexahydro-1H,12H-benzo[de]pyrano[3',4':6,7]indolizino[1,2-b]quinolin-1-yl]amino}-4-oxobutyl)glycinamide in dimethyl sulfoxide (1.13 mL; 10 equivalents per antibody molecule) was added thereto, and the mixture was incubated at 15°C for 1 hour to conjugate the drug linker to the antibody. Subsequently, an aqueous solution of 100 mM NAC (Sigma-Aldrich Co. LLC) (0.113 mL; 10 equivalents per antibody molecule) was added thereto, and the mixture was further stirred at room temperature for 20 minutes to terminate the reaction of the drug linker.

Purification: The solution was purified by common procedure D described in production method 1 to obtain 59.5 mL of a solution containing the title antibody-drug conjugate "hMAb1B-ADC".

Characterization: Using common procedure E (using $\varepsilon_{D,280}$ = 7642 and $\varepsilon_{D,370}$ = 24111) described in production method 1, the following characteristic values were obtained.

Antibody concentration: 2.32 mg/mL, antibody yield: 138 mg (84%), average number of conjugated drug molecules (n) per antibody molecule measured by common procedure E: 4.4; average number of conjugated drug molecules (n) per antibody molecule measured by common procedure F: 7.7.

(Example 18) Property of hMAb1-ADC

18)-1 Cell proliferation suppressive activity of hMAb1A-ADC and hMAb1B-ADC

18)-1-1 Cell culture

A CD25-positive human tumor cell line Karpas-299 was subcultured in RPMI1640 (Thermo Fisher Scientific Inc.) medium containing 2 mM glutamine (Life Technologies Corp.) and 20% FBS under conditions of 37°C and 5% $CO_2$. A CD25-positive human tumor cell line EoL-1 was subcultured in RPMI1640 (Thermo Fisher Scientific Inc.) medium containing 10% FBS under conditions of 37°C and 5% $CO_2$. A CD25-negative human tumor cell line Daudi was subcultured in RPMI1640 medium containing 10% FBS under conditions of 37°C and 5% $CO_2$.

18)-1-2 Cell proliferation suppression assay

The test was carried out in accordance with 9)-1-2. However, the incubation period was set to 6 days. EoL-1 was inoculated at $1 \times 10^4$ cells/50 μL/well in RPMI1640 medium containing 10% FBS to 96 well clear U bottom microplate (Corning Inc.). The plate was incubated for 6 days under conditions of 37°C and 5% $CO_2$. The plate thus incubated was brought back to room temperature. 100 μL of Celltiter-Glo Reagent of CellTiter-Glo luminescent Cell Viability Assay (Promega Corp.) kit was added to each well, and the plate was stirred for 10 minutes using a plate shaker. 100 μL/well of the reaction solution was transferred to 96-well White Flat Bottom plate, and luminescence intensity (relative light unit (RLU)) was measured using a plate reader (Ensight: PerkinElmer, Inc.) and used as an index for the number of cells. As a result, hMAb1A or hMAb1B added to the CD25-positive human tumor cell line Karpas-299 or EoL-1 exhibited no cell proliferation suppressive activity. On the other hand, hMAb1A-ADC or hMAb1B-ADC exhibited cell proliferation suppressive activity at a lower concentration than that of human IgG1-ADC or human IgG1LALA-ADC (Figure 22). hMAb1A-ADC and hMAb1B-ADC exhibited no cell proliferation suppressive activity against the CD25-negative human tumor cell line Daudi (Figure 22). This demonstrated that hMAb1A-ADC and hMAb1B-ADC have proliferation suppressive activity against CD25 expression-positive cells.

18)-2 *In vivo* immunoenhancing effect of hMAb1A-ADC

18)-2-1 Harvest of tumor tissue and preparation of cell suspension

$5 \times 10^6$ human breast adenocarcinoma-derived MDA-MB-231 cells (American Type Culture Collection) subcultured in Leibovitz's L-15 medium containing 10% FBS were suspended in Matrigel and subcutaneously transplanted to each female immune-humanized NOG mouse (huNOG mouse) engrafted with human CD34 cells. The mice were grouped on the basis of their tumor volumes. 7 days after transplantation, a vehicle ABS or 0.1 mg/kg, 0.3 mg/kg, 1 mg/kg, 3 mg/kg, or 10 mg/kg hMAb1A-ADC was intravenously administered to each mouse (n = 5). 23 days after ADC administration, tumor was harvested. The harvested tumor mass was treated with gentleMACS (Miltenyi Biotec) to recover cells. The cells were washed with PBS and resuspended therein. Then, the cell suspension was used as a sample for flow cytometry. The amount of PBS in resuspending the cells was the following amount depending on a tumor weight for addition: 0.2 mL for a tumor weight up to 50 mg; 0.4 mL for a tumor weight of 50 to 80 mg; 0.8 mL for a tumor weight of 80 to 100 mg; 1.2 mL for a tumor weight of 100 to 120 mg; 1.5 mL for a tumor weight of 120 to 160 mg; and 2.5 mL for a tumor weight of 200 to 250 mg.

18)-2-2 Preparation of staining solution for flow cytometry analysis

Zombie NIR dye solution was prepared by diluting Zombie NIR dye solution attached to Zombie NIR Fixable Viability Kit (BioLegend, Inc.) 1000-fold with PBS. Fc Block solution was prepared by adding and mixing Mouse BD Fc Block (BD Biosciences) solution, Human BD Fc Block solution, and MACS buffer at a ratio of 1:1:48. Foxp3 Fixation/-Permeabilization buffer was prepared by mixing Concentrate and Diluent of Foxp3 Fixation/Permeabilization Concentrate and Diluent (eBioscience, Inc.) at a ratio of 1:3. Perm wash buffer was prepared by diluting Intracellular Staining Perm Wash Buffer (BioLegend, Inc.) 10-fold with $H_2O$. A cell surface marker antibody cocktail solution was used by adding 2 μL of FITC anti-human CD25 Antibody (BioLegend, Inc.), 1 μL of APC anti-human CD8 Antibody (BioLegend, Inc.), 3 μL of Brilliant Violet 421 anti-human CD4 Antibody (BioLegend, Inc.), 2.5 μL of PE/Cyanine7 anti-human CD45RA Antibody (BioLegend, Inc.), 2 μL of Alexa Fluor 700 anti-mouse CD45 Antibody (BioLegend, Inc.), 2 μL of Brilliant Violet 605 anti-human CD45 Antibody (BioLegend, Inc., and 2 μL of Brilliant Violet 650 anti-human CD3 Antibody (BioLegend, Inc.), and adjusting the whole amount to 50 μL with MACS buffer. An intracellular marker antibody cocktail solution was used by adding 3 μL of PE Mouse anti-Human FoxP3 (BD Biosciences), 3 μL of PerCP/Cyanine5.5 anti-human/mouse Granzyme B Recombinant Antibody (BioLegend, Inc.), 1 μL of Mouse BD Fc Block (BD Biosciences), and 1 μL of Human BD Fc Block, and adjusting the whole amount to 100 μL with Perm wash buffer.

18)-2-3 Antibody staining of sample for flow cytometry analysis

0.2 mL of the sample for flow cytometry prepared in 18)-2-1 was centrifuged to remove a supernatant. Then, 0.1 mL of Zombie NIR dye diluting solution was added to the resulting sample, and the mixture was incubated at 4°C for 30 minutes. 0.1 mL of MACS buffer was added thereto, and the mixture was centrifuged, followed by the removal of a supernatant by suction. 0.1 mL of Fc Block solution was added to the remaining cells, which were then incubated at 4°C for 5 minutes. 0.1 mL of the cell surface marker antibody cocktail solution prepared in 18)-2-2 was added to the cells, which were then incubated at 4°C for 30 minutes. Subsequently, 0.1 mL of MACS buffer was added thereto, and the mixture was centrifuged, followed by the removal of a supernatant by suction. 0.2 mL of MACS buffer was added to the remaining cells, and the mixture was further centrifuged to remove a supernatant. This operation was further carried out twice for the washing of the cells. 0.2 mL of Foxp3 Fixation/Permeabilization buffer was added to the cells, which were then incubated at room temperature for 1 hour. After centrifugation, a supernatant was removed by suction, and 0.2 mL of Perm wash buffer was added to the remaining cells, which were further centrifuged to remove a supernatant. This operation was further carried out twice for the washing of the cells. 0.2 mL of the intracellular marker antibody cocktail solution was added to the washed cells, which were then incubated at 4°C for 1 hour and then washed twice with Perm wash buffer. A 4% paraformaldehyde solution was added to the cells, which were then incubated at 4°C for 15 minutes. The cells were washed twice with MACS buffer, and 0.18 mL of MACS buffer was then added to the cells. The sample was incubated overnight at 4°C, followed by flow cytometry analysis.

18)-2-4 Flow cytometry analysis

The sample prepared in 18)-2-3 was applied to a flow cytometer (BD LSR Fortessa X-20 cell analyzer: BD Biosciences) to measure the numbers of human CD45-positive cells, human FoxP3-, CD4-, CD3-, and CD45-positive cells (human Treg cells), human FoxP3-negative and CD4-, CD3-, and CD45-positive cells (human FoxP3-negative and CD4-positive T cells), human CD8-, CD3-, and CD45-positive cells (human CD8-positive T cells), and human granzyme-positive CD8T cells (human GZMB-positive and CD8-positive T cells) in a Zombie NIR dye-negative fraction. Then, the respective numbers of human Treg cells, human FoxP3-negative and CD4-positive T cells, human CD8-positive T cells, and human GZMB-positive and CD8-positive T cells were divided by the number of human CD45-positive cells to calculate the ratios of the numbers of human Treg cells, human FoxP3-negative and CD4-positive T cells, human CD8-positive T cells, and human GZMB-positive and CD8-positive T cells to the number of human CD45 cells (% of hCD45). As a result, in the hMAb1A-ADC administration group compared with the vehicle group, the ratio of the number of human Treg cells to the number of human CD45 cells was decreased in a dose-dependent manner, and increase in the ratios of the numbers of human FoxP3-negative and CD4-positive T cells, human CD8-positive T cells, and human GZMB-positive and CD8-positive T cells was observed (Figure 23). These results demonstrated that hMAb1A-ADC activates human FoxP3-negative and CD4-positive T cells or human CD8-positive T cells by decreasing the number of human Treg.

18)-3 *In vivo* immunoenhancing effects of hMAb1A-ADC and hMAb1B-ADC

18)-3-1 Harvest of tumor tissue and preparation of cell suspension

The procedures were carried out in accordance with 18)-2-1. However, 7 days after transplantation, 3 mg/kg

hMAb1A-ADC, hMAb1B-ADC, or human IgG1LALA-ADC was intravenously administered (n = 4 or 5). 21 days after ADC administration, a tumor tissue was harvested.

18)-3-2 Preparation of staining solution for flow cytometry analysis

The procedures were carried out in accordance with 18)-2-2.

18)-3-3 Antibody staining of sample for flow cytometry analysis

The procedures were carried out in accordance with 18)-2-3.

18)-3-4 Flow cytometry analysis

The test was carried out in accordance with 18)-2-4. As a result, in the hMAb1A-ADC and hMAb1B-ADC administration groups compared with the human IgG1LALA-ADC group, the ratio of the number of human Treg cells to the number of human CD45 cells was decreased, and increase in the ratios of the numbers of human CD8-positive T cells and human GZMB-positive and CD8-positive T cells was observed (Figure 24). These results demonstrated that hMAb1A-ADC and hMAb1B-ADC activate human CD8-positive T cells by decreasing the number of human Treg.

(Example 19) Preparation of RG-6292

The antibody was prepared in accordance with the method of the patent (WO2019/175222).

(Example 20) Activity comparison of RG-6292 with hMAb1A-ADC

20)-1 ADCC assay

20)-1-1 Preparation of iTreg

In the preparation of iTreg, frozen human PBMC (Cellular Technology Limited) was thawed and used as peripheral blood mononuclear cells (PBMC). Naive CD4 T cells were purified from PBMC using EasySep human naive CD4 T cell Isolation kit (STEMCELL Technologies). The obtained naive CD4 T cells were cultured for 4 days under conditions of 37°C and 5% $CO_2$ in X-VIVO(TM) 15 (Lonza KK) medium containing 10% FBS, 1% Glutamax (Thermo Fisher Scientific Inc.), 1 × Sodium pyruvate (Thermo Fisher Scientific Inc.), 1 × MEM Non-Essential Amino Acids Solution (Thermo Fisher Scientific Inc.), 25 mM HEPES (Thermo Fisher Scientific Inc.), 30 ng/mL Recombinant Human IL-2 (PeproTech, Inc.), 10 ng/mL Recombinant Human TGF-β, 50 μM 2-mercaptomethanol (Thermo Fisher Scientific Inc.), and the same number of Dynabeads Human T-Activator CD3/28 (Thermo Fisher Scientific Inc.) as the number of cells. As a result, the obtained cells were used as iTreg.

20)-1-2 Preparation of NK cell

PBMC of a healthy individual was separated from blood using Ficoll-Paque PLUS (Cytiva). NK cells were purified from PBMC using EasySep(TM) Human NK cell isolation Kit (STEMCELL Technologies). The obtained NK cells were cultured overnight under conditions of 37°C and 5% $CO_2$ in IMDM (Thermo Fisher Scientific Inc.) medium containing 4 ng/mL recombinant human IL-2 (PeproTech, Inc.) and 10% CTS Immune Cell SR (Thermo Fisher Scientific Inc.).

20)-1-3 ADCC assay

100 μL of Diluent C included in PKH26 Red Fluorescent Cell linear Kits (Sigma-Aldrich Co., LLC) was added to the prepared iTreg. Further, 100 μL of Dye Solution prepared by adding 4 μL of PKH26 ethanolic dye solution to 1 mL of Diluent C was added thereto. Then, the cells were incubated at room temperature for 5 minutes. 8 mL of RPMI1640 medium (Thermo Fisher Scientific Inc.) supplemented with 10% FBS was added thereto, and the cells were centrifuged at 300 × g at 20°C for 10 minutes to remove a supernatant. This operation was further carried out twice for the washing of the cells. Then, the cells were adjusted to $4 \times 10^5$ cells/mL with IMDM (Thermo Fisher Scientific Inc.) medium containing 10% CTS Immune Cell SR (Thermo Fisher Scientific Inc.). This cell suspension was added at 50 μL/well to 96 well clear U bottom microplate (Corning Inc.). Likewise, the NK cells prepared in 20)-1-2 were adjusted to $2 \times 10^6$ cells/mL with IMDM (Thermo Fisher Scientific Inc.) medium containing 10% CTS Immune Cell SR (Thermo Fisher Scientific Inc.) and added at 50 μL/well. Control antibodies hIgG1 (Sigma-Aldrich Co., LLC) and RG-6292 were each diluted into 0.5 and 5 μg/mL (final

concentration) with IMDM (Thermo Fisher Scientific Inc.) medium containing 10% CTS Immune Cell SR (Thermo Fisher Scientific Inc.) and added at 100 μL/well. Then, the cells were incubated for 4 hours under conditions of 37°C and 5% $CO_2$.

20)-1-4 Preparation of staining solution for flow cytometry analysis

Zombie NIR dye solution was prepared by diluting Zombie NIR dye solution attached to Zombie NIR Fixable Viability Kit (BioLegend, Inc.) 1000-fold with PBS. Fc Block solution was prepared by adding and mixing Human BD Fc Block solution and PBS containing 2 mM EDTA (Nacalai Tesque, Inc.) and 2% FBS (hereinafter, referred to as "FACS Buffer") at a ratio of 1:9.

A cell surface marker antibody cocktail solution was used by adding 2.5 μL of AF700 anti-human CD3 Antibody (BioLegend, Inc.), 2.5 μL of BV421 anti-human CD25 Antibody (BD Biosciences), and 2.5 μL of APC anti-human OX40 Antibody (eBioscience, Inc.), and adjusting the whole amount to 50 μL with FACS Buffer.

20)-1-5 Antibody staining of sample for flow cytometry analysis

The plate incubated in 20)-1-3 was centrifuged at 300 × g at 4°C for 5 minutes to remove a supernatant. PBS was added at 200 μL/well, and the plate was centrifuged at 300 × g at 4°C for 5 minutes to remove a supernatant for the washing of the cells. 0.1 mL of Zombie NIR dye diluting solution was added to each well, and the plate was incubated at room temperature for 15 minutes. 0.1 mL of FACS buffer was added to each well, the plate was centrifuged at 300 × g at 4°C for 5 minutes to remove a supernatant. Then, the operation of adding 0.1 mL of FACS buffer to each well, and centrifuging the plate at 300 × g at 4°C for 5 minutes to remove a supernatant was carried out twice.

0.05 mL of Fc Block solution was added to cells that remained by removing the supernatant by suction, and the cells were incubated at room temperature for 10 minutes. Further, 0.05 mL of the cell surface marker antibody cocktail solution prepared in 20)-1-4 was added thereto, and the cells were incubated at 4°C for 1 hour under light shielding. 0.1 mL of FACS buffer was added thereto, and the cells were centrifuged at 300 × g at 4°C for 5 minutes to remove a supernatant. Then, 0.1 mL of FACS buffer was added thereto, and the cells were centrifuged at 300 × g at 4°C for 5 minutes to remove a supernatant for the washing of the cells. Then, 0.2 mL of FACS Buffer was added thereto, and the resulting sample was used in flow cytometry analysis.

20)-1-6 Flow cytometry analysis

The sample prepared in 20)-1-5 was applied to a flow cytometer (BD LSR Fortessa X-20 cell analyzer: BD Biosciences) to respectively measure the numbers of cells in Zombie NIR dye-positive and -negative fractions in a PKH26-positive fraction. The ratio of dead cells was calculated therefrom according to the following expression.

Dead cells (%) = (The number of cells in the PKH26-positive Zombie NIR dye-positive fraction) / (The number of cells in the PKH26-positive fraction) × 100

As a result, the prepared RG-6292 was found to exhibit ADCC activity against iTreg (Figure 25).

20)-2 *In vitro* cell proliferation suppression evaluation using iTreg

20)-2-1 Preparation of iTreg

The cells were prepared in accordance with the method of 20)-1-1 except that culturing was carried out for 3 days.

20)-2-2 Preparation of NK cells

PBMC of a healthy individual was separated from blood using Ficoll-Paque PLUS (Cytiva). NK cells were purified from PBMC using EasySep(TM) Human NK cell isolation Kit (STEMCELL Technologies). The obtained NK cells were cultured overnight under conditions of 37°C and 5% $CO_2$ in IMDM (Thermo Fisher Scientific Inc.) medium containing 10% CTS Immune Cell SR (Thermo Fisher Scientific Inc.) (hereinafter, referred to as 10% CTS + IMDM medium).

20)-2-3 Cell killing assay

The target cells iTreg were adjusted with 10% CTS + IMDM medium and inoculated at 5 × $10^3$ cells/25 μL/well to 96 well clear U bottom microplate (Corning Inc.). Subsequently, the effector NK cells were adjusted with 10% CTS + IMDM

medium and inoculated in the range of 25 to 1.25 × $10^3$ cells/25 μL/well. The antibody or the antibody-drug conjugate was added at 50 μL/well in the final concentration range of 10 to 0.001 μg/mL. The cells were cultured for 3 days under conditions of 37°C and 5% $CO_2$. The incubated plate was left standing at room temperature for 30 minutes, and Reagent solution of CellTiter-Glo(TM) Luminescent Cell Viability Assay (Promega Corp.) was added at 100 μL/well, followed by mixing using a plate mixer (Taitec Corp.) at room temperature for 10 minutes under light shielding. 100 μL of the solution in each well was added to 96 well white flat bottom microplate (Corning Inc.), and luminescence intensity was measured using a plate reader (EnSight: PerkinElmer, Inc.).

A cell survival rate was calculated according to the following expression.

Cell survival rate (RLU) = (Value of the well in which iTreg and NK cells were cocultured) - (Average value from the wells in which only NK cells were cultured)

As a result, the proliferation suppressive effect of RG-6292 on iTreg was no longer able to be confirmed with decrease in the amount of NK cells, whereas hMAb1A-ADC exhibited a proliferation suppressive effect on iTreg, irrespective of NK cells (Figure 26). It has been reported that the ratio of NK cell/Treg is smaller than that of peripheral blood in tumor. This suggested that even in tumor, hMAb1A-ADC has higher ability to remove Treg than that of RG-6292.

(Example 21) Preparation of ADCT-301

21)-1 Preparation of Camidanlumab

The antibody was prepared in accordance with the method described in WO2004/045512.

21)-2 Preparation of ADCT-301

The antibody was synthesized in accordance with the method described in WO 2014/057119.

(Example 22) Activity comparison between ADCT-301 and hMAb1A-ADC

22)-1 Comparison of *in vivo* immunoenhancing effect between hMAb1A-ADC and ADCT-301

22)-1-1 Harvest of tumor tissue and preparation of cell suspension

5 × $10^6$ human breast adenocarcinoma-derived MDA-MB-231 cells (American Type Culture Collection) subcultured in Leibovitz's L-15 medium containing 10% FBS were suspended in Matrigel and subcutaneously transplanted to each female immune-humanized NOG mouse (huNOG mouse) engrafted with human CD34 cells. The mice were grouped on the basis of their tumor volumes. 7 days after transplantation, 3 mg/kg hMAb1A-ADC or 0.015 mg/kg, 0.05 mg/kg, 0.15 mg/kg, 0.5 mg/kg, or 1.5 mg/kg ADCT-301 was intravenously administered to each mouse (n = 4 or 5). 21 or 23 days after ADC administration, tumor was harvested. The harvested tumor mass was treated with gentleMACS (Miltenyi Biotec) to recover cells. The cells were washed with PBS and resuspended therein. Then, the cell suspension was used as a sample for flow cytometry. The amount of PBS in resuspending the cells was the following amount depending on a tumor weight for addition: 0.3 mL for a tumor weight up to 50 mg; 0.6 mL for a tumor weight of 50 to 80 mg; 0.9 mL for a tumor weight of 80 to 100 mg; 1.2 mL for a tumor weight of 100 to 120 mg; 1.5 mL for a tumor weight of 120 to 160 mg; 2.0 mL for a tumor weight of 160 to 200 mg; and 2.5 mL for a tumor weight of 200 to 250 mg.

22)-1-2 Preparation of staining solution for flow cytometry analysis

Zombie NIR dye solution was prepared by diluting Zombie NIR dye solution attached to Zombie NIR Fixable Viability Kit (BioLegend, Inc.) 1000-fold with PBS. Fc Block solution was prepared by adding and mixing Mouse BD Fc Block (BD Biosciences) solution, Human BD Fc Block solution, and MACS buffer at a ratio of 1:1:48. Foxp3 Fixation/-Permeabilization buffer was prepared by mixing 3 mL of Concentrate and 9 mL of Diluent of Foxp3 Fixation/Permeabilization Concentrate and Diluent (eBioscience, Inc.). Perm wash buffer was prepared by diluting Intracellular Staining Perm Wash Buffer (BioLegend, Inc.) 10-fold with $H_2O$. A cell surface marker antibody cocktail solution was used by adding 2 μL of FITC anti-human CD25 Antibody (BioLegend, Inc.), 1 μL of APC anti-human CD8 Antibody (BioLegend, Inc.), 3 μL of Brilliant Violet 421 anti-human CD4 Antibody (BioLegend, Inc.), 2.5 μL of PE/Cyanine7 anti-human CD45RA Antibody (BioLegend, Inc.), 2 μL of Alexa Fluor 700 anti-mouse CD45 Antibody (BioLegend, Inc.), 2 μL of Brilliant Violet 605 anti-human CD45 Antibody (BioLegend, Inc.), and Brilliant Violet 650 anti-human CD3 Antibody, and adjusting the whole

amount to 50 μL with MACS buffer. An intracellular marker antibody cocktail solution was used by adding 3 μL of PE Mouse anti-Human FoxP3 (BD Biosciences), 3 μL of PerCP/Cyanine5.5 anti-human/mouse Granzyme B Recombinant Antibody (BioLegend, Inc.), 1 μL of Mouse BD Fc Block (BD Biosciences), and 1 μL of Human BD Fc Block, and adjusting the whole amount to 100 μL with Perm wash buffer.

22)-1-3 Antibody staining of sample for flow cytometry analysis

0.1 mL of Zombie NIR dye diluting solution was added to 0.2 mL of the sample for flow cytometry prepared in 22)-1-1, and the mixture was incubated at 4°C for 30 minutes. 0.1 mL of MACS buffer was added thereto, and the mixture was centrifuged, followed by the removal of a supernatant by suction. 0.1 mL of Fc Block solution was added to the remaining cells, which were then incubated at 4°C for 5 minutes. 0.1 mL of the cell surface marker antibody cocktail solution prepared in 22)-1-2 was added to the cells, which were then incubated at 4°C for 30 minutes. Subsequently, 0.1 mL of MACS buffer was added thereto, and the mixture was centrifuged, followed by the removal of a supernatant by suction. 0.2 mL of MACS buffer was added to the remaining cells, and the mixture was further centrifuged to remove a supernatant. This operation was further carried out twice for the washing of the cells. 0.2 mL of Foxp3 Fixation/Permeabilization buffer was added to the cells, which were then incubated at room temperature for 1 hour. After centrifugation, a supernatant was removed by suction, and 0.2 mL of Perm wash buffer was added to the remaining cells, which were further centrifuged to remove a supernatant. This operation was further carried out twice for the washing of the cells. 0.2 mL of the intracellular marker antibody cocktail solution was added to the washed cells, which were then incubated at 4°C for 1 hour and then washed twice with Perm wash buffer. A 4% paraformaldehyde solution was added to the cells, which were then incubated at 4°C for 15 minutes. The cells were washed twice with MACS buffer, and 0.18 mL of MACS buffer was then added to the cells. The sample was incubated overnight at 4°C, followed by flow cytometry analysis.

22)-1-4 Flow cytometry analysis

The sample prepared in 22)-1-3 was applied to a flow cytometer (BD LSR Fortessa X-20 cell analyzer: BD Biosciences) to measure the numbers of human CD45-positive cells and human granzyme-positive CD8T cells (human GZMB-positive and CD8-positive T cells) in a Zombie NIR dye-negative fraction. Then, the number of human GZMB-positive and CD8-positive T cells was assigned to the following expression to calculate the number of human GZMB-positive and CD8-positive T cells per mg of the tumor tissue (cells/mg).

[Expression 6]

$$\text{The number of cells measured} \times \frac{\text{Total amount of the sample measured in the flow cytometer}}{\text{Amount of the solution measured in the flow cytometer}} \times \frac{\text{Total amount of the cell suspension obtained from the tumor tissue}}{0.2} \times \frac{1}{\text{Weight of the tumor tissue}}$$

As a result, increase in the number of human GZMB-positive and CD8-positive T cells per mg of the tumor tissue was observed in the hMAb1A-ADC administration group compared to the ADCT-301 administration group (Figure 27). These results demonstrated that hMAb1A-ADC activates human CD8-positive T cells as compared to ADCT-301. This suggested that hMAb1A-ADC is superior to ADCT-301 in the activation of CTL by the removal of Treg.

(Example 23) Combined effect of cMAb2_hIgG1-ADC and anti-PD-1 antibody

23)-1 Antitumor activity evaluation in syngeneic model

$2 \times 10^5$ mouse colorectal cancer-derived CT26.WT cells (American Type Culture Collection, subcultured in RPMI1640 containing 10% FBS) were subcutaneously transplanted to each female BALB/C mouse. The mice were grouped on the basis of their tumor volumes. 10 days after transplantation, 5 mg/kg human IgG1-ADC or cMAb2_hIgG1-ADC was intravenously administered to each mouse (n = 8 or 10). 6, 9, and 11 days after transplantation, 5 mg/kg rat IgG2a control antibody (clone 2A3, Bio X Cell) or anti-PD-1 antibody (clone RMP1-14, Bio X Cell) was intravenously administered thereto.

$3 \times 10^5$ mouse breast cancer-derived EMT6 cells (American Type Culture Collection, subcultured in Waymouth's containing 15% FBS) were subcutaneously transplanted to each female BALB/C mouse. The mice were grouped on the basis of their tumor volumes. 5 days after transplantation, 5 mg/kg human IgG1-ADC or cMAb2_hIgG1-ADC was intravenously administered to each mouse (n = 8). 6, 8, and 11 days after transplantation, 5 mg/kg rat IgG2a control antibody or anti-PD-1 antibody was intravenously administered thereto.

$2 \times 10^5$ mouse colorectal cancer-derived MC38 cells (kindly provided by Dr. S.A. Rosenberg, National Cancer Institute, subcultured in RPMI1640 containing 10% FBS and 50 $\mu$M 2-mercaptoethanol) were subcutaneously transplanted to each female C57BL/6J mouse. The mice were grouped on the basis of their tumor volumes. 5 days after transplantation, 5 mg/kg human IgG1-ADC or cMAb2_hIgG1-ADC was intravenously administered to each mouse (n = 6). 6, 8, and 11 days after transplantation, 5 mg/kg rat IgG2a control antibody or anti-PD-1 antibody was intravenously administered thereto.

$3 \times 10^5$ mouse kidney cancer-derived RENCA cells (American Type Culture Collection, subcultured in RPMI1640 containing 10% FBS, nonessential amino acids for MEM, and sodium pyruvate) were subcutaneously transplanted to each male BALB/C mouse. The mice were grouped on the basis of their tumor volumes. 6 days after transplantation, 5 mg/kg human IgG1-ADC or cMAb2_hIgG1-ADC was intravenously administered to each mouse (n = 3 to 7). 7, 9, and 12 days after transplantation, 5 mg/kg rat IgG2a control antibody or anti-PD-1 antibody was intravenously administered thereto.

$3 \times 10^5$ mouse melanoma-derived B16F10 cells (American Type Culture Collection, subcultured in Dulbecco's Modified Eagle Medium containing 10% FBS) were subcutaneously transplanted to each female C57BL/6J mouse. The mice were grouped on the basis of their tumor volumes. 4 days after transplantation, 5 mg/kg human IgG1-ADC or cMAb2_hIgG1-ADC was intravenously administered to each mouse (n = 4 to 8). 5, 7, and 10 days after transplantation, 5 mg/kg rat IgG2a control antibody or anti-PD-1 antibody was intravenously administered thereto.

The major axis and minor axis of the transplanted tumor were measured two or three times a week using electronic digital calipers (manufactured by Mitutoyo Corp.), and the tumor volume was calculated according to the following expression.

$$\text{Tumor volume (mm}^3\text{)} = 1/2 \times \text{Minor axis (mm)} \times \text{Minor axis (mm)} \times \text{Major axis (mm)}$$

As a result, in the CT26.WT cell-, MC38 cell-, RENCA cell-, or B16F10 cell-transplanted syngeneic models, the cMAb2_hIgG1-ADC + anti-PD-1 antibody administration group (combination group) more strongly exhibited delayed tumor growth and tumor regression than the cMAb2_hIgG1-ADC + rat IgG2a control antibody administration group (cMAb2_hIgG1-ADC single-agent group) or the anti-PD-1 antibody + human IgG1-ADC administration group (anti-PD-1 antibody single-agent group) (Figure 32).

In the EMT-6 cell-transplanted syngeneic mouse models, the combination group more strongly exhibited delayed tumor growth and tumor regression than the anti-PD-1 antibody single-agent group (Figure 32). In the EMT-6 cell-transplanted syngeneic mouse models, the combination group had a complete response rate of 88%, and the cMAb2_hIgG1-ADC + rat IgG2a control antibody administration group had a complete response rate of 63%. Thus, the cMAb2_hIgG1-ADC + anti-PD-1 antibody administration group exhibited a higher complete response rate. This demonstrated that cMAb2_hIgG1-ADC used in combination with an anti-PD-1 antibody exhibits stronger antitumor activity than that of each single agent.

In both the RENCA cell- and B16F10 cell-transplanted syngeneic mouse models, the anti-PD-1 antibody single-agent group exhibited no antitumor effect whereas the cMAb2_hIgG1-ADC + anti-PD-1 antibody administration group (combination group) exhibited an antitumor effect. This result suggested that the anti-CD25 antibody-drug conjugate of the present invention used in combination with an immune checkpoint inhibitor is effective even for cancers that exhibit resistance to the immune checkpoint inhibitor.

(Example 24) Preparation of human IgG1LALA-PA chimeric MAb2 (cMAb2_hIgG1LALA-PA)

24)-1 Construction of light chain expression vector pCMA-LK

A fragment of approximately 5.4 kb obtained by the digestion of a plasmid pcDNA3.3-TOPO/LacZ (manufactured by Thermo Fisher Scientific Inc.) with restriction enzymes XbaI and PmeI, and a DNA fragment comprising a nucleotide sequence (shown in SEQ ID NO: 51) encoding a human light chain signal sequence and a human $\kappa$ chain constant region were ligated using In-Fusion HD PCR cloning kit (manufactured by Thermo Fisher Scientific Inc.) to prepare pcDNA3.3/LK.

A neomycin expression unit was removed from pcDNA3.3/LK to construct pCMA-LK.

24)-2 Construction of heavy chain expression vector pCMA-G1LALA-PA

A DNA fragment that lost the nucleotide sequence encoding the light chain signal sequence and the human κ chain constant region by the digestion of pCMA-LK with XbaI and PmeI, and a DNA fragment comprising a nucleotide sequence (shown in SEQ ID NO: 58) encoding a human heavy chain signal sequence and a human IgG1LALA-PA heavy chain constant region were digested with XbaI and PmeI and ligated using Ligation High (manufactured by Toyobo Co., Ltd.) to construct pCMA-G1LALA-PA.

24)-3 Construction of cMAb2_hIgG1LALA-PA light chain expression vector

A DNA fragment represented by nucleotide positions 61 to 402 of the signal sequence and cMAb2_hIgG1LALA-PA light chain nucleotide sequence represented by SEQ ID NO: 56 was synthesized (manufactured by Thermo Fisher Scientific Inc.). The synthesized DNA fragment was inserted into a cleavage site of pCMA-LK with a restriction enzyme BsiWI using In-Fusion HD PCR cloning kit (manufactured by Thermo Fisher Scientific Inc.) to construct a cMAb2_hIgG1LALA light chain expression vector. The amino acid sequence of the signal sequence and the cMAb2_hIgG1LALA-PA light chain is shown in SEQ ID NO: 54.

24)-4 Construction of cMAb2_hIgG1LALA-PA heavy chain expression vector

A DNA fragment represented by nucleotide positions 36 to 416 of the signal sequence and cMAb2_hIgG1LALA-PA heavy chain nucleotide sequence represented by SEQ ID NO: 57 was amplified. Then, the amplified DNA fragment was inserted into a cleavage site of pCMA-G1LALA-PA with a restriction enzyme BlpI using In-Fusion HD PCR cloning kit (manufactured by Thermo Fisher Scientific Inc.) to construct a cMAb2_hIgG1LALA-PA heavy chain expression vector. The amino acid sequence of the signal sequence and the cMAb2_hIgG1LALA-PA heavy chain is shown in SEQ ID NO: 55.

24)-5 Preparation of cMAb2_hIgG1LALA-PA

24)-5-1 Production of cMAb2_hIgG1LALA-PA

FreeStyle 293F cells (manufactured by Thermo Fisher Scientific Inc.) were passaged and cultured in accordance with the manual. The FreeStyle 293F cells in the logarithmic growth phase were diluted into $2.0 \times 10^6$ cells/mL with FreeStyle 293 expression medium (manufactured by Thermo Fisher Scientific Inc.), and 1.2 L of the dilution was inoculated to 5 L Optimum Growth Flask (manufactured by Thomson Instrument Company). 3.6 mg of Polyethyleneimine (manufactured by Polyscience) was added to 40 mL of Opti-Pro SFM medium (manufactured by Thermo Fisher Scientific Inc.). Subsequently, 600 μg of the heavy chain expression vector and 600 μg of the light chain expression vector were added to 40 mL of Opti-Pro SFM medium. The expression vector/Opti-Pro SFM mixed solution was added to the Polyethyleneimine/Opti-Pro SFM mixed solution, and the mixture was gently stirred, further left standing for 5 minutes, and then added to the FreeStyle 293F cells. The cells were shake-cultured at 105 rpm in an 8% $CO_2$ incubator at 37°C for 4 hours. Then, 1.2 L of EX-CELL VPRO medium (manufactured by SAFC Biosciences Inc.) and 60 mL of 43.4 g/L BD Recharge CD (manufactured by BD Biosciences) were added thereto, and the cells were shake-cultured at 105 rpm in an 8% $CO_2$ incubator at 37°C for 6 days. The obtained culture supernatant was centrifuged and then filtrated through a filter having a pore size of 0.2 μm (manufactured by Pall Corp.).

24)-5-2 Purification of cMAb2_hIgG1LALA-PA

From the culture supernatant obtained in 24)-5-1, the antibody was purified by rProtein A affinity chromatography. The culture supernatant was applied to a column (manufactured by Cytiva) packed with MabSelectSuRe equilibrated with PBS, and the column was then washed with PBS in an amount of two or more times the volume of the column. Subsequently, the antibody was eluted with a 2 M arginine hydrochloride solution (pH 4.0). The eluted fraction was dialyzed (manufactured by Thermo Fisher Scientific Inc., Slide-A-Lyzer Dialysis Cassette) for buffer replacement with HBSor (25 mM histidine/5% sorbitol, pH 6.0). Finally, the antibody solution was filtered through Minisart-Plus filter (manufactured by Sartorius Stedim Biotech SA) to prepare a purified sample.

(Example 25) Combined effect of cMAb2_hIgG1-ADC and anti-PD-1 antibody in syngeneic model with mouse Lewis lung cancer 3LL cell

$3 \times 10^5$ mouse Lewis lung cancer-derived 3LL cells (JCRB Cell Bank, subcultured in RPMI1640 containing 10% FBS) were subcutaneously transplanted to each female C57BL/6J mouse. The mice were grouped on the basis of their

tumor volumes. 6 days after transplantation, 5 mg/kg human IgG1-ADC or cMAb2_hIgG1-ADC was intravenously administered to each mouse (n = 8). 7, 9, and 12 days after transplantation, 5 mg/kg rat IgG2a control antibody (clone 2A3, Bio X Cell) or anti-PD-1 antibody (clone RMP1-14, Bio X Cell) was intravenously administered thereto.

The major axis and minor axis of the transplanted tumor were measured two or three times a week using electronic digital calipers (manufactured by Mitutoyo Corp.), and the tumor volume was calculated according to the following expression.

Tumor volume ($mm^3$) = 1/2 $\times$ Minor axis (mm) $\times$ Minor axis (mm) $\times$ Major axis (mm)

As a result, in the 3LL cell-transplanted syngeneic models, the cMAb2_hIgG1-ADC + anti-PD-1 antibody administration group (combination group) more strongly exhibited delayed tumor growth and tumor regression than the cMAb2_hIgG1-ADC + rat IgG2a control antibody administration group (cMAb2_hIgG1-ADC single-agent group) or the anti-PD-1 antibody + human IgG1-ADC administration group (anti-PD-1 antibody single-agent group) (Figure 33).

(Example 26) Combined effect of cMAb2_hIgG1-ADC and anti-CTLA-4 antibody in syngeneic model with mouse colorectal cancer MC38 cell

2 $\times$ $10^5$ mouse colorectal cancer-derived MC38 cells were subcutaneously transplanted to each female C57BL/6J mouse. The mice were grouped on the basis of their tumor volumes. 5 days after transplantation, 3 mg/kg human IgG1-ADC or cMAb2_hIgG1-ADC was intravenously administered to each mouse (n = 7 or 8). 6, 8, and 11 days after transplantation, 5 mg/kg rat IgG2b control antibody (clone: LTF-2, Bio X Cell) or anti-CTLA-4 antibody (clone: 9D9, Bio X Cell) was intravenously administered thereto.

The major axis and minor axis of the transplanted tumor were measured two or three times a week using electronic digital calipers (manufactured by Mitutoyo Corp.), and the tumor volume was calculated according to the following expression.

Tumor volume ($mm^3$) = 1/2 $\times$ Minor axis (mm) $\times$ Minor axis (mm) $\times$ Major axis (mm)

As a result, the cMAb2_hIgG1-ADC + anti-CTLA-4 antibody administration group (combination group) more strongly exhibited delayed tumor growth than the cMAb2_hIgG1-ADC + rat IgG2b control antibody administration group (cMAb2_hIgG1-ADC single-agent group) or the anti-CTLA-4 antibody + human IgG1-ADC administration group (anti-CTLA-4 antibody single-agent group) (Figure 34).

Industrial Applicability

The present invention has provided an anti-CD25 antibody having internalization activity without having the ability to block IL-2, and an antibody-drug conjugate comprising the antibody. The antibody-drug conjugate can be used as a therapeutic drug or the like for cancers.

Free Text of Sequence Listing

SEQ ID NO: 1: Amino acid sequence of MAb1 CDRL1
SEQ ID NO: 2: Amino acid sequence of MAb1 CDRL2
SEQ ID NO: 3: Amino acid sequence of MAb1 CDRL3
SEQ ID NO: 4: Amino acid sequence of MAb2 CDRL1
SEQ ID NO: 5: Amino acid sequence of MAb2 CDRL2
SEQ ID NO: 6: Amino acid sequence of MAb2 CDRL3
SEQ ID NO: 7: Amino acid sequence of MAb1 CDRH1
SEQ ID NO: 8: Amino acid sequence of MAb1 CDRH2
SEQ ID NO: 9: Amino acid sequence of MAb1 CDRH3
SEQ ID NO: 10: Amino acid sequence of MAb2 CDRH1
SEQ ID NO: 11: Amino acid sequence of MAb2 CDRH2
SEQ ID NO: 12: Amino acid sequence of MAb2 CDRH3
SEQ ID NO: 13: Full-length amino acid sequence of hMAb1A-L light chain
SEQ ID NO: 14: Full-length amino acid sequence of hMAb1B-L light chain

SEQ ID NO: 15: Full-length amino acid sequence of hMAb1AH heavy chain
SEQ ID NO: 16: Full-length amino acid sequence of hMAb1B-H heavy chain
SEQ ID NO: 17: Amino acid sequence of human CD25
SEQ ID NO: 18: Amino acid sequence of MAb1 light chain variable region
SEQ ID NO: 19: Nucleotide sequence encoding MAb1 light chain variable region
SEQ ID NO: 20: Amino acid sequence of MAb1 heavy chain variable region
SEQ ID NO: 21: Nucleotide sequence encoding MAb1 heavy chain variable region
SEQ ID NO: 22: Amino acid sequence of MAb2 light chain variable region
SEQ ID NO: 23: Nucleotide sequence encoding MAb2 light chain variable region
SEQ ID NO: 24: Amino acid sequence of MAb2 heavy chain variable region
SEQ ID NO: 25: Nucleotide sequence encoding MAb2 heavy chain variable region
SEQ ID NO: 26: Amino acid sequence of signal sequence and cMAb1_hIgG1LALA light chain
SEQ ID NO: 27: Amino acid sequence of signal sequence and cMAb1_hIgG1LALA heavy chain
SEQ ID NO: 28: Nucleotide sequence encoding signal sequence and cMAb1_hIgG1LALA light chain
SEQ ID NO: 29: Nucleotide sequence encoding signal sequence and cMAb1_hIgG1LALA heavy chain
SEQ ID NO: 30: Amino acid sequence of signal sequence and cMAb2_hIgG1LALA light chain
SEQ ID NO: 31: Amino acid sequence of signal sequence and cMAb2_hIgG1LALA heavy chain
SEQ ID NO: 32: Amino acid sequence of signal sequence and cMAb2_hIgG1 light chain
SEQ ID NO: 33: Amino acid sequence of signal sequence and cMAb2_hIgG1 heavy chain
SEQ ID NO: 34: Nucleotide sequence encoding signal sequence and cMAb2_hIgG1LALA light chain
SEQ ID NO: 35: Nucleotide sequence encoding signal sequence and cMAb2_hIgG1LALA heavy chain
SEQ ID NO: 36: Nucleotide sequence encoding signal sequence and cMAb2_hIgG1 light chain
SEQ ID NO: 37: Nucleotide sequence encoding signal sequence and cMAb2_hIgG1 heavy chain
SEQ ID NO: 38: Nucleotide sequence encoding signal sequence and hMAb1A light chain
SEQ ID NO: 39: Nucleotide sequence encoding signal sequence and hMAb1B light chain
SEQ ID NO: 40: Nucleotide sequence encoding signal sequence and hMAb1A heavy chain
SEQ ID NO: 41: Nucleotide sequence encoding signal sequence and hMAb1B heavy chain
SEQ ID NO: 42: Nucleotide sequence of human CD25
SEQ ID NO: 43: Nucleotide sequence of mouse CD25
SEQ ID NO: 44: Amino acid sequence of mouse CD25
SEQ ID NO: 45: Nucleotide sequence of primer 1F
SEQ ID NO: 46: Nucleotide sequence of primer 1R
SEQ ID NO: 47: Nucleotide sequence of cynomolgus monkey CD25
SEQ ID NO: 48: Amino acid sequence of cynomolgus monkey CD25
SEQ ID NO: 49: Nucleotide sequence of primer 2F
SEQ ID NO: 50: Nucleotide sequence of primer 2R
SEQ ID NO: 51: Nucleotide sequence encoding human light chain signal sequence and human κ chain constant region SEQ ID NO: 52: Nucleotide sequence encoding human heavy chain signal sequence and human IgG1LALA constant region SEQ ID NO: 53: Nucleotide sequence encoding heavy chain signal sequence and human G1 chain constant region SEQ ID NO: 54: Amino acid sequence of signal sequence and cMAb2_hIgG1LALA-PA light chain
SEQ ID NO: 55: Amino acid sequence of signal sequence and cMAb2_hIgG1LALA-PA heavy chain
SEQ ID NO: 56: Nucleotide sequence of signal sequence and cMAb2_hIgG1LALA-PA light chain
SEQ ID NO: 57: Nucleotide sequence of signal sequence and cMAb2_hIgG1LALA-PA heavy chain
SEQ ID NO: 58: Nucleotide sequence encoding human heavy chain signal sequence and human IgG1LALA-PA heavy chain constant region

## Claims

1. An anti-CD25 antibody or an antigen binding fragment of the antibody, having the following characteristics:

   (1) having no ability to block IL-2; and
   (2) exhibiting the ability to remove human regulatory T cells and/or the ability to promote the proliferation of human granzyme-positive CD8-positive cells by conjugation to a cytotoxic active compound.

2. The antibody or the antigen binding fragment of the antibody according to claim 1, wherein the antibody or the antigen binding fragment has activity of being internalized in CD25-expressing cells by binding to CD25.

**3.** The antibody or the antigen binding fragment of the antibody according to claim 1 or 2, wherein the CD25 is human CD25, cynomolgus monkey CD25, or mouse CD25.

**4.** The antibody or the antigen binding fragment of the antibody according to claim 3, wherein the CD25 is human CD25 or cynomolgus monkey CD25.

**5.** The antibody or the antigen binding fragment of the antibody according to claim 3, wherein the CD25 is human CD25.

**6.** The antibody or the antigen binding fragment of the antibody according to claim 3, wherein the CD25 is mouse CD25.

**7.** The antibody or the antigen binding fragment of the antibody according to any one of claims 1 to 6, wherein the antibody or the antigen binding fragment has competitive inhibitory activity, for binding to CD25, against at least any one antibody selected from the group consisting of the following antibodies (1) to (6):

(1) an antibody comprising a light chain consisting of an amino acid sequence from positions 21 to 232 of SEQ ID NO: 26 and a heavy chain consisting of an amino acid sequence from positions 20 to 476 of SEQ ID NO: 27,
(2) an antibody comprising a light chain consisting of an amino acid sequence from positions 21 to 233 of SEQ ID NO: 13 and a heavy chain consisting of an amino acid sequence from positions 20 to 476 of SEQ ID NO: 15,
(3) an antibody comprising a light chain consisting of an amino acid sequence from positions 21 to 233 of SEQ ID NO: 14 and a heavy chain consisting of an amino acid sequence from positions 20 to 476 of SEQ ID NO: 16,
(4) an antibody comprising a light chain consisting of an amino acid sequence from positions 21 to 232 of SEQ ID NO: 30 and a heavy chain consisting of an amino acid sequence from positions 20 to 463 of SEQ ID NO: 31,
(5) an antibody comprising a light chain consisting of an amino acid sequence from positions 21 to 239 of SEQ ID NO: 32 and a heavy chain consisting of an amino acid sequence from positions 20 to 463 of SEQ ID NO: 33, and
(6) an antibody comprising a light chain consisting of an amino acid sequence from positions 21 to 239 of SEQ ID NO: 54 and a heavy chain consisting of an amino acid sequence from positions 20 to 463 of SEQ ID NO: 55.

**8.** The antibody or the antigen binding fragment of the antibody according to any one of claims 1 to 7, wherein the antibody comprises

CDRL1, CDRL2, and CDRL3 in any one combination selected from the group consisting of the following combinations (1) and (2):

(1) CDRL1 consisting of the amino acid sequence represented by SEQ ID NO: 1, CDRL2 consisting of the amino acid sequence represented by SEQ ID NO: 2 (KAS), and CDRL3 consisting of the amino acid sequence represented by SEQ ID NO: 3, and
(2) CDRL1 consisting of the amino acid sequence represented by SEQ ID NO: 4, CDRL2 consisting of the amino acid sequence represented by SEQ ID NO: 5 (FVS), and CDRL3 consisting of the amino acid sequence represented by SEQ ID NO: 6, and

CDRH1, CDRH2, and CDRH3 in any one combination selected from the group consisting of the following combinations (3) and (4):

(3) CDRH1 consisting of the amino acid sequence represented by SEQ ID NO: 7, CDRH2 consisting of the amino acid sequence represented by SEQ ID NO: 8, and CDRH3 consisting of the amino acid sequence represented by SEQ ID NO: 9, and
(4) CDRH1 consisting of the amino acid sequence represented by SEQ ID NO: 10, CDRH2 consisting of the amino acid sequence represented by SEQ ID NO: 11, and CDRH3 consisting of the amino acid sequence represented by SEQ ID NO: 12.

**9.** The antibody or the antigen binding fragment of the antibody according to any one of claims 1 to 8, wherein the antibody comprises
CDRL1, CDRL2, and CDRL3, and CDRH1, CDRH2, and CDRH3 in any one combination selected from the group consisting of the following combinations (1) and (2):

(1) CDRL1 consisting of the amino acid sequence represented by SEQ ID NO: 1, CDRL2 consisting of the amino acid sequence represented by SEQ ID NO: 2 (KAS), and CDRL3 consisting of the amino acid sequence represented by SEQ ID NO: 3, and CDRH1 consisting of the amino acid sequence represented by SEQ ID

NO: 7, CDRH2 consisting of the amino acid sequence represented by SEQ ID NO: 8, and CDRH3 consisting of the amino acid sequence represented by SEQ ID NO: 9, and
(2) CDRL1 consisting of the amino acid sequence represented by SEQ ID NO: 4, CDRL2 consisting of the amino acid sequence represented by SEQ ID NO: 5 (FVS), and CDRL3 consisting of the amino acid sequence represented by SEQ ID NO: 6, and CDRH1 consisting of the amino acid sequence represented by SEQ ID NO: 10, CDRH2 consisting of the amino acid sequence represented by SEQ ID NO: 11, and CDRH3 consisting of the amino acid sequence represented by SEQ ID NO: 12.

**10.** The antibody or the antigen binding fragment of the antibody according to any one of claims 1 to 9, wherein the antibody or the antigen binding fragment is humanized.

**11.** The antibody or the antigen binding fragment of the antibody according to any one of claims 1 to 10, wherein the antibody comprises

any one light chain variable region selected from the group consisting of the following light chain variable regions (1) to (4):

(1) a light chain variable region consisting of an amino acid sequence from positions 21 to 127 of SEQ ID NO: 13,
(2) a light chain variable region consisting of an amino acid sequence from positions 21 to 127 of SEQ ID NO: 14,
(3) a light chain variable region consisting of an amino acid sequence having at least 95% or higher sequence identity to the sequences of framework regions except for CDR sequences in each of the amino acid sequences of (1) and (2), and
(4) a light chain variable region consisting of an amino acid sequence derived by the deletion, substitution, or addition of one or several amino acids from the sequences of framework regions except for CDR sequences in each of the amino acid sequences of (1) and (2), and

any one heavy chain variable region selected from the group consisting of the following heavy chain variable regions (5) to (8):

(5) a heavy chain variable region consisting of an amino acid sequence from positions 20 to 146 of SEQ ID NO: 15,
(6) a heavy chain variable region consisting of an amino acid sequence from positions 20 to 146 of SEQ ID NO: 16,
(7) a heavy chain variable region consisting of an amino acid sequence having at least 95% or higher sequence homology to the sequences of framework regions except for CDR sequences in each of the amino acid sequences of (5) and (6), and
(8) a heavy chain variable region consisting of an amino acid sequence derived by the deletion, substitution, or addition of one or several amino acids from the sequences of framework regions except for CDR sequences in each of the amino acid sequences of (5) and (6).

**12.** The antibody or the antigen binding fragment of the antibody according to any one of claims 1 to 11, wherein the antibody comprises a light chain variable region and a heavy chain variable region in any of the following combinations (1) and (2):

(1) a light chain variable region consisting of an amino acid sequence from positions 21 to 127 of SEQ ID NO: 13 and a heavy chain variable region consisting of an amino acid sequence from positions 20 to 146 of SEQ ID NO: 15, and
(2) a light chain variable region consisting of an amino acid sequence from positions 21 to 127 of SEQ ID NO: 14 and a heavy chain variable region consisting of an amino acid sequence from positions 20 to 146 of SEQ ID NO: 16.

**13.** The antibody or the antigen binding fragment of the antibody according to any one of claims 1 to 12, wherein the antibody comprises any of the following combinations (1) and (2):

(1) a light chain consisting of an amino acid sequence from positions 21 to 233 of SEQ ID NO: 13 and a heavy chain consisting of an amino acid sequence from positions 20 to 476 of SEQ ID NO: 15, and

(2) a light chain consisting of an amino acid sequence from positions 21 to 233 of SEQ ID NO: 14 and a heavy chain consisting of an amino acid sequence from positions 20 to 476 of SEQ ID NO: 16.

**14.** The antibody or the antigen binding fragment of the antibody according to claim 13, wherein the antibody comprises a light chain consisting of an amino acid sequence from positions 21 to 233 of SEQ ID NO: 13 and a heavy chain consisting of an amino acid sequence from positions 20 to 476 of SEQ ID NO: 15.

**15.** The antibody or the antigen binding fragment of the antibody according to claim 13, wherein the antibody comprises a light chain consisting of an amino acid sequence from positions 21 to 233 of SEQ ID NO: 14 and a heavy chain consisting of an amino acid sequence from positions 20 to 476 of SEQ ID NO: 16.

**16.** The antigen binding fragment of the antibody according to any one of claims 1 to 15, wherein the antigen binding fragment is selected from the group consisting of Fab, F(ab')2, Fab', and Fv.

**17.** The antigen binding fragment of the antibody according to any one of claims 1 to 15, wherein the antigen binding fragment is scFv.

**18.** A humanized antibody comprising a light chain variable region consisting of an amino acid sequence from positions 21 to 127 of SEQ ID NO: 13 and a heavy chain variable region consisting of an amino acid sequence from positions 20 to 146 of SEQ ID NO: 15.

**19.** A humanized antibody comprising a light chain consisting of an amino acid sequence from positions 21 to 233 of SEQ ID NO: 13 and a heavy chain consisting of an amino acid sequence from positions 20 to 476 of SEQ ID NO: 15.

**20.** A humanized antibody comprising a light chain variable region consisting of an amino acid sequence from positions 21 to 127 of SEQ ID NO: 14 and a heavy chain variable region consisting of an amino acid sequence from positions 20 to 146 of SEQ ID NO: 16.

**21.** A humanized antibody comprising a light chain consisting of an amino acid sequence from positions 21 to 233 of SEQ ID NO: 14 and a heavy chain consisting of an amino acid sequence from positions 20 to 476 of SEQ ID NO: 16.

**22.** A polynucleotide encoding the antibody or the antigen binding fragment of the antibody according to any one of claims 1 to 21.

**23.** The polynucleotide according to claim 22, wherein the polynucleotide comprises
polynucleotides in any one combination selected from the group consisting of the following combinations (1) and (2):

(1) a polynucleotide encoding a light chain variable region comprising CDRL1 consisting of the amino acid sequence represented by SEQ ID NO: 1, CDRL2 consisting of the amino acid sequence represented by SEQ ID NO: 2 (KAS), and CDRL3 consisting of the amino acid sequence represented by SEQ ID NO: 3, and a polynucleotide encoding a heavy chain variable region comprising CDRH1 consisting of the amino acid sequence represented by SEQ ID NO: 7, CDRH2 consisting of the amino acid sequence represented by SEQ ID NO: 8, and CDRH3 consisting of the amino acid sequence represented by SEQ ID NO: 9, and
(2) a polynucleotide encoding a light chain variable region comprising CDRL1 consisting of the amino acid sequence represented by SEQ ID NO: 4, CDRL2 consisting of the amino acid sequence represented by SEQ ID NO: 5 (FVS), and CDRL3 consisting of the amino acid sequence represented by SEQ ID NO: 6, and a polynucleotide encoding a heavy chain variable region comprising CDRH1 consisting of the amino acid sequence represented by SEQ ID NO: 10, CDRH2 consisting of the amino acid sequence represented by SEQ ID NO: 11, and CDRH3 consisting of the amino acid sequence represented by SEQ ID NO: 12.

**24.** The polynucleotide according to claim 22 or 23, wherein the polynucleotide comprises a polynucleotide encoding a light chain consisting of an amino acid sequence from positions 21 to 233 of SEQ ID NO: 13 and a polynucleotide encoding a heavy chain consisting of an amino acid sequence from positions 20 to 476 of SEQ ID NO: 15.

**25.** The polynucleotide according to claim 22 or 23, wherein the polynucleotide comprises a polynucleotide encoding a light chain consisting of an amino acid sequence from positions 21 to 233 of SEQ ID NO: 14 and a polynucleotide encoding a heavy chain consisting of an amino acid sequence from positions 20 to 476 of SEQ ID NO: 16.

**26.** An expression vector comprising the polynucleotide according to any one of claims 22 to 25.

**27.** A host cell transformed with the expression vector according to claim 26.

**28.** The host cell according to claim 27, wherein the host cell is a eukaryotic cell.

**29.** A method for producing an antibody or an antigen binding fragment of the antibody, comprising the steps of: culturing the host cell according to claim 27 or 28; and collecting the antibody or the antigen binding fragment of the antibody of interest from the cultures obtained in the preceding step.

**30.** The antibody or the antigen binding fragment of the antibody according to any one of claims 1 to 21, wherein a heavy chain or a light chain has one or two or more modifications selected from the group consisting of N-linked glycosylation, O-linked glycosylation, N-terminal processing, C-terminal processing, deamidation, isomerization of aspartic acid, oxidation of methionine, addition of a methionine residue to the N terminus, amidation of a proline residue, conversion of N-terminal glutamine or N-terminal glutamic acid to pyroglutamic acid, and deletion of one or two amino acids from the carboxyl terminus.

**31.** The antibody according to claim 30, wherein one or two amino acids are deleted from the carboxyl terminus of a heavy chain.

**32.** The antibody according to claim 31, wherein one amino acid is deleted from each of the carboxyl termini of both of two heavy chains.

**33.** The humanized antibody according to any one of claims 18 to 21, wherein one amino acid is deleted from each of the carboxyl termini of both of two heavy chains.

**34.** The humanized antibody according to claim 33, wherein the deleted amino acid is lysine.

**35.** The antibody according to claim 30, wherein a proline residue at the carboxyl terminus of a heavy chain is further amidate.

**36.** The antibody or the antigen binding fragment of the antibody according to any one of claims 1 to 21 and 30 to 35, wherein the antibody or the antigen binding fragment has the modification of a glycan for enhancing antibody-dependent cellular cytotoxic activity.

**37.** An antibody-drug conjugate in which the antibody or the antigen binding fragment of the antibody according to any one of claims 1 to 21 and 30 to 36 is conjugated to a drug.

**38.** The antibody-drug conjugate according to claim 37, wherein the drug is any one or more drugs selected from the group consisting of a substance having cytotoxic activity, a cytotoxic active compound, a substance having antitumor activity, an antitumor active compound, a chemotherapeutic, a molecular targeting drug, an immunoactivator, an immunosuppressant, a toxin, a photosensitive substance, a drug for diagnosis, a protein, a peptide, an amino acid, a nucleic acid, an antigen, a vitamin, a hormone, a substance effective for blood disease, a substance effective for autoimmune disease, an anti-inflammatory substance, an antibacterial substance, an antifungal substance, an antiparasitic substance, an antiviral substance, and an anti-anesthetic substance.

**39.** The antibody-drug conjugate according to claim 38, wherein the drug is a cytotoxic active compound.

**40.** The antibody-drug conjugate according to claim 39, wherein the cytotoxic active compound is a cytotoxic active compound represented by the following formula:

[Formula 1]

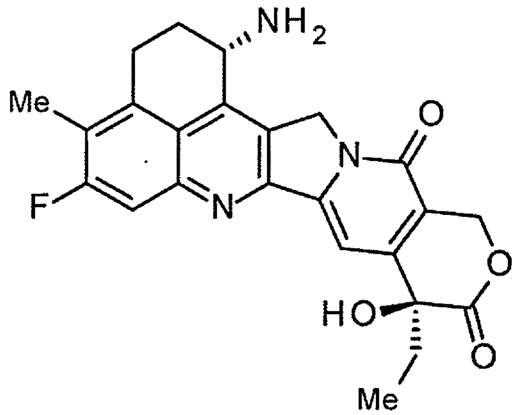

**41.** The antibody-drug conjugate according to any one of claims 37 to 40, wherein the antibody is conjugated to the drug via a linker having any structure selected from the group consisting of the following formulas (a) to (f) :

(a) -(Succinimid-3-yl-N)-$CH_2CH_2$-C(=O)-GGFG-NH-$CH_2CH_2CH_2$-C(=O)-,

(b) - (Succinimid-3-yl-N)-$CH_2CH_2CH_2CH_2CH_2$-C(=O) -GGFG-NH-$CH_2CH_2CH_2$-C(=O)-,

(c) -(Succinimid-3-yl-N)-$CH_2CH_2CH_2CH_2CH_2$-C(=O)-GGFG-NH-$CH_2$-O-$CH_2$-C(=O)-,

(d) -(Succinimid-3-yl-N)-$CH_2CH_2CH_2CH_2CH_2$-C(=O) -GGFG-NH-$CH_2CH_2$-O-$CH_2$-C(=O)-,

(e) -(Succinimid-3-yl-N)-$CH_2CH_2$-C(=O)-NH-$CH_2CH_2O$-$CH_2CH_2O$-$CH_2CH_2$-C(=O)-GGFG-NH-$CH_2CH_2CH_2$-C(=O)-, and

(f) -(Succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-GGFG-NH-$CH_2CH_2CH_2$-C(=O)-,

wherein the antibody is connected to the end of - (Succinimid-3-yl-N), the drug is connected to the carbonyl group at another end, GGFG represents an amino acid sequence consisting of glycine-glycine-phenylalanine-glycine linked through peptide bonds, and -(Succinimid-3-yl-N)- has a structure represented by the following formula:

[Formula 2]

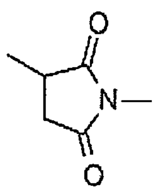

which is connected to the antibody at the 3-position thereof and is connected to a methylene group in the linker structure containing this structure on the nitrogen atom at the 1-position.

**42.** The antibody-drug conjugate according to any one of claims 37 to 41, wherein the linker is represented by any formula selected from the group consisting of the following formulas (c), (d), and (e):

(c) -(Succinimid-3-yl-N)-$CH_2CH_2CH_2CH_2CH_2$-C(=O)-GGFG-NH-$CH_2$-O-$CH_2$-C(=O)-,

(d) -(Succinimid-3-yl-N)-$CH_2CH_2CH_2CH_2CH_2$-C(=O) -GGFG-NH-$CH_2CH_2$-O-$CH_2$-C(=O)-,

(e) -(Succinimid-3-yl-N)-$CH_2CH_2$-C(=O)-NH-$CH_2CH_2$O-$CH_2CH_2$O-$CH_2CH_2$-C(=O)-GGFG-NH-$CH_2CH_2CH_2$-C(=O)-

**43.** The antibody-drug conjugate according to any one of claims 37 to 42, wherein the linker is represented by the following formula (c) or (e):

(c) -(Succinimid-3-yl-N)-$CH_2CH_2CH_2CH_2CH_2$-C(=O)-GGFG-NH-$CH_2$-O-$CH_2$-C(=O)-,

(e) -(Succinimid-3-yl-N)-$CH_2CH_2$-C(=O)-NH-$CH_2CH_2$O-$CH_2CH_2$O-$CH_2CH_2$-C(=O)-GGFG-NH-$CH_2CH_2CH_2$-C(=O)-

**44.** The antibody-drug conjugate according to any one of claims 37 to 43, wherein the antibody-drug conjugate has a structure represented by the following formula:

[Formula 3]

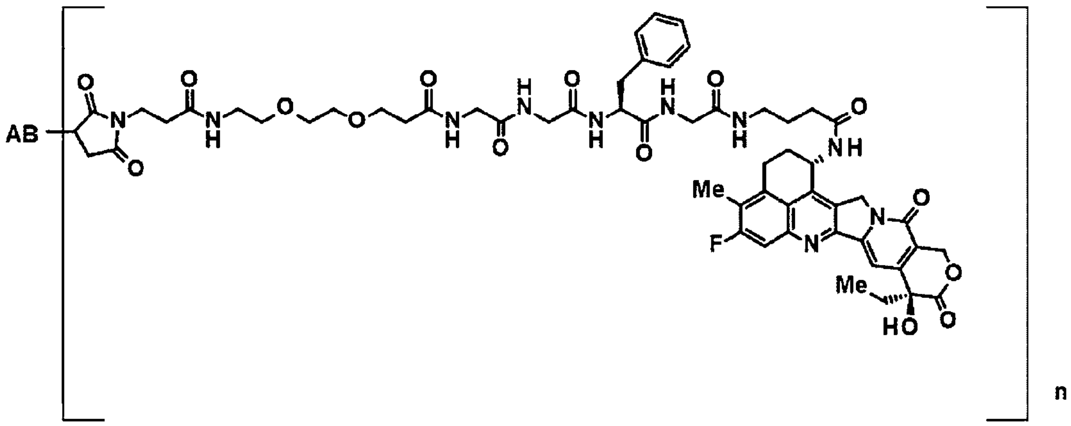

wherein AB represents the antibody or the antigen binding fragment of the antibody, n represents an average number of units of the drug-linker structure conjugated to the antibody or the antigen binding fragment of the antibody per antibody or per antigen binding fragment of the antibody, and the antibody or the antigen binding fragment of the antibody is connected to the linker via a sulfhydryl group derived from the antibody.

**45.** The antibody-drug conjugate according to any one of claims 37 to 43, wherein the antibody-drug conjugate has a structure represented by the following formula:

[Formula 4]

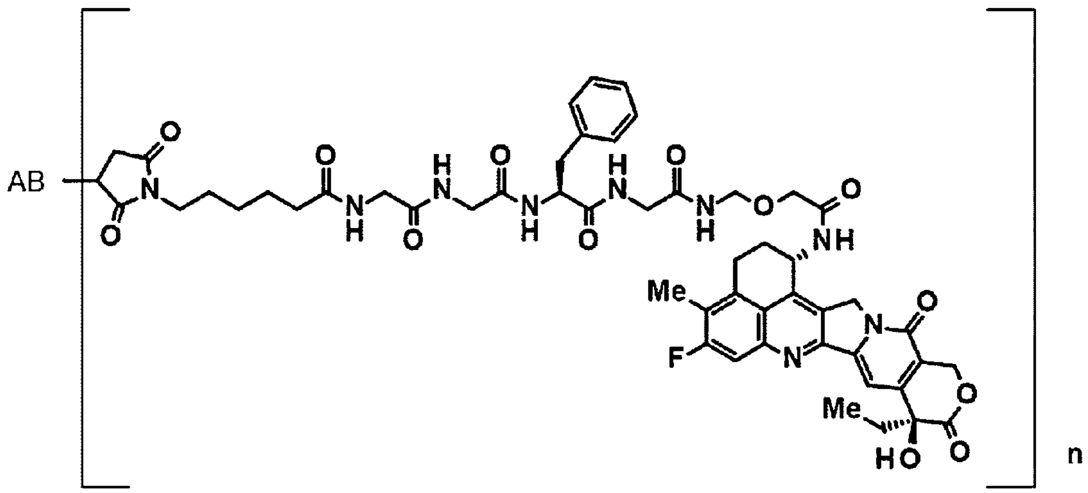

wherein AB represents the antibody or the antigen binding fragment of the antibody, n represents an average number of units of the drug-linker structure conjugated to the antibody or the antigen binding fragment of the antibody per antibody or per antigen binding fragment of the antibody, and the antibody or the antigen binding fragment of the antibody is connected to the linker via a sulfhydryl group derived from the antibody or the antigen binding fragment of the antibody.

**46.** The antibody-drug conjugate according to any one of claims 37 to 45, wherein the antibody is an antibody comprising a light chain variable region and a heavy chain variable region in any one combination selected from the group consisting of the following combinations (1) and (2):

(1) a light chain variable region consisting of an amino acid sequence from positions 21 to 127 of SEQ ID NO: 13 and a heavy chain variable region consisting of an amino acid sequence from positions 20 to 146 of SEQ ID NO: 15, and
(2) a light chain variable region consisting of an amino acid sequence from positions 21 to 127 of SEQ ID NO: 14 and a heavy chain variable region consisting of an amino acid sequence from positions 20 to 146 of SEQ ID NO: 16, or an antigen binding fragment of the antibody.

**47.** The antibody-drug conjugate according to claim 46, wherein the antibody is an antibody comprising a light chain variable region consisting of an amino acid sequence from positions 21 to 127 of SEQ ID NO: 13 and a heavy chain variable region consisting of an amino acid sequence from positions 20 to 146 of SEQ ID NO: 15, or an antigen binding fragment of the antibody.

**48.** The antibody-drug conjugate according to claim 46, wherein the antibody is an antibody comprising a light chain variable region consisting of an amino acid sequence from positions 21 to 127 of SEQ ID NO: 14 and a heavy chain variable region consisting of an amino acid sequence from positions 20 to 146 of SEQ ID NO: 16, or an antigen binding fragment of the antibody.

**49.** The antibody-drug conjugate according to any one of claims 37 to 45, wherein the antibody is an antibody comprising a light chain and a heavy chain in any one combination selected from the group consisting of the following combinations (1) and (2):

(1) a light chain consisting of an amino acid sequence from positions 21 to 233 of SEQ ID NO: 13 and a heavy chain consisting of an amino acid sequence from positions 20 to 476 of SEQ ID NO: 15, and
(2) a light chain consisting of an amino acid sequence from positions 21 to 233 of SEQ ID NO: 14 and a heavy chain consisting of an amino acid sequence from positions 20 to 476 of SEQ ID NO: 16,
or an antigen binding fragment of the antibody.

**50.** The antibody-drug conjugate according to claim 49, wherein the antibody is an antibody comprising a light chain consisting of an amino acid sequence from positions 21 to 233 of SEQ ID NO: 13 and a heavy chain consisting of an

amino acid sequence from positions 20 to 476 of SEQ ID NO: 15, or an antigen binding fragment of the antibody.

**51.** The antibody-drug conjugate according to claim 49, wherein the antibody is an antibody comprising a light chain consisting of an amino acid sequence from positions 21 to 233 of SEQ ID NO: 14 and a heavy chain consisting of an amino acid sequence from positions 20 to 476 of SEQ ID NO: 16, or an antigen binding fragment of the antibody.

**52.** The antibody-drug conjugate according to any one of claims 37 to 51, wherein a heavy chain or a light chain has one or two or more modifications selected from the group consisting of N-linked glycosylation, O-linked glycosylation, N-terminal processing, C-terminal processing, deamidation, isomerization of aspartic acid, oxidation of methionine, addition of a methionine residue to the N terminus, amidation of a proline residue, conversion of N-terminal glutamine or N-terminal glutamic acid to pyroglutamic acid, and deletion of one or two amino acids from the carboxyl terminus.

**53.** The antibody-drug conjugate according to claim 52, wherein one or two amino acids are deleted from the carboxyl terminus of a heavy chain.

**54.** The antibody-drug conjugate according to claim 53, wherein one amino acid is deleted from each of the carboxyl termini of both of two heavy chains.

**55.** The antibody-drug conjugate according to claim 52, wherein a proline residue at the carboxyl terminus of a heavy chain is further amidate.

**56.** The antibody-drug conjugate according to any one of claims 37 to 55, wherein the antibody-drug conjugate has the modification of a glycan for enhancing antibody-dependent cellular cytotoxic activity.

**57.** The antibody-drug conjugate according to any one of claims 37 to 56, wherein the average number of units of the drug-linker structure conjugated per antibody is in the range of 1 to 10.

**58.** The antibody-drug conjugate according to claim 57, wherein the average number of units of the drug-linker structure conjugated per antibody is in the range of 2 to 8.

**59.** The antibody-drug conjugate according to claim 58, wherein the average number of units of the drug-linker structure conjugated per antibody is in the range of 5 to 8.

**60.** The antibody-drug conjugate according to claim 59, wherein the average number of units of the drug-linker structure conjugated per antibody is 7 to 8.

**61.** An antibody-drug conjugate having a structure represented by the following formula:

[Formula 5]

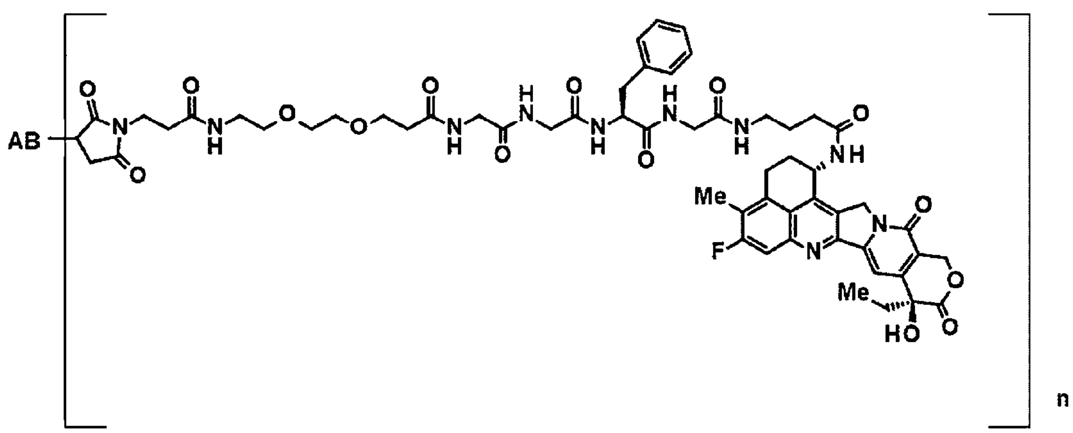

wherein AB represents a humanized antibody comprising a light chain variable region consisting of an amino acid sequence from positions 21 to 127 of SEQ ID NO: 13 and a heavy chain variable region consisting of an amino acid

sequence from positions 20 to 146 of SEQ ID NO: 15, n represents an average number of units of the drug-linker structure conjugated to the antibody per antibody and is in the range of 7 to 8, and the antibody is connected to the linker via a sulfhydryl group derived from the antibody.

**62.** An antibody-drug conjugate having a structure represented by the following formula:

[Formula 6]

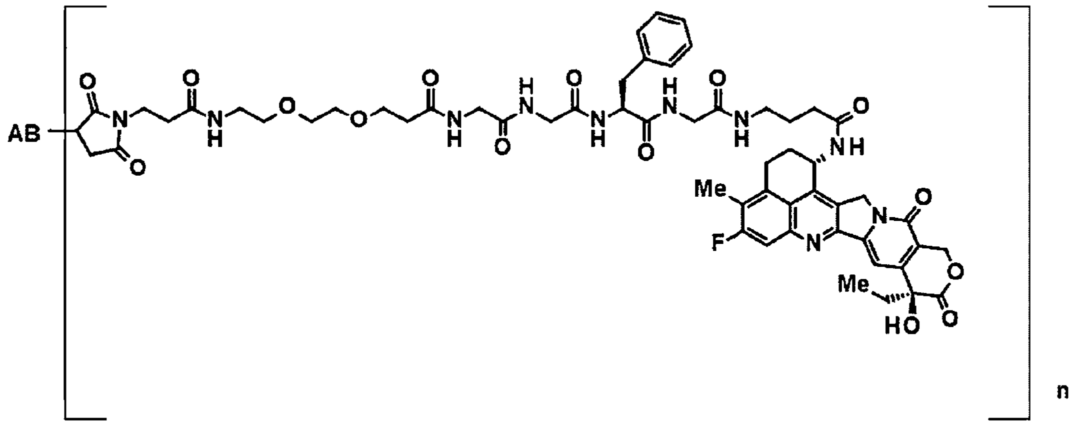

wherein AB represents a humanized antibody comprising a light chain consisting of an amino acid sequence from positions 21 to 233 of SEQ ID NO: 13 and a heavy chain consisting of an amino acid sequence from positions 20 to 476 of SEQ ID NO: 15, n represents an average number of units of the drug-linker structure conjugated to the antibody per antibody and is in the range of 7 to 8, and the antibody is connected to the linker via a sulfhydryl group derived from the antibody.

**63.** An antibody-drug conjugate having a structure represented by the following formula:

[Formula 7]

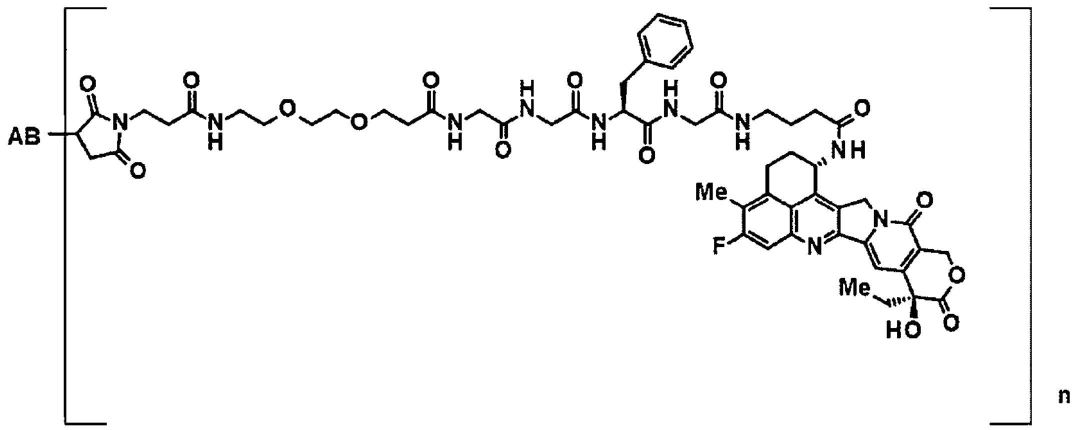

wherein AB represents a humanized antibody comprising a light chain variable region consisting of an amino acid sequence from positions 21 to 127 of SEQ ID NO: 14 and a heavy chain variable region consisting of an amino acid sequence from positions 20 to 146 of SEQ ID NO: 16, n represents an average number of units of the drug-linker structure conjugated to the antibody per antibody and is in the range of 7 to 8, and the antibody is connected to the linker via a sulfhydryl group derived from the antibody.

**64.** An antibody-drug conjugate having a structure represented by the following formula:

[Formula 8]

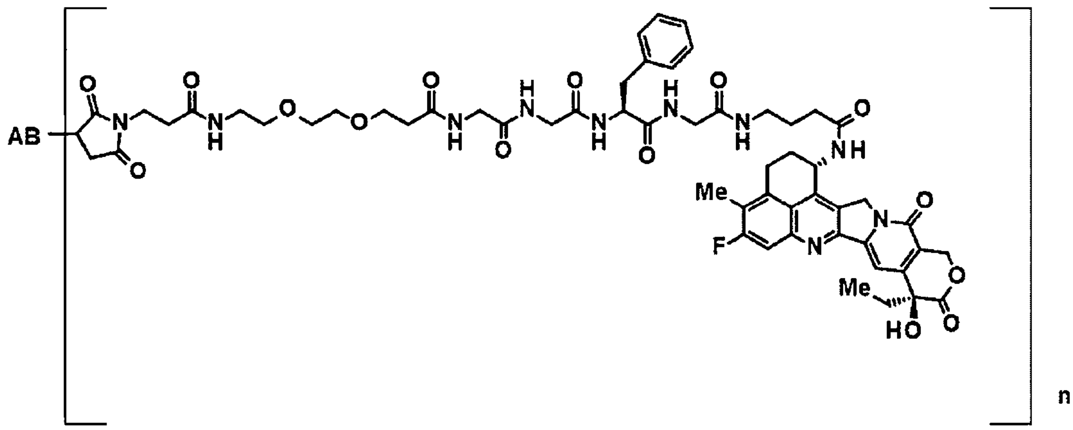

wherein AB represents a humanized antibody comprising a light chain consisting of an amino acid sequence from positions 21 to 233 of SEQ ID NO: 14 and a heavy chain consisting of an amino acid sequence from positions 20 to 476 of SEQ ID NO: 16, n represents an average number of units of the drug-linker structure conjugated to the antibody per antibody and is in the range of 7 to 8, and the antibody is connected to the linker via a sulfhydryl group derived from the antibody.

**65.** An antibody-drug conjugate having a structure represented by the following formula:

[Formula 9]

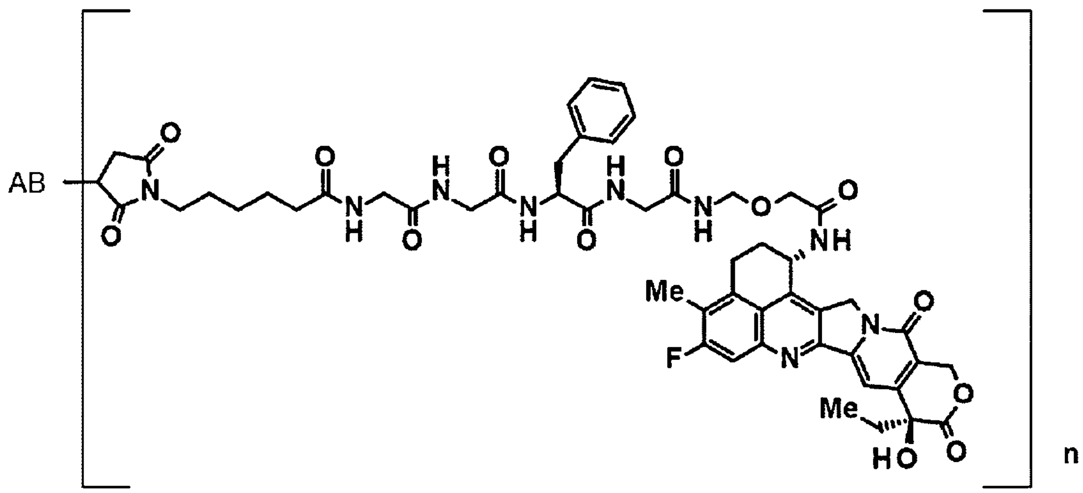

wherein AB represents an antibody comprising a light chain variable region consisting of the amino acid sequence of SEQ ID NO: 18 and a heavy chain variable region consisting of the amino acid sequence of SEQ ID NO: 20, n represents an average number of units of the drug-linker structure conjugated to the antibody per antibody and is in the range of 7 to 8, and the antibody is connected to the linker via a sulfhydryl group derived from the antibody.

**66.** An antibody-drug conjugate having a structure represented by the following formula:

[Formula 10]

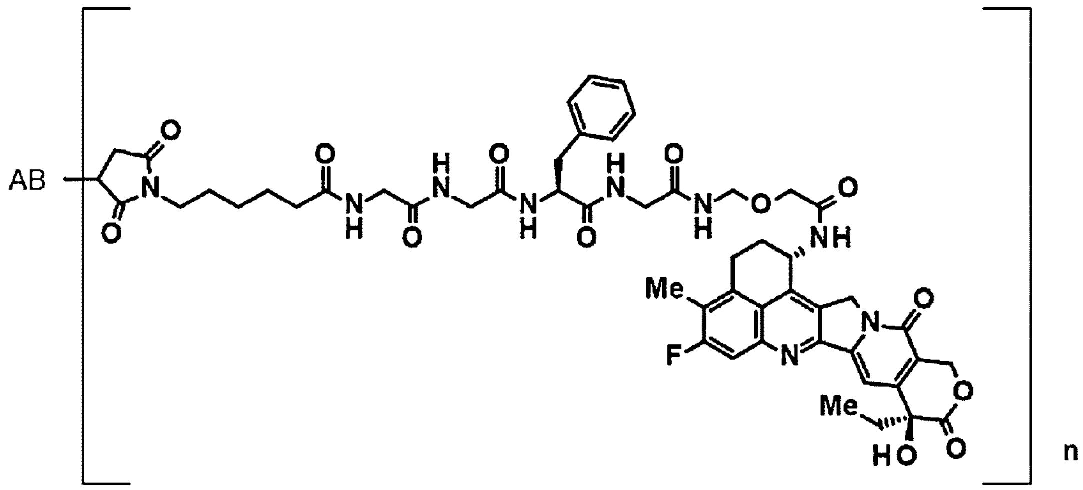

wherein AB represents an antibody comprising a light chain consisting of an amino acid sequence from positions 21 to 232 of SEQ ID NO: 26 and a heavy chain consisting of an amino acid sequence from positions 20 to 476 of SEQ ID NO: 27, n represents an average number of units of the drug-linker structure conjugated to the antibody per antibody and is in the range of 7 to 8, and the antibody is connected to the linker via a sulfhydryl group derived from the antibody.

**67.** The antibody-drug conjugate according to any one of claims 61 to 66, wherein one amino acid is deleted from each of the carboxyl termini of both of two heavy chains of the antibody.

**68.** The antibody-drug conjugate according to claim 67, wherein the deleted amino acid is lysine.

**69.** A pharmaceutical composition comprising the antibody or the antigen binding fragment of the antibody according to any one of claims 1 to 21 and 30 to 36, the antibody-drug conjugate according to any one of claims 37 to 68, a salt of the component, or a hydrate of the component or the salt.

**70.** The pharmaceutical composition according to claim 69, wherein the pharmaceutical composition is an antitumor drug.

**71.** The pharmaceutical composition according to claim 70, wherein tumor cells express CD25, or tumor immunity is suppressed by regulatory T cells.

**72.** The pharmaceutical composition according to claim 71, wherein the tumor environment and/or the lymph node contains regulatory T cells.

**73.** The pharmaceutical composition according to any one of claims 69 to 72, wherein the tumor is blood cancer, breast cancer, small-cell lung cancer, non-small cell lung cancer, head and neck cancer, brain tumor, glioma, eye tumor, esophageal cancer, stomach cancer, colorectal cancer, colon cancer, liver cancer, pancreatic cancer, renal cancer, kidney cancer, urinary bladder cancer, adrenal cortex cancer, prostate cancer, uterine cervical cancer, endometrial cancer, ovary cancer, melanoma, or sarcoma.

**74.** A method for treating tumor, comprising administering any member selected from the antibody or the antigen binding fragment of the antibody according to any one of claims 1 to 21 and 30 to 36, the antibody-drug conjugate according to any one of claims 37 to 68, a salt of the component, and a hydrate of the component or the salt to an individual.

**75.** The treatment method according to claim 74, wherein tumor cells express CD25, or tumor immunity is suppressed by regulatory T cells.

**76.** The treatment method according to claim 74 or 75, wherein the tumor environment and/or the lymph node contains regulatory T cells.

**77.** The treatment method according to any one of claims 74 to 76, wherein the tumor is blood cancer, breast cancer, small-cell lung cancer, non-small cell lung cancer, head and neck cancer, brain tumor, glioma, eye tumor, esophageal cancer, stomach cancer, colorectal cancer, colon cancer, liver cancer, pancreatic cancer, renal cancer, kidney cancer,

urinary bladder cancer, adrenal cortex cancer, prostate cancer, uterine cervical cancer, endometrial cancer, ovary cancer, melanoma, or sarcoma.

**78.** A method for treating tumor, comprising administering a pharmaceutical composition comprising at least one member selected from the antibody or the antigen binding fragment of the antibody according to any one of claims 1 to 21 and 30 to 36, the antibody-drug conjugate according to any one of claims 37 to 68, a salt of the component, and a hydrate of the component or the salt, and at least one antitumor drug to an individual simultaneously, separately, or continuously.

**79.** A method for producing an antibody-drug conjugate, comprising the steps of: culturing the host cell according to claim 27 or 28; collecting the antibody or the antigen binding fragment of the antibody of interest from the cultures obtained in the preceding step; and reacting the antibody or the antigen binding fragment of the antibody obtained in the preceding step with a drug-linker intermediate compound.

**80.** A pharmaceutical composition in which the antibody-drug conjugate according to any one of claims 37 to 68 and an immune checkpoint inhibitor are administered in combination.

**81.** The pharmaceutical composition according to claim 80, wherein the antibody-drug conjugate and the immune checkpoint inhibitor are separately contained as active ingredients in different formulations and administered simultaneously or at different times.

**82.** The pharmaceutical composition according to claim 80, wherein the antibody-drug conjugate and the immune checkpoint inhibitor are contained as active ingredients in the same formulation.

**83.** The pharmaceutical composition according to any one of claims 80 to 82, wherein the immune checkpoint inhibitor is any one or more members selected from the group consisting of an anti-PD-1 antibody, an anti-PD-L1 antibody, and an anti-CTLA-4 antibody.

**84.** The pharmaceutical composition according to claim 83, wherein the immune checkpoint inhibitor is an anti-PD-1 antibody.

**85.** The pharmaceutical composition according to claim 84, wherein the anti-PD-1 antibody is any one or more members selected from the group consisting of nivolumab, pembrolizumab, sintilimab, spartalizumab, dostarlimab, serplulimab, tislelizumab, penpulimab, toripalimab, zimberelimab, camrelizumab, retifanlimab, cemiplimab, prolgolimab, geptanolimab, enlonstobart, QL-1604, pucotenlimab, and finotonlimab.

**86.** The pharmaceutical composition according to claim 84, wherein the anti-PD-1 antibody is nivolumab or pembrolizumab.

**87.** The pharmaceutical composition according to claim 83, wherein the immune checkpoint inhibitor is an anti-PD-L1 antibody.

**88.** The pharmaceutical composition according to claim 87, wherein the anti-PD-L1 antibody is any one or more members selected from the group consisting of atezolizumab, durvalumab, cosibelimab, adebrelimab, sugemalimab, avelumab, socazolimab, KL-A167, and envafolimab.

**89.** The pharmaceutical composition according to claim 87, wherein the anti-PD-L1 antibody is any one or more members selected from the group consisting of atezolizumab, durvalumab, and avelumab.

**90.** The pharmaceutical composition according to claim 83, wherein the immune checkpoint inhibitor is an anti-CTLA-4 antibody.

**91.** The pharmaceutical composition according to claim 90, wherein the anti-CTLA-4 antibody is any one or more members selected from the group consisting of ipilimumab, botensilimab, and tremelimumab.

**92.** The pharmaceutical composition according to claim 90, wherein the anti-CTLA-4 antibody is ipilimumab.

**93.** The pharmaceutical composition according to any one of claims 80 to 92 for the treatment of a cancer.

**94.** The pharmaceutical composition according to any one of claims 80 to 92 for the treatment of at least one cancer selected from the group consisting of blood cancer, breast cancer, small-cell lung cancer, non-small cell lung cancer, head and neck cancer, brain tumor, glioma, eye tumor, esophageal cancer, stomach cancer, colorectal cancer, colon cancer, liver cancer, pancreatic cancer, renal cancer, kidney cancer, urinary bladder cancer, adrenal cortex cancer, prostate cancer, uterine cervical cancer, endometrial cancer, ovary cancer, melanoma, and sarcoma.

**95.** The pharmaceutical composition according to any one of claims 80 to 92 for the treatment of a cancer that exhibits resistance to the immune checkpoint inhibitor.

Fig. 1

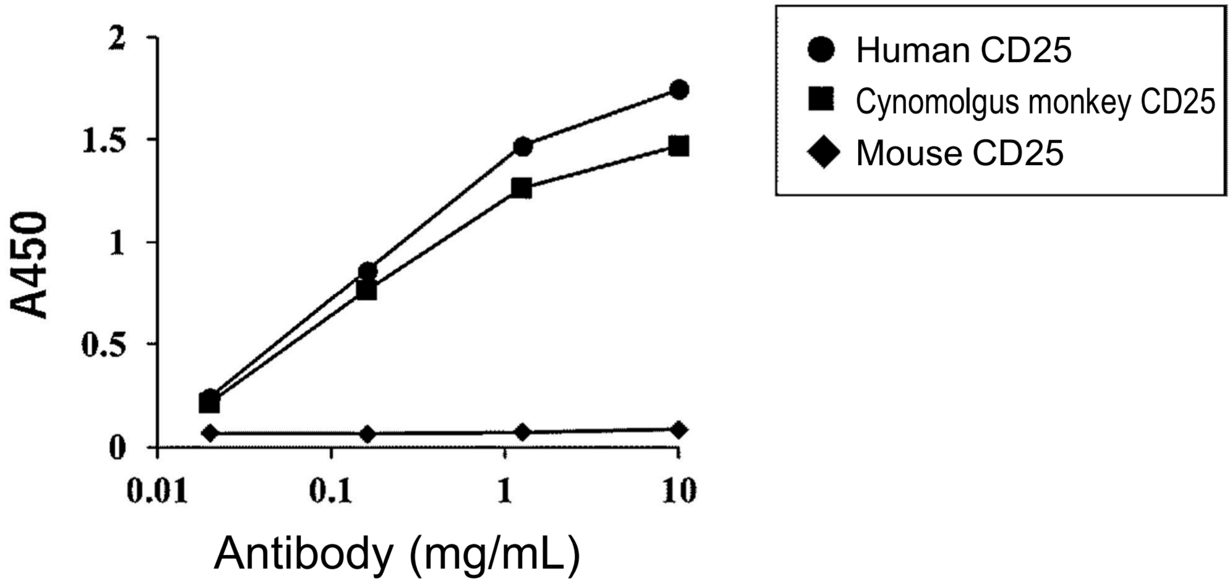
2
1.5
1
0.5
0
A450
0.01
0.1
1
10
Antibody (mg/mL)
Human CD25
Cynomolgus monkey CD25
Mouse CD25

Fig. 2

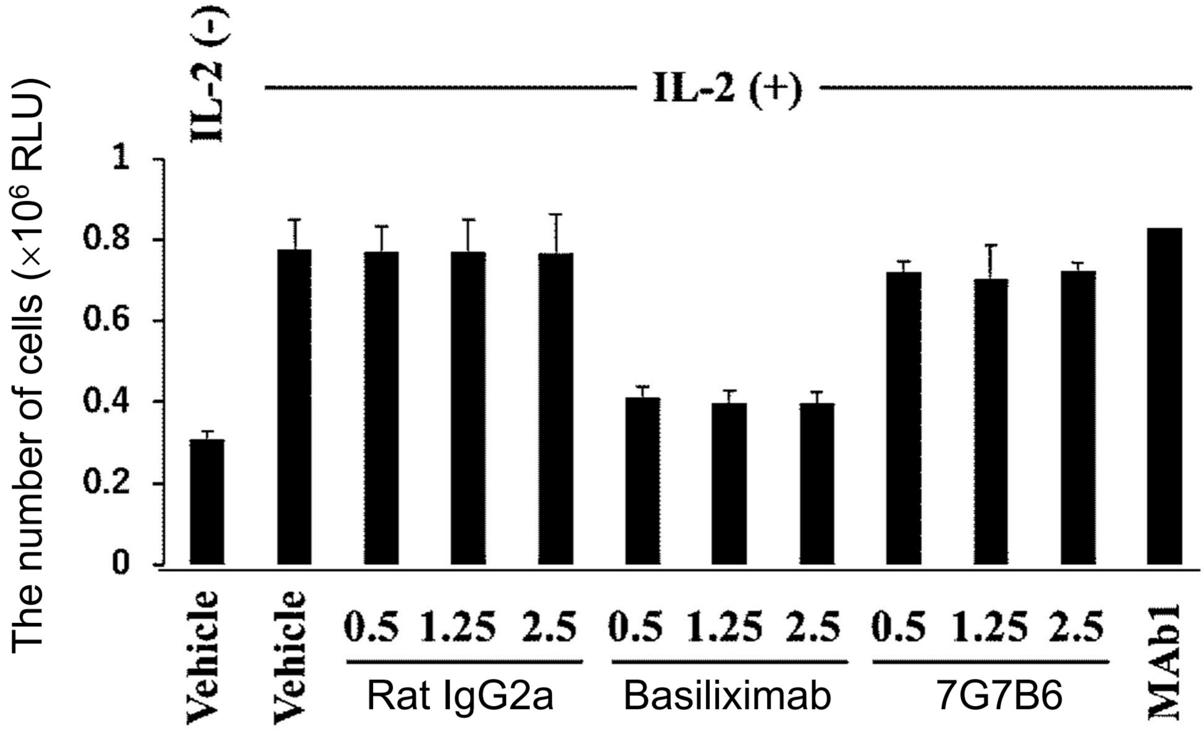
IL-2 (-)
IL-2 (+)
The number of cells (×10⁶ RLU)
1
0.8
0.6
0.4
0.2
0
Vehicle
Vehicle
0.5 1.25 2.5
Rat IgG2a
0.5 1.25 2.5
Basiliximab
0.5 1.25 2.5
7G7B6
MAb1

Fig. 3

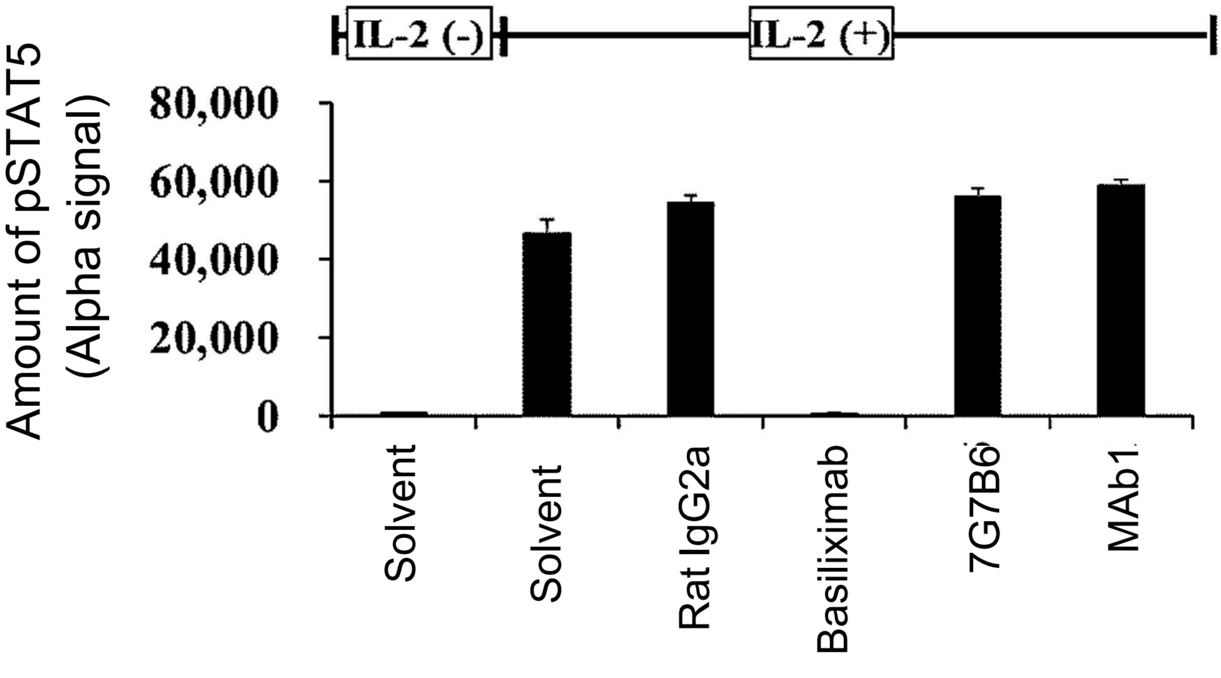
IL-2 (-)
IL-2 (+)
Amount of pSTAT5 (Alpha signal)
80,000
60,000
40,000
20,000
0
Solvent
Solvent
Rat IgG2a
Basiliximab
7G7B6
MAb1

Fig. 4

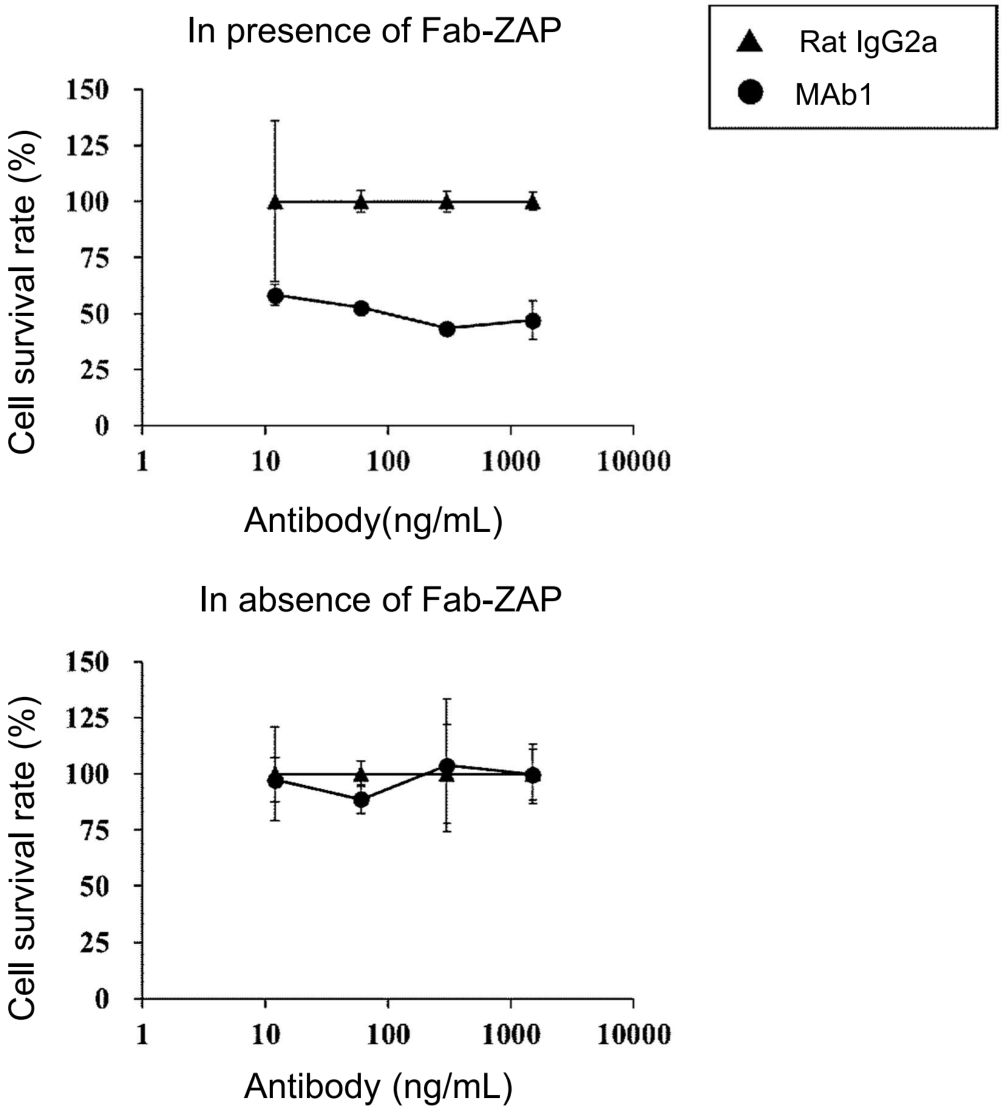
In presence of Fab-ZAP
Rat IgG2a
MAb1
Cell survival rate (%)
150
125
100
75
50
25
0
1
10
100
1000
10000
Antibody(ng/mL)
In absence of Fab-ZAP
Cell survival rate (%)
150
125
100
75
50
25
0
1
10
100
1000
10000
Antibody (ng/mL)

Fig. 5

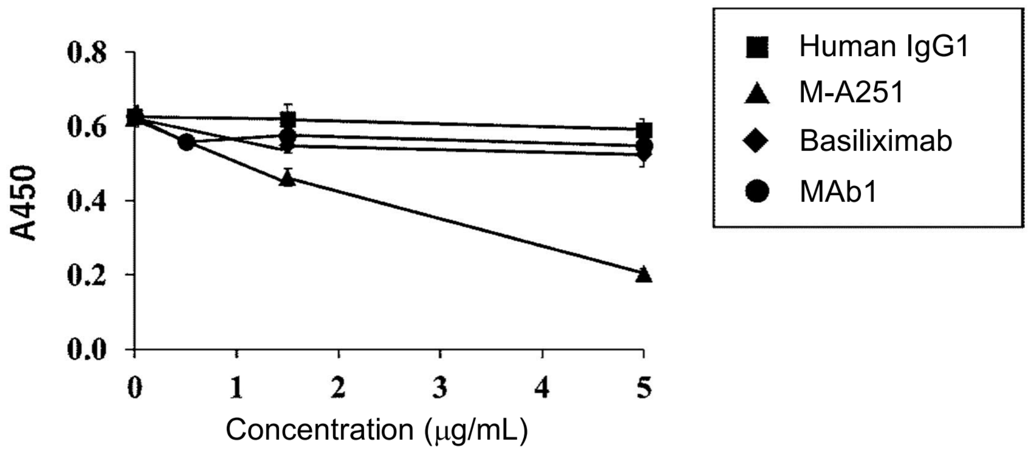
Human IgG1
M-A251
Basiliximab
MAb1
A450
0.8
0.6
0.4
0.2
0.0
0
1
2
3
4
5
Concentration (μg/mL)

Fig. 6

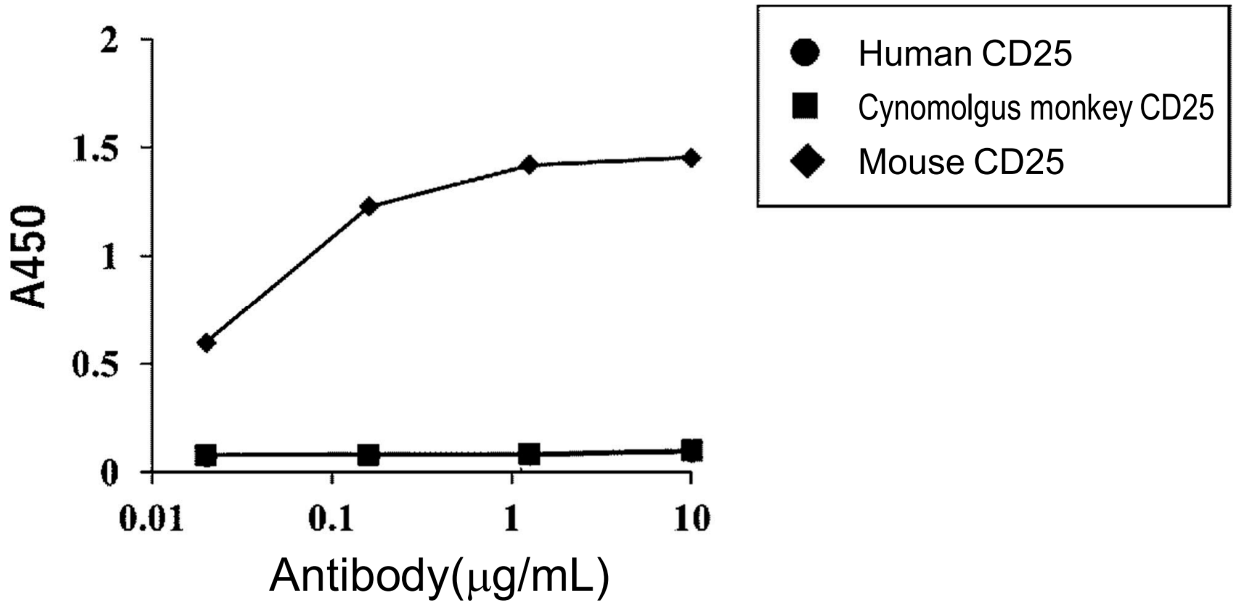
2
1.5
1
0.5
0
A450
0.01
0.1
1
10
Antibody(μg/mL)
Human CD25
Cynomolgus monkey CD25
Mouse CD25

Fig. 7

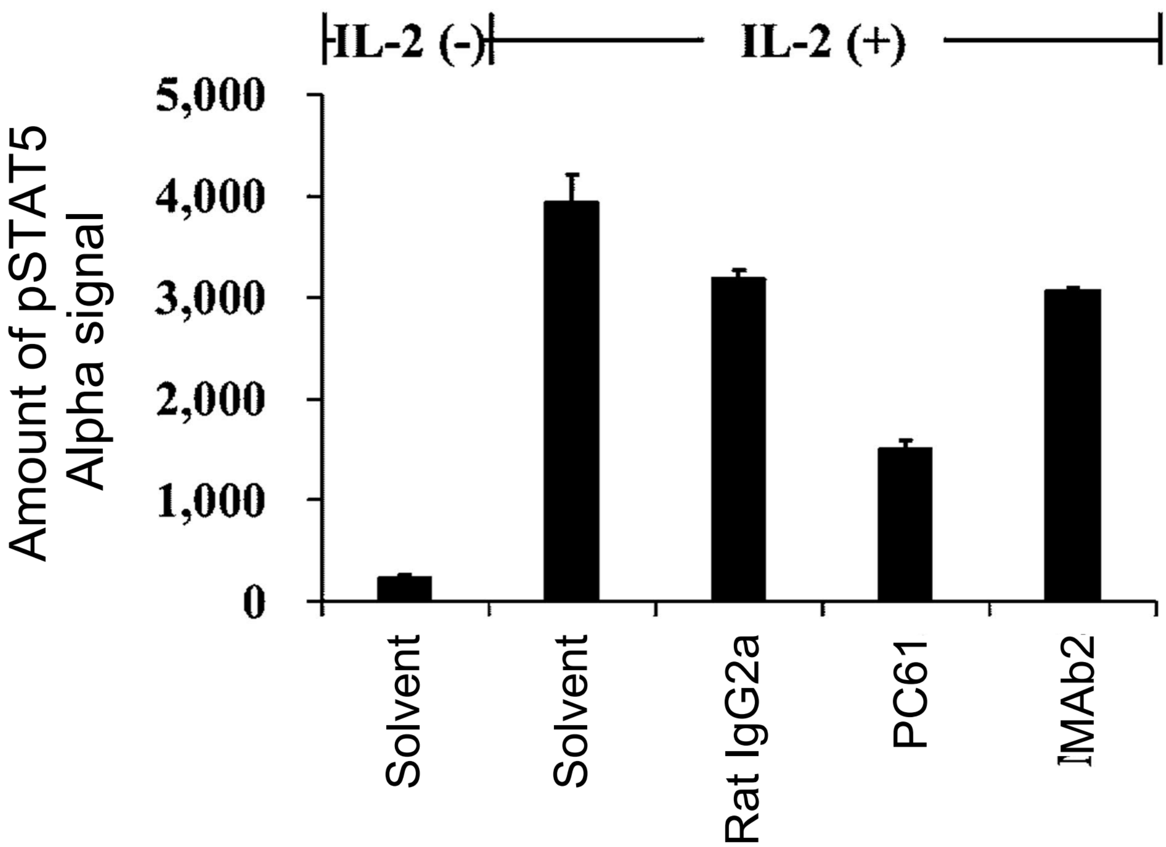
IL-2 (-)
IL-2 (+)
5,000
4,000
3,000
2,000
1,000
0
Amount of pSTAT5
Alpha signal
Solvent
Solvent
Rat IgG2a
PC61
IMAb2

Fig. 8

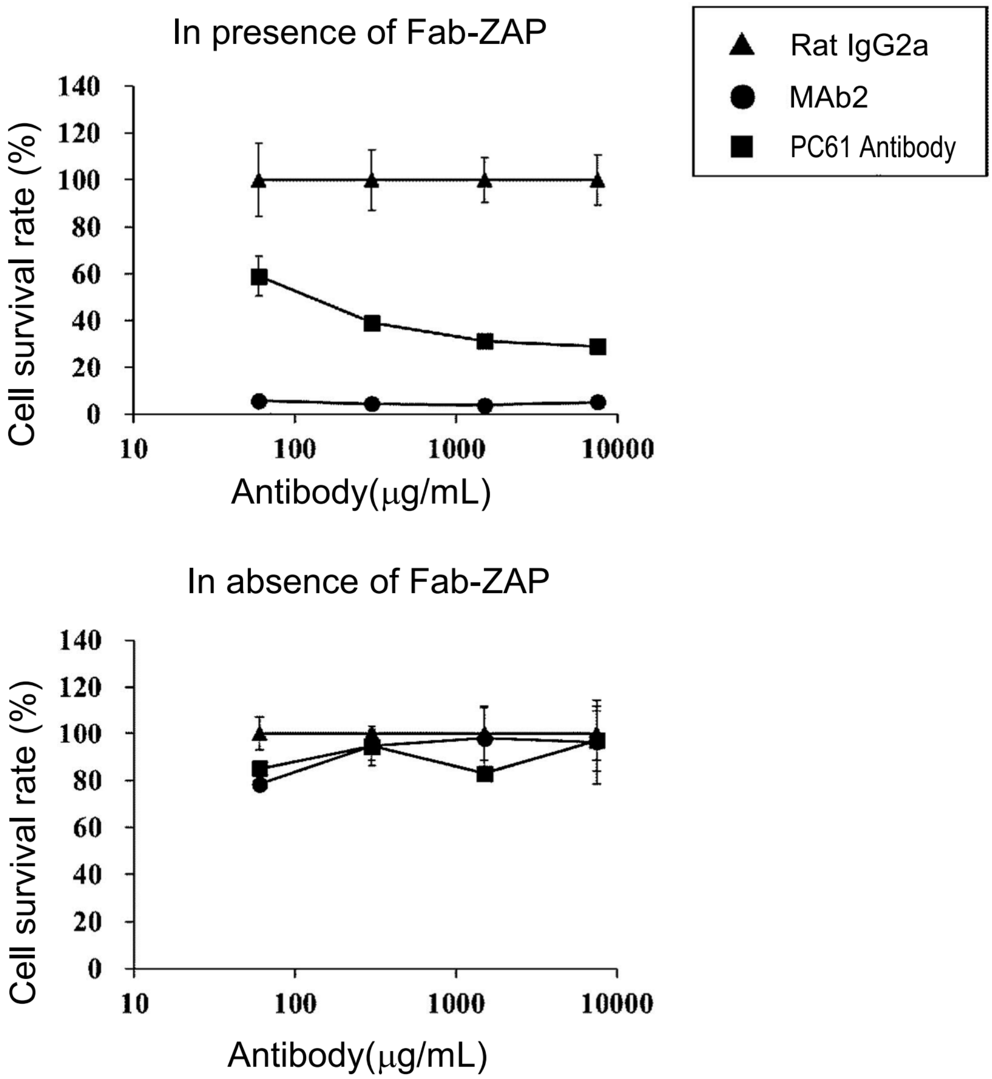
In presence of Fab-ZAP
Rat IgG2a
MAb2
PC61 Antibody
Cell survival rate (%)
140
120
100
80
60
40
20
0
10
100
1000
10000
Antibody(μg/mL)
In absence of Fab-ZAP
Cell survival rate (%)
140
120
100
80
60
40
20
0
10
100
1000
10000
Antibody(μg/mL)

Fig. 9

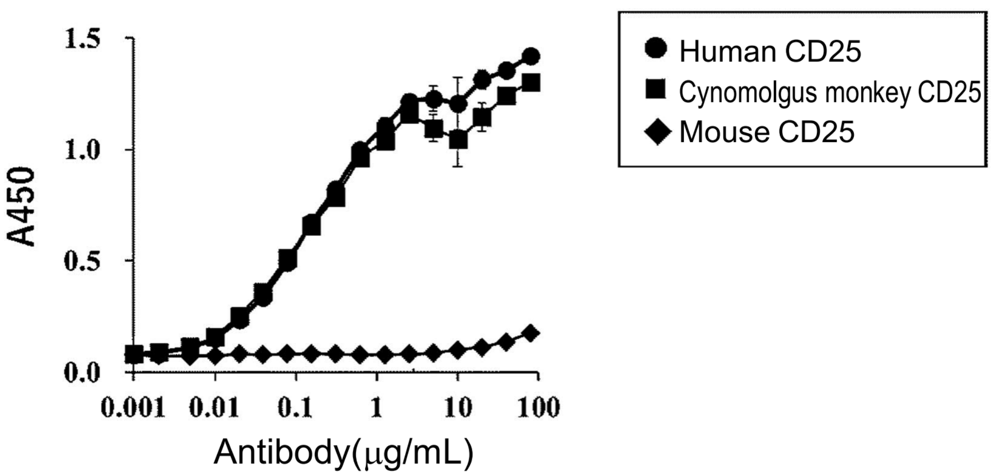
Human CD25
Cynomolgus monkey CD25
Mouse CD25
A450
1.5
1.0
0.5
0.0
0.001
0.01
0.1
1
10
100
Antibody(μg/mL)

Fig. 10

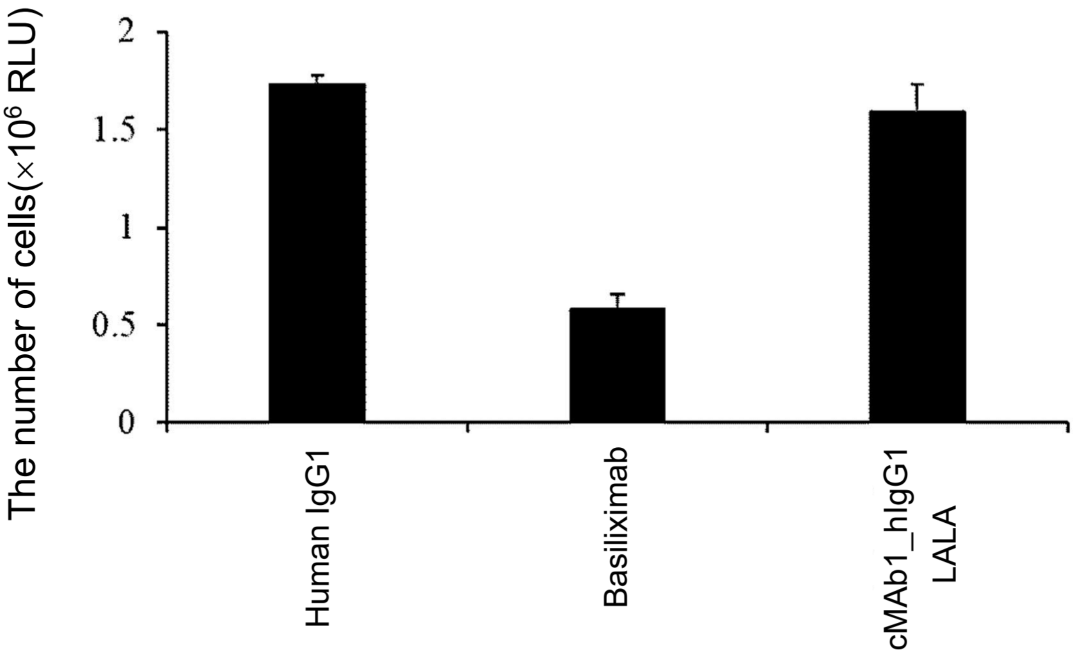
The number of cells(×10^6 RLU)
2
1.5
1
0.5
0
Human IgG1
Basiliximab
cMAb1_hIgG1 LALA

Fig. 11

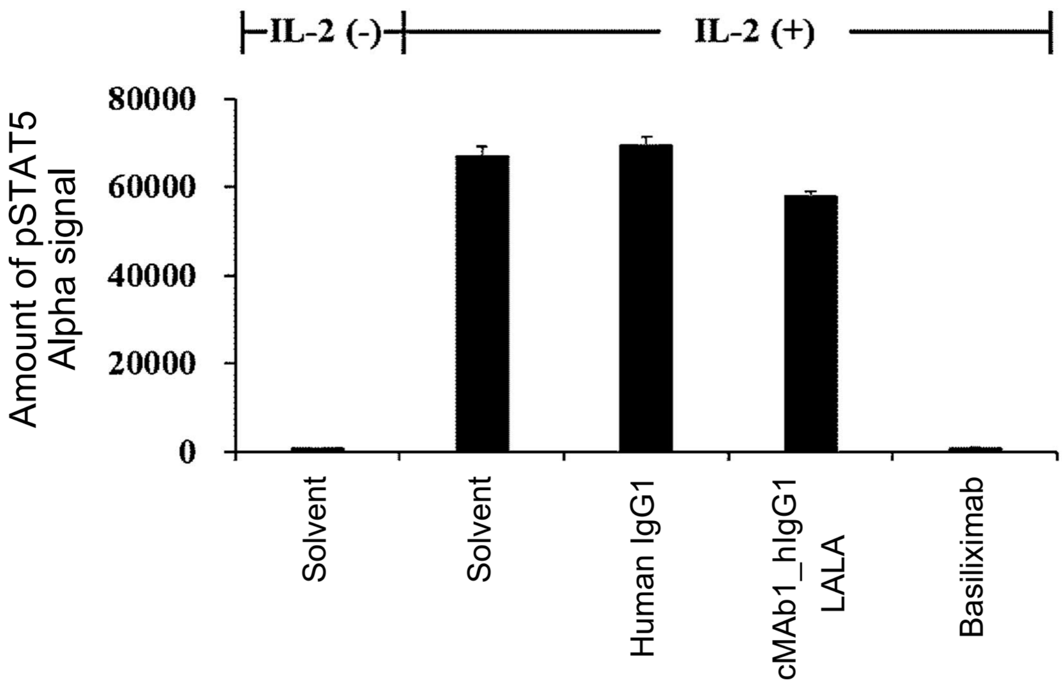
IL-2 (-)
IL-2 (+)
Amount of pSTAT5 Alpha signal
80000
60000
40000
20000
0
Solvent
Solvent
Human IgG1
cMAb1_hIgG1 LALA
Basiliximab

Fig. 12

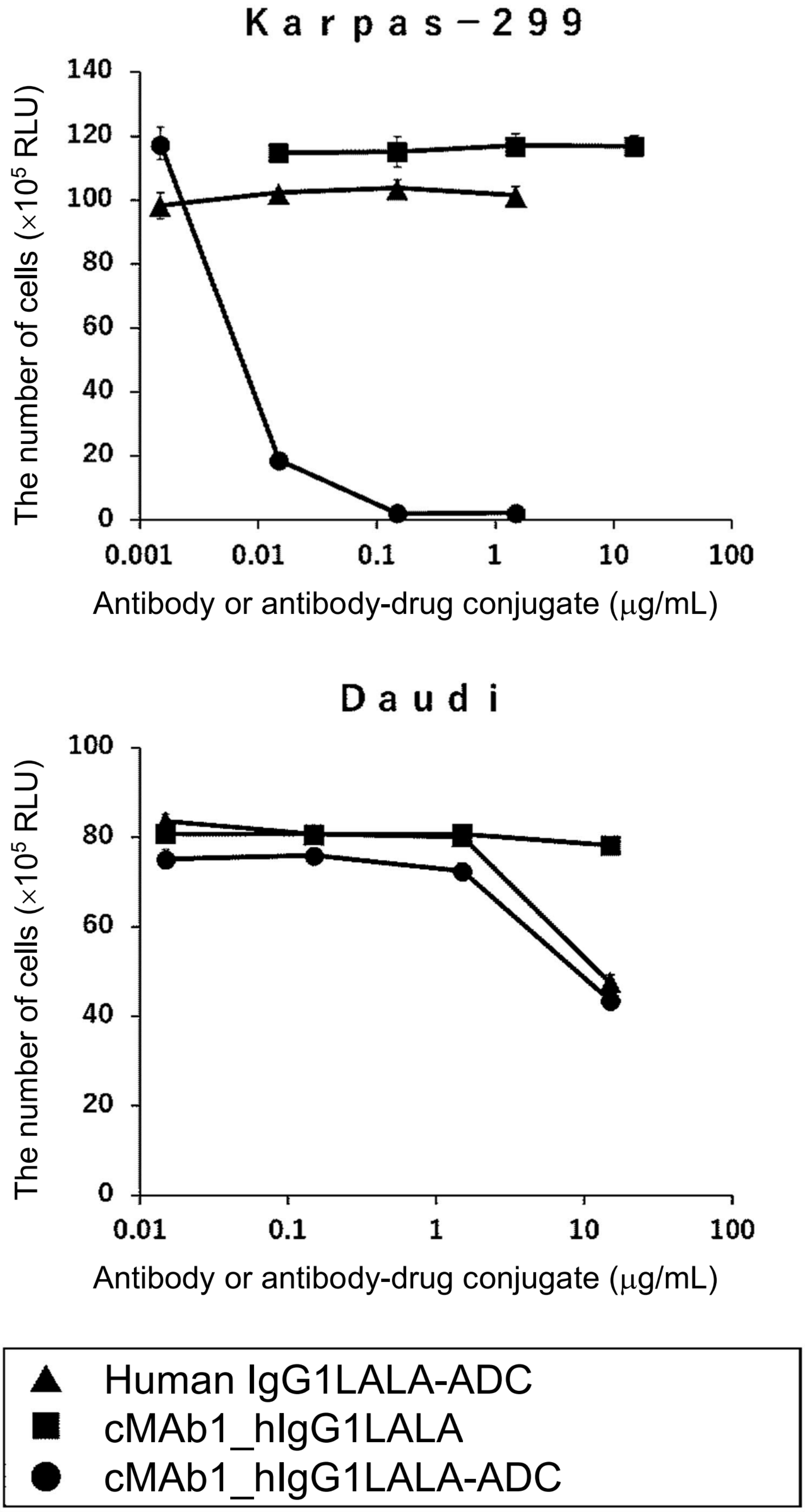
Karpas−299
The number of cells (×10^5 RLU)
140
120
100
80
60
40
20
0
0.001
0.01
0.1
1
10
100
Antibody or antibody-drug conjugate (μg/mL)
Daudi
The number of cells (×10^5 RLU)
100
80
60
40
20
0
0.01
0.1
1
10
100
Antibody or antibody-drug conjugate (μg/mL)
Human IgG1LALA-ADC
cMAb1_hIgG1LALA
cMAb1_hIgG1LALA-ADC

Fig. 13

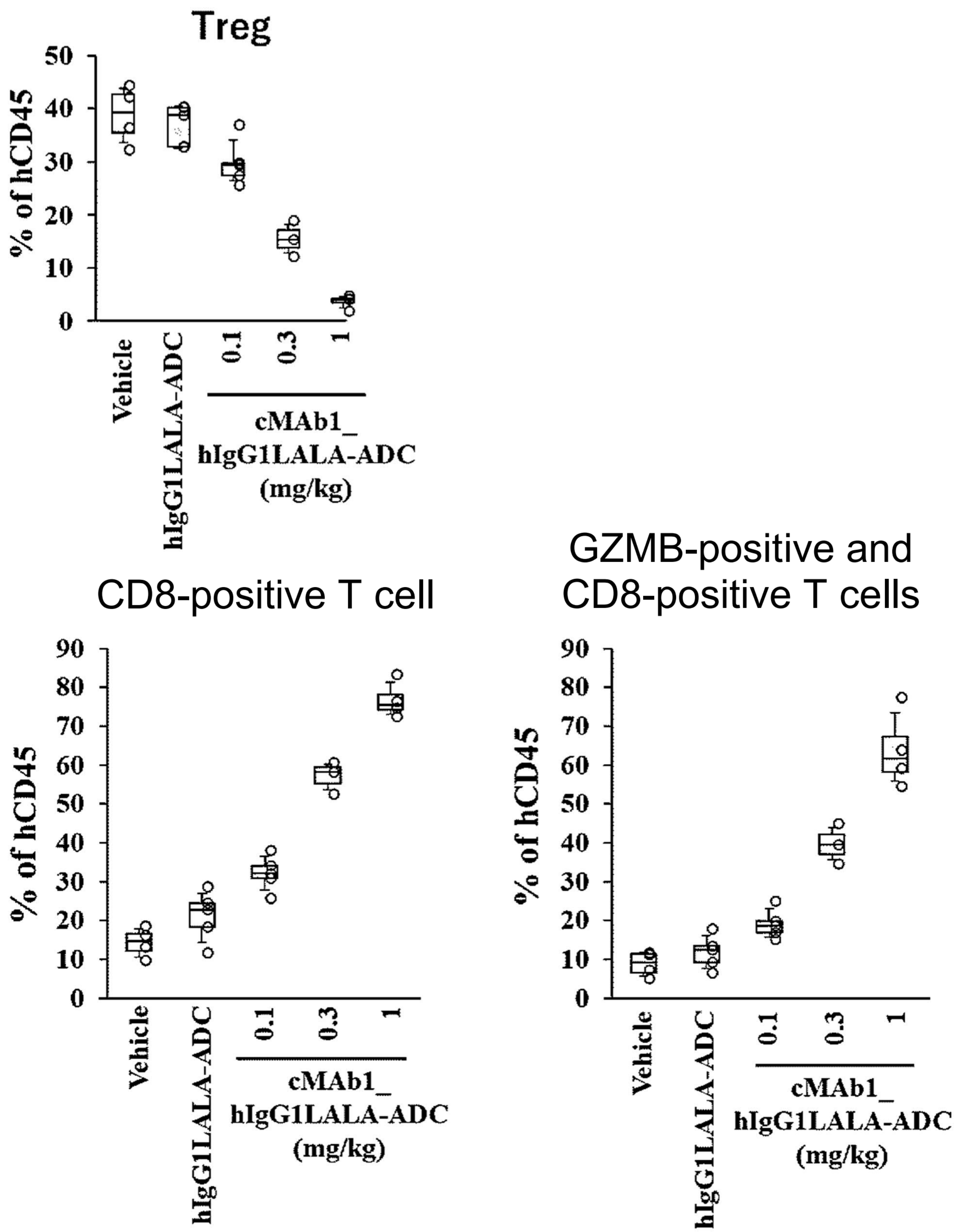
Treg
% of hCD45
50
40
30
20
10
0
Vehicle
hIgG1LALA-ADC
0.1
0.3
1
cMAb1_
hIgG1LALA-ADC
(mg/kg)
CD8-positive T cell
GZMB-positive and
CD8-positive T cells
% of hCD45
90
80
70
60
50
40
30
20
10
0
Vehicle
hIgG1LALA-ADC
0.1
0.3
1
cMAb1_
hIgG1LALA-ADC
(mg/kg)

Fig. 14

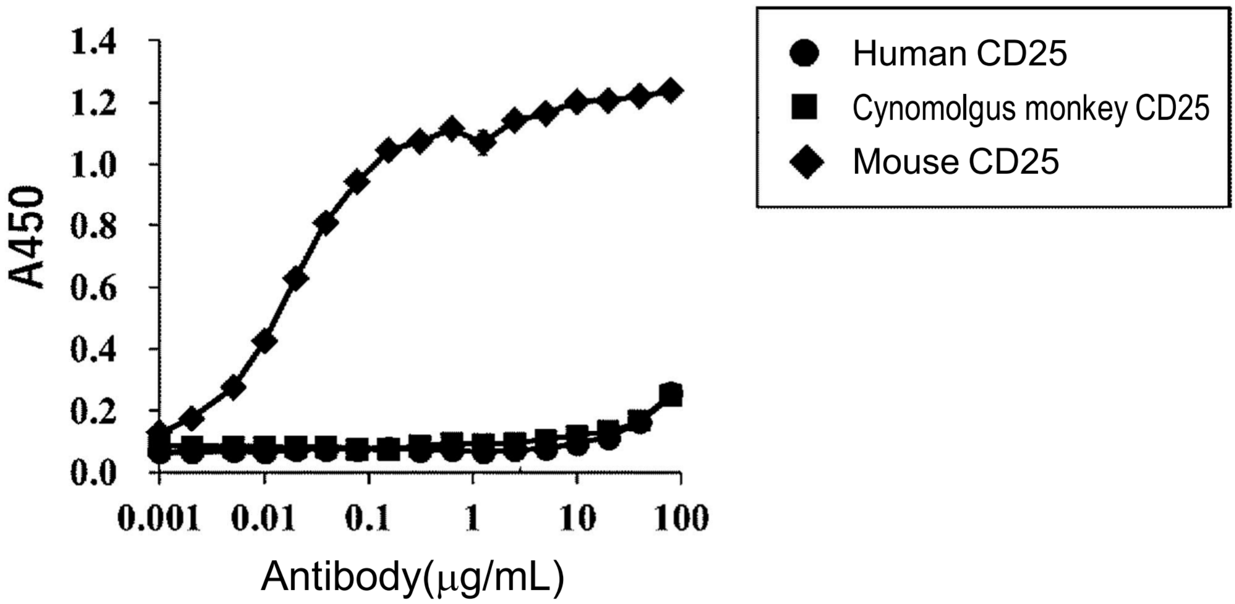
1.4
1.2
1.0
0.8
0.6
0.4
0.2
0.0
A450
0.001
0.01
0.1
1
10
100
Antibody(μg/mL)
Human CD25
Cynomolgus monkey CD25
Mouse CD25

Fig. 15

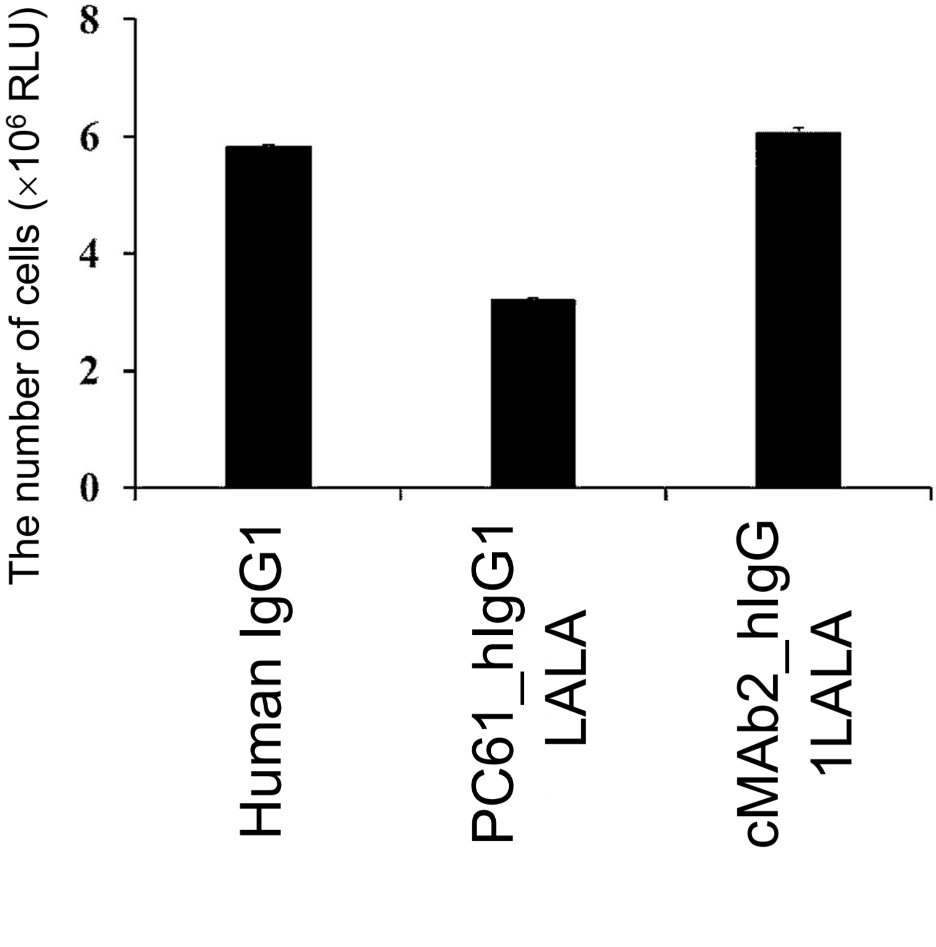
8
6
4
2
0
The number of cells (×10^6 RLU)
Human IgG1
PC61_hIgG1 LALA
cMAb2_hIgG 1LALA

Fig. 16

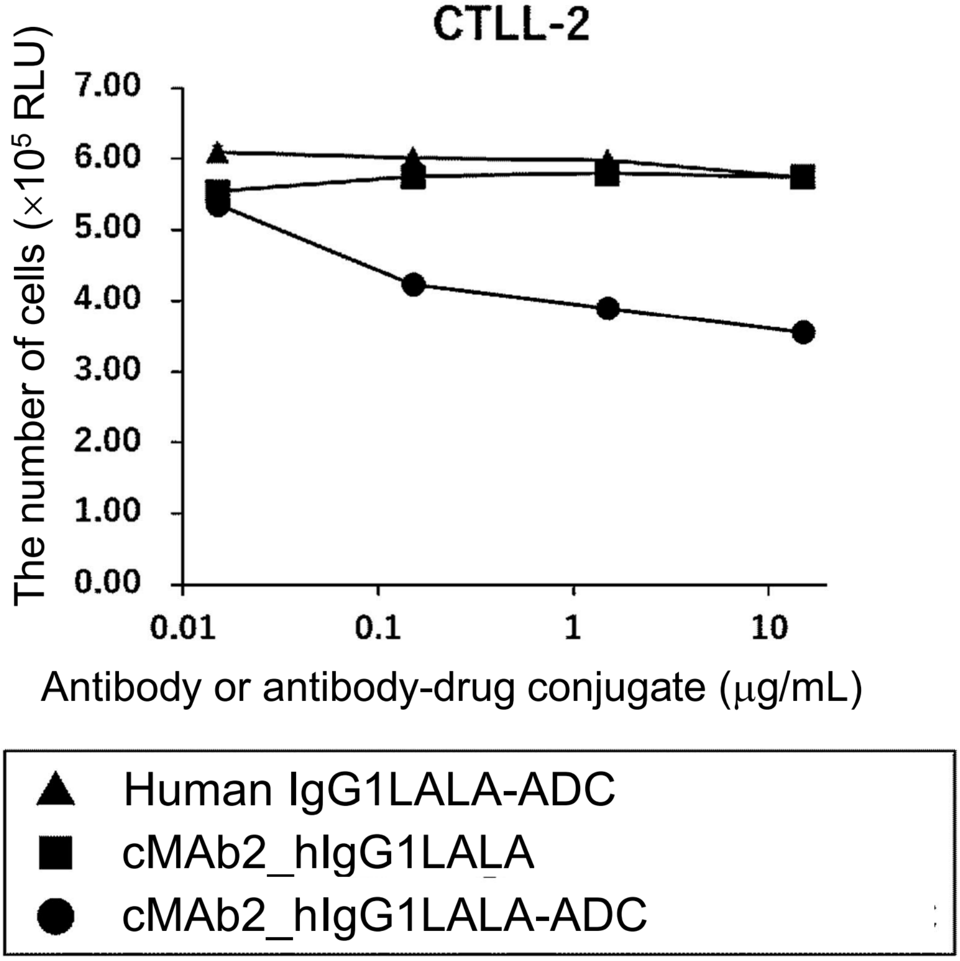
CTLL-2
The number of cells (×10^5 RLU)
7.00
6.00
5.00
4.00
3.00
2.00
1.00
0.00
0.01
0.1
1
10
Antibody or antibody-drug conjugate (μg/mL)
Human IgG1LALA-ADC
cMAb2_hIgG1LALA
cMAb2_hIgG1LALA-ADC

Fig. 17

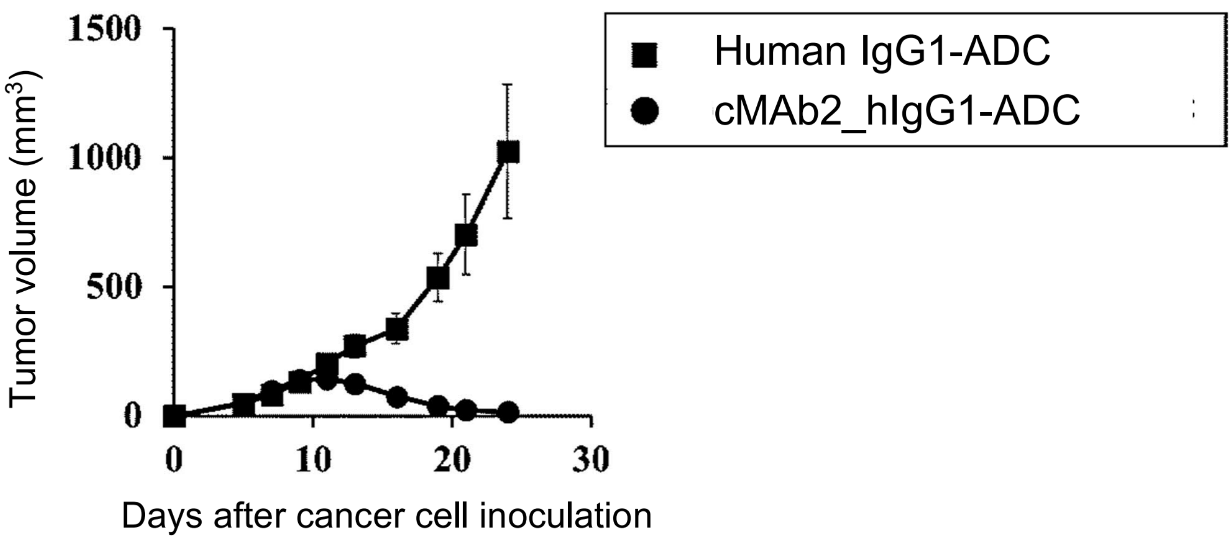
Tumor volume (mm3)
1500
1000
500
0
0
10
20
30
Days after cancer cell inoculation
Human IgG1-ADC
cMAb2_hIgG1-ADC

Fig. 18

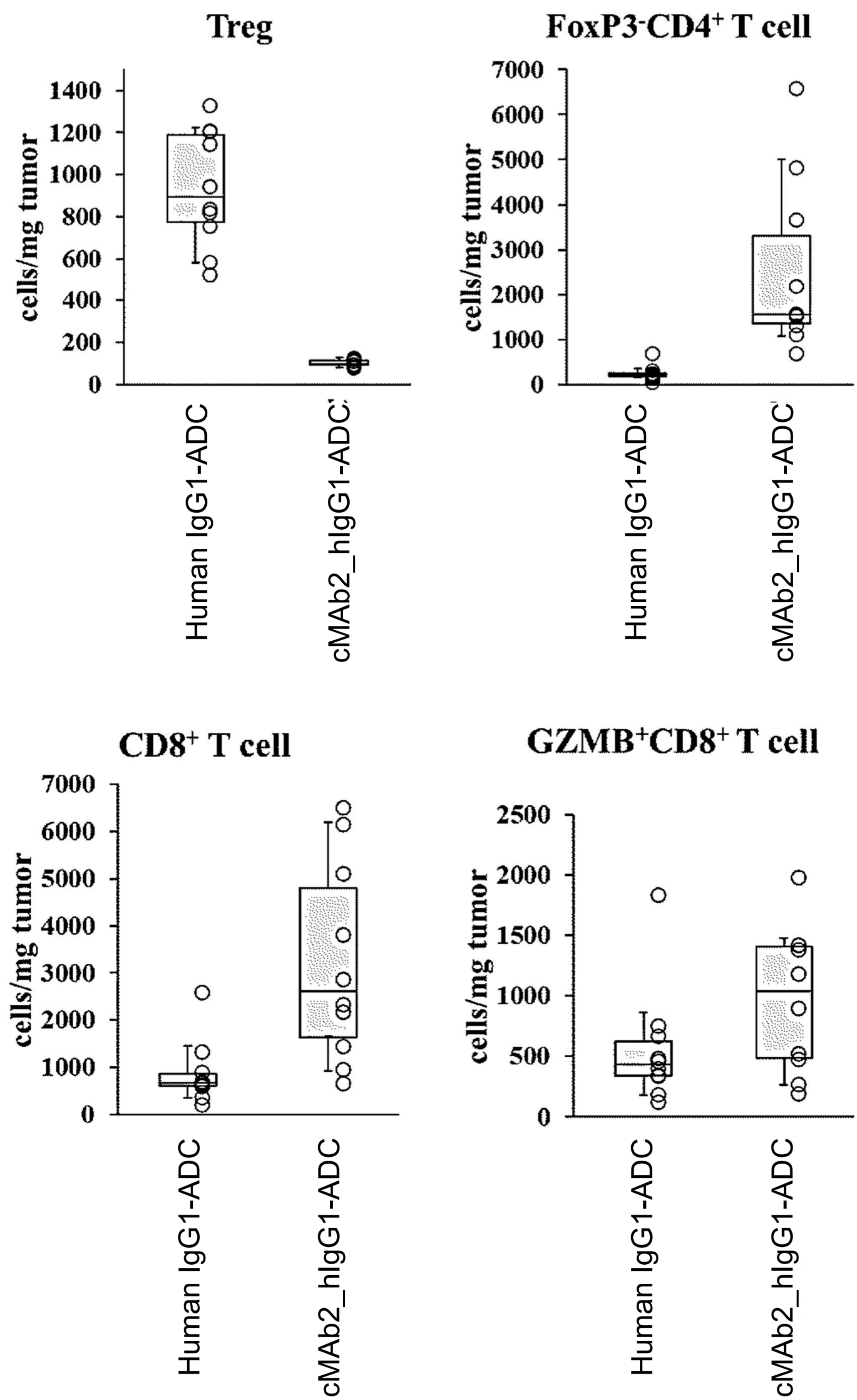
Treg
cells/mg tumor
1400
1200
1000
800
600
400
200
0
Human IgG1-ADC
cMAb2_hIgG1-ADC
FoxP3⁻CD4⁺ T cell
7000
6000
5000
4000
3000
2000
1000
0
CD8⁺ T cell
GZMB⁺CD8⁺ T cell
2500
2000
1500
1000
500

Fig. 19

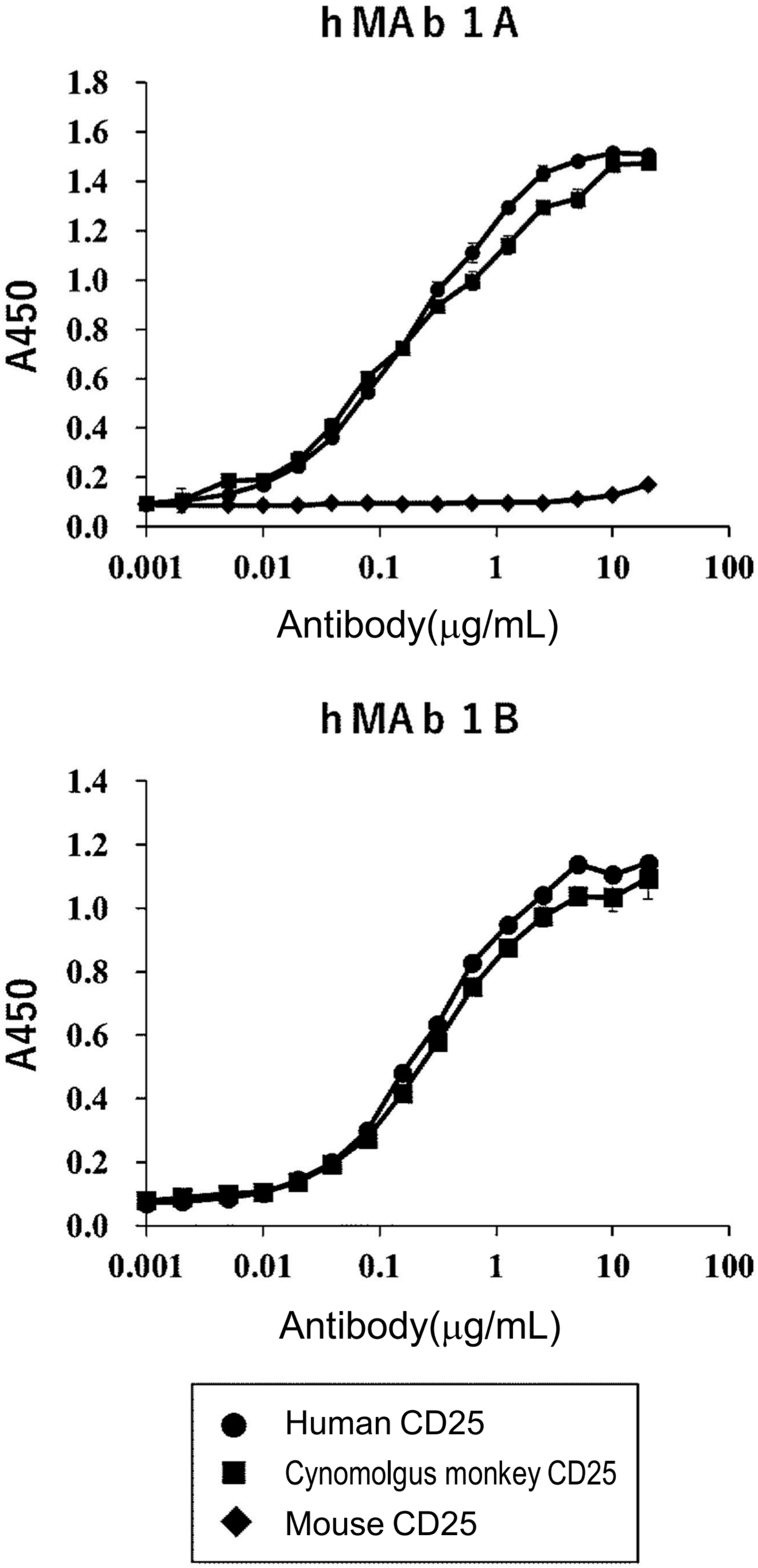
hMAb 1A
1.8
1.6
1.4
1.2
1.0
0.8
0.6
0.4
0.2
0.0
A450
0.001
0.01
0.1
1
10
100
Antibody(μg/mL)
hMAb 1B
1.4
1.2
1.0
0.8
0.6
0.4
0.2
0.0
A450
0.001
0.01
0.1
1
10
100
Antibody(μg/mL)
Human CD25
Cynomolgus monkey CD25
Mouse CD25

Fig. 20

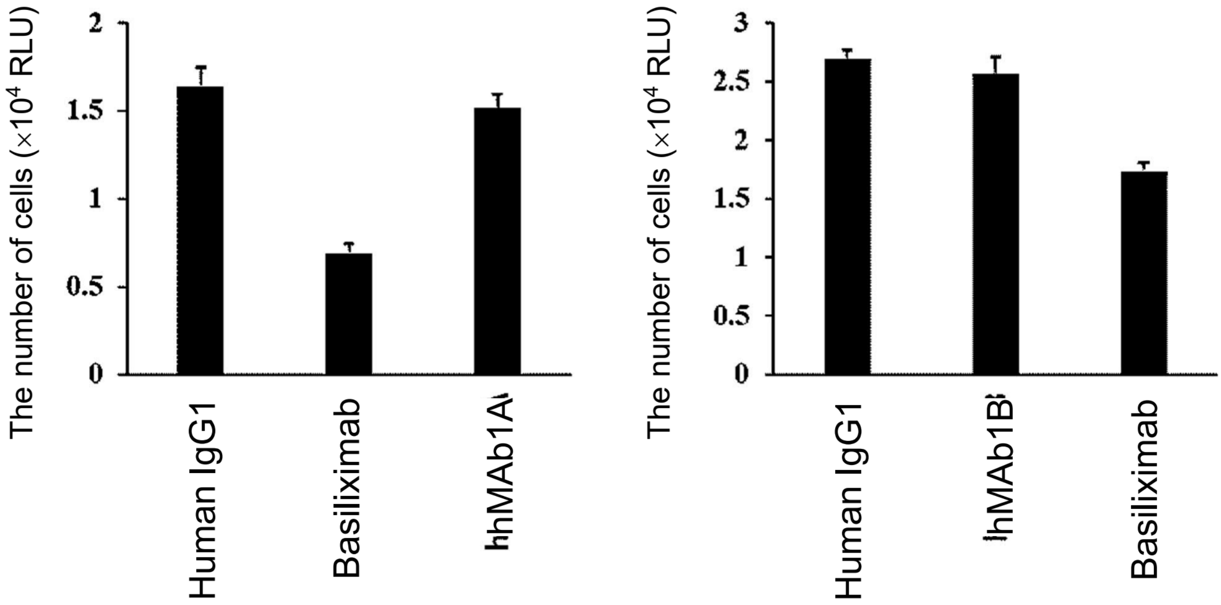
The number of cells (×10⁴ RLU)
2
1.5
1
0.5
0
Human IgG1
Basiliximab
hMAb1A
The number of cells (×10⁴ RLU)
3
2.5
2
1.5
1
0.5
0
Human IgG1
hMAb1B
Basiliximab

Fig. 21

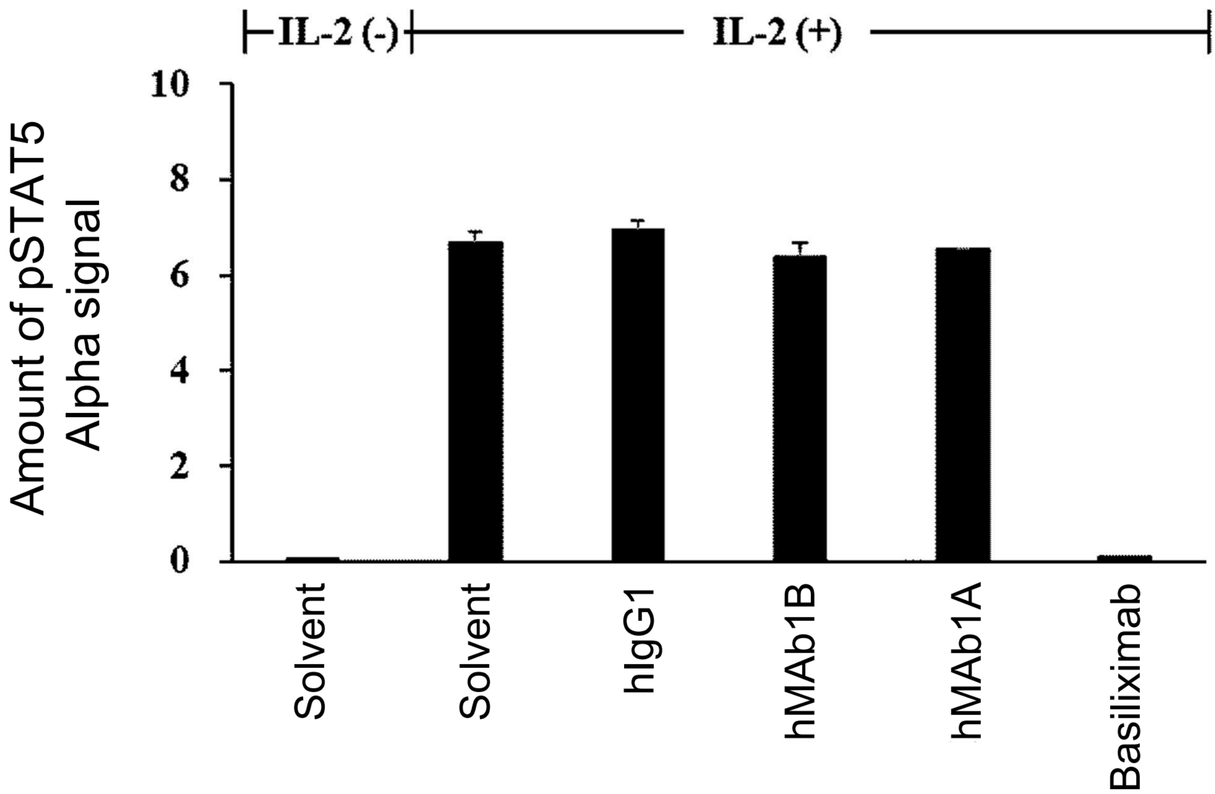
IL-2 (-)
IL-2 (+)
Amount of pSTAT5 Alpha signal
10
8
6
4
2
0
Solvent
Solvent
hIgG1
hMAb1B
hMAb1A
Basiliximab

Fig. 22-1

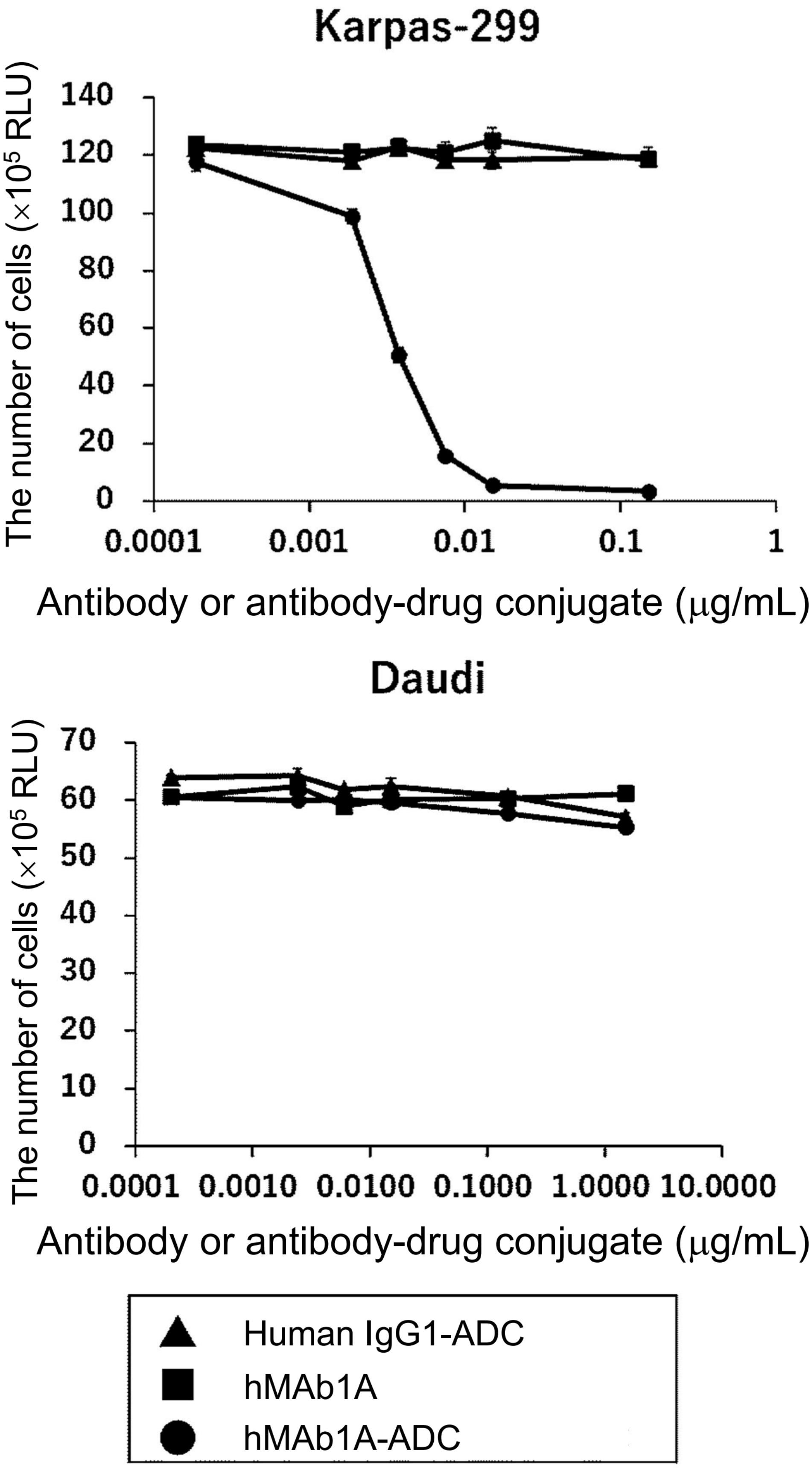
Karpas-299
The number of cells (×10⁵ RLU)
140
120
100
80
60
40
20
0
0.0001
0.001
0.01
0.1
1
Antibody or antibody-drug conjugate (μg/mL)
Daudi
The number of cells (×10⁵ RLU)
70
60
50
40
30
20
10
0
0.0001
0.0010
0.0100
0.1000
1.0000
10.0000
Antibody or antibody-drug conjugate (μg/mL)
Human IgG1-ADC
hMAb1A
hMAb1A-ADC

Fig. 22-2

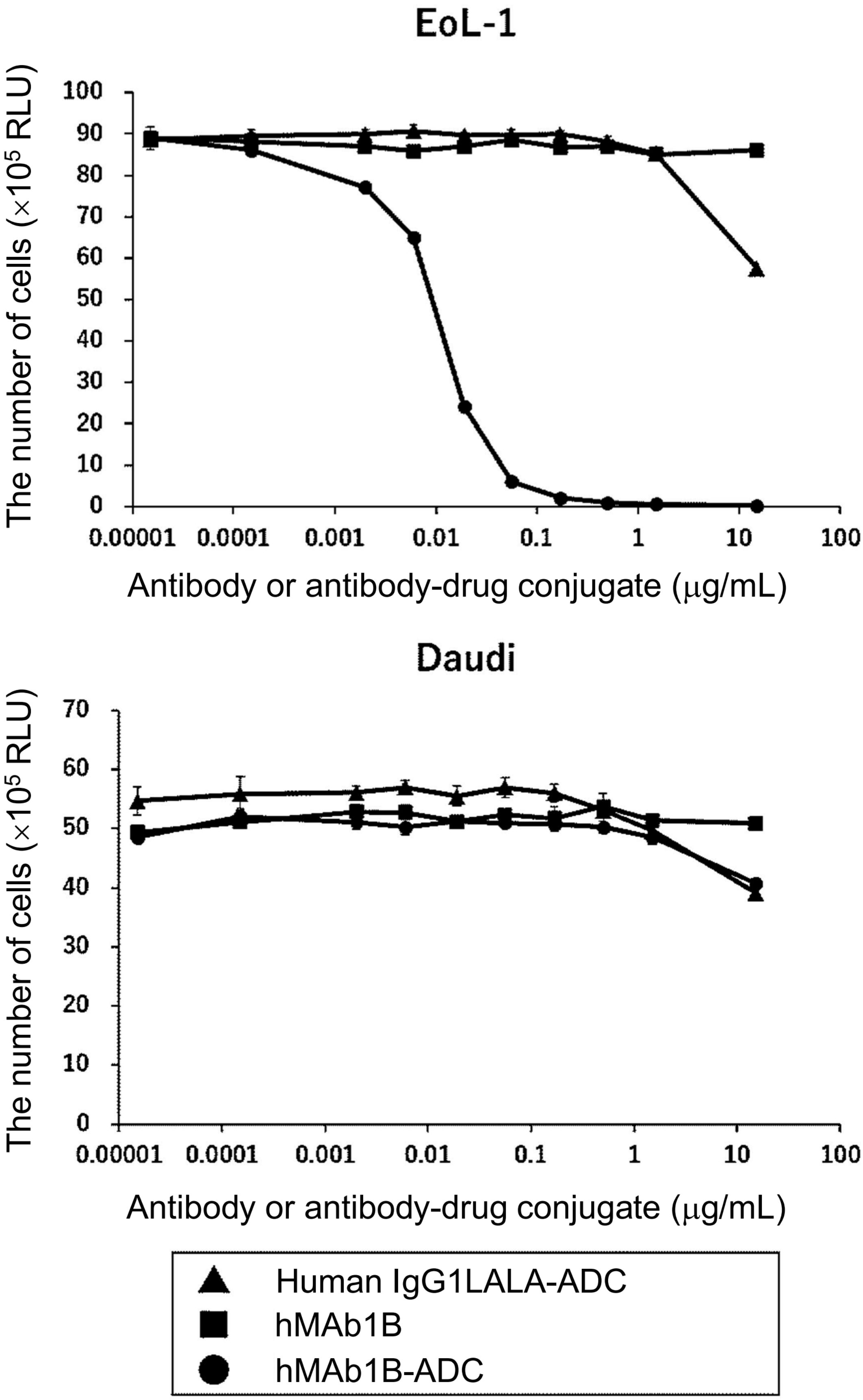
EoL-1
The number of cells (×10^5 RLU)
100
90
80
70
60
50
40
30
20
10
0
0.00001
0.0001
0.001
0.01
0.1
1
10
100
Antibody or antibody-drug conjugate (μg/mL)
Daudi
The number of cells (×10^5 RLU)
70
60
50
40
30
20
10
0
0.00001
0.0001
0.001
0.01
0.1
1
10
100
Antibody or antibody-drug conjugate (μg/mL)
Human IgG1LALA-ADC
hMAb1B
hMAb1B-ADC

Fig. 23

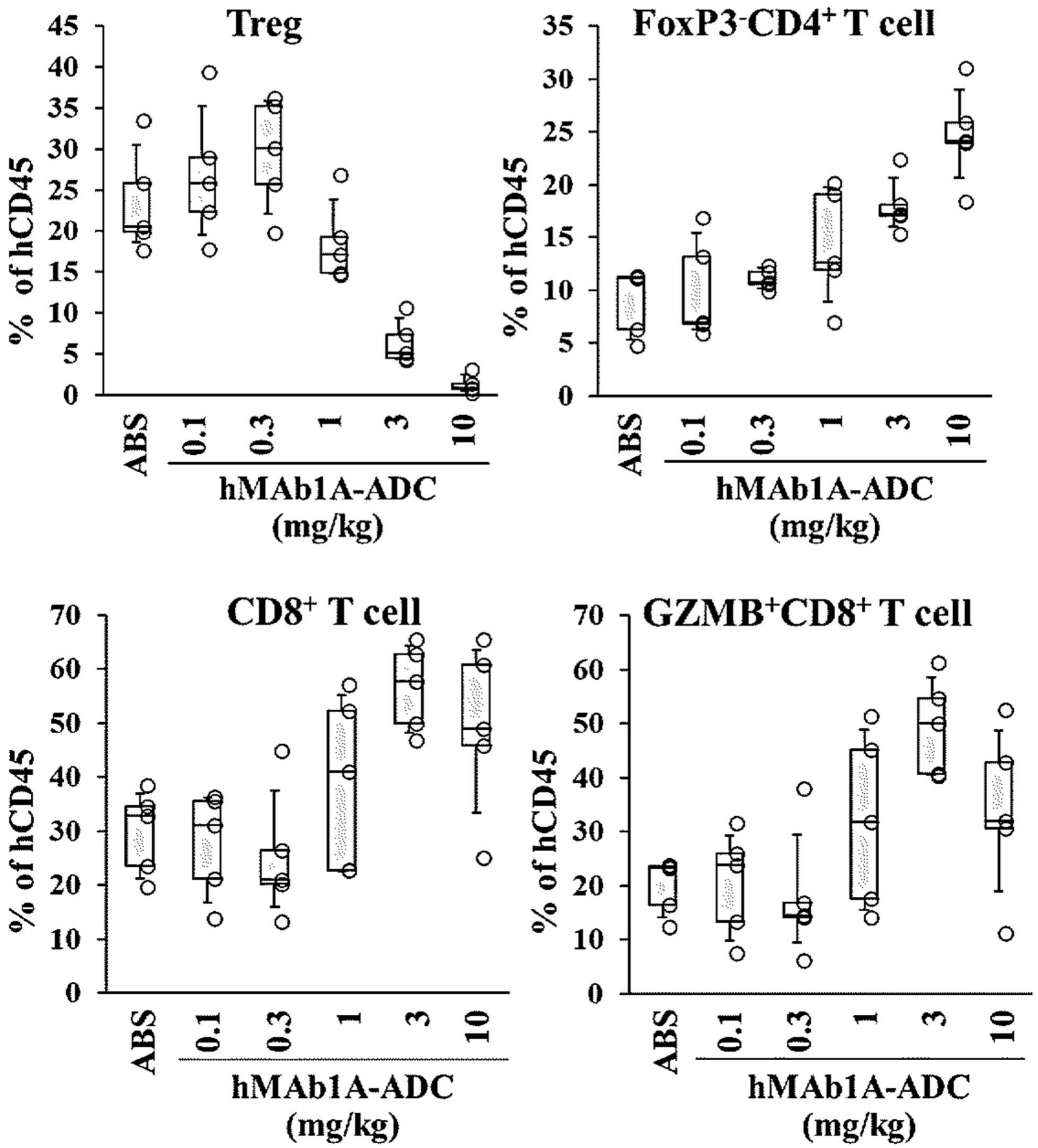
Treg
% of hCD45
45
40
35
30
25
20
15
10
5
0
ABS
0.1
0.3
1
3
10
hMAb1A-ADC
(mg/kg)
FoxP3-CD4+ T cell
% of hCD45
35
30
25
20
15
10
5
0
ABS
0.1
0.3
1
3
10
hMAb1A-ADC
(mg/kg)
CD8+ T cell
% of hCD45
70
60
50
40
30
20
10
0
ABS
0.1
0.3
1
3
10
hMAb1A-ADC
(mg/kg)
GZMB+CD8+ T cell
% of hCD45
70
60
50
40
30
20
10
0
ABS
0.1
0.3
1
3
10
hMAb1A-ADC
(mg/kg)

Fig. 24

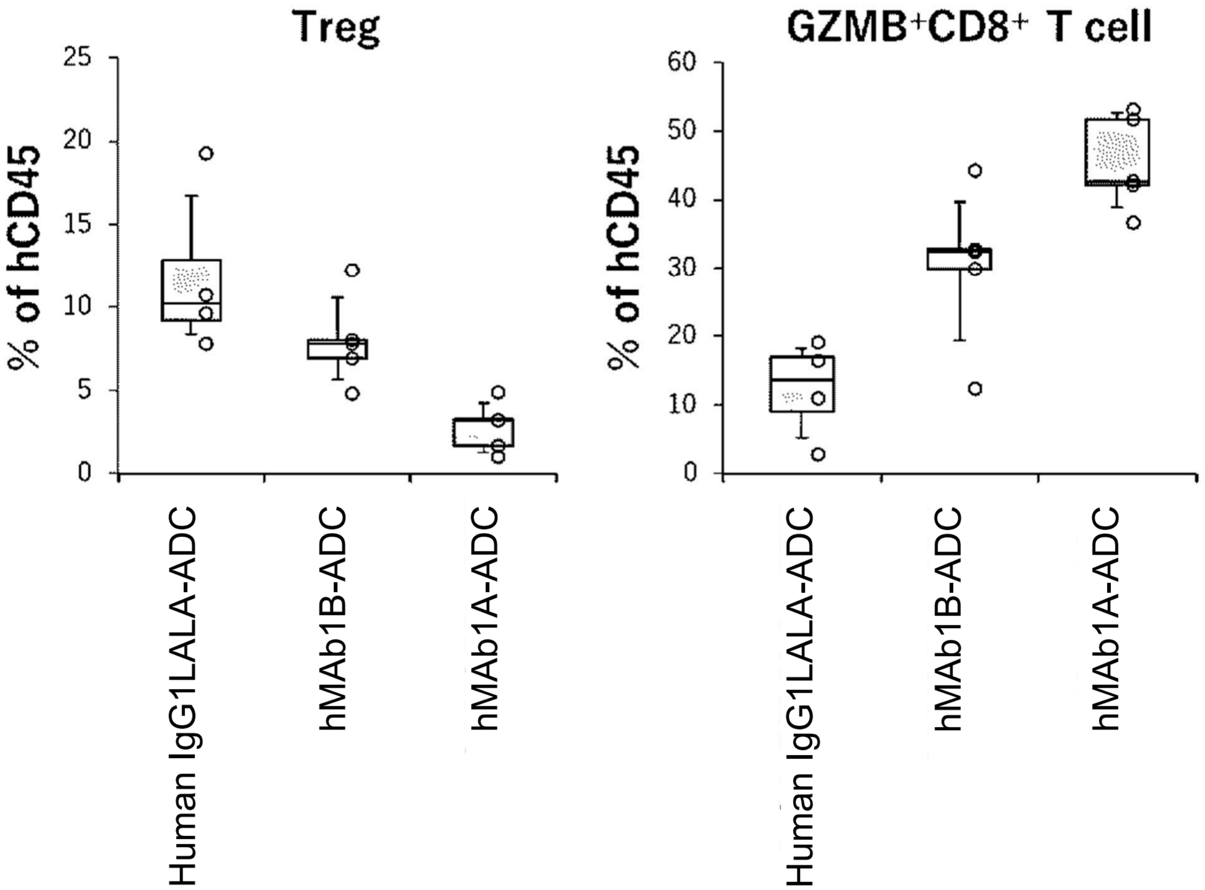
Treg
25
20
15
10
5
0
% of hCD45
Human IgG1LALA-ADC
hMAb1B-ADC
hMAb1A-ADC
GZMB+CD8+ T cell
60
50
40
30
20
10
0
% of hCD45
Human IgG1LALA-ADC
hMAb1B-ADC
hMAb1A-ADC

Fig. 25

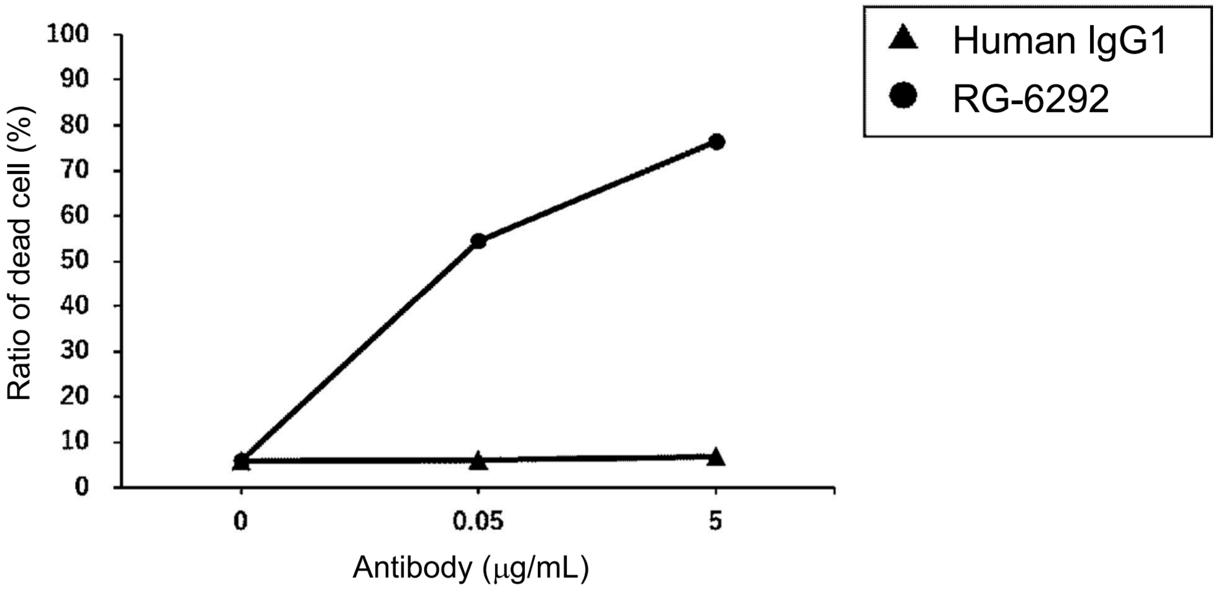
Human IgG1
RG-6292
Ratio of dead cell (%)
100
90
80
70
60
50
40
30
20
10
0
0
0.05
5
Antibody (μg/mL)

Fig. 26

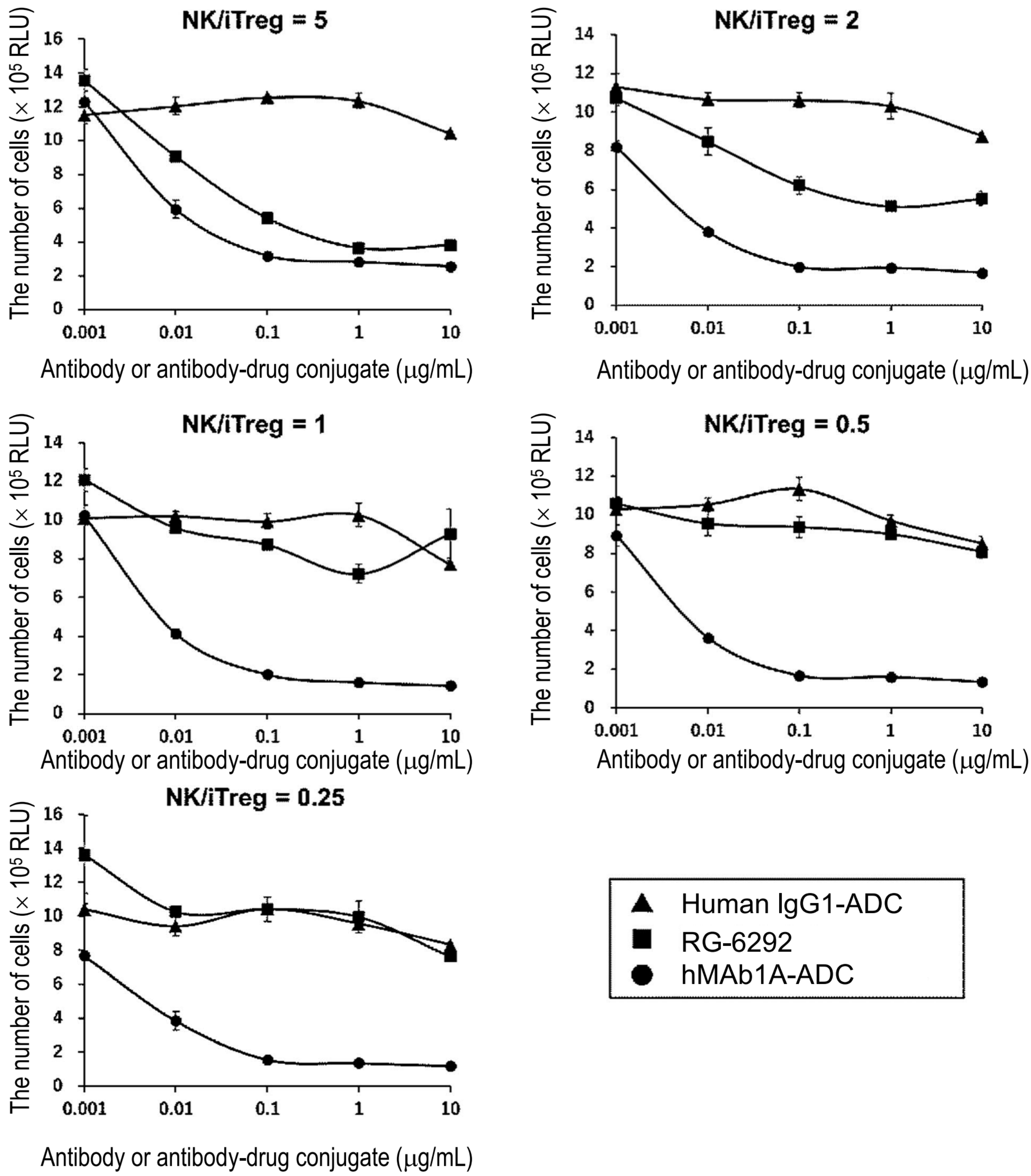
NK/iTreg = 5
NK/iTreg = 2
NK/iTreg = 1
NK/iTreg = 0.5
NK/iTreg = 0.25
The number of cells (× 10^5 RLU)
Antibody or antibody-drug conjugate (μg/mL)
0.001
0.01
0.1
1
10
Human IgG1-ADC
RG-6292
hMAb1A-ADC

Fig. 27

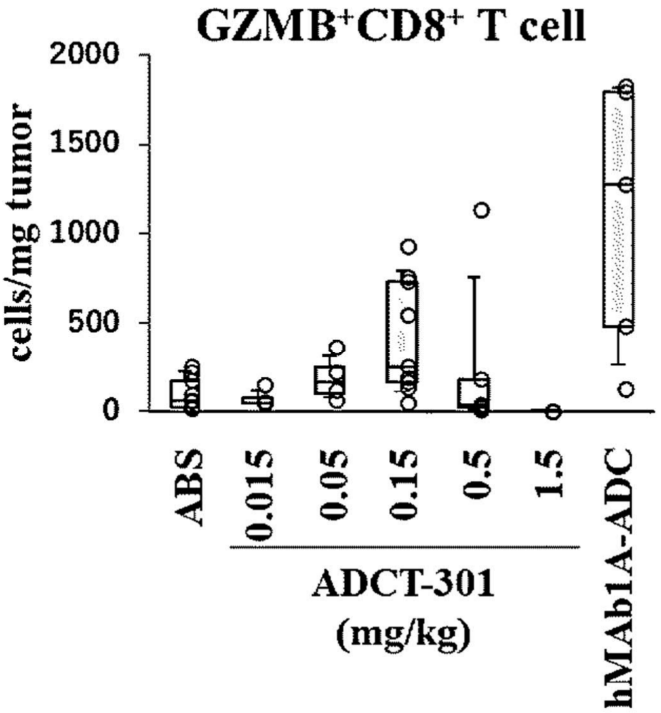


Fig. 28

SEQ ID NO13: Amino acid sequence of signal sequence and hMAb1A light chain

MVLQTQVFISLLLWISGAYGDIVMTQSPDSLAVSLGERATINCKASESVSTRMH

WYQQKPGQPPKLLIYKASNLASGVPARFSGSGSGTDFTLTISSLQAEDVAVYYC

QQSWNGWAFGQGTKVEIKRTVAAPSVFIFPPSDEQLKSGTASVVCLLNNFYPRE

AKVQWKVDNALQSGNSQESVTEQDSKDSTYSLSSTLTLSKADYEKHKVYACEV

THQGLSSPVTKSFNRGEC

Signal sequence(1-20), Light chain variable region(21-127), Light chain constant region(128-233)

# Fig. 29

SEQ ID NO15: Amino acid sequence of signal sequence and hMAb1A heavy chain

MKHLWFFLLLVAAPRWVLSEVQLVESGGGLVQPGGSLRLSCAASGFTFNNYYM

VWVRQAPGKGLEWVAYISTGGGTTYYRESVKGRFTISRDNSKSTLYLQMNSLR

AEDTAVYYCTTSVYFSDGSSHYDGEWFAYWGQGTLVTVSSASTKGPSVFPLAPS

SKSTSGGTAALGCLVKDYFPEPVTVSWNSGALTSGVHTFPAVLQSSGLYSLSSV

VTVPSSSLGTQTYICNVNHKPSNTKVDKKVEPKSCDKTHTCPPCPAPELLGGPS

VFLFPPKPKDTLMISRTPEVTCVVVDVSHEDPEVKFNWYVDGVEVHNAKTKPR

EEQYNSTYRVVSVLTVLHQDWLNGKEYKCKVSNKALPAPIEKTISKAKGQPREP

QVYTLPPSREEMTKNQVSLTCLVKGFYPSDIAVEWESNGQPENNYKTTPPVLD

SDGSFFLYSKLTVDKSRWQQGNVFSCSVMHEALHNHYTQKSLSLSPGK

Signal sequence(1-19), Heavy chain variable region(20-146), Heavy chain constant region(147-476)

# Fig. 30

SEQ ID NO14: Amino acid sequence of signal sequence and hMAb1B light chain

MVLQTQVFISLLLWISGAYGEIVLTQSPGTLSLSPGERATLSCKASESVSTRMHW

YQQKPGQAPRLLIYKASNLASGVPARFSGSGSGTDFTLTISRLEPEDFAVYFCQQ

SWNGWAFGQGTKVEIKRTVAAPSVFIFPPSDEQLKSGTASVVCLLNNFYPREAK

VQWKVDNALQSGNSQESVTEQDSKDSTYSLSSTLTLSKADYEKHKVYACEVTH

QGLSSPVTKSFNRGEC

Signal sequence(1-20), Light chain variable region(21-127), Light chain constant region(128-233)

Fig. 31

SEQ ID NO16: Amino acid sequence of signal sequence and hMAb1B heavy chain

MKHLWFFLLLVAAPRWVLSEVQLVESGGGLVQPGGSLRLSCAASGFTFNNYYM
VWVRQAPGKGLEWVAYISTGGGTTYYADSVKGRFTISRDNSKSTLYLQMNSLR
AEDTAVYYCTTSVYFSDGSSHYDGEWFAYWGQGTLVTVSSASTKGPSVFPLAPS
SKSTSGGTAALGCLVKDYFPEPVTVSWNSGALTSGVHTFPAVLQSSGLYSLSSV
VTVPSSSLGTQTYICNVNHKPSNTKVDKKVEPKSCDKTHTCPPCPAPELLGGPS
VFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREP
QVYTLPPSREEMTKNQVSLTCLVKGFYPSDIAVEWESNGQPENNYKTTPPVLD
SDGSFFLYSKLTVDKSRWQQGNVFSCSVMHEALHNHYTQKSLSLSPGK

Signal sequence(1-19), Heavy chain variable region(20-146), Heavy chain constant region(147-476)

Fig. 32

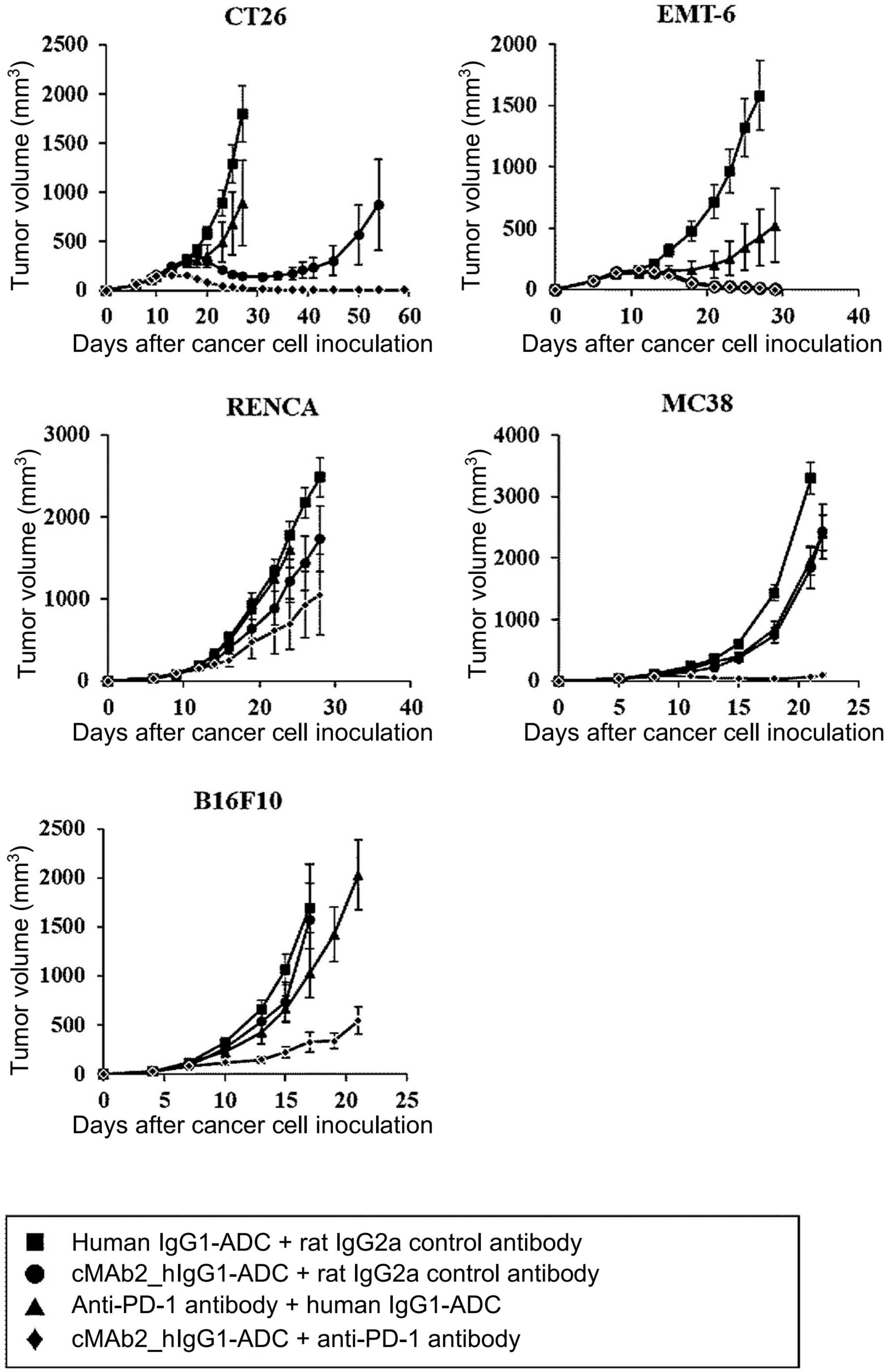
CT26
Tumor volume (mm3)
2500
2000
1500
1000
500
0
0 10 20 30 40 50 60
Days after cancer cell inoculation
EMT-6
Tumor volume (mm3)
2000
1500
1000
500
0
0 10 20 30 40
Days after cancer cell inoculation
RENCA
Tumor volume (mm3)
3000
2000
1000
0
0 10 20 30 40
Days after cancer cell inoculation
MC38
Tumor volume (mm3)
4000
3000
2000
1000
0
0 5 10 15 20 25
Days after cancer cell inoculation
B16F10
Tumor volume (mm3)
2500
2000
1500
1000
500
0
0 5 10 15 20 25
Days after cancer cell inoculation
Human IgG1-ADC + rat IgG2a control antibody
cMAb2_hIgG1-ADC + rat IgG2a control antibody
Anti-PD-1 antibody + human IgG1-ADC
cMAb2_hIgG1-ADC + anti-PD-1 antibody

Fig. 33

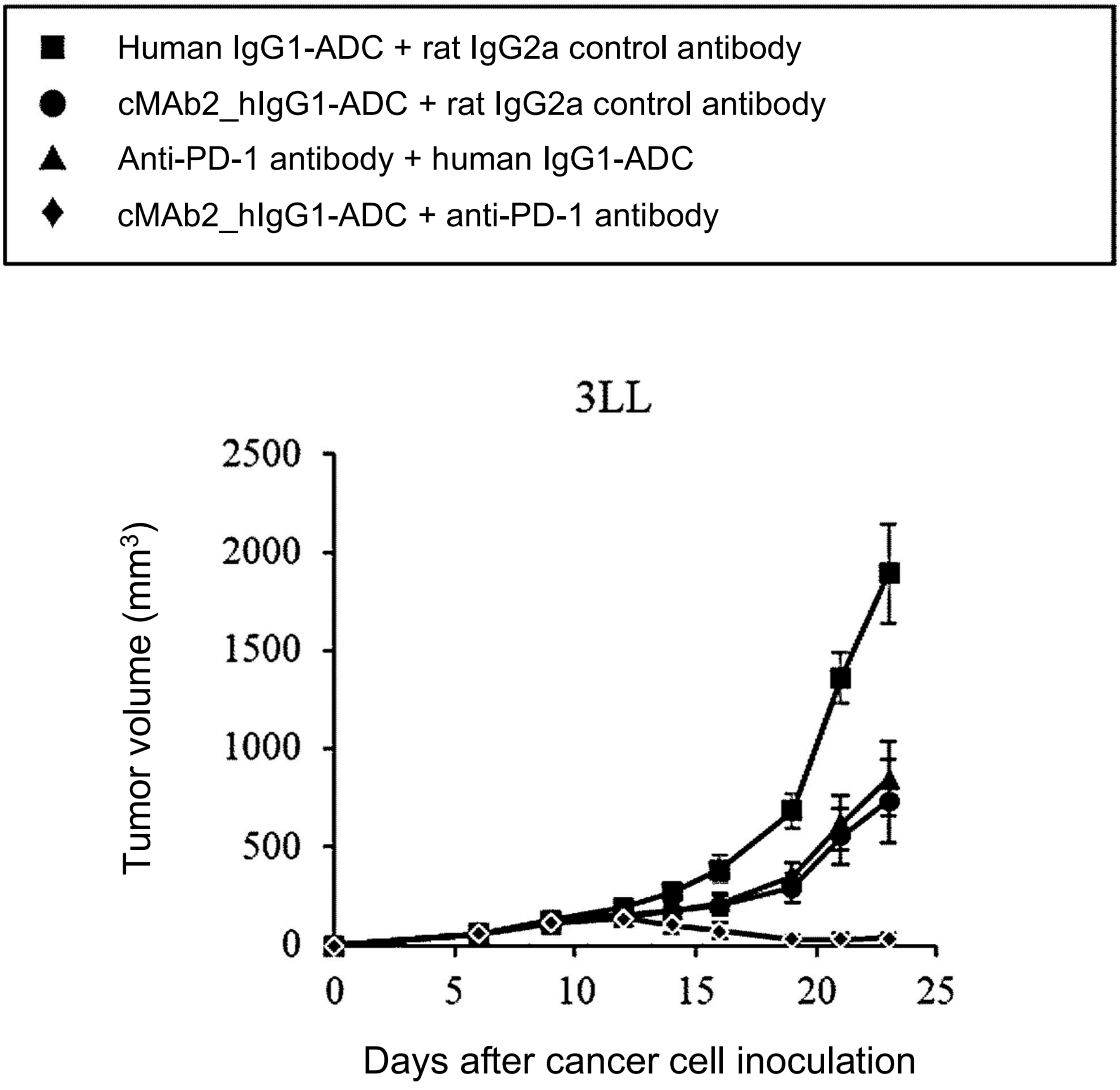
Human IgG1-ADC + rat IgG2a control antibody
cMAb2_hIgG1-ADC + rat IgG2a control antibody
Anti-PD-1 antibody + human IgG1-ADC
cMAb2_hIgG1-ADC + anti-PD-1 antibody
3LL
Tumor volume (mm3)
2500
2000
1500
1000
500
0
0
5
10
15
20
25
Days after cancer cell inoculation

Fig. 34

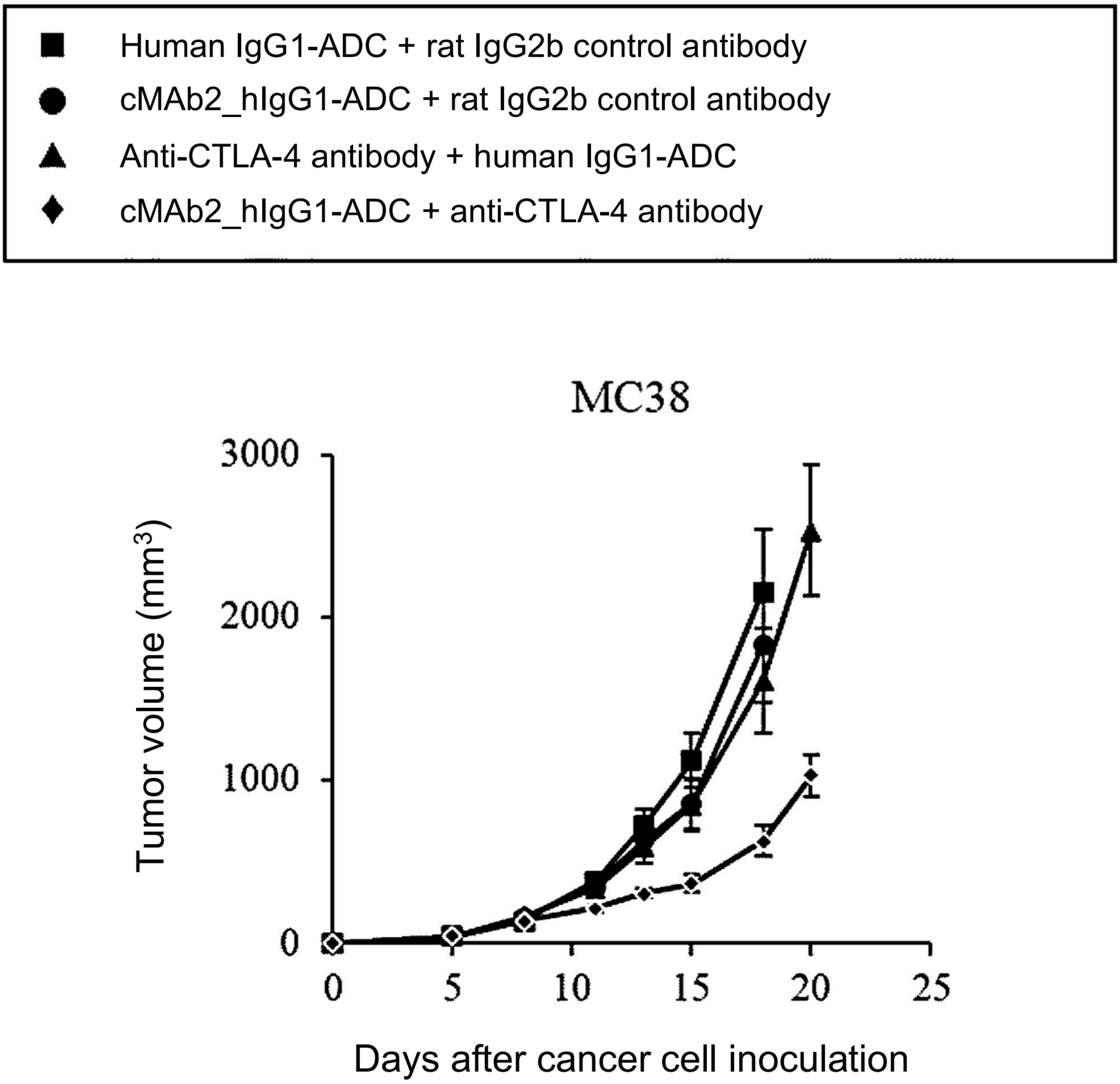
Human IgG1-ADC + rat IgG2b control antibody
cMAb2_hIgG1-ADC + rat IgG2b control antibody
Anti-CTLA-4 antibody + human IgG1-ADC
cMAb2_hIgG1-ADC + anti-CTLA-4 antibody
MC38
Tumor volume (mm3)
3000
2000
1000
0
0
5
10
15
20
25
Days after cancer cell inoculation

**INTERNATIONAL SEARCH REPORT**

International application No.
**PCT/JP2024/012855**

**A. CLASSIFICATION OF SUBJECT MATTER**

***C12N 15/13***(2006.01)i; ***A61K 31/4745***(2006.01)i; ***A61K 39/395***(2006.01)i; ***A61K 45/00***(2006.01)i; ***A61K 47/68***(2017.01)i; ***A61P 35/00***(2006.01)i; ***A61P 43/00***(2006.01)i; ***C07K 16/28***(2006.01)i; ***C12N 1/15***(2006.01)i; ***C12N 1/19***(2006.01)i; ***C12N 5/10***(2006.01)i; ***C12N 15/63***(2006.01)i; ***C12P 21/08***(2006.01)i

FI: C07K16/28; C12N15/63 Z; C12N1/15; C12N1/19; C12N5/10; C12P21/08; A61P35/00; A61P43/00 121; A61K45/00; A61K39/395 D; A61K39/395 N; A61K39/395 C; A61K39/395 L; A61K31/4745; A61K47/68; C12N1/21; A61K39/395 U; A61K39/395 T; A61K39/395 E; A61P37/04; A61K39/395 G; A61P43/00 107; C12N15/13 ZNA

According to International Patent Classification (IPC) or to both national classification and IPC

**B. FIELDS SEARCHED**

Minimum documentation searched (classification system followed by classification symbols)

C12N15/13; A61K31/4745; A61K39/395; A61K45/00; A61K47/68; A61P35/00; A61P43/00; C07K16/28; C12N1/15; C12N1/19; C12N5/10; C12N15/63; C12P21/08

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN); GenBank/EMBL/DDBJ/GeneSeq

**C. DOCUMENTS CONSIDERED TO BE RELEVANT**

| Category* | Citation of document, with indication, where appropriate, of the relevant passages | Relevant to claim No. |
|---|---|---|
| X | WO 2022/182483 A1 (RAKUTEN MEDICAL, INC.) 01 September 2022 (2022-09-01) claims, examples 1-7 | 1-7, 10, 16, 17, 22, 26-29, 37-39, 69-95 |
| Y | | 30-45, 52-60, 69-95 |
| A | | 8, 9, 11-15, 18-21, 23-25, 46-51, 61-68 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **26 April 2024** | **21 May 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

## INTERNATIONAL SEARCH REPORT

International application No.
**PCT/JP2024/012855**

### C. DOCUMENTS CONSIDERED TO BE RELEVANT

| Category* | Citation of document, with indication, where appropriate, of the relevant passages | Relevant to claim No. |
|---|---|---|
| X | JP 2022-538733 A (INSERM (INST. NAT. SANTE RECH. MED.)) 06 September 2022 (2022-09-06) claims, paragraphs [0285]-[0287], [0407]-[0411], examples | 1-7, 10, 16, 17, 22, 26-29, 37-39, 69-95 |
| Y | | 30-45, 52-60, 69-95 |
| A | | 8, 9, 11-15, 18-21, 23-25, 46-51, 61-68 |
| X | JP 2021-533726 A (TUSK THERAPEUTICS LTD.) 09 December 2021 (2021-12-09) claims, examples 1-4 | 1-7, 10, 16, 17, 22, 26-29, 37-39, 69-95 |
| Y | | 30-45, 52-60, 69-95 |
| A | | 8, 9, 11-15, 18-21, 23-25, 46-51, 61-68 |
| Y | WO 2018/135501 A1 (DAIICHI SANKYO CO., LTD.) 26 July 2018 (2018-07-26) claims, paragraphs [0107], [0108], [0121]-[0156], examples 1-6 | 30-45, 52-60, 69-95 |
| Y | WO 2018/212136 A1 (DAIICHI SANKYO CO., LTD.) 22 November 2018 (2018-11-22) claims, paragraphs [0123], [0124], [0137]-[0173], examples 1-8 | 30-45, 52-60, 69-95 |

Form PCT/ISA/210 (second sheet) (July 2022)

**INTERNATIONAL SEARCH REPORT**

International application No.
**PCT/JP2024/012855**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/JP2024/012855**

| Patent document cited in search report | Publication date (day/month/year) | Patent family member(s) | Publication date (day/month/year) |
|---|---|---|---|
| WO 2022/182483 A1 | 01 September 2022 | EP 4288100 A1<br>JP 2024-505556 A | |
| JP 2022-538733 A | 06 September 2022 | US 2022/0251232 A1<br>claims, paragraphs [0385]-[0387], [0545]-[0549], examples<br>WO 2020/234399 A1<br>EP 3972997 A1<br>CN 114630838 A | |
| JP 2021-533726 A | 09 December 2021 | US 2019/0284287 A1<br>claims, examples 1-4<br>WO 2019/175217 A1<br>EP 3765506 A1<br>KR 10-2020-0131286 A<br>CN 112020512 A | |
| WO 2018/135501 A1 | 26 July 2018 | US 2019/0225686 A1<br>claims, paragraphs [0168], [0169], [0182]-[0253], examples 1-6<br>EP 3572428 A1<br>KR 10-2019-0104160 A<br>CN 110382535 A | |
| WO 2018/212136 A1 | 22 November 2018 | US 2020/0171163 A1<br>claims, paragraphs [0149], [0150], [0163]-[0233], examples 1-8<br>EP 3626825 A1<br>CN 110651045 A<br>KR 10-2020-0006061 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9007861 A
- WO 9201047 A
- WO 9220791 A
- WO 9306213 A
- WO 9311236 A
- WO 9319172 A
- WO 9501438 A
- WO 9515388 A
- WO 199954342 A
- WO 200061739 A
- WO 200231140 A
- WO 2013120066 A
- WO 88007089 A
- WO 9428027 A
- WO 9429351 A
- US 20160297890 A
- US 2016297890 A
- WO 2006121168 A
- WO 2008156712 A
- WO 2010077634 A
- WO 2011066389 A
- WO 2013079174 A
- WO 2001014424 A
- WO 2000037504 A
- WO 2017174331 A
- WO 2014057687 A
- WO 2015115091 A
- WO 2019175222 A
- WO 2004045512 A
- WO 2014057119 A


### Non-patent literature cited in the description


- **SAKAGUCHI S et al.** *Annu Rev Immunol.*, 2004, vol. 22, 531-562
- **SAKAGUCHI S et al.** *Annu Rev Immunol.*, 2020, vol. 38, 541-566
- **SAKAGUCHI S et al.** *Cell.*, 2008, vol. 133 (5), 775-787
- **BACCHETTA R et al.** *Ann N Y Acad Sci.*, 2016, vol. 1417 (1), 5-22
- **SUNG H et al.** Global Cancer Statistics 2020: GLOBOCAN Estimates of Incidence and Mortality Worldwide for 36 Cancers in 185 Countries. *CA Cancer J Clin.*, 2021, vol. 71 (3), 209-249
- **HANAHAN D et al.** *Cell*, 2011, vol. 144 (5), 646-674
- **CHEN DS et al.** *Immunity.*, 2013, vol. 39 (1), 1-10
- **HUANG AC et al.** *Nat Immunol. Published online*, 2022, vol. 23 (5), 660-670
- **LARKIN J et al.** *N Engl J Med.*, 2015, vol. 373 (1), 23-34
- **MOK TSK et al.** *Lancet.*, 2019, vol. 393 (10183), 1819-1830
- **O'CALLAGHAN DS et al.** *Eur Respir J.*, 2015, vol. 46 (6), 1762-1772
- **RICKERT M** ; **RICKERT M, WANG X** ; **BOULANGER MJ** ; **GORIATCHEVA N** ; **GARCIA KC et al.** *The Structure of Interleukin-2 Complexed with Its Alpha Receptor*, 1477-1481
- **WANG X et al.** *Science*, 2005, vol. 310 (5751), 1159-1163
- **SPOLSKI R et al.** *Nat Rev Immunol.*, 2018, vol. 18 (10), 648-659
- **TSUDO M et al.** *J Immunol.*, 1982, vol. 129 (2), 592-595
- **BRISSLERT M et al.** *Immunology*, 2006, vol. 117 (4), 548-557
- **MIYARA M et al.** *Immunity*, 2009, vol. 30 (6), 899-911
- **CHINEN T et al.** *Nat Immunol.*, 2016, vol. 17 (11), 1322-1333
- **FLYNN MJ et al.** *Br J Haematol.*, 2017, vol. 179 (1), 20-35
- **RUBIN LA. et al.** *A monoclonal antibody 7G7/B6, binds to an epitope on the human interleukin-2 (IL-2) receptor that is distinct from that recognized by IL-2 or anti-Tac Hybridoma*, 1985, vol. 4 (2), 91-102
- **SOLOMON I et al.** *Nat Cancer.*, 2020, vol. 1 (12), 1153-1166
- **SAITO T et al.** *Nat Med.*, 2016, vol. 22 (6), 679-684
- **CHARI RVJ et al.** *Angew Chemie - Int Ed.*, 2014, vol. 53 (15), 3796-3827
- **NIWA R et al.** *Cancer Res.*, 2004, vol. 64 (6), 2127-2133
- **ISHIDA T et al.** *J Clin Oncol.*, 2012, vol. 30 (8), 837-842
- **LO NIGRO C et al.** *Ann Transl Med.*, 2019, vol. 7 (5), 105-105
- **MARECHAL R et al.** *BMC Cancer.*, 2010, 10

- **LEFRANC et al.** *Dev Comparat Immunol*, 2003, vol. 27, 55-77
- **KABAT et al.** Sequences of Proteins of Immunological Interest. National Institutes of Health, 1991
- **AL-LAZIKANI et al.** *J. Mol. Biol*, 1997, vol. 273, 927-948
- *Cell Death and Differentiation*, 2008, vol. 15 (2), 751-761
- *Molecular Biology of the Cell*, December 2004, vol. 15 (3), 5268-5282
- **KOHLER** ; **MILSTEIN**. *Nature*, 1975, vol. 256, 495-497
- Monoclonal Antibodies. Plenum Press, 1980, 365-367
- *Proc. Natl. Acad. Sci. U.S.A.*, 1984, vol. 81, 6851-6855
- *Nature*, 1986, vol. 321, 522-525
- **TOMIZUKA, K et al.** *Nature Genetics*, 1997, vol. 16, 133-143
- **KUROIWA, Y. et al.** *Nucl. Acids Res.*, 1998, vol. 26, 3447-3448
- **YOSHIDA, H. et al.** Animal Cell Technology: Basic and Applied Aspects. Kluwer Academic Publishers, 1999, vol. 10, 69-73
- **TOMIZUKA, K. et al.** *Proc. Natl. Acad. Sci. USA*, 2000, vol. 97, 722-727
- **WORMSTONE, I.M et al.** *Investigative Ophthalmology & Visual Science.*, 2002, vol. 43 (7), 2301-2308
- **CARMEN, S. et al.** *Briefings in Functional Genomics and Proteomics*, 2002, vol. 1 (2), 189-203
- **SIRIWARDENA, D. et al.** *Ophthalmology*, 2002, vol. 109 (3), 427-431
- *Nature Biotechnology*, 2005, vol. 23 (9), 1105-1116
- *Annu. Rev. Immunol*, 1994, vol. 12, 433-455
- **GLUZMAN, Y.** *Cell*, 1981, vol. 23, 175-182
- **URLAUB, G.** ; **CHASIN, L. A.** *Proc. Natl. Acad. Sci. U.S.A.*, 1980, vol. 77, 4126-4220
- *Journal of Chromatography A*, 1995, vol. 705, 129-134
- *Analytical Biochemistry*, 2007, vol. 360, 75-83
- Strategies for Protein Purification and Characterization: A Laboratory Course Manual. Cold Spring Harbor Laboratory Press, 1996
- Antibodies: A Laboratory Manual. Cold Spring Harbor Laboratory, 1988
- *Pharmacological Reviews*, 2016, vol. 68, 3-19
- Protein Cell. *Pharmacol Rev*, January 2016, vol. 68, 3-19
- **HERMANSON, G.T**. Bioconjugate Techniques. Academic Press, 1996, 56-136, 456-493
- *Protein Science*, 1995, vol. 4, 2411-2423
- **SAMBROOK, J.** ; **FRITSCH, E. F.** ; **MANIATIS, T.** Molecular Cloning. Cold Spring Harbor Laboratory Press, 1989
- **HUSS et al.** *Immunology*, 2016, vol. 148 (3), 276-286
- *Methods in Enzymology*, 1991, vol. 203, 121-153
- *Nuc. Acid Res.*, 2007, vol. 35, D301-D303
- *Proc. Natl. Acad. Sci. USA*, 1989, vol. 86, 10029-10033
- **KABAT et al.** Sequences of Proteins of Immunological Interest. Public Health Service National Institutes of Health, 1991
- **QUEEN et al.** *Proc. Natl. Acad. Sci. USA*, 1989, vol. 86, 10029-10033